# EUROPEAN PATENT APPLICATION

(11) **EP 4 610 278 A2**
(43) Date of publication of application: **03.09.2025**
(21) Application number: 25186753.7
(22) Date of filing: 20.12.2022
(51) Int. Cl.: C07K 16/28

(54) **TARGETED SAPONIN-NUCLEIC ACID CONJUGATES FOR TREATMENT OF MUSCLE WASTING DISORDERS**

(30) Priority: 22.12.2021 NL 2030215
(62) Divisional of application: 22830621.3
(71) Applicant: Sapreme Technologies B.V., 3584 CM Utrecht (NL)
(72) Inventor: Bujny, Miriam Verena, 3584 CM Utrecht (NL); Postel, Ruben, 3584 CM Utrecht (NL); Hermans, Guy, 9820 Merelbeke (BE)
(74) Representative: Nederlandsch Octrooibureau

(57) **Abstract**

The invention lies in the field of treatment and prophylaxis of muscle wasting disorders, in particular the ones involving a genetic factor that can be targeted by a delivery of a therapeutic nucleic acid into the muscle cells. In line with the latter aspect, disclosed herein are pharmaceutical compositions and advantageous components thereof that substantially enhance the effective delivery and release of a therapeutic nucleic acid into the correct internal compartment of the muscle cell, such as the cytosol and/or the nucleus, in which compartment it can reach and act upon its genetic target. As disclosed herein, this substantially enhanced delivery and release is achieved by a provision of an endosomal-escape-enhancing saponin in the disclosed herein pharmaceutical compositions comprising therapeutic nucleic acids and muscle cell-targeting ligands. As for the first time demonstrated herein, these saponin types surprisingly not only appear to retain their endosomal-escape-enhancing properties in fully differentiated muscle cells, but also successfully reach and enter muscle cells *in vivo* through endothelial transcytosis route followed by endocytic capture at the sarcolemma.

## Description

### TECHNOLOGICAL FIELD

The invention lies in the field of treatment and prophylaxis of muscle wasting disorders, in particular the ones involving a genetic factor that can be targeted by a delivery of a therapeutic nucleic acid into the muscle cells. In line with the latter aspect, disclosed herein are pharmaceutical compositions and advantageous components thereof that substantially enhance the effective delivery and release of a therapeutic nucleic acid into the correct internal compartment of the muscle cell, such as the cytosol and/or the nucleus, in which compartment it can reach and act upon its genetic target. As disclosed herein, this substantially enhanced delivery and release is achieved by a provision of an endosomal-escape-enhancing saponin in the disclosed herein pharmaceutical compositions comprising therapeutic nucleic acids and muscle cell-targeting ligands. As for the first time demonstrated herein, these saponin types surprisingly not only appear to retain their endosomal-escape-enhancing properties in fully-differentiated muscle cells but also successfully reach and enter muscle cells *in vivo* through endothelial transcytosis route followed by endocytic capture at the sarcolemma.

### BACKGROUND

Muscle wasting disorders represent a major cause of human diseases worldwide. They can be caused by an underlying genetic condition, such as in various types of muscular dystrophies or congenital myopathies [Cardamone, 2008], or can be related to aging, like the age-related loss of muscle mass known as sarcopenia, or result from a traumatic muscle injury, among others.

Both the hereditary and non-hereditary muscle wasting disorders primarily manifest as a debilitating weakening or loss of striated muscle tissue, which is the tissue responsible, among others, for whole-body oxygen supply, metabolic balance, and locomotion. The striated muscle tissue is built by two types of striated muscle cells, namely the skeletal muscle cells and the cardiac muscle cells [Shadrin, 2016]. Skeletal muscles comprise 30 to 40% of total human body mass and can regenerate in response to small muscle tears that occur during exercise or daily activity owing to the presence of resident muscle stem cells called satellite cells (SCs), which upon injury activate, proliferate, and fuse to repair damaged or form new muscle fibres [Dumont, 2016]. In contrast, cardiac muscle does not possess a cardiomyogenic stem cell pool and has little to no regenerative ability, with injury resulting in the formation of a fibrotic scar and, eventually, impaired pump function [Uygur, 2016].

Both of these cell types are terminally differentiated and highly structurally- and functionally-specialised and these characteristics usually correlate with an increased difficulty of targeting payloads into such cells' inner compartments. In particular, muscle cells are covered by a unique type of a cell membrane termed sarcolemma that, just like in neurons, is excitable. Furthermore, these cells are particularly resilient characterised by contractibility, extensibility, and elasticity, which are the key features required for fulfilling their primary function in the muscle tissue, which is the production of tension resulting in the generation of force that contracts the muscle cells in order to produce voluntary or involuntary movement of different body parts.

In line with this, despite a widespread recognition that certain therapeutic payloads can yield beneficial effects when delivered into the muscle cells, it is also very well known that targeting these cells has proven notoriously challenging, as acknowledged in e.g. WO2021142227. This is particularly true for cardiac muscle cells where any on- and off-target activity of a drug cause serious safety liabilities [Slordalm 2006]. Systemic delivery to heart of even specifically muscle-targeted therapeutics is known to have very limited efficacy, and even direct intramuscular injections into the heart have shown restricted heart exposure [Ebner, 2015].

In addition to the above limitations, very few treatment options and strategies are available for individuals suffering from acquired advanced-stage or genetic muscle wasting disorders. In fact, for majority of them, there are no drugs available with palliative treatment frequently being the only applicable solution to ease the suffering of the affected individual.

In particular, an incredible large spectrum of muscle cell-related genetic disorders (sometimes collectively termed as hereditary myopathies) has been described to date, and although due to relatively low prevalence majority of them are catalogued as "rare diseases", the sum of the different forms makes these disorders a relatively common health problem that affects the life quality of millions of patients worldwide, causing debilitating complications that frequently lead to death [González-Jamett, 2017].

One common classification of muscle cell-related genetic disorders is based on the location of the mutated protein product originating from the muscle cell. Namely, congenital myopathies are considered to be caused by genetic defects in the contractile apparatus within the muscle cell, and are defined by distinctive static histochemical or ultrastructural changes on muscle biopsy. In contrast, muscular dystrophies are described as diseases of the muscle membrane or its supporting proteins and are generally characterised by pathological evidence of ongoing muscle degeneration and regeneration [Cardamone, 2008].

In brief, the contractile apparatus includes myofibrils comprised of actin and myosin that form myofilaments which slide past each other producing tension that changes the shape of the muscle cell. The function of the contractile apparatus heavily relies on its interaction with the reinforced muscle cell cytoskeleton and the highly specialised structures within and around the sarcolemma that, unlike most of the cell membranes in the human body, is heavily coated by a polysaccharide material termed glycocalyx that contacts the basement membrane around the muscle cells. This basement membrane contains numerous collagen fibrils and specialized extracellular matrix proteins such as laminin. The matrix proteins provide a scaffold to which the muscle fibre can adhere. Through transmembrane proteins in the sarcolemma, the actin skeleton inside the muscle cells is connected to the basement membrane and the cell's exterior. Such anchored numerous muscle cells make up the muscle tissue and by synchronous and controlled production of tension they can generate significant force.

This structural and functional complexity of the muscle cells, including their intracellular contractile apparatus, the network of proteins reinforcing and accounting for specific function and architecture of the sarcolemma, and the multi-component scaffolding outside of it, is a product of a large muscle cell-specific proteome.

A substantial part of this proteome are large structural proteins that are translated from purely-muscle-cell-specific transcripts originating from frequently very large multi-exonic genes that tend to undergo extensive alternative splicing events [Savarese, 2020]. In fact, various mutations scattered along some of the largest genes of the human genome, notably including DMD, TTN, NEB, RYR1, are recognized as underlying causes of the best characterised muscle cell-related genetic disorders.

Perhaps and arguably the most investigated genetic muscle cell-related disorder is the Duchenne muscular dystrophy (DMD) that results from a mutation in the DMD gene encoding for dystrophin protein, which prevents the production of the muscle isoform of dystrophin (Dp427m). DMD is a particularly severe disease characterized by progressive wasting and replacement of skeletal muscles with fibrous, bony, or fatty tissue, which eventually leads to death due to usually heart-muscle or respiratory failure. DMD is recessive and X-chromosome-linked (X-linked). Consequently, most patients are males. On average, they develop the earliest symptoms around 2-3 years of age, become wheelchair dependent around 10-12 years, and with even with optimal care die between 20 and 40 years of age. The different spectra can be explained by the fact that DMD is not caused by a precise defined site-specific or single hot-spot mutation in the DMD gene. To the contrary, DMD, like many other muscle-cell related genetic disorders caused by different mutations in large multi-exonic genes, can be seen as a spectrum of disorders which severity of the phenotype depending on the extent to which the reading frame of transcript was affected. DMD cases usually harbour frameshifting or nonsense mutations that cause premature truncation leading to non-functional and unstable dystrophin. In contrast to this, a milder dystrophinopathy called Becker muscular dystrophy (BMD) is caused by in frame mutations of the DMD gene, i.e. mutations that maintain the reading frame and lead to a production of a dystrophin mutant protein that is merely internally truncated.

Based on the observation that most out-of-frame mutations result in severe DMD, whereas the vast majority of in-frame mutations result in milder BMD, different antisense oligonucleotide (ASO)-based therapeutics have been tested and developed for DMD with the aim of restoring the reading frame of dystrophin transcripts causing the production of a protein that is at least partially functional. These ASOs are short (20-30 nucleotides), frequently chemically-modified nucleic acids or nucleic acid analogues that specifically bind to a target exon during pre-mRNA splicing causing a so called skipping of the faulty exon, i.e. prevention of its inclusion into the mRNA. The exon-skipping-ASO approach is mutation specific as different exons need to be skipped depending on the mutation location. However, skipping of certain exons is applicable to larger groups of patients, including the skipping of exon 51 (14%), exon 45 (8%), exon 53 (8%), and exon 44 (6%) [Bladen, 2013]. To date four different morpholino-type ASOs that are designed to skip exon 51 (eteplirsen), exon 53 (golodirsen and viltolarsen), or exon 45 (casimersen) have shown some evidence of induced dystrophin restoration in small cohorts of patients and, despite exhibition of certain systemic side-effects, were granted FDA-approvals. Another exon 51-skipping ASO with a 2'-O-methyl phosphorothioate modification (drisapersen) was evaluated in placebo-controlled trials but eventually was not approved by the FDA, due to e.g. occurrence of injection site reactions, proteinuria and, in a subset of patients, thrombocytopenia [Goemans, 2018]. Alternative compositions for induction of exon skipping can be found in WO2018129384.

Other nucleic acid-based approaches in DMD included attempted delivery of micro-dystrophin cDNA at high vector dose, for which clinical trials are under way with some already reported success of micro-dystrophin expression but not without observation of severe adverse effects in a subset of patients, including transient renal failure likely due to an innate immune response [Mendell, 2020]. Alternatively, efforts are also ongoing to deliver cDNA of genes that encode proteins that can improve muscle mass, such as follistatin [Mendell, 2020] or that target disease mechanisms, such as SERCA2a [Wasala, 2020] f). Further considerations involve the use of microRNAs, miRNA mimic products, antimiRs, antagomiRs and the line either alone or for co-administration with other nucleic acid-based therapies for the stimulation of growth and/or regeneration of the wakened muscle cells [Aránega, 2021]. For example, WO2018080658 discloses miR-128-1 as LNA-based ASO therapeutic for the treatment of DMD. Yet another alternative approach was proposed based on CRISPR/Cas9 technology with guide RNAs designed for restoring the reading frame e.g. by exon deletion or by abolishing of a splice site, a proof of concept of which was tried in DMD cell lines and animal models [Chemello, 2020; Nelson 2017]. However, all the genome-editing work is still in a preclinical phase and multiple challenges have to be overcome to apply it systemically in humans, including optimal delivery of the genome-editing components.

Thousands of different mutations in DMD have been found in patients with DMD or BMD [Blanden, 2015]. Similar situation exists for many other genetic muscle cell-related disorders where gene mutation targets are identified. These include but are not limited to other muscular dystrophies including facioscapulohumeral muscular dystrophy (affected genes: DUX4/double homeobox 4), myotonic dystrophy type 1 (DMPK), Emery-Dreifuss muscular dystrophy (affected genes: EMD/emerin and LMNA/lamin A/C), limb-girdle muscular dystrophy 1 (affected genes: MYOT/myotilin, LMNA/lamin A/C etc.), congenital muscular dystrophy (affected genes: LAMA2/merosin or laminin-α2 chain/ any of COL6A genes encoding for collagen 6A); or dilated familial cardiomyopathy (affected genes: LMNA/lamin A/C), as well as congenital myopathies notably including nemalin myopathy (affected genes: NEB/nebulin, ACTA/skeletal muscle alpha-actin, TPM3/alpha-tropomyosin-3, TPM2/beta-tropomyosin-2, TNNT1/troponin T1, LMOD3/leiomodin-3, MYPN/myopalladin etc.) or congenital fibre-type disproportion myopathy (affected genes: TPM3/alpha-tropomyosin-3 , CTA/skeletal muscle alpha-actin, RYR1/ryanodine receptor channel), as well as any syndromes involving mutations e.g. in TTN gene (titin). Consequently, due to the variability of the mutations in even defined genetic targets underlying many different muscle wasting disorders, use of therapeutic nucleic acids, such ASOs or antagomirs [Cerro-Herreros, 2020], appears to be a logical and practical way forward for the development of new therapies for various muscle-wasting disorder.

However, as already explained above, despite considerable advantages, even when coupled with various delivery systems, it is difficult to efficiently bring such nucleic acid-based therapeutics into the appropriate compartments inside the muscle-cells, like for example into the muscle cell cytosol for antisense-therapy or, therefrom, into the nucleus for direct gene-editing. This low efficiency of muscle cell transfection and *in vivo* naturally results in the concentrations of nucleic acid-based therapeutics being too low at their target site for achieving effective and sustained outcomes. This in turn results in the need to increase the administered dose, which then causes off-target effects. Most common of such side-effects include activation of the complement cascade, the inhibition of the clotting cascade, and toll-like receptor mediated stimulation of the immune system. Naturally, these effects are highly undesired and bear the risk of inducing health- or even life-threatening side effects in the patient including organ failure. The occurrence of such and similar adverse events had caused many nucleic acid-based therapeutics, like the DMD exon-skipping ASO drisapersen, to fail in clinical trials.

Therefore, there is a strong desire to provide novel nucleic acid-based drug formulations for the treatment of muscle cell wasting disorder, which in particular would show efficient nucleic acid-delivery rates into the muscle cells in vivo. Consequently, formulations are needed wherein the effect of the therapeutic nucleic acid would be (1) highly specific for its genetic target implicated in or causing a muscle cell wasting disorder, (2) sufficiently safe, (3) efficacious, (4) specifically directed to the muscle cells with little to none off-target activity on other cells, (5) have a sufficiently timely mode of action (e.g. the administered drug should reach the targeted site in the human patient within a certain time frame and should remain at the targeted site for a certain time frame), and/or (6) have sufficiently long lasting therapeutic activity in the patient's body, amongst others.

A comprehensive review of different drug delivery approaches into the striated muscle cells can be found in DC Ebner et al., 2015, Curr Pharm Des, 21(10):1327-36. doi: 10.2174/1381612820666140929095755, which mentions muscle targeting peptides, microbubbles, nanoparticles, viral-, transporter-, and antibody-based targeting techniques as well as highlights the fact that cellular uptake remains a major issue in muscle cells in general. With regard to the transporter-, and antibody-based targeting techniques, delivery of nucleic acids by a ligand-mediated targeting of endocytic (or internalizing) receptors on muscle cell surface is e.g. shown in WO2018129384 or WO2020028857.

However, to the inventors' knowledge and experience, none of the known muscle-specific delivery approaches achieves all or at least a substantial part of the above outlined beneficial characteristics (1)-(6). Consequently, despite the long-lasting and intensive research and the progress made in several areas of the field individually, there still exists a dire need for improvement of the efficiency of delivery of nucleic acid-based therapeutics into the muscle cells of the patients suffering from muscle wasting disorders.

### SUMMARY

To address this need, the inventors have developed and hereby describe novel pharmaceutical compositions comprising muscle cell-targeted therapeutic nucleic acids in combination with triterpenoid saponins of the 12,13-dehydrooleanane type. These specific saponin types were characterised and reported in e.g. WO2020126620 as possessing an endosomal-escape enhancing activity towards various antibody-drug conjugates (ADCs) in several cancer cell types.

In the context of tumour cells, these saponins were further disclosed in WO2020126626, WO2020126620, WO2020126627, WO2020126064 and WO2020126609 that describe the silencing of the HSP27 gene in tumour models, with a conjugate of a monoclonal antibody directed to a tumour-cell marker, a saponin and a BNA for silencing HSP27. Then, WO2020126627, WO2020126064, WO2020126604, WO2020126600 and WO2020126609 describe the silencing of the HSP27 gene in tumour models, with a combination of a first conjugate of a monoclonal antibody directed to a tumour-cell marker and a saponin, and a second conjugate of a monoclonal antibody directed to a tumour-cell marker and a BNA for silencing HSP27. Lastly, WO2020126609 describes the silencing of the HSP27 gene in tumour models, with a combination of a saponin and a BNA for silencing HSP27 or with a combination of a saponin with a conjugate of a monoclonal antibody directed to a tumour-cell marker and a BNA for silencing HSP27.

Terminally differentiated muscle cells however, cardiac muscle cells in particular, are much different in metabolism as well as in cell membrane architecture and endocytic activity from the genetically unstable and constantly dividing tumour cells. Furthermore, due to perturbations in cell signalling pathways and chaotic proliferation activity, tumours are known to be supplied by permeable and leaky vascularisation [Hanahan and Weinberg, 2011], which is very different from the healthy and tight-junction-rich blood vessels that supply the muscle tissue.

Despite these differences, the inventors have observed a surprising and robust effect on exon-skipping efficiency in human and murine DMD transcript, both *in vitro* and *in vivo,* respectively, when combining muscle-endocytic-receptor-ligand-conjugated ASOs with the 12,13-dehydrooleanane type triterpenoid saponins. Without wishing to be bound by any theory, the inventors hypothesised that these findings indicate that said specific group of endosomal-escape enhancing saponins not is only capable of stimulating efficient exiting of the therapeutic nucleic acids' from the muscle cells' endosomes into the appropriate muscle cell inner compartments (in a very much desired for therapeutics but not fully understood phenomenon termed *endosomal escape*)*,* but also, and surprisingly, when formulated with the ligands and ASOs, they successfully underwent endothelial cell transcytosis *in vivo* from blood to the external environment of the muscle cells, without having caused via endosomal escape any apparent loss of the payload-containing cargo into the inner compartments of the endothelial cells.

To the inventors' knowledge, the only instance of combining saponins with nucleic acids for the treatment of muscle cell wasting disorders was attempted by Wang et al. [Wang, 2018, Molecular Therapy: Nucleic Acids; Wang, 2018 - Drug Design, Development and Therapy]. In these reports, the authors however concentrated on membrane-piercing transfection activity of non-covalently bound and non-targeted nucleic acid complexes with mainly steroidal saponins such as the known *in vitro* transfection agent digitonin. None of the saponins as investigated by Wang et al. however was an endosomal-escape enhancing saponin of the 12,13-dehydrooleanane type, and none of the complexes was specifically muscle-cell targeted via an endocytic receptor ligand. Consequently, both the saponin as well as nucleic acid concentrations as used by Wang et al. for the induction of exon-skipping activity in their DMD model systems, greatly exceeded the concentrations of the respective components in the novel compositions as presented herein in the continuation.

In sum, to address the drawbacks of the prior art, presented herein are novel pharmaceutical compositions for the use in in the treatment or prophylaxis of a muscle wasting disorders, muscle cell-related genetic disorders in particular, as well as novel muscle-specific endocytic receptor targeted-therapeutic nucleic acid conjugates with a covalently linked 12,13-dehydrooleanane type endosomal-escape enhancing saponins for the delivery of a therapeutic nucleic acid into a muscle cell, which the inventors observed have the unique ability to efficiently deliver the therapeutic nucleic acids into striated muscle cells *in vivo,* possibly by facilitating the endosomal escape specifically in the target muscle cells and surprisingly also successfully undergoing endothelial transcytosis from blood to said cells. The findings presented herein open the venue of developing novel low-therapeutic-load and thus safer treatment methods for the patients suffering from muscle wasting disorders. These and other advantages are presented further in the continuation.

Disclosed herein are improved biologically active compounds and pharmaceutical compositions comprising covalently-linked conjugates of therapeutic nucleic acids, muscle cell-surface endocytic receptor-targeting ligands and endocytic-escape enhancing saponins. The disclosed herein conjugates possess the particular advantage of exhibiting the highly desired property of enhanced and effective delivery of therapeutic nucleic acids, such as antisense oligonucleotides, into differentiated muscles cells, striated muscle cells in particular, notably including heart muscle cells.

The innovative concepts presented herein will be described with respect to particular embodiments that should be regarded as descriptive and not limiting beyond of what is described in the claims. These embodiments as described herein can operate in combination and cooperation, unless specified otherwise.

It is one of several objectives of embodiments of present disclosure is to provide a solution to the problem of non-specificity, encountered when administering nucleic acid-based therapeutics to human patients suffering from a muscle-wasting disorder and in need of such therapeutics.

It a further one of several objectives of the embodiments to provide a solution to the problem of insufficient safety characteristics of current nucleic-acid-based drugs, when administered to human patients in need thereof, in particular at side-effect inducing excessive doses.

It is yet a further one of several objectives of embodiments of the current invention to provide a solution to the problem of current nucleic acid-based therapies being less efficacious than desired, when administered to human patients in need thereof, due to not being sufficiently capable to reach and/or enter into to the diseased muscle cell with little to no off-target activity on non-diseased cells, when administered to human patients in need thereof.

At least one of the above objectives is achieved by providing a pharmaceutical composition for use in the treatment or prophylaxis of a muscle wasting disorder, in particular being a muscle cell-related genetic disorder such as congenital myopathy or a muscular dystrophy notably including Duchenne muscular dystrophy, the composition comprising
a covalently-linked conjugate comprising
   a saponin,
   a nucleic acid, and
   a ligand of an endocytic receptor on a muscle cell,
wherein the saponin is an endosomal-escape-enhancing triterpenoid 12,13-dehydrooleanane-type saponin.

In a further aspect, at least one of the above objectives is achieved by providing a covalently linked conjugate for delivery of a therapeutic nucleic acid into a muscle cell, the conjugate comprising
a saponin,
a nucleic acid, and
a ligand of an endocytic receptor on a muscle cell,
wherein the saponin is an endosomal-escape-enhancing triterpenoid 12,13-dehydrooleanane-type saponin, and wherein the conjugate comprises 1 - 16 molecules of the saponin and 1 - 5 molecules of the nucleic acid per 1 molecule of the ligand.

In further aspect provided are further embodiments of the composition for the disclosed herein therapeutic or prophylactic uses and/or of the conjugate according to the disclosure, which embodiments further address one or more of the above-stated objectives.

In particularly advantageous aspects, different embodiments of the disclosure are provided comprising advantageous conjugates comprising one selected from advantageous endosomal-escape-enhancing saponins, different therapeutic nucleic acid such as antisense oligonucleotides for example configured to induce skipping of faulty exons of a wasting muscle cell disorder-associated gene transcript, advantageous ligands or combinations thereof for targeting endocytic receptors on muscle-cells, and advantageous covalent linkers for connecting any of the above together, possibly also configured for being cleavable under conditions present in human endosomes.

These and other aspects of the disclosure are presented in detail in continuation.

### DEFINITIONS

The term "saponin" has its regular established meaning and refers herein to a group of amphipathic glycosides which comprise one or more hydrophilic saccharide chains combined with a lipophilic aglycone core which is termed a sapogenin. The saponin may be naturally occurring or synthetic (i.e. non-naturally occurring). The term "saponin" includes naturally-occurring saponins, functional derivatives of naturally-occurring saponins as well as saponins synthesized *de novo* through chemical and/or biotechnological synthesis routes. Saponin according to the conjugate of the invention has a triterpene backbone, which is a pentacyclic C30 terpene skeleton, also referred to as sapogenin or aglycone. Within the conjugate of the invention saponin is not considered an effector molecule nor an effector moiety in the conjugates according to the invention. Thus, in the conjugates comprising a saponin and an effector moiety, the effector moiety is a different molecule than the conjugated saponin. In the context of the conjugate of the invention, the term saponin refers to those saponins which exert an endosomal/lysosomal escape enhancing activity, when present in the endosome and/or lysosome of a mammalian cell such as a human cell, towards an effector moiety comprised by the conjugate of the invention and present in said endosome/lysosome together with the saponin.

As used herein, the term "saponin derivative" (also known as "modified saponin") shall be understood as referring to a compound corresponding to a naturally-occurring saponin (preferably being endosomal/lysosomal escape enhancing activity towards a therapeutic molecule such as nucleic acid, when present together in the endosome or lysosome of a mammalian cell) which has been derivatised by one or more chemical modifications, such as the oxidation of a functional group, the reduction of a functional group and/or the formation of a covalent bond with another molecule (also referred to as "conjugation" or as "covalent conjugation"). Preferred modifications include derivatisation of an aldehyde group of the aglycone core; of a carboxyl group of a saccharide chain or of an acetoxy group of a saccharide chain. Typically, the saponin derivative does not have a natural counterpart, i.e. the saponin derivative is not produced naturally by e.g. plants or trees. The term "saponin derivative" includes derivatives obtained by derivatisation of naturally-occurring saponins as well as derivatives synthesized *de novo* through chemical and/or biotechnological synthesis routes resulting in a compound corresponding to a naturally-occurring saponin which has been derivatised by one or more chemical modifications. A saponin derivative in the context of the invention should be understood as a saponin functional derivative. "Functional" in the context of the saponin derivative is understood as the capacity or activity of the saponin or the saponin derivative to enhance the endosomal escape of an effector molecule which is contacted with a cell together with the saponin or the saponin derivative.

The term "aglycone core structure" shall be understood as referring to the aglycone core of a saponin without the carbohydrate antennae or saccharide chains (glycans) bound thereto. For example, quillaic acid is the aglycone core structure for SO1861, QS-7 and QS21. Typically, the glycans of a saponin are mono-saccharides or oligo-saccharides, such as linear or branched glycans.

The term "saccharide chain" has its regular scientific meaning and refers to any of a glycan, a carbohydrate antenna, a single saccharide moiety (mono-saccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

As used herein, the terms "nucleic acid" and "polynucleotide" are synonymous to one another and are to be construed as encompassing any polymeric molecule made of units, wherein a unit comprises a nucleobase (or simply "base" e.g. being a canonical nucleobase like adenine (A), cytosine (C), guanine (G), thymine (T), or uracil (U), or any known non-canonical, modified, or synthetic nucleobase like 5-methylcytosine, 5-hydroxymethylcytosine, xanthine, hypoxanthine, 7-methylguanine; 5,6-dihydrouracil etc.) or a functional equivalent thereof, which renders said polymeric molecule capable of engaging in hydrogen bond-based nucleobase pairing (such as Watson-Crick base pairing) under appropriate hybridisation conditions with naturally-occurring nucleic acids such as deoxyribonucleic acid (DNA) or ribonucleic acid (RNA), which naturally-occurring nucleic acids are to be understood being polymeric molecules made of units being nucleotides.

Hence, from a chemistry perspective, the term nucleic acid under the present definition can be construed as encompassing polymeric molecules that chemically are DNA or RNA, as well as polymeric molecules that are nucleic acid analogues, also known as xeno nucleic acids (XNA) or artificial nucleic acids, which are polymeric molecules wherein one or more (or all) of the units are modified nucleotides or are functional equivalents of nucleotides. Nucleic acid analogues are well known in the art and due to various properties, such as improved specificity and/or affinity, higher binding strength to their target and/or increased stability in vivo, they are extensively used in research and medicine. Typical examples of nucleic acid analogues include but are not limited to locked nucleic acid (LNA) (that is also known as bridged nucleic acid (BNA)), phosphorodiamidate morpholino oligomer (PMO also known as Morpholino), peptide nucleic acid (PNA), glycol nucleic acid (GNA), threose nucleic acid (TNA), hexitol nucleic acid (HNA), 2'-deoxy-2'-fluoroarabinonucleic acid (FANA or FNA), 2'-deoxy-2'-fluororibonucleic acid (2'-F RNA or FRNA); altritol nucleic acids (ANA), cyclohexene nucleic acids (CeNA) etc.

In accordance with the canon, length of a nucleic acid is expressed herein the number of units from which a single strand of a nucleic acid is build. Because each unit corresponds to exactly one nucleobase capable of engaging in one base pairing event, the length is frequently expressed in so called "base pairs" or "bp" regardless of whether the nucleic acid in question is a single stranded (ss) or double stranded (ds) nucleic acid. In naturally-occurring nucleic acids 1 bp corresponds to 1 nucleotide, abbreviated to 1 nt. For example, a single stranded nucleic acid made of 1000 nucleotides (or a double stranded nucleic acid made of two complementary strands each of which is made of 1000 nucleotides) is described as having a length of 1000 base pairs or 1000 bp, which length can also be expressed as 1000 nt or as 1 kilobase that is abbreviated to 1 kb. 2 kilobases or 2 kb are equal to the length of 2000 base pairs which equates 2000 nucleotides of a single stranded RNA or DNA. To avoid confusion however, in view of the fact the nucleic acids as defined herein may comprise or consist of units not only chemically being nucleotides but also being functional equivalents thereof, the length of nucleic acids will preferentially be expressed herein in "bp" or "kb" rather than in the equally common in the art denotation "nt".

In advantageous embodiments as disclosed herein, the nucleic acid are no longer than 1kb, preferably no longer than 500 bp, most preferably no longer than 250 bp.

In particularly advantageous embodiments, the nucleic acid is an oligonucleotide (or simply an oligo) defined as nucleic acid being no longer than 150 bp, i.e. in accordance with the above provided definition, being any polymeric molecule made of no more than 150 units, wherein each unit comprises a nucleobase or a functional equivalent thereof, which renders said oligonucleotide capable of engaging in hydrogen bond-based nucleobase pairing under appropriate hybridisation conditions with DNA or RNA. Within the ambit of said definition, it will immediately be appreciated that the disclosed herein oligonucleotides can comprise or consist of units not only being nucleotides but also being synthetic equivalents thereof. In other words, from a chemistry perspective, as used herein the term oligonucleotide will be construed as possibly comprising or consisting of RNA, DNA, or a nucleic acid analogue such as but not limited to LNA (BNA), PMO (Morpholino), PNA, GNA, TNA, HNA, FANA, FRNA, ANA, CeNA and/or the like.

As used herein, the term "an endocytic receptor on a muscle cell" is to be understood as referring to surface molecules, likely receptors or transporters that accessible to their specific ligands from the external side or surface of the sarcolemma of the muscle cells and capable of undergoing internalisation via endocytic pathway e.g., upon external stimulation, such as ligand binding to the receptor. In some embodiments, an endocytic receptor on a muscle cell is internalized by clathrin-mediated endocytosis, but can also be internalized by a clathrin-independent pathway, such as, for example, phagocytosis, macropinocytosis, caveolae- and raft-mediated uptake or constitutive clathrin-independent endocytosis. In some embodiments, the endocytic receptor on a muscle cell comprises an intracellular domain, a transmembrane domain, and/or (e.g., and) an extracellular domain, which may optionally further comprise a ligand-binding domain. In some embodiments, the endocytic receptor on a muscle cell becomes internalized by the muscle cell after ligand binding. In some embodiments, a ligand may be a muscle-targeting agent or a muscle-targeting antibody. In some embodiments, an internalizing cell surface receptor is a transferrin receptor (CD71) or for example, CD63 (also known as LAMP-3) belonging to the tetraspanin family.

The term "antibody-oligonucleotide conjugate" or "AOC" has its regular scientific meaning and here refers to any conjugate of an antibody such as an IgG, a Fab, an scFv, an immunoglobulin, an immunoglobulin fragment, one or multiple V_{H} domains, single-domain antibodies, a V_{HH}, a camelid V_{H}, *etc.,* and any polynucleotide (oligonucleotide) molecule that can exert a therapeutic effect when contacted with cells of a subject such as a human patient, such as an oligonucleotide selected from a natural or synthetic string of nucleic acids encompassing DNA, modified DNA, RNA, mRNA, modified RNA, synthetic nucleic acids, presented as a single-stranded molecule or a double-stranded molecule, such as a BNA, an antisense oligonucleotide (ASO, AON), a short or small interfering RNA (siRNA; silencing RNA), an anti-sense DNA, anti-sense RNA, *etc.*

As used herein, the term "antibody or a binding fragment thereof" refers to a polypeptide that includes at least one immunoglobulin variable domain or at least one antigenic determinant, e.g., paratope that specifically binds to an antigen. In some embodiments, an antibody is a full length antibody. In some embodiments, an antibody is a chimeric antibody. In some embodiments, an antibody is a humanized antibody. However, in some embodiments, an antibody is a Fab fragment, a F(ab') fragment, a F(ab')2 fragment, a Fv fragment or a scFv fragment. In some embodiments, an antibody is a nanobody derived from a camelid antibody or a nanobody derived from a shark antibody. In some embodiments, an antibody is a diabody. In some embodiments, an antibody comprises a framework having a human germline sequence. In another embodiment, an antibody comprises a heavy chain constant domain selected from the group consisting of IgG, IgG1, IgG2, IgG2A, IgG2B, IgG2C, IgG3, IgG4, IgAl, IgA2, IgD, IgM, and IgE constant domains. In some embodiments, an antibody comprises a heavy (H) chain variable region (abbreviated herein as VH), and/or (e.g., and) a light (L) chain variable region (abbreviated herein as VL). In some embodiments, an antibody comprises a constant domain, e.g., an Fc region. An immunoglobulin constant domain refers to a heavy or light chain constant domain. Human IgG heavy chain and light chain constant domain amino acid sequences and their functional variations are known. With respect to the heavy chain, in some embodiments, the heavy chain of an antibody described herein can be an alpha (a), delta (D), epsilon (e), gamma (g) or mu (m) heavy chain. In some embodiments, the heavy chain of an antibody described herein can comprise a human alpha (a), delta (D), epsilon (e), gamma (g) or mu (m) heavy chain. In a particular embodiment, an antibody described herein comprises a human gamma 1 CHI, CH2, and/or (e.g., and) CH3 domain. In some embodiments, the amino acid sequence of the VH domain comprises the amino acid sequence of a human gamma (g) heavy chain constant region, such as any known in the art. Non-limiting examples of human constant region sequences have been described in the art, e.g., see U.S. Pat. No. 5,693,780 and Kabat E A et al, (1991) supra. In some embodiments, the VH domain comprises an amino acid sequence that is at least 70%, 75%, 80%, 85%, 90%, 95%, 98%, or at least 99% identical to any of the variable chain constant regions provided herein. In some embodiments, an antibody is modified, e.g., modified via glycosylation, phosphorylation, sumoylation, and/or (e.g., and) methylation. In some embodiments, an antibody is a glycosylated antibody, which is conjugated to one or more sugar or carbohydrate molecules. In some embodiments, the one or more sugar or carbohydrate molecule are conjugated to the antibody via N-glycosylation, O-glycosylation, C-glycosylation, glypiation (GPI anchor attachment), and/or (e.g., and) phosphoglycosylation. In some embodiments, the one or more sugar or carbohydrate molecule are monosaccharides, disaccharides, oligosaccharides, or glycans. In some embodiments, the one or more sugar or carbohydrate molecule is a branched oligosaccharide or a branched glycan. In some embodiments, the one or more sugar or carbohydrate molecule includes a mannose unit, a glucose unit, an N-acetylglucosamine unit, an N-acetylgalactosamine unit, a galactose unit, a fucose unit, or a phospholipid unit. In some embodiments, an antibody is a construct that comprises a polypeptide comprising one or more antigen binding fragments of the disclosure linked to a linker polypeptide or an immunoglobulin constant domain. Linker polypeptides comprise two or more amino acid residues joined by peptide bonds and are used to link one or more antigen binding portions. Examples of linker polypeptides have been reported (see e.g., Holliger, P, et al. (1993) Proc. Natl. Acad. Sci. USA 90:6444-6448; Poljak, R. J., et al. (1994) Structure 2:1121-1123). Still further, an antibody may be part of a larger immunoadhesion molecule, formed by covalent or noncovalent association of the antibody or antibody portion with one or more other proteins or peptides. Examples of such immunoadhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058).

The term "single domain antibody", or "sdAb", in short, or 'nanobody', has its regular scientific meaning and here refers to an antibody fragment consisting of a single monomeric variable antibody domain, unless referred to as more than one monomeric variable antibody domain such as for example in the context of a bivalent sdAb, which comprises two of such monomeric variable antibody domains in tandem. A bivalent nanobody is a molecule comprising two single domain antibodies targeting epitopes on molecules present at the extracellular side of a cell, such as epitopes on the extracellular domain of a cell surface molecule that is present on the cell. Preferably the cell-surface molecule is a cell-surface receptor. A bivalent nanobody is also named a bivalent single domain antibody. Preferably the two different single domain antibodies are directly covalently bound or covalently bound through an intermediate molecule that is covalently bound to the two different single domain antibodies. Preferably the intermediate molecule of the bivalent nanobody has a molecular weight of less than 10,000 Dalton, more preferably less than 5000 Dalton, even more preferably less than 2000 Dalton, most preferably less than 1500 Dalton.

As used herein, the term "covalently linked" refers to a characteristic of two or more molecules being linked together via at least one covalent bond, i.e. directly, or via a chain of covalent bonds, i.e. via a linker comprising at least one or more atoms.

As used herein, the term "conjugate" is to be construed as a combination of two or more different molecules that have been and are covalently bound. For example, different molecules forming a conjugate as disclosed herein may include one or more saponins or saponin molecules, one or more nucleic acid or oligonucleotide molecules, one or more ligands that bind to an endocytic receptor present on a surface of a muscle cell, preferably wherein the ligand is an antibody or a binding fragment thereof, such as an IgG, a monoclonal antibody (mAb), a VHH domain or anther nanobody type, a bivalent nanobody molecule comprising two single domain antibodies, etc. In some aspects, the disclosed herein conjugates may be made by covalently linking different molecules via one or more intermediate molecules such as linkers, such as for example via linking to a central or further linker. In a conjugate, not all of the two or more, such as three, different molecules need to be directly covalently bound to each other. Different molecules in the conjugate may also be covalently bound by being both covalently bound to the same intermediate molecule such as a linker or each by being covalently bound to an intermediate molecule such as a further linker or a central linker wherein these two intermediate molecules such as two (different) linkers, are covalently bound to each other. According to this definition even more intermediate molecules, such as linkers, may be present between the two different molecules in the conjugate as long as there is a chain of covalently bound atoms in between.

As used herein, the terms "administering" or "administration" means to provide a complex to a subject in a manner that is physiologically and/or (e.g., and) pharmacologically useful (e.g., to treat a condition in the subject)

As used herein, the term "approximately" or "about," as applied to one or more values of interest, refers to a value that is similar to a stated reference value. In certain embodiments, the term "approximately" or "about" refers to a range of values that fall within 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments as described herein can operate in combination and cooperation, unless specified otherwise. Furthermore, the various embodiments, although referred to as "preferred" or "e.g." or "for example" or "in particular" and the like are to be construed as exemplary manners in which the disclosed herein concepts may be implemented rather than as limiting.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

The term "Saponinum album" has its normal meaning and here refers to a mixture of saponins produced by Merck KGaA (Darmstadt, Germany) containing saponins from *Gypsophila paniculata* and *Gypsophila arostii,* containing SA1657 and mainly SA1641.

The term "Quillaja saponin" has its normal meaning and here refers to the saponin fraction of Quillaja saponaria and thus the source for all other QS saponins, mainly containing QS-18 and QS-21.

"QS-21" or "QS21" has its regular scientific meaning and here refers to a mixture of QS-21 A-apio (~63%), QS-21 A-xylo (~32%), QS-21 B-apio (~3.3%), and QS-21 B-xylo (~1.7%).

Similarly, "QS-21A" has its regular scientific meaning and here refers to a mixture of QS-21 A-apio (~65%) and QS-21 A-xylo (~35%).

Similarly, "QS-21B" has its regular scientific meaning and here refers to a mixture of QS-21 B-apio (~65%) and QS-21 B-xylo (~35%).

The term "Quil-A" refers to a commercially available semi-purified extract from Quillaja saponaria and contains variable quantities of more than 50 distinct saponins, many of which incorporate the triterpene-trisaccharide substructure Gal-(1→2)-[Xyl-(1→3)]-GlcA- at the C-3beta-OH group found in QS-7, QS-17, QS-18, and QS-21. The saponins found in Quil-A are listed in van Setten (1995), Table *2 [*Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)]. Quil-A and also Quillaja saponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et *al.* (2019) in Table 1, row no. 28 *[*Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171*].* The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

The terms "SO1861" and "SO1862" refer to the same saponin of *Saponaria officinalis,* though in deprotonated form or api form, respectively. The molecular mass is 1862 Dalton as this mass is the formal mass including a proton at the glucuronic acid. At neutral pH, the molecule is deprotonated. When measuring the mass using mass spectrometry in negative ion mode, the measured mass is 1861 Dalton.

### BRIEF DESCRIPTION OF THE DRAWINGS

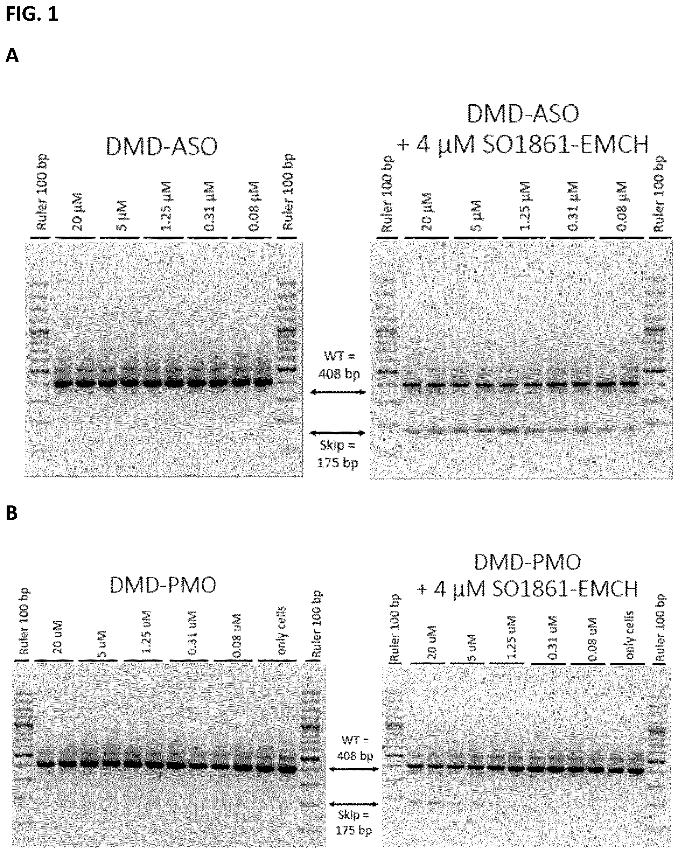

**Figure 1****:** Exon skip using (A) DMD-ASO without (left panel) or with (right panel) co-administration of SO1861-EMCH and (B) DMD-PMO without (left panel) or with (right panel) co-administration of SO1861-EMCH in differentiated human myotubes from a non-DMD (healthy) donor (KM155)

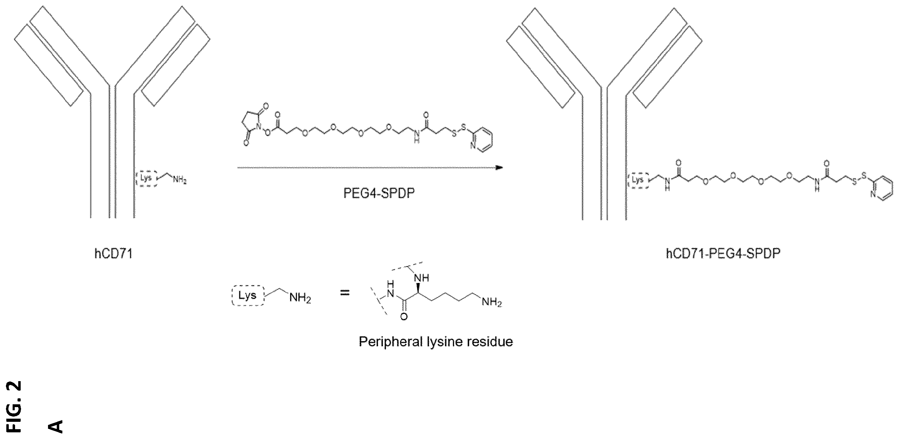

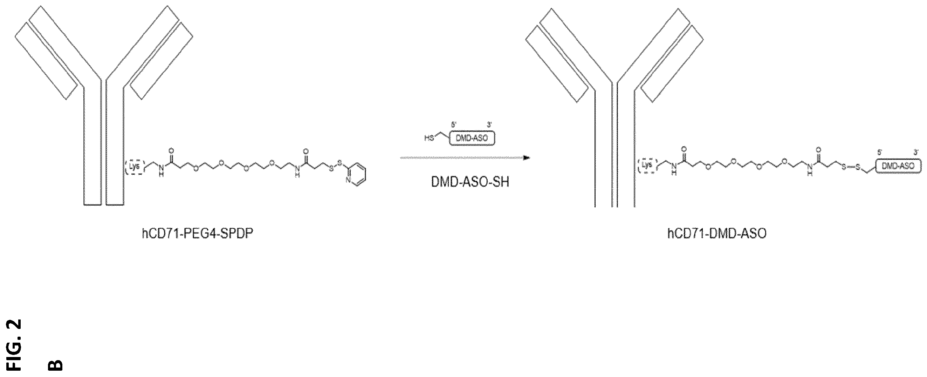

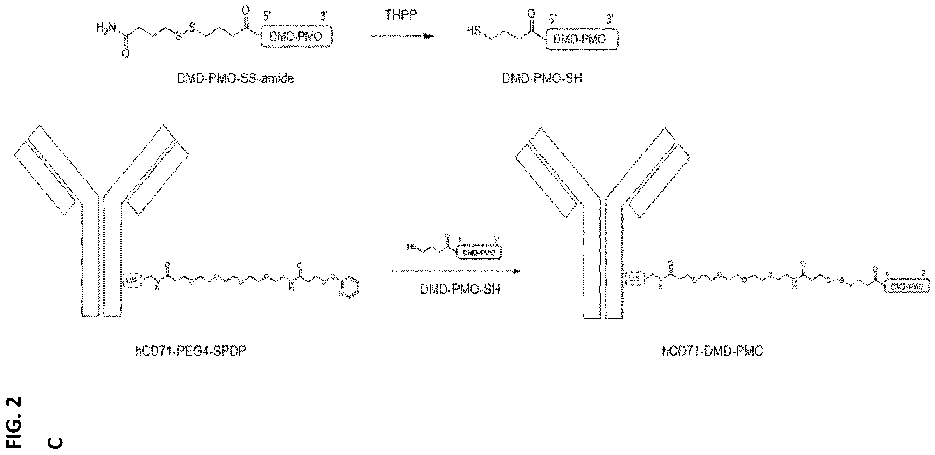

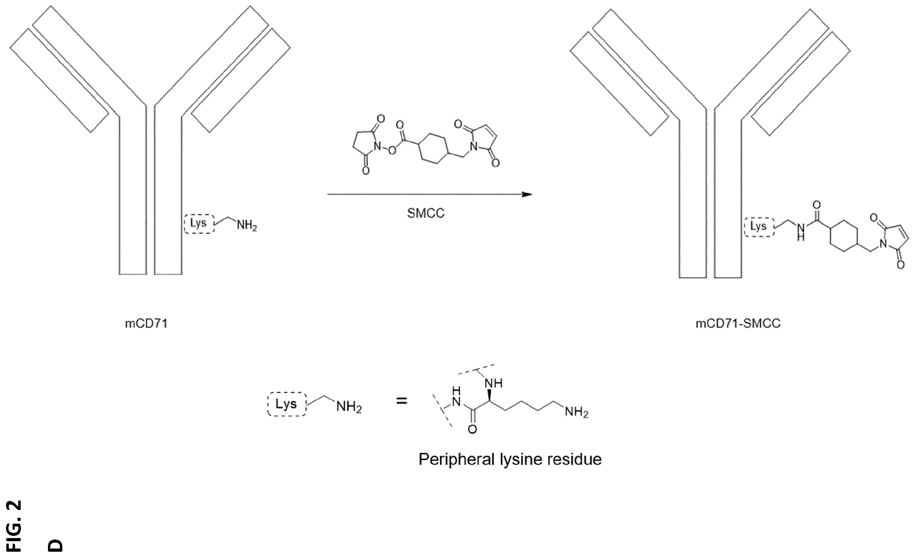

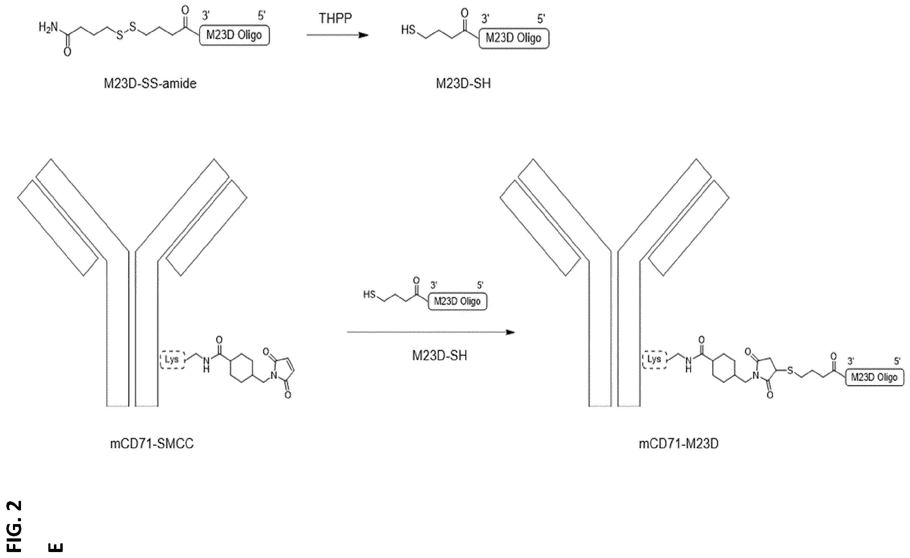

**Figure 2****:** (A) Synthesis of hCD71-PEG4-SPDP precursor to produce (B) hCD71-DMD-ASO and (C) hCD71-DMD-PMO; (D) synthesis of mCD71-SMCC; (E) synthesis of mCD71-M23D

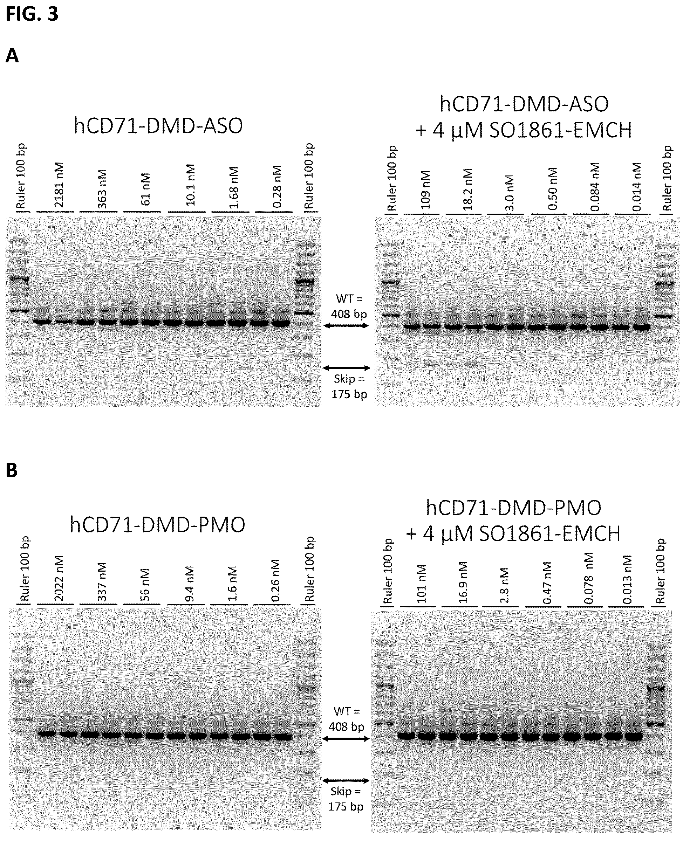

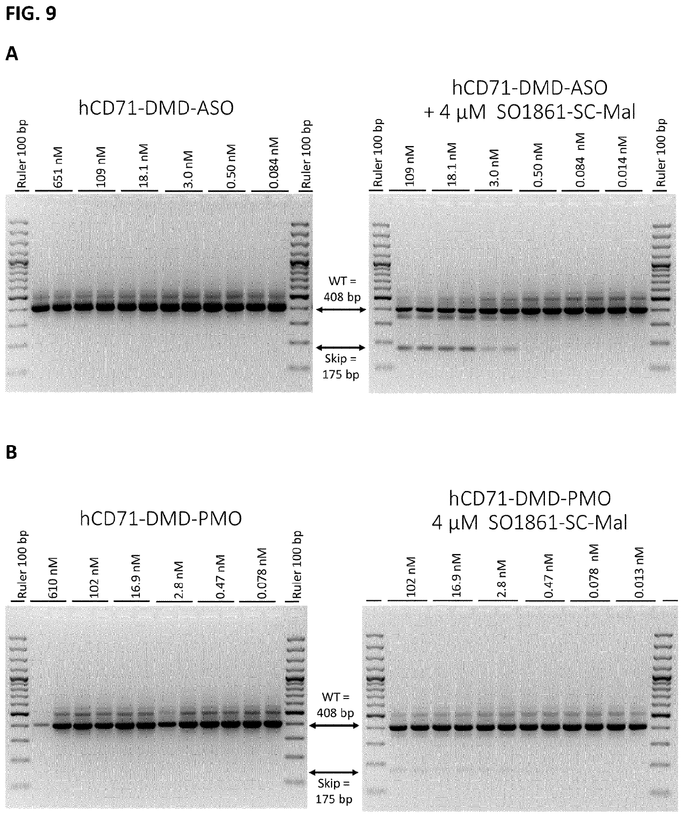

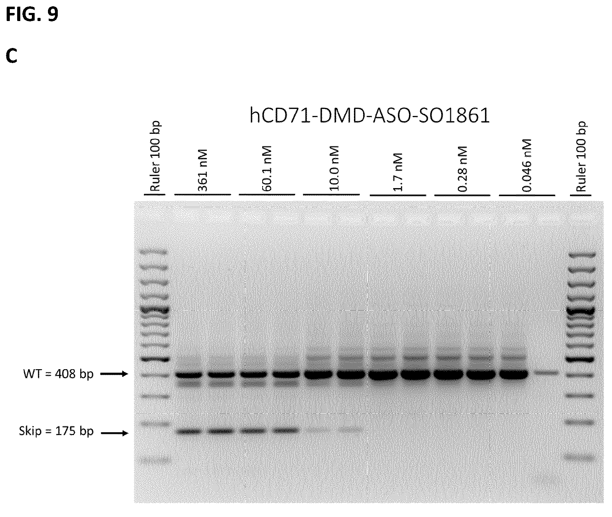

**Figure 3****:** Exon skip using (A) hCD71-DMD-ASO (DAR2.1) without (left panel) or with (right panel) co-administration of SO1861-EMCH and (B) hCD71-DMD-PMO (DAR3.2) without (left panel) or with (right panel) co-administration of SO1861-EMCH in differentiated human myotubes from a non-DMD (healthy) donor (KM155); see also Figure 9

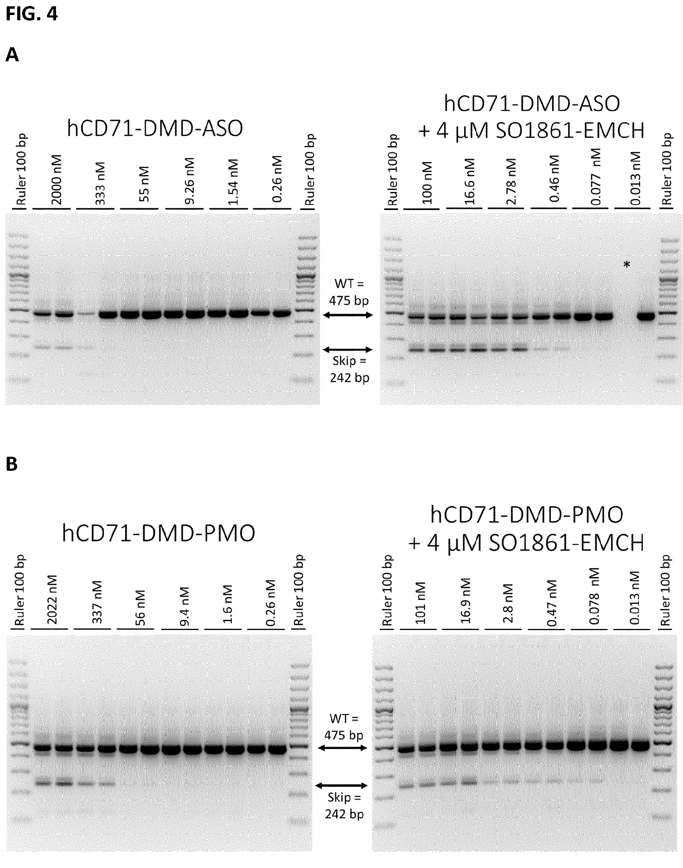

**Figure 4****:** Exon skip using (A) hCD71-DMD-ASO without (left panel) or with (right panel) co-administration of SO1861-EMCH and (B) hCD71-DMD-PMO without (left panel) or with (right panel) co-administration of SO1861-EMCH in differentiated human myotubes from a DMD-affected donor (DM8036); NB, in (A, right panel) the first sample at 0.013 nM (asterisk) shows an empty lane

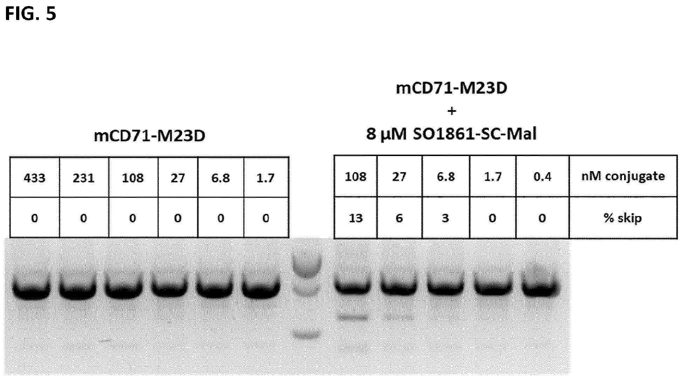

**Figure 5****:** Exon skip using mCD71-M23D PMO without (left panel) or with (right panel) co-administration of SO1861-SC-Mal in differentiated murine C2C12 myotubes

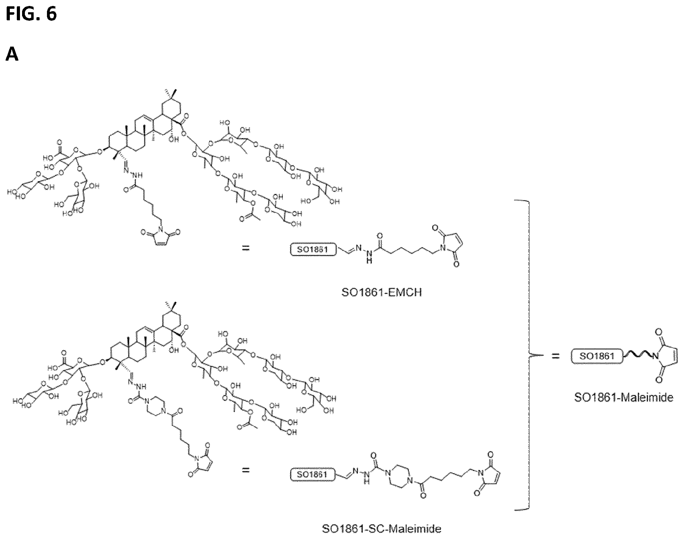

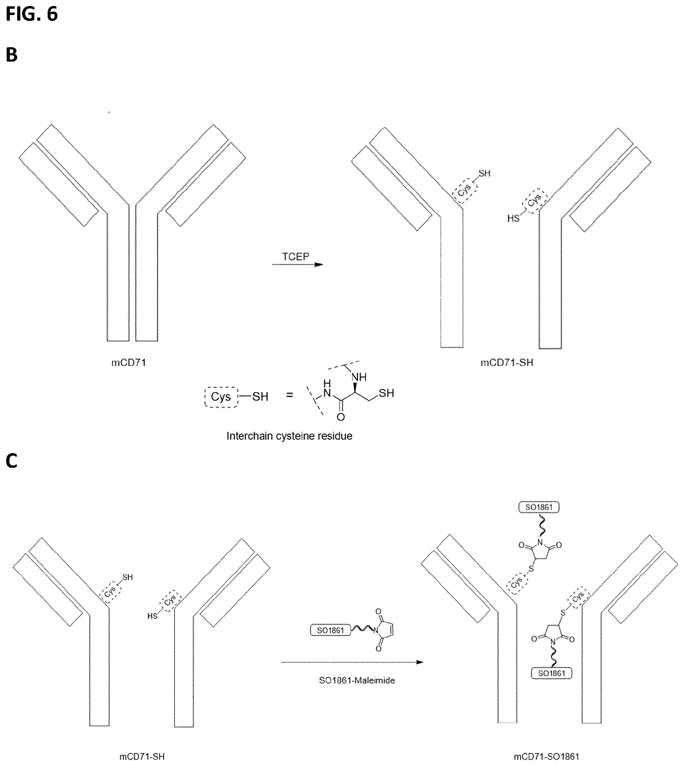

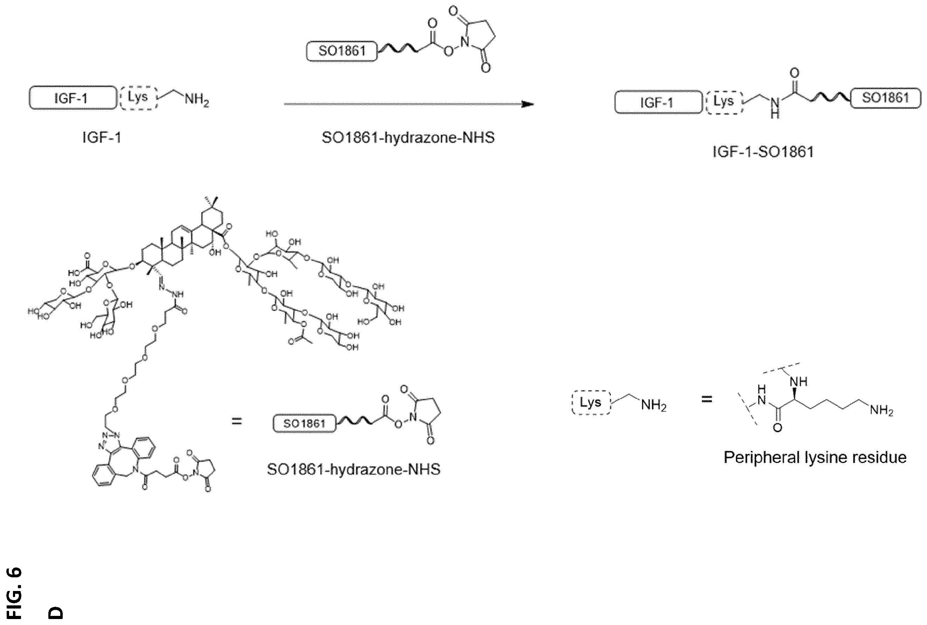

**Figure 6****:** (A-C) Synthesis of mCD71-SO1861 yielding an intact mAb conjugated with SO1861 via interchain cysteines and held together by various forces as known in the art using (A) either SO1861-EMCH or SO1861-SC-Maleimide, (B) generation of the mCD71-SH intermediate, (C) synthesis of mCD71-SO1861; NB: for clarity of schematic representation, the space between heavy chains was enlarged in the figures; (D) IGF-1 ligand was conjugated to SO1861-hydrazone-NHS to produce IGF-1-SO1861

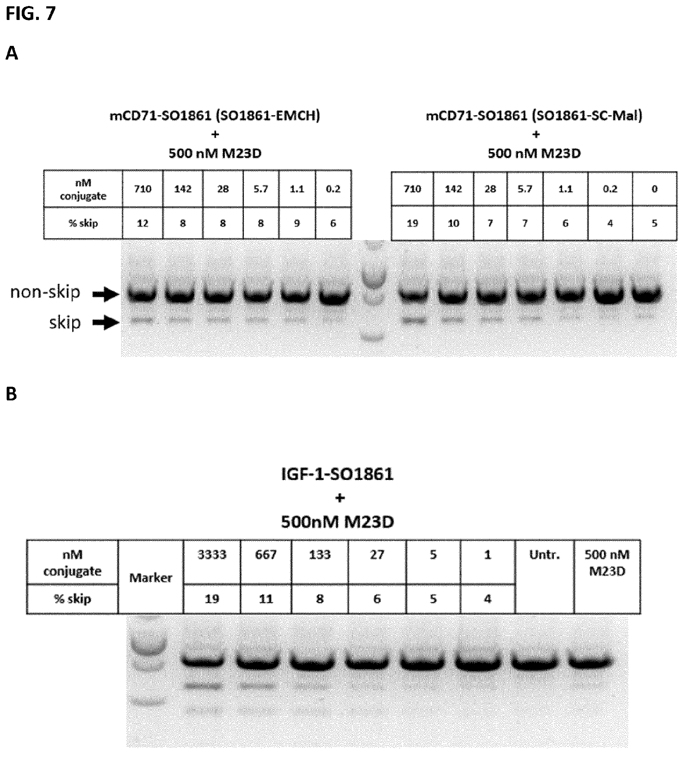

**Figure 7****:** Exon skip using (A) mCD71-SO1861 (synthesized with SO1861-EMCH) + M23D (left panel) and mCD71-SO1861 (synthesized with SO1861-SC-Mal) + M23D (right panel), and (B) IGF-1-SO1861 + M23D (left panel) and controls (right panel) in differentiated murine C2C12 myotubes

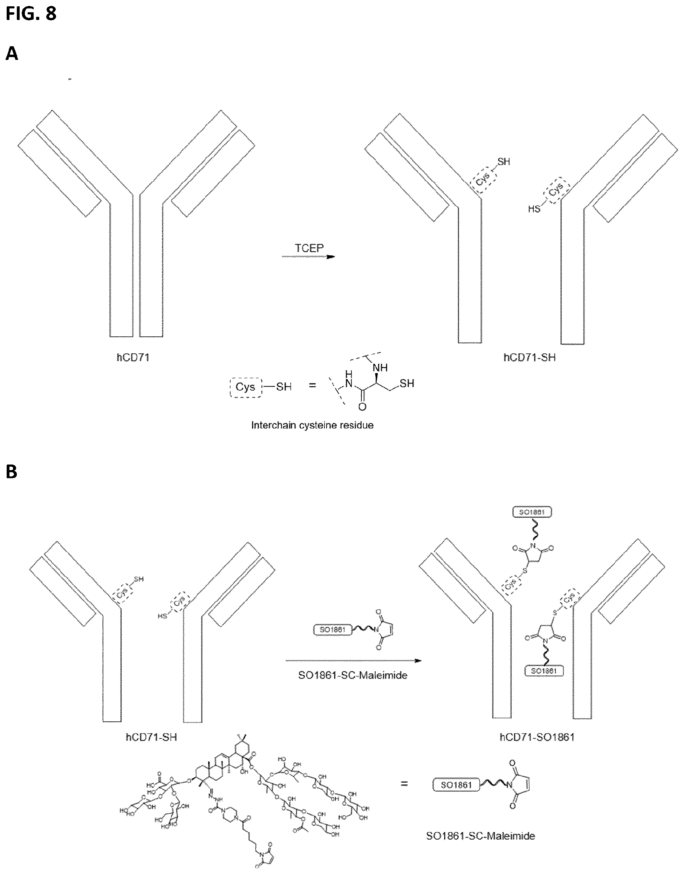

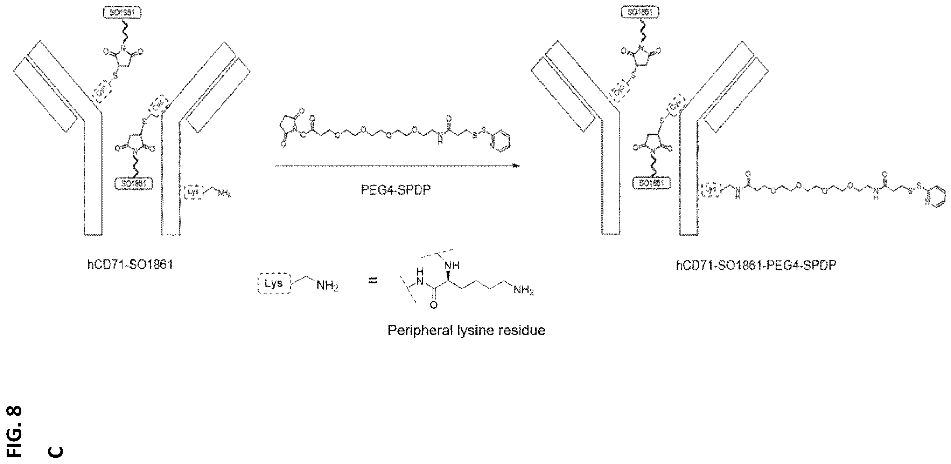

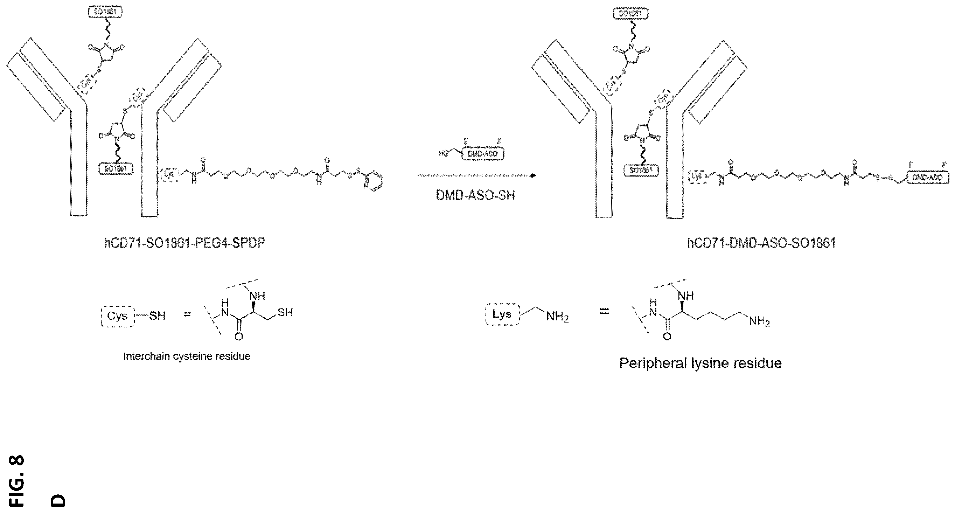

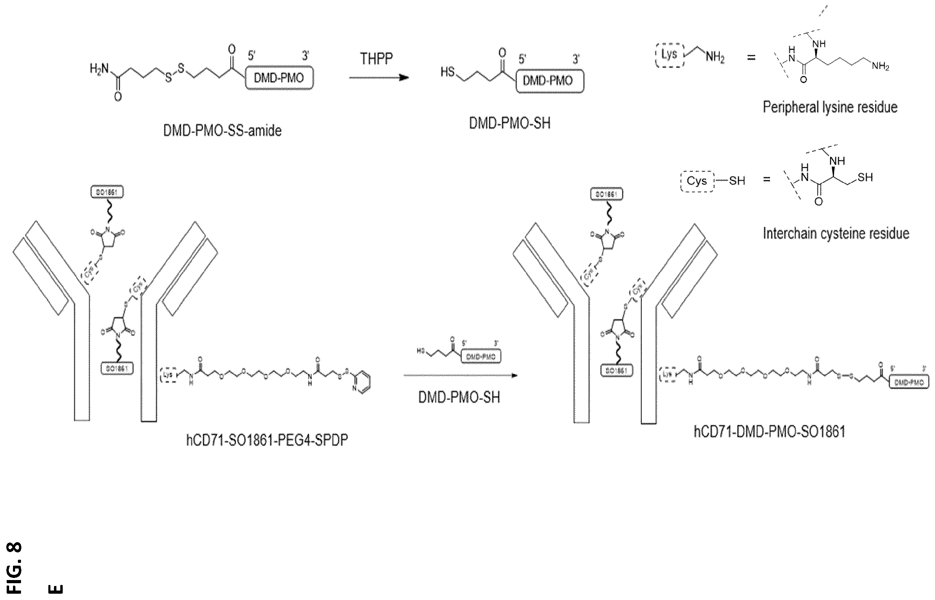

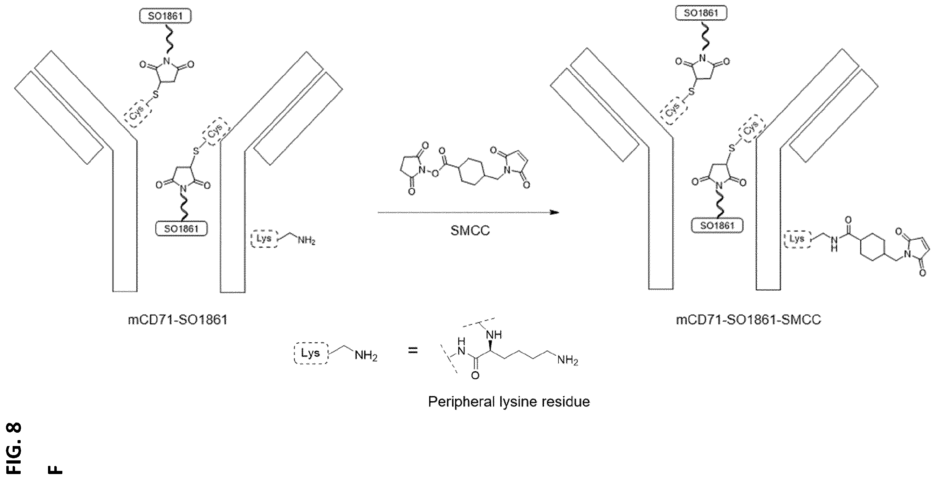

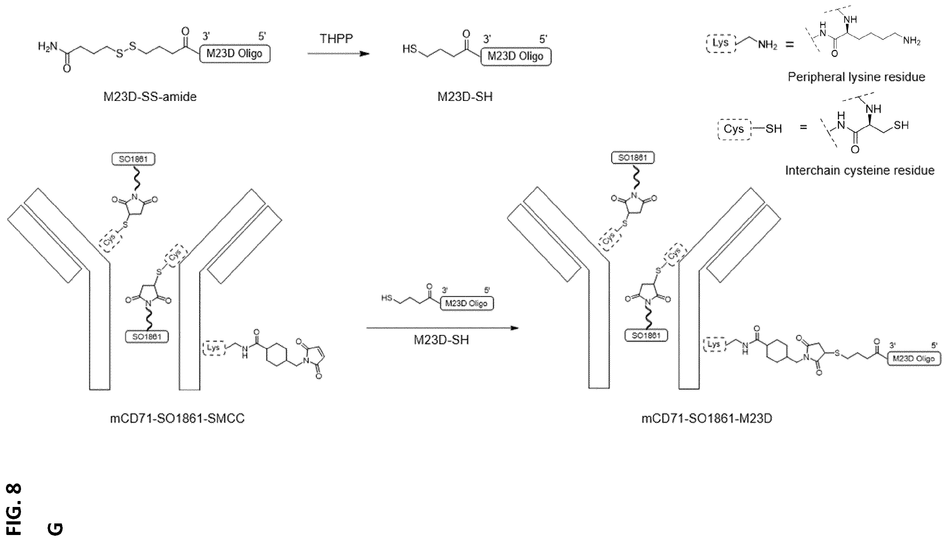

**Figure 8****:** (A-E) Synthesis of hCD71-DMD-ASO-SO1861 and hCD71-DMD-PMO-SO1861, yielding intact mAbs conjugated with SO1861 via interchain cysteines and held together by various forces as known in the art, with (A) interchain disulfide reduction of hCD71, (B) synthesis of hCD71-SO1861, (C) synthesis of hCD71-SO1861-PEG4-SPDP, (D) synthesis of hCD71-DMD-ASO-SO1861, and (E) synthesis of hCD71-DMD-PMO-SO1861, respectively; (F) synthesis of mCD71-M23D-SMCC, and (G) synthesis of mCD71-M23D-SO1861, yielding intact mAbs conjugated with SO1861 via interchain cysteines and held together by various forces as known in the art; NB: for clarity of schematic representation, the space between heavy chains was enlarged in the figures
**Figure 9****:** Exon skip assessment using (A) hCD71-DMD-ASO (DAR2.2) without (left panel) or with (right panel) co-administration of SO1861-SC-Mal and (B) hCD71-DMD-PMO (DAR3.1) without (left panel) or with (right panel) co-administration of SO1861-SC-Mal and (C) hCD71-DMD-ASO-SO1861 1-component in differentiated human myotubes from a non-DMD (healthy) donor (KM155)

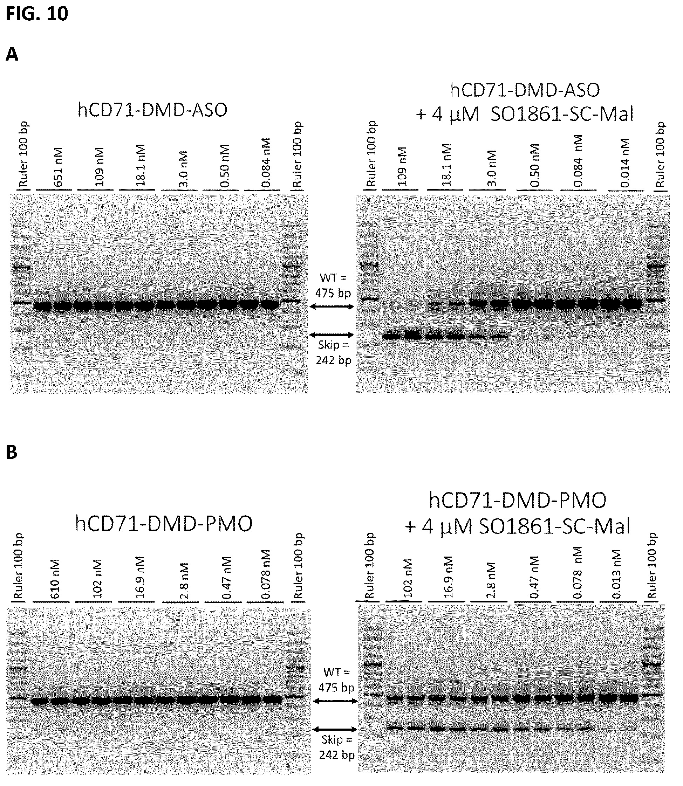

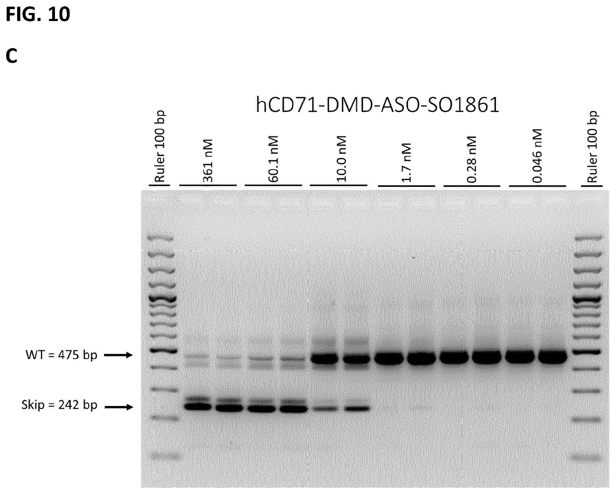

**Figure 10****:** Exon skip assessment using (A) hCD71-DMD-ASO without (left panel) or with (right panel) co-administration of SO1861-SC-Mal and (B) hCD71-DMD-PMO without (left panel) or with (right panel) co-administration of SO1861-SC-Mal and (C) hCD71-DMD-ASO-SO1861 1-component in differentiated human myotubes from a DMD-affected donor (DM8036)

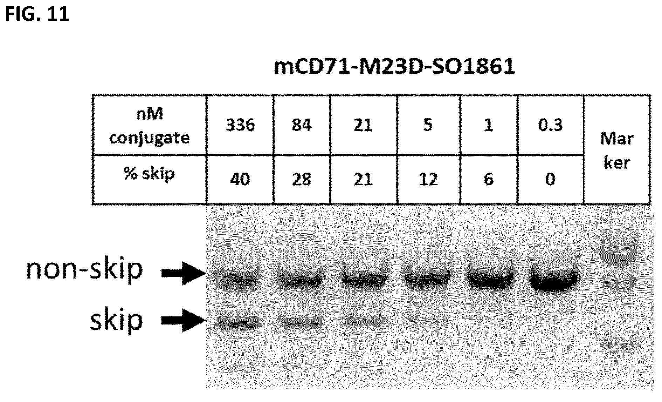

**Figure 11****:** Assessment of exon skip using mCD71-M23D-SO1861 in murine C2C12 differentiated myotubes

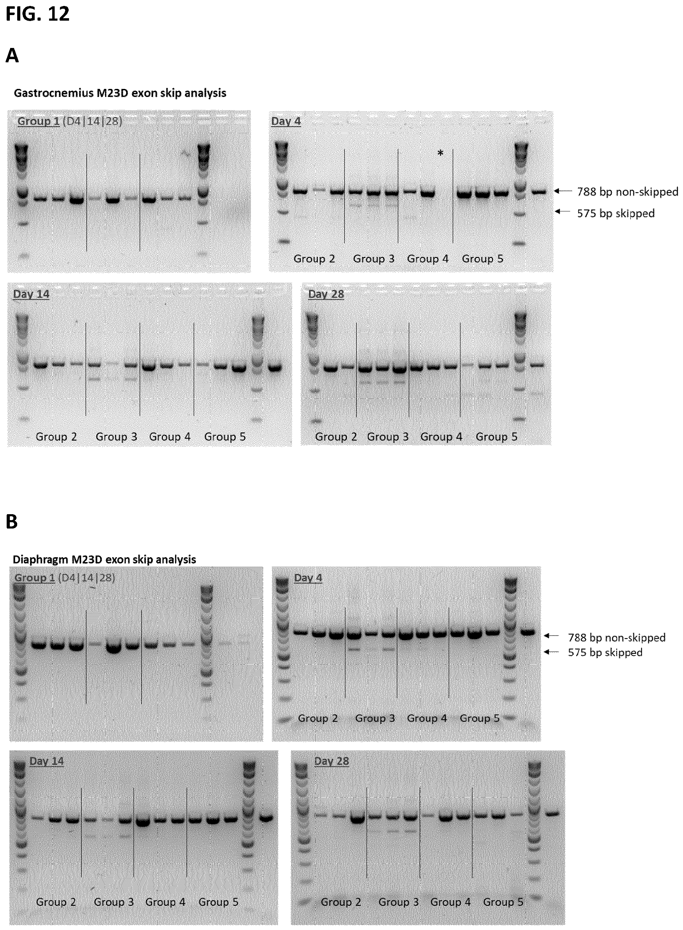

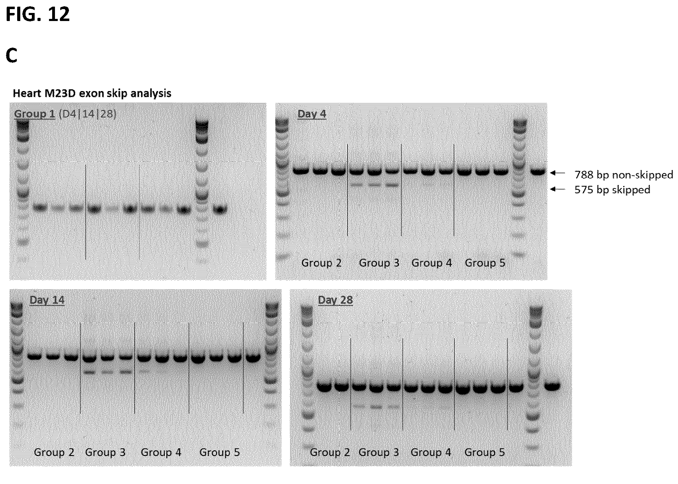

**Figure 12****:** Exon skip analysis in mice from a single dose mCD71-M23D (group 2) or single dose mCD71-M23D-SO1861 (3 dose groups, group 3, group 4, group 5) and a vehicle control (group 1) in (A) gastrocnemius, (B) diaphragm, and (C) heart at day 4, day 14, and day 28 post treatment; NB, in (A), the PCR amplification of sample 3 in group 4, day 4 (asterisk), showing an empty lane, failed initially but a technical repeat of the cDNA synthesis/PCR showed full-length product and no skip measurable in this animal

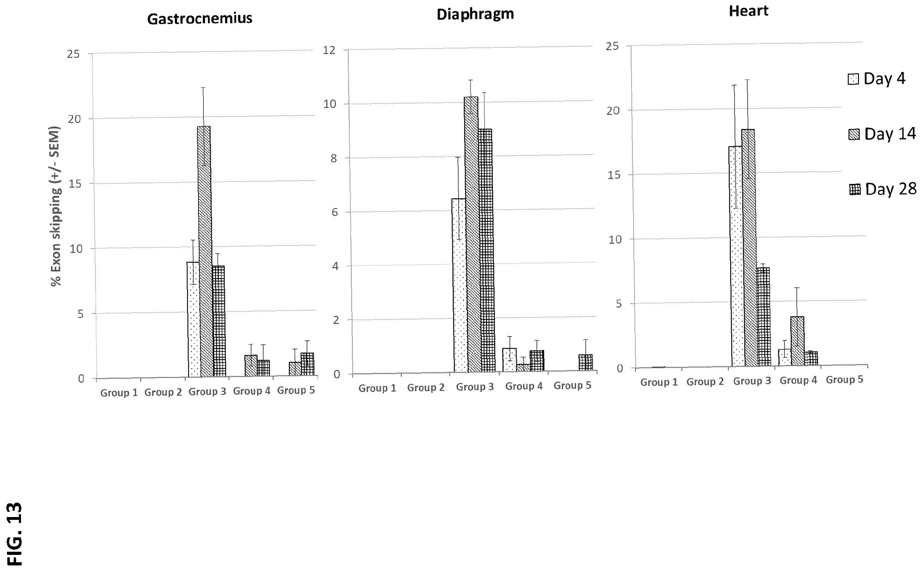

**Figure 13****:** Densitometric quantification of mean exon skip in mice (N=3 per group, per timepoint; except for gastrocnemius group 4, day 4, where N=2) from a single dose mCD71-M23D (group 2) or single dose mCD71-M23D-SO1861 (3 dose groups, group 3, group 4, group 5) in (A) gastrocnemius, (B) diaphragm, and (C) heart at day 4, day 14, and day 28 post treatment; (A-C) quantification of band intensities with a ChemiDoc^{™} XRS+ System with Image Lab^{™} Software (BioRad)

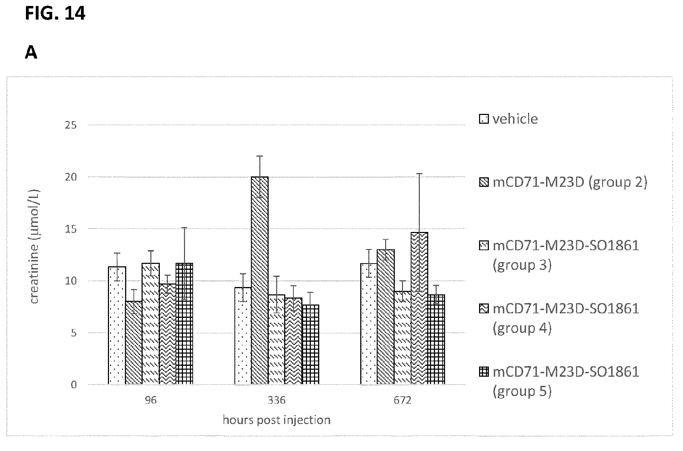

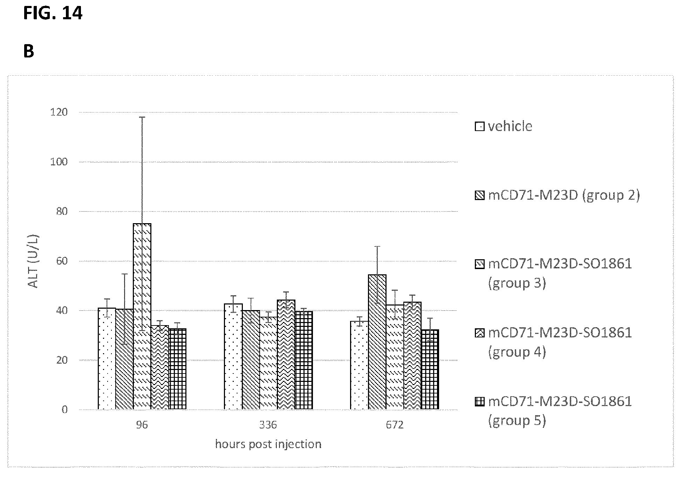

**Figure 14****:** (A) Serum creatinine and (B) serum ALT analysis from a single dose study with mCD71-M23D (group 2) or single dose mCD71-M23D-SO1861 (3 dose groups: group 3, group 4, group 5) and a vehicle control group at day 4, day 14, day 28 (N=3 per dose/timepoint, except mCD71-M23D, day 28, where N=2).

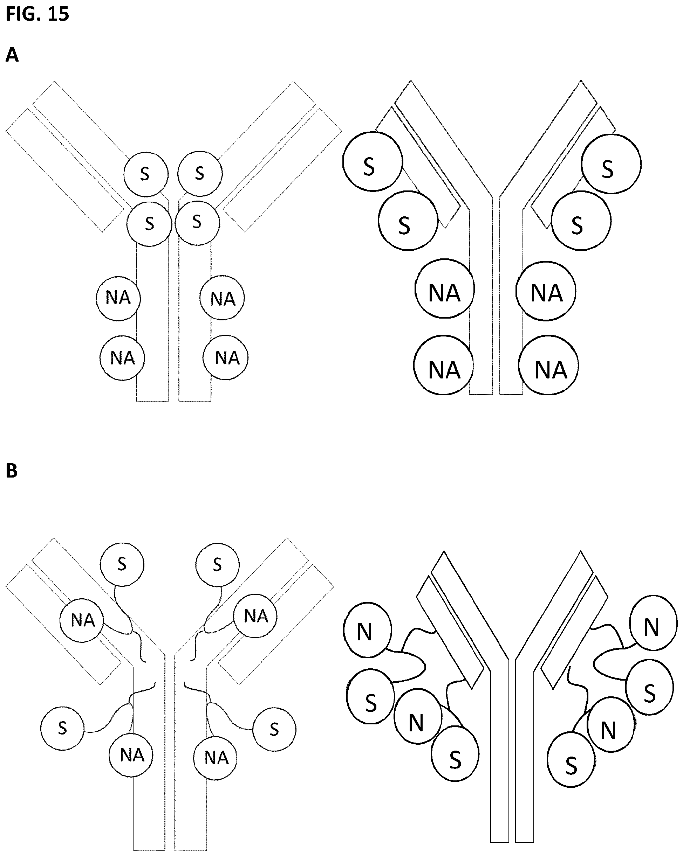

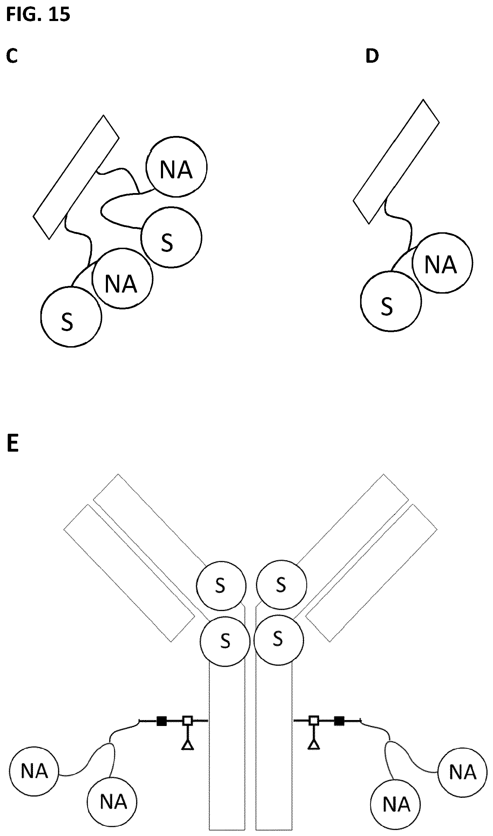

**Figure 15****:** (A) Schematic representation of possible conjugates of the present invention. Shown is an IgG antibody covalently conjugated with four saponin molecules 'S', bound to either the light chains or the hinge region of the antibody (both options schematically represented), and with four effector nucleic acid molecules 'NA' that are covalently bound to the constant domains of the heavy chain of the antibody. (B) Schematic representation of a possible embodiment of a conjugate of the present invention. Shown is an IgG antibody covalently conjugated with four trivalent linkers, wherein each linker is covalently bound to a saponin and is covalently bound to a nucleic acid molecule. The trivalent linkers are covalently bound to the antibody for example by the hinge region or by the light chains (both options shown). (C) Schematic representation of a possible embodiment of a conjugate of the present invention. Shown is a single domain antibody covalently conjugated with two trivalent linkers, wherein each linker is covalently bound to a saponin and is covalently bound to a nucleic acid molecule. (D) Schematic representation of a possible embodiment of a conjugate of the present invention. Shown is a single domain antibody covalently conjugated with a trivalent linker, wherein the trivalent linker is covalently bound to a saponin and is covalently bound to a nucleic acid molecule. (E) Schematic representation of a possible embodiment of a conjugate of the present invention. Shown is an IgG antibody covalently conjugated with four saponin molecules 'S', bound to the hinge region of the antibody, and with four effector nucleic acid molecules 'NA' that are covalently bound to the constant domains of the heavy chain of the antibody via glycan remodelling and click-chemistry.

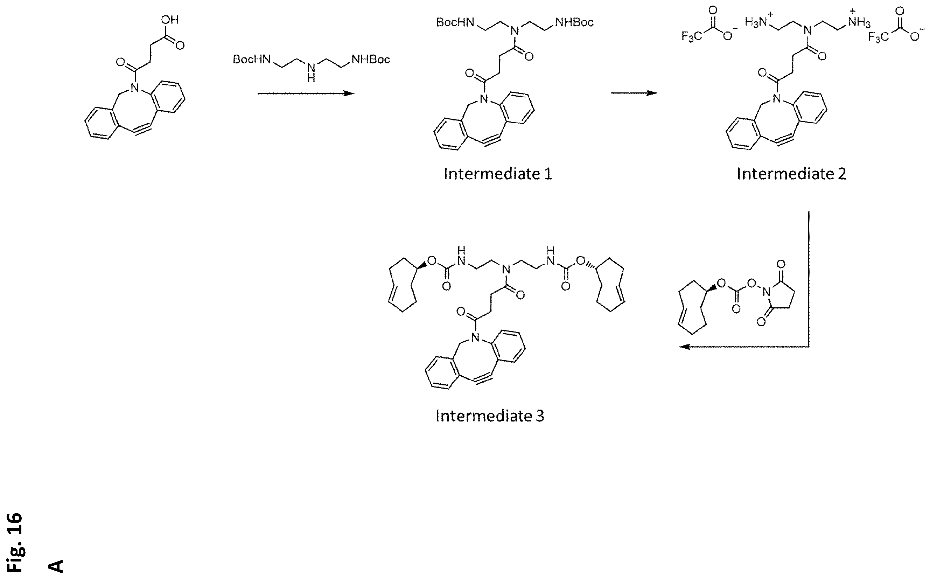

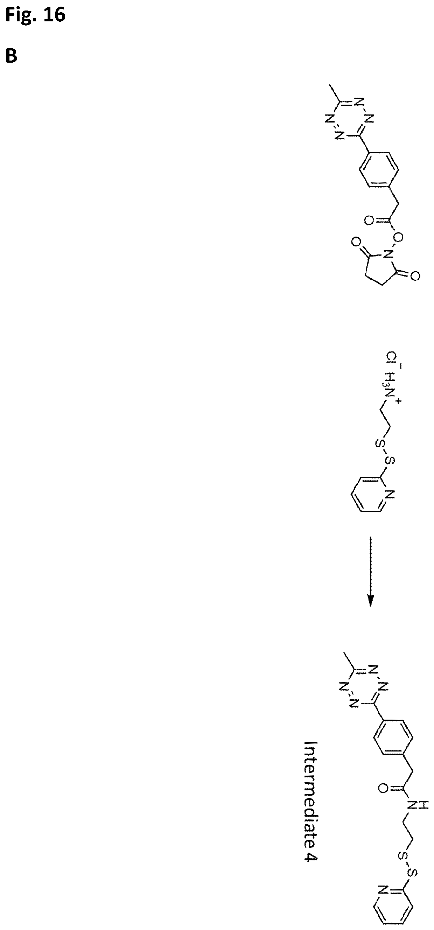

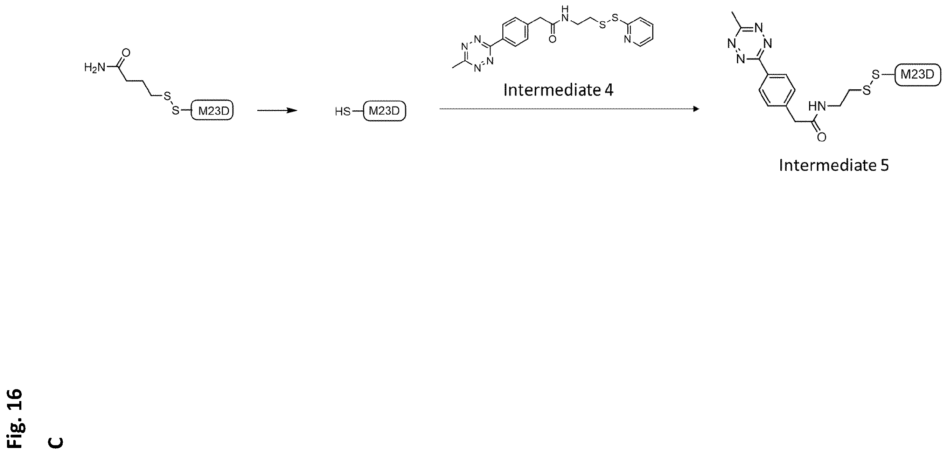

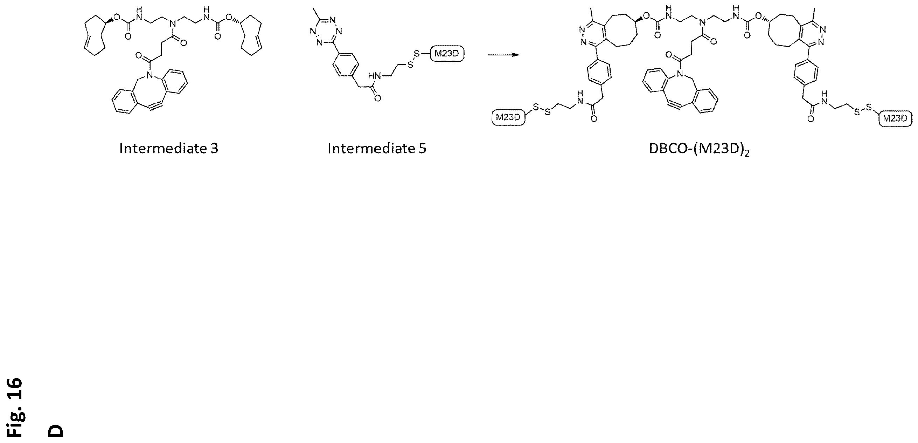

**Figure 16****:** Synthesis of DBCO-(M23D)₂ via a synthesis scheme involving (A) the synthesis of intermediate 3 (via intermediates 1 and 2); (B) the synthesis of intermediate 4; (C) coupling of intermediate 4 with M23D-SH (reduced form) to achieve the synthesis of intermediate 5; and (D) coupling of intermediates 3 and 5 to yield the desired final product DBCO-(M23D)₂, i.e. a branched scaffold bearing two M23D PMO oligonucleotide payloads.

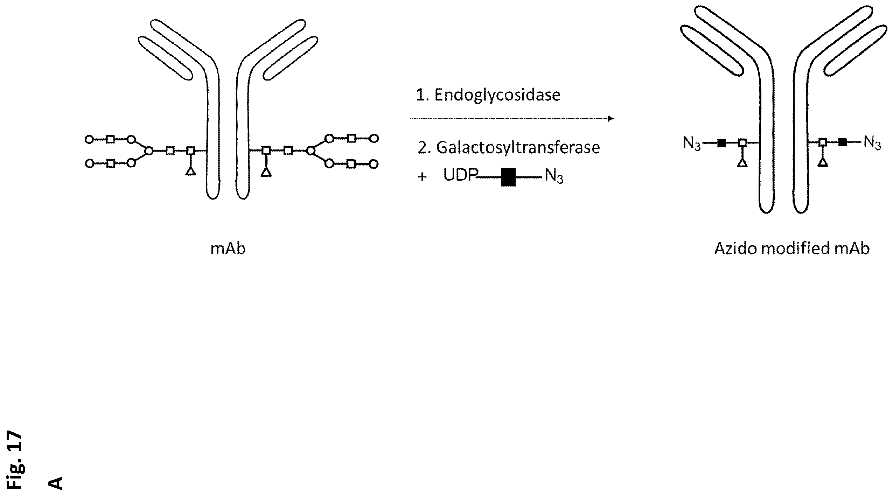

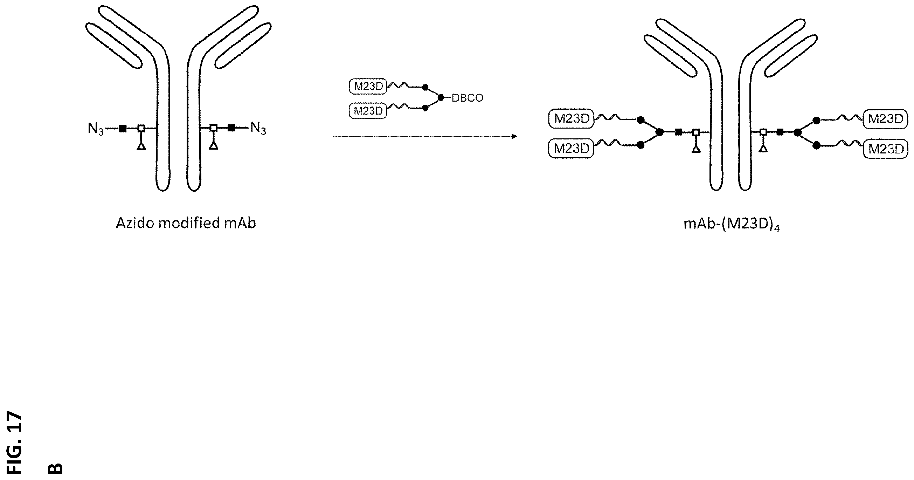

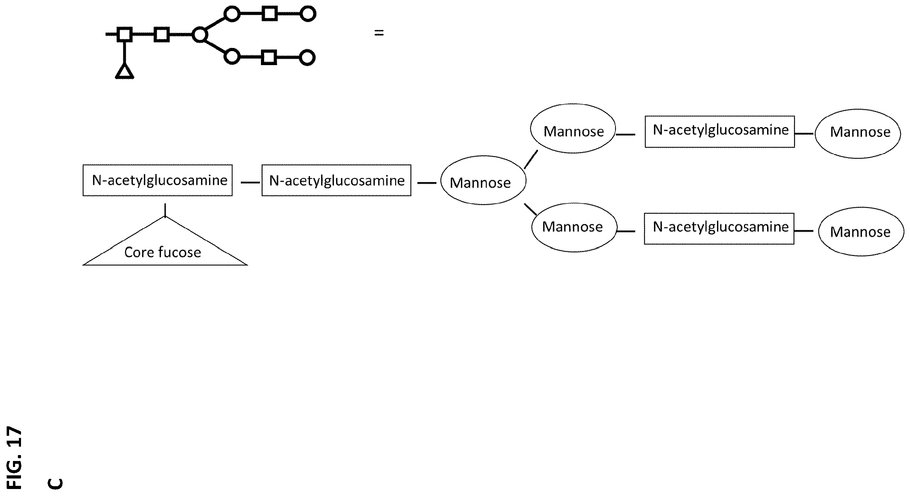

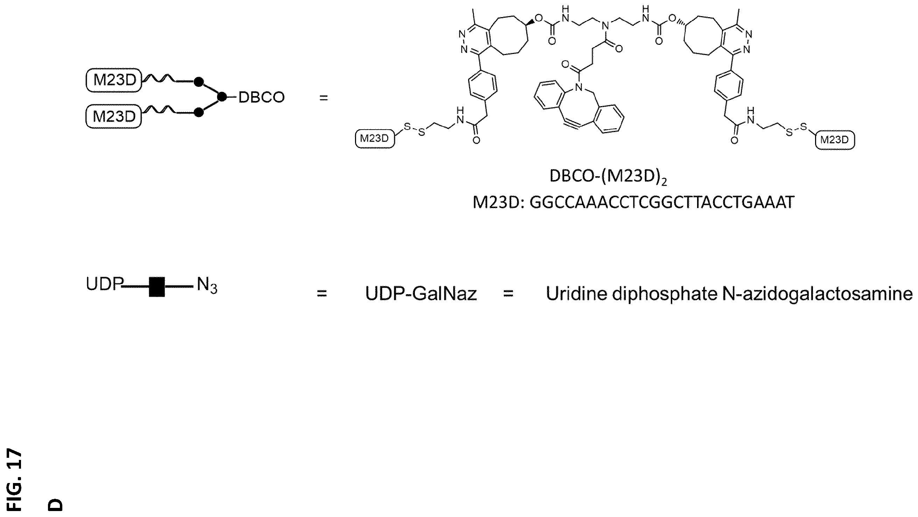

**Figure 17****:** Schematic representation of the conjugation procedure for mAb-M23D, such as mCD71-M23D and mCD63-M23D. (A) Preparation of a trimmed and azido modified mAb glycan. (B) Conjugation, via strain-promoted azide-alkyne click reaction, between the trimmed and azido modified mAb glycan and DBCO-(M23D)₂, yielding mAb-(M23D)₄. NB: for clarity of schematic representation, mAb-M23D is shown with DAR 4. (C) Legend explaining symbolically represented glycan residues. (D) Legend explaining symbolically represented molecules.

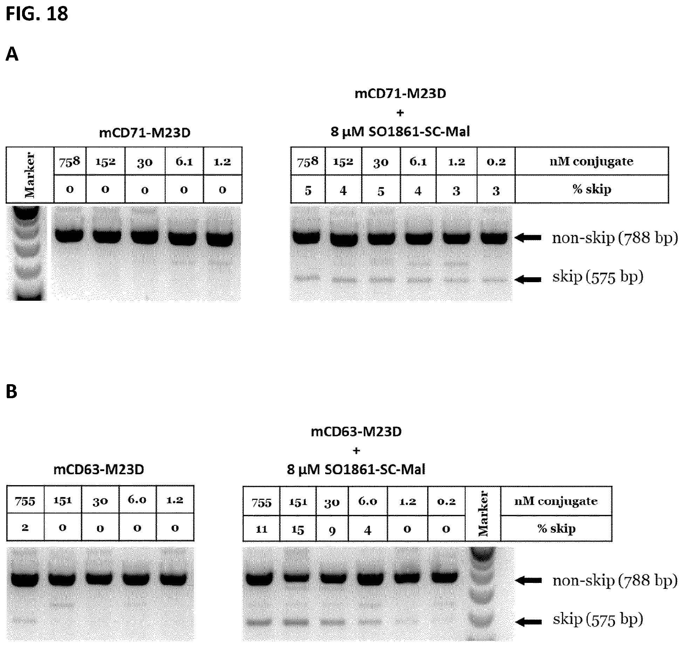

**Figure 18****:** Exon 23 skip analysis of (A) mCD71-M23D without (left panel) or with (right panel) co-administration of SO1861-SC-Mal, and (B) mCD63-M23D without (left panel) or with (right panel) co-administration of SO1861-SC-Mal in differentiated murine C2C12 myotubes.

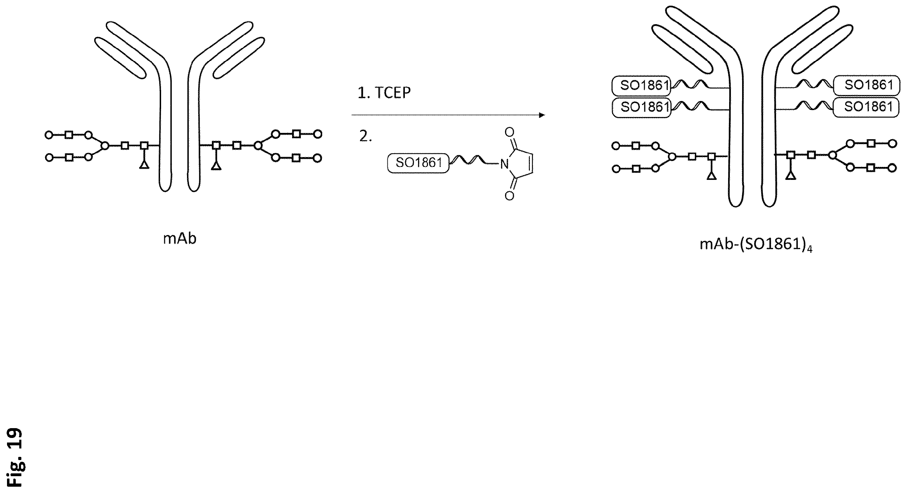

**Figure 19****:** Schematic representation of the conjugation procedure for mAb-SO1861, such as mCD63-SC-SO1861. Conjugation between a mAb and SO1861 on the reduced interchain disulfide bonds, yielding mAb-(SO1861)₄. NB: for clarity of schematic representation, mAb-SO1861 is shown with DAR 4.

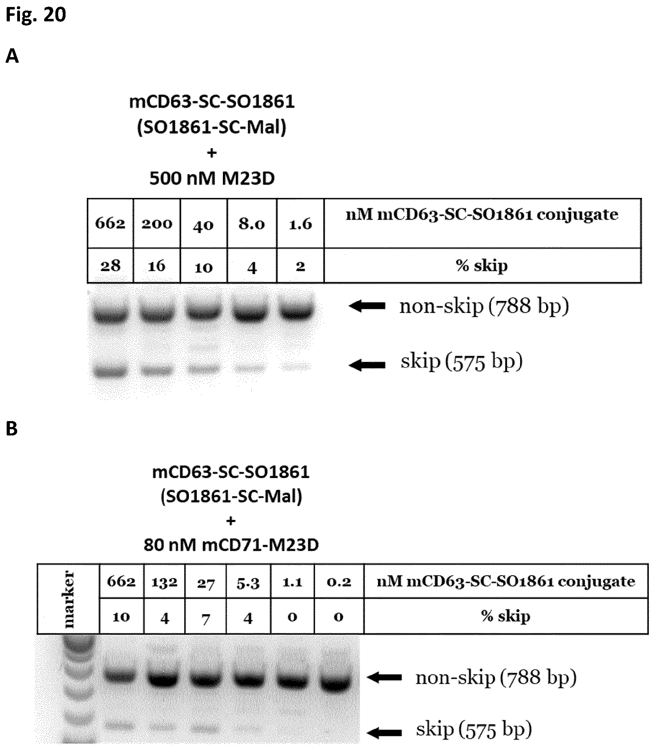

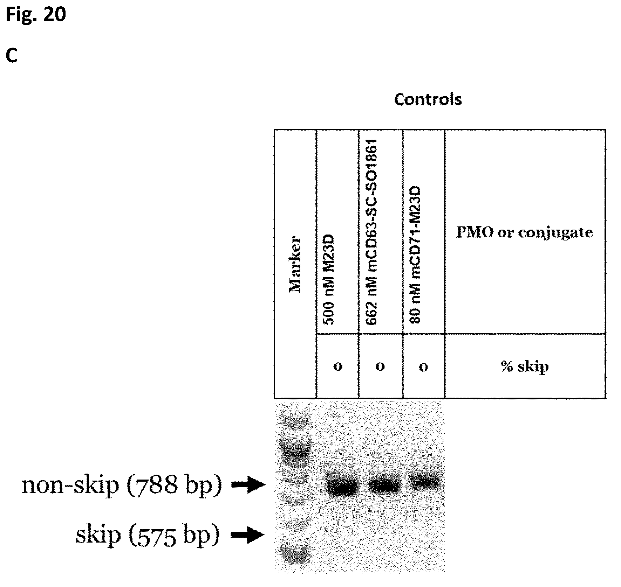

**Figure 20****:** Exon 23 skip analysis of (A) mCD63-SC-SO1861 + M23D and (B) mCD63-SC-SO1861 + mCD71-M23D in differentiated murine C2C12 myotubes. (C) Exon skip using M23D, mCD63-SC-SO1861, or mCD71-M23D, alone, as a control, in differentiated murine C2C12 myotubes.

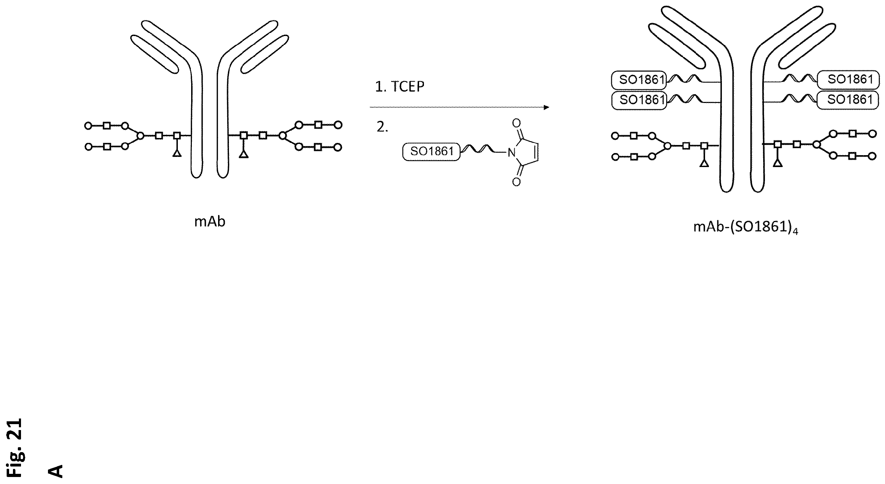

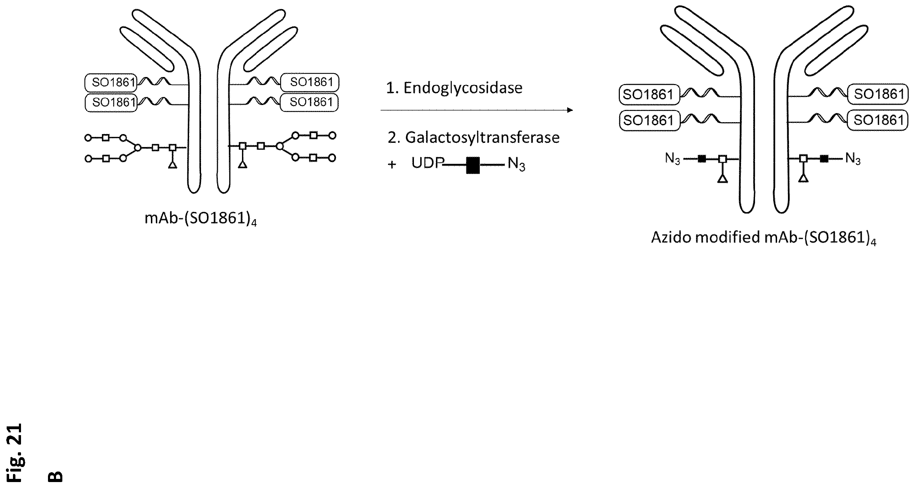

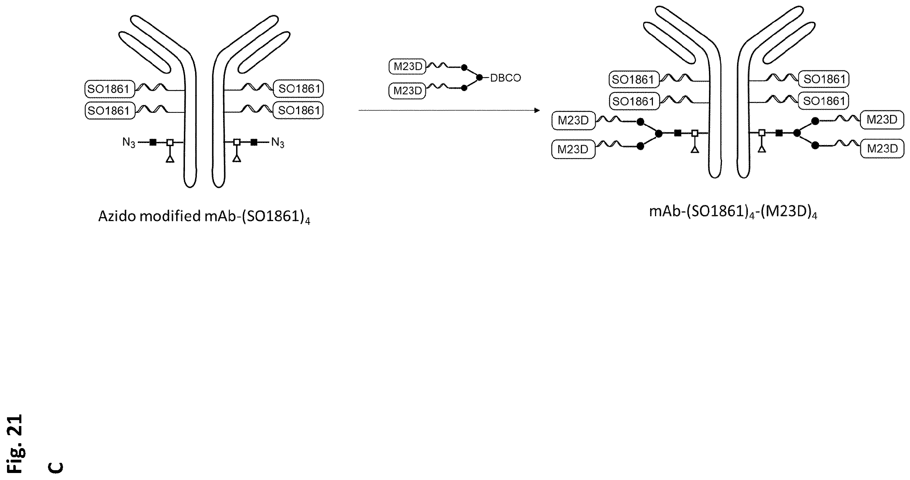

**Figure 21****:** Schematic representation of the conjugation procedure for mAb-M23D-SO1861, such as mCD63-M23D-SC-SO1861. (A) Conjugation between a mAb and SO1861 on the reduced interchain disulfide dibonds, yielding mAb-(SO1861)₄, followed by (B) preparation of a trimmed and azido modified mAb-(SO1861)₄ glycan and (C) conjugation, via strain-promoted azide-alkyne click reaction, between the trimmed mAb-(SO1861)₄ glycan and DBCO-(M23D)₂, yielding mAb-(SO1861)₄-(M23D)₄. NB: for clarity of schematic representation, mAb-M23D-SO1861 is shown with DAR 4/4. For explanation of symbolically represented glycan residues or symbolically represented molecules see Figure 17C and D, respectively.

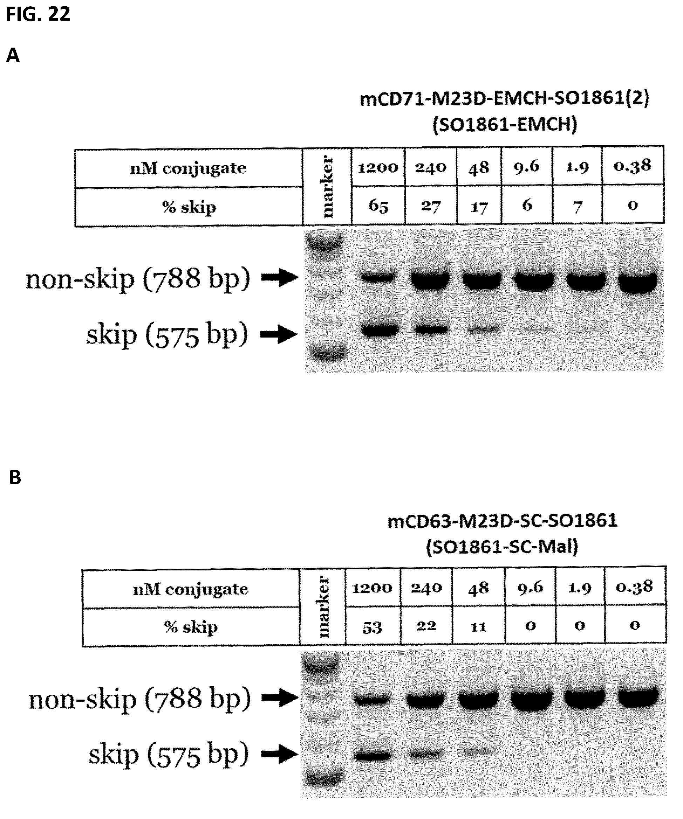

**Figure 22****:** Assessment of exon 23 skip using (A) mCD71-M23D-EMCH-SO1861(2) or (B) mCD63-M23D-SC-SO1861 in differentiated murine C2C12 myotubes.

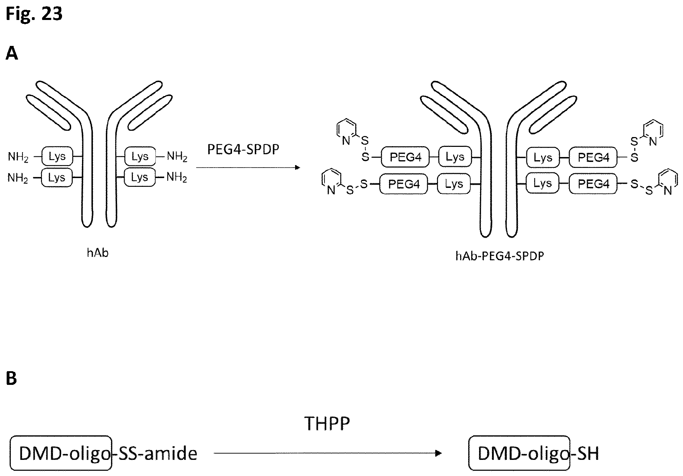

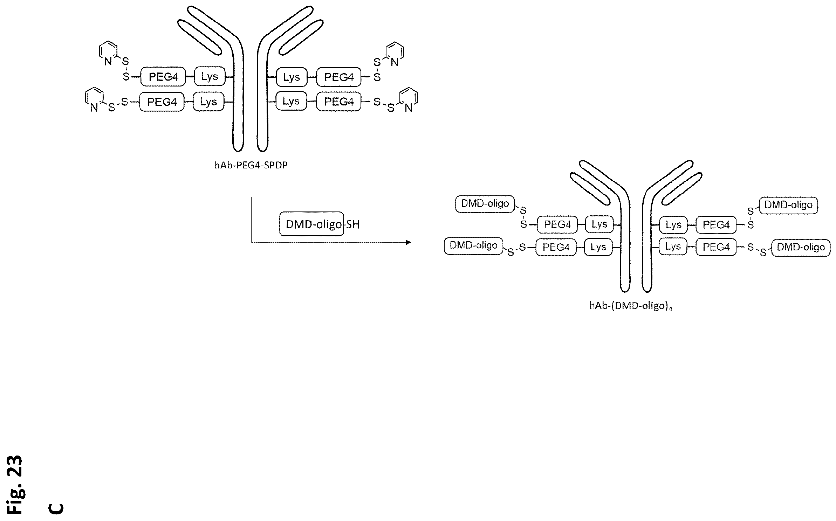

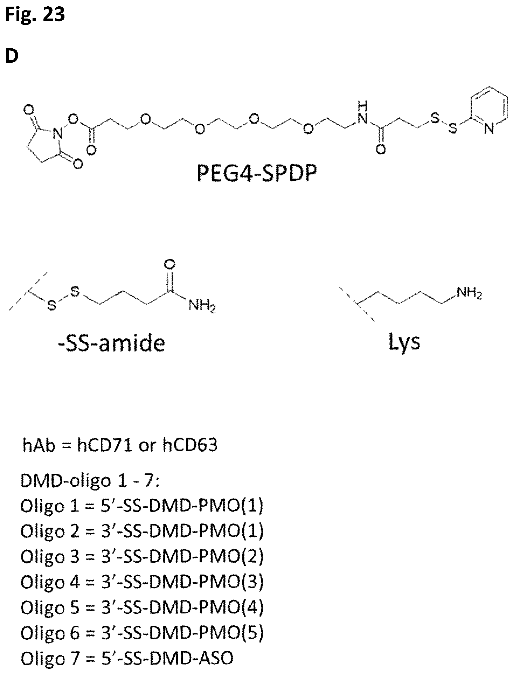

**Figure 23****:** (A-C) Schematic representation of the conjugation procedure for hAb-DMD-oligo, such as hCD71-5'-SS-DMD-ASO, hCD71-5'-SS-DMD-PMO(1), hCD71-3'-SS-DMD-PMO(1-5), and hCD63-5'-SS-DMD-ASO, involving (A) hAb functionalization with PEG4-SPDP at activated lysine (Lys) residues, (B) activation of the protected DMD-oligo, (C) disulfide bond formation between the activated DMD-oligo-SH and hAb-PEG4-SPDP, and (D) figure legend. NB: for clarity of schematic representation, hAb-DMD-oligo is shown with DAR 4.

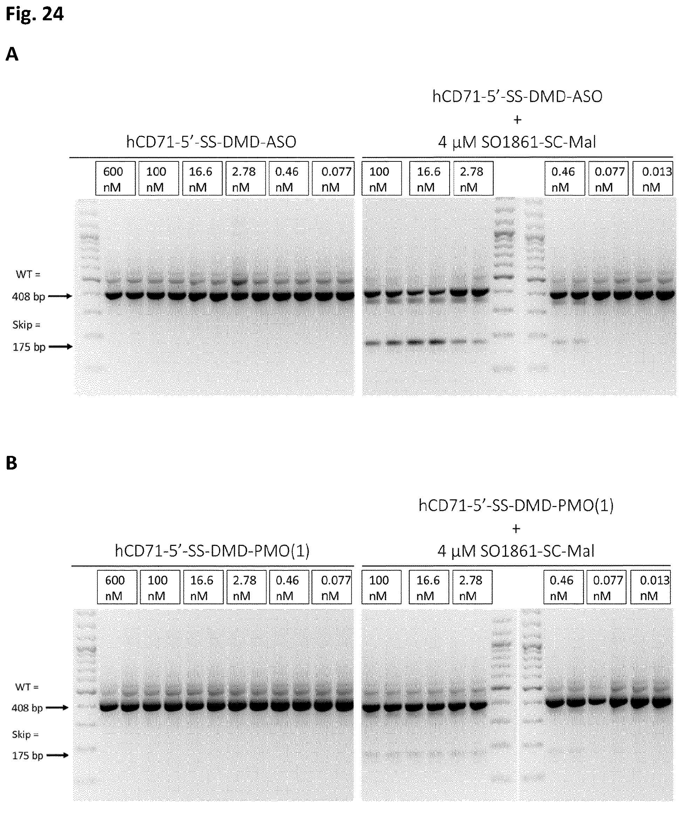

**Figure 24****:** Exon 51 skip analysis of (A) hCD71-5'-SS-DMD-ASO (DAR2.1) without (left panel) or with (right panel) co-administration of SO1861-SC-Mal, and (B) hCD71-5'-SS-DMD-PMO(1) (DAR2.2) without (left panel) or with (right panel) co-administration of SO1861-SC-Mal, in differentiated human myotubes from a non-DMD (healthy) donor (KM155).

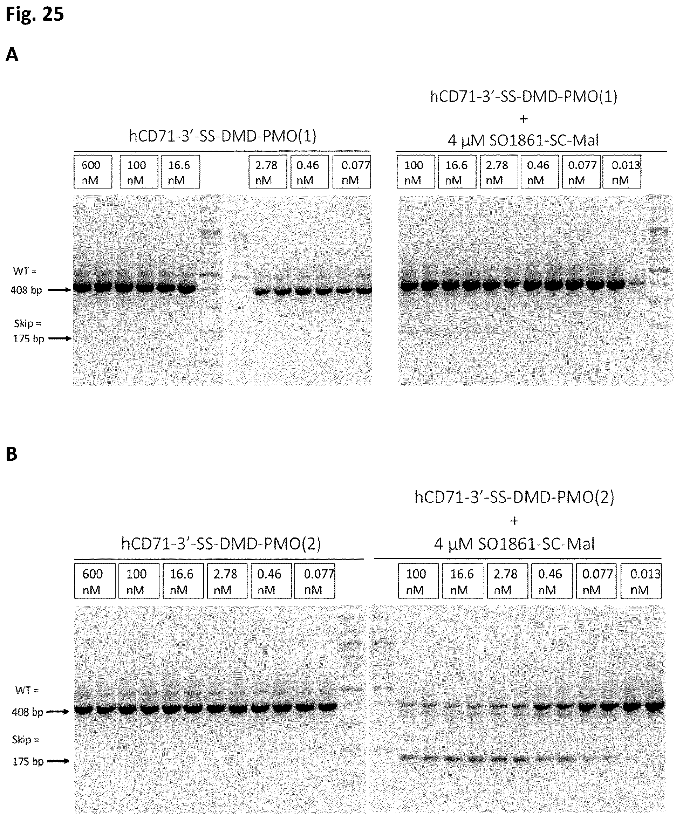

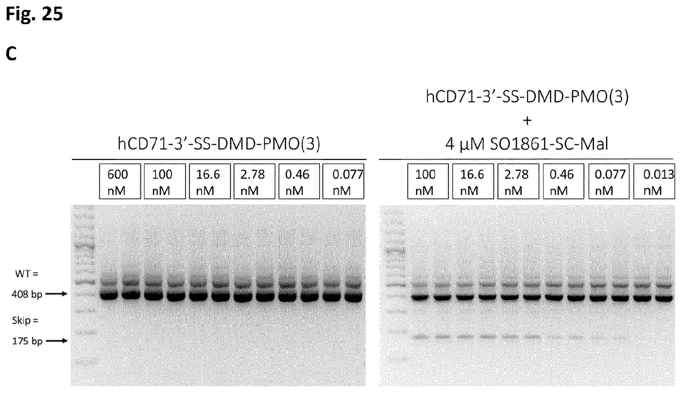

**Figure 25****:** Exon 51 skip analysis of (A) hCD71-3'-SS-DMD-PMO(1) (DAR2.1) without (left panel) or with (right panel) co-administration of SO1861-SC-Mal, (B) hCD71-3'-SS-DMD-PMO(2) (DAR3.0) without (left panel) or with (right panel) co-administration of SO1861-SC-Mal, and (C) hCD71-3'-SS-DMD-PMO(3) (DAR2.6) without (left panel) or with (right panel) co-administration of SO1861-SC-Mal, in differentiated human myotubes from a non-DMD (healthy) donor (KM155).

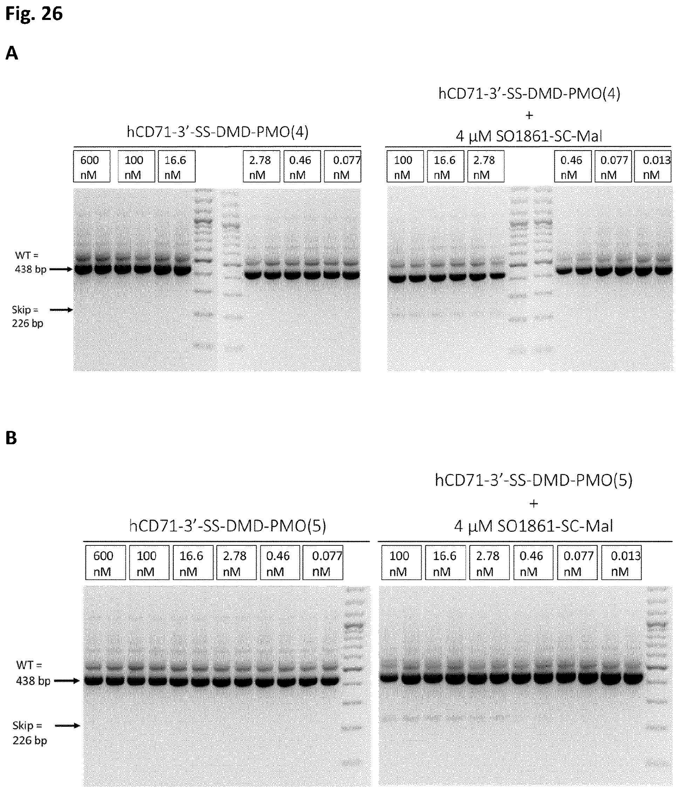

**Figure 26****:** Exon 53 skip analysis of (A) hCD71-3'-SS-DMD-PMO(4) (DAR2.3) without (left panel) or with (right panel) co-administration of SO1861-SC-Mal, and (B) hCD71-3'-SS-DMD-PMO(5) (DAR2.1) without (left panel) or with (right panel) co-administration of SO1861-SC-Mal, in differentiated human myotubes from a non-DMD (healthy) donor (KM155).

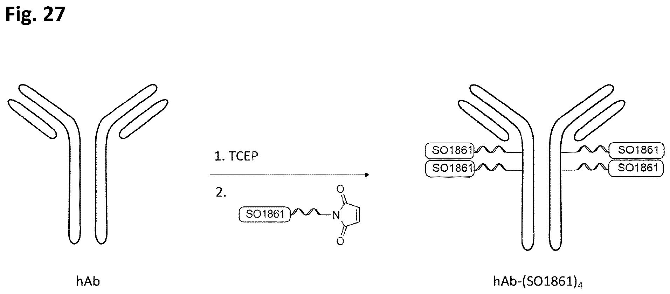

**Figure 27****:** Schematic representation of the conjugation procedure for hAb-SO1861, such as hCD71-SC-SO1861 and hCD63-SC-SO1861. Conjugation between a hAb and SO1861 on the interchain disulfide bonds, yielding hAb-(SO1861)₄. NB: for clarity of schematic representation, hAb-SO1861 is shown with DAR 4.

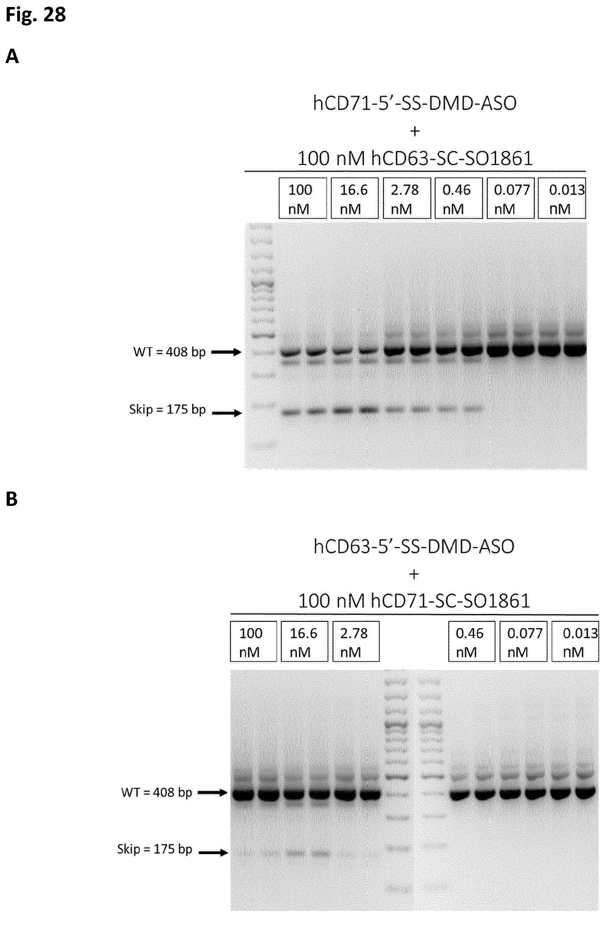

**Figure 28****:** Exon 51 skip analysis of (A) hCD71-5'-SS-DMD-ASO (DAR2.1) with co-administration of hCD63-SC-SO1861 (DAR4.8), and (B) hCD63-5'-SS-DMD-ASO (DAR2.3) with co-administration of hCD71-SC-SO1861 (DAR4.0), in differentiated human myotubes from a non-DMD (healthy) donor (KM155).

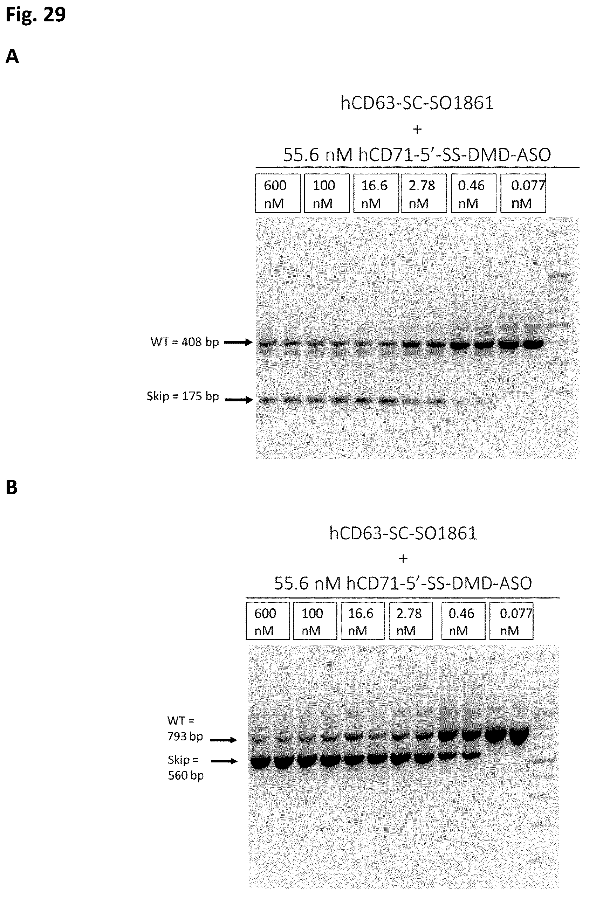

**Figure 29****:** Exon 51 skip analysis of hCD63-SC-SO1861 (DAR4.8) with co-administration of hCD71-5'-SS-DMD-ASO (DAR2.1) in (A) differentiated human myotubes from a non-DMD (healthy) donor (KM155), and (B) differentiated human myotubes from a DMD-affected donor (KM1328).

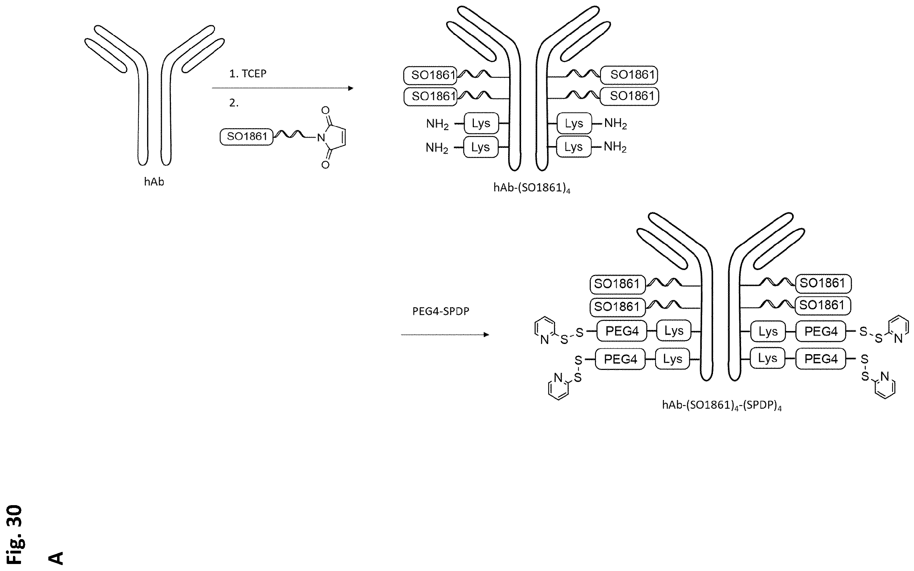

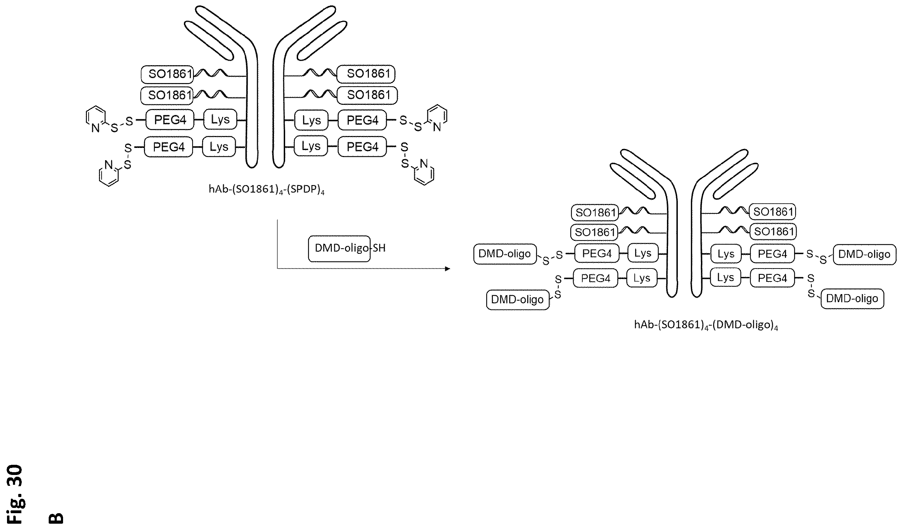

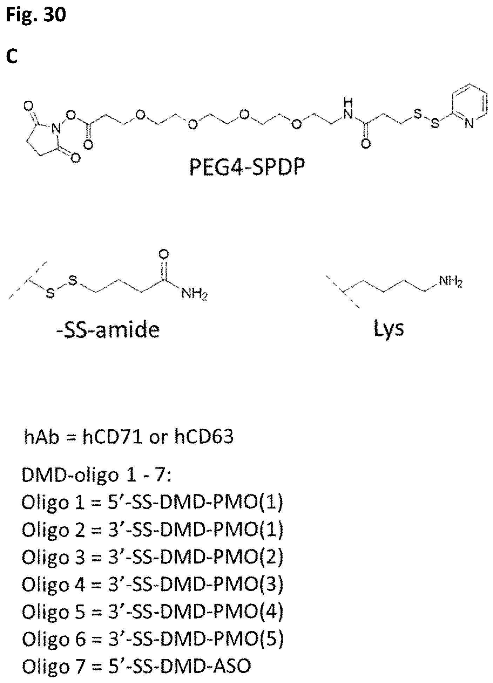

**Figure 30****:** (A-C) Schematic representation of the conjugation procedure for hAb-DMD-oligo-SO1861, such as hCD71-5'-SS-DMD-ASO-SC-SO1861, hCD63-5'-SS-DMD-ASO-SC-SO1861, hCD71-3'-SS-DMD-PMO(1-5)-SC-SO1861 and hCD63-3'-SS-DMD-PMO(1-4)-SC-SO1861. Conjugation between (A) a hAb and SO1861 on the interchain disulfide bonds, yielding hAb-(SO1861)₄, followed by hAb functionalization with PEG4-SPDP at activated lysine (Lys) residues, and (B) disulfide bond formation between the activated DMD-oligo-SH and hAb-(SO1861)₄-(SPDP)₄ to yield hAb-(SO1861)₄-(DMD-oligo)₄. (C) Figure legend. NB: for clarity of schematic representation, hAb-DMD-oligo-SO1861 is shown with DAR 4/4.

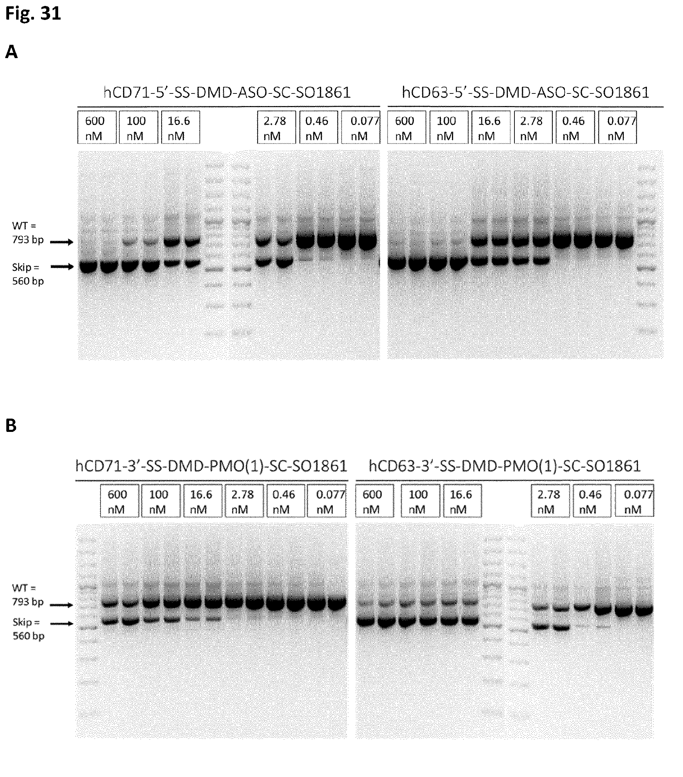

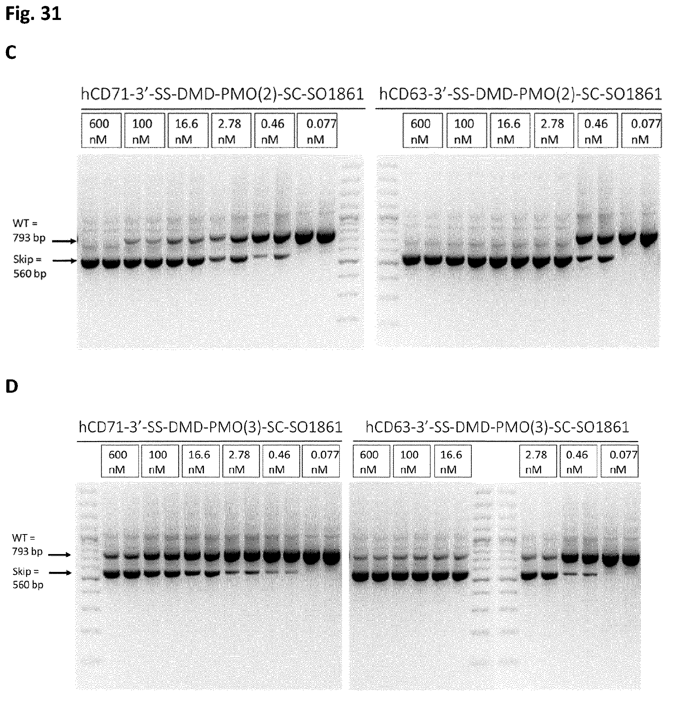

**Figure 31****:** Exon 51 skip analysis of (A) hCD71-5'-SS-DMD-ASO-SC-SO1861 (DAR2.3/4.0) (left panel) and hCD63-5'SS-DMD-ASO-SC-SO1861 (DAR2.2/4.8) (right panel), (B) hCD71-3'-SS-DMD-PMO(1)-SC-SO1861 (DAR2.4/4.0) (left panel) and hCD63-3'-SS-DMD-PMO(1)-SC-SO1861 (DAR2.7/4.8) (right panel), (C) hCD71-3'-SS-DMD-PMO(2)-SC-SO1861 (DAR3.1/4.0) (left panel) and hCD63-3'-SS-DMD-PMO(2)-SC-SO1861 (DAR2.7/4.8) (right panel), and (D) hCD71-3'-SS-DMD-PMO(3)-SC-SO1861 (DAR2.5/4.0) (left panel) and hCD63-3'-SS-DMD-PMO(3)-SC-SO1861 (DAR2.8/4.8) (right panel), in differentiated human myotubes from a DMD-affected donor (KM1328).

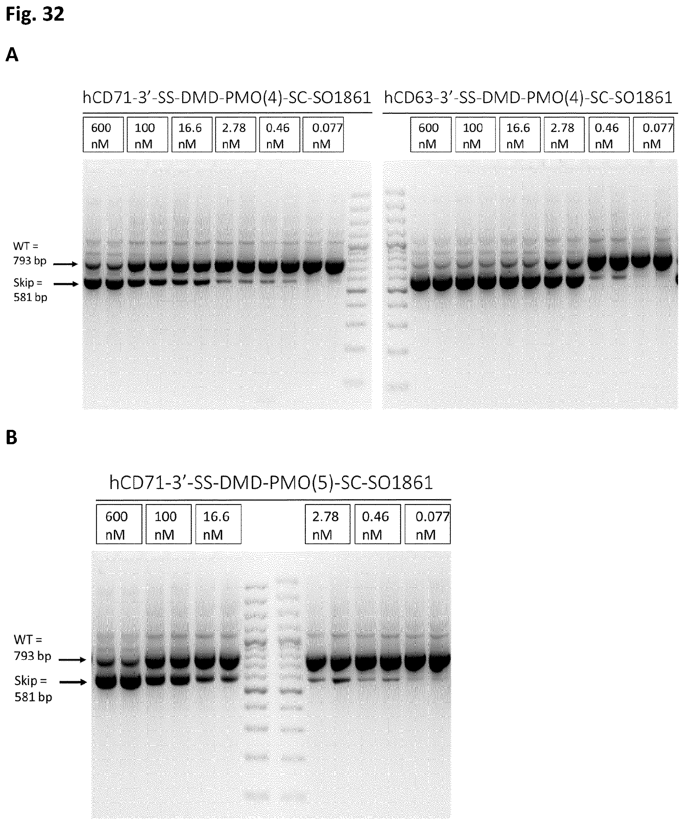

**Figure 32****:** Exon 53 skip analysis of (A) hCD71-3'-SS-DMD-PMO(4)-SC-SO1861 (DAR2.0/4.0) (left panel) and hCD63-3'-SS-DMD-PMO(4)-SC-SO1861 (DAR2.4/4.8) (right panel), and (B) hCD71-3'-SS-DMD-PMO(5)-SC-SO1861 (DAR2.0/4.0), in differentiated human myotubes from a DMD-affected donor (KM1328).

### DETAILED DESCRIPTION

Disclosed herein are improved biologically active pharmaceutical compositions comprising covalently-linked conjugates of therapeutic nucleic acids, muscle cell-surface endocytic receptor-targeting ligands and endocytic-escape enhancing saponins. The disclosed herein conjugates possess the particular advantage of exhibiting the highly desired property of enhanced and effective delivery of therapeutic nucleic acids, such as antisense oligonucleotides, into differentiated muscles cells, striated muscle cells in particular, notably including heart muscle cells.

The innovative concepts presented herein will be described with respect to particular embodiments or aspects of the disclosure, that should be regarded as descriptive and not limiting beyond of what is described in the claims. The particular aspects as described herein can operate in combination and cooperation, unless specified otherwise. While the invention has been described with reference to these embodiments, it is contemplated that alternatives, modifications, permutations and equivalents thereof will become apparent to one having ordinary skill in the art upon reading the specification and upon study of the drawings and graphs. The invention is not limited in any way to the illustrated embodiments. Changes can be made without departing from the scope which is defined by the appended claims.

It is one of several objectives of embodiments of present disclosure is to provide a solution to the problem of non-specificity, encountered when administering nucleic acid-based therapeutics to human patients suffering from a muscle-wasting disorder and in need of such therapeutics. It a further one of several objectives of the embodiments to provide a solution to the problem of insufficient safety characteristics of current nucleic-acid-based drugs, when administered to human patients in need thereof, in particular at side-effect inducing excessive doses. It is yet a further one of several objectives of embodiments of the current invention to provide a solution to the problem of current nucleic acid-based therapies being less efficacious than desired, when administered to human patients in need thereof, due to not being sufficiently capable to reach and/or enter into to the diseased muscle cell with little to no off-target activity on non-diseased cells, when administered to human patients in need thereof.

Without wishing to be bound by any theory, the disclosed herein novel pharmaceutical compositions were conceived based on the observation that a specific group of triterpenoid 12,13-dehydrooleanane-type saponins appears to exhibit potent endosomal-escape enhancing properties for nucleic acid based therapeutics that are targeted into muscle cells by endocytic-receptor mediated endocytosis.

Endocytic pathways are complex and not fully understood. Nowadays, it is hypothesized that they involve stable compartments connected by vesicular traffic. A compartment is a complex, multifunctional membrane organelle that is specialized for a particular set of essential functions for the cell. Vesicles are considered to be transient organelles, simpler in composition, and are defined as membrane-enclosed containers that form *de novo* by budding from a pre-existing compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles.

An endocytic vesicle, which arises at the plasma membrane, most prominently from clathrin-coated pits, first fuses with the early endosome, which is a major sorting compartment of approximately pH 6.5. A large part of the internalized cargo and membranes are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of the endocytosed molecules occurs inside the endolysosomes.

*Endosomal escape* is the active or passive release of a substance from the inner lumen of any kind of compartment or vesicle from the endocytic pathway, preferably from clathrin-mediated endocytosis, or recycling pathway into the cytosol. Endosomal escape thus includes but is not limited to release from endosomes, endolysosomes or lysosomes, including their intermediate and hybrid organelles. After entering the cytosol, said substance might move to other cell units such as the nucleus.

As will be demonstrated be the presented herein data, not only did the inclusion of triterpenoid 12,13-dehydrooleanane-type saponin in the therapeutic compositions and conjugates of the disclosure appeared to stimulate efficient exiting of the therapeutic nucleic acids' from the muscle cells' endosomes into the appropriate muscle cell inner compartments, but also, and surprisingly, it also did not interfere with endothelial cell transcytosis *in vivo* from blood to the external environment of the muscle cells, thus allowing efficient *in vivo* delivery of the saponin-containing therapeutic compositions and conjugates of the disclosure to the muscle cells following intravenous delivery.

In line with these promising observations and findings, in a first general aspect, the invention provides a pharmaceutical composition for use in the treatment or prophylaxis of a muscle wasting disorder, the composition comprising a covalently-linked conjugate, the conjugate comprising
a saponin,
a nucleic acid, and
a ligand of an endocytic receptor on a muscle cell,
wherein the saponin is a triterpenoid 12,13-dehydrooleanane-type saponin.

As used herein, the term "covalently-linked conjugate" in the context of the covalently-linked conjugate comprising the saponin, the nucleic acid, and ligand of an endocytic receptor on a muscle cell, is to be construed as referring to a conjugate wherein the saponin, the nucleic acid, and the ligand are covalently bound together.

As used herein, it will be understood from the context that the nucleic acids forming part of the disclosed herein compositions for the therapeutic purposes are selected such to possess a therapeutic activity for treating or performing prophylaxis of a selected muscle wasting disorder. That is to say, a nucleic acid as comprised in a conjugate as disclosed herein will be a therapeutic nucleic acid for one disorder, whereas for another disorder it may not cause any benefit. A skilled person aiming to perform a specific treatment of a selected disorder will know how to perform selection of a promising therapeutic nucleic acid and will be able to decide, based by either their knowledge of mutations causing such disorders or based on genetic mutation screening results of a given patient, which therapeutic nucleic acid should be comprised in the novel conjugates as disclosed herein, for performing improved treatment.

In preferred embodiments, composition for the disclosed herein therapeutic or prophylactic use are provided, wherein the muscle wasting disorder is a muscle cell-related genetic disorder, preferably being a congenital myopathy or a muscular dystrophy; preferably wherein the congenital myopathy is selected from nemaline myopathy or congenital fiber-type disproportion myopathy, and/or wherein the muscular dystrophy is selected from a dystrophinopathy, facioscapulohumeral muscular dystrophy, myotonic dystrophy, Emery-Dreifuss muscular dystrophy, limb-girdle muscular dystrophy 1B, congenital muscular dystrophy; or dilated familial cardiomyopathy; most preferably wherein the muscle wasting disorder is a muscle cell-related genetic disorder being a dystrophinopathy, preferably being Duchenne muscular dystrophy.

In a particularly advantageous embodiments, composition for the disclosed herein therapeutic or prophylactic use is provided, wherein the treatment or prophylaxis of the muscle wasting disorder involves antisense therapy, preferably involving exon skipping.

It has interestingly been observed that advantageous nucleic acid delivery characteristics can be correlated with the number of the nucleic acid and saponin molecules that are conjugated together per one (1) molecule of the endocytic receptor-targeting ligand present in the conjugate.

In line with the above observation, in an advantageous embodiment, a composition for the disclosed herein therapeutic or prophylactic use is provided, wherein the conjugate comprises 1 - 16 molecules of the saponin and 1 - 5 molecules of the nucleic acid per 1 molecule of the ligand.

In another advantageous embodiment, further disclosed herein is a covalently linked conjugate for delivery of a therapeutic nucleic acid into a muscle cell, the conjugate comprising
a saponin,
a nucleic acid, and
a ligand of an endocytic receptor on a muscle cell,

wherein the saponin is a triterpenoid 12,13-dehydrooleanane-type saponin, and
wherein the conjugate comprises 1 - 16 molecules of the saponin and 1 - 5 molecules of the nucleic acid per 1 molecule of the ligand.

In a related aspect, a composition for the disclosed herein therapeutic or prophylactic use or a conjugate according to the disclosure is provided, wherein the conjugate comprises 2 - 8 molecules of the saponin per 1 molecule of the ligand;
preferably 3 - 6 molecules of the saponin per 1 molecule of the ligand;
more preferably 4 - 5 molecules of the saponin per 1 molecule of the ligand;
most preferably wherein the conjugate comprises on average 4-4.5 molecules of the saponin per 1 molecule of the ligand.

In a further related aspect, a composition for the disclosed herein therapeutic or prophylactic use or a conjugate according to the disclosure is provided, wherein the conjugate comprises 2 - 5 molecules of the nucleic acid per 1 molecule of the ligand;
preferably 3 - 4 molecules of the nucleic acid per 1 molecule of the ligand;
more preferably wherein the conjugate comprises on average 4 molecules of the nucleic acid per 1 molecule of the ligand.

Typically, the saponins suitable for application in the disclosed herein conjugates are saponins that display endosomal escape enhancing activity. As can be seen from Table 1, such saponins have a triterpene 12,13-dehydrooleanane-type backbone wherein the basic structure of the triterpene backbone is a pentacyclic C30 terpene skeleton (also referred to as sapogenin or aglycone).

| **TABLE 1. Saponins displaying (late) endosomal/lysosomal escape enhancing activity, and with a aglycone core of the 12,13-dehydrooleanane type⁴⁾** | | | | |
|---|---|---|---|---|
| **Saponin Name** | **Aglycone core with an aldehyde group at the C-23 position** | | **Carbohydrate substituent at the C-3beta-OH group** | **Carbohydrate substituent at the C-28-OH group** |
| NP-017777 | Gypsogenin | | Gal-(1→2)-[Xyl-(1→3)]-GlcA- | Xyl-(1→4)-Rha-(1→2)-[R-(→4)]-Fuc- (R = 4E-Methoxycinnamic acid) |
| NP-017778 | Gypsogenin | | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | | Gal-(1→2)-[Xyl-(1→3)]-GlcA- | Xyl-(1→4)-[Gal-(1→3)]-Rha-(1→2)-4-OAc-Fuc- |
| NP-018110^{c}, NP-017772^{d} | Gypsogenin | | Gal-(1→2)-[Xyl-(1→3)]-GlcA- | Xyl-(1→4)-[Glc-(1→3)]-Rha-(1→2)-3,4-di-OAc-Fuc- |
| NP-018109 | Gypsogenin | | Gal-(1→2)-[Xyl-(1→3)]-GlcA- | Xyl-(1→4)-[Glc-(1→3)]-Rha-(1→2)-[R-(→4)]-3-OAc-Fuc- (R = 4E-Methoxycinnamic acid) |
| NP-017888 | Gypsogenin | | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1→4)]-Rha/Fuc- |
| SA1641^{a}, AE X55^{b} | Gypsogenin | | Gal-(1→2)-[Xyl-(1→3)]-GlcA- | Xyl-(1→3)-Xyl-(1→4)-Rha-(1→2)-[Qui-(1→4)]-Fuc- |
| SO1658 | Gypsogenin | | Gal-(1→2)-[Xyl-(1→3)]-GlcA- | Glc-(1→3)-[Xyl-(1→3)-Xyl-(1→4)]-Rha-(1→2)-Fuc- |
| gypsoside A⁶⁾ | Gypsogenin | | Gal-(1→4)-Glc (1→4)-[Ara-(1→3)]-GlcA- | Xyl-(1→3)-Fuc-(1→4)-[Xyl-(1→3)-Xyl-(1→3)]-Rha- |
| phytolaccagenin | Gypsogenin | | Absent | absent |
| | | | | |
| Gypsophila saponin 1 (Gyp1) | Quillaic acid | | Gal-(1→2)-[Xyl-(1→3)]-GlcA- | Glc-(1→3)-[Xyl-(1→4)]-Rha-(1→2)-Fuc- |
| NP-017674 | Quillaic acid | | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | | Gal-(1→2)-[Xyl-(1→3)]-GlcA- | Xyl-(1→4)-[Gal-(1→3)]-Rha-(1→2)-Fuc- |
| AG1 | Quillaic acid | | Gal-(1→2)-[Xyl-(1→3)]-GlcA- | Xyl-(1→4)-[Glc-(1→3)]-Rha-(1→2)-Fuc- |
| NP-003881 | Quillaic acid | | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | | Gal-(1→2)-[Xyl-(1→3)]-GlcA- | Api-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017677 | Quillaic acid | | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017706 | Quillaic acid | | Gal-(1→2)-[Xyl-(1→3)]-GlcA- | Api-(1→3)-Xyl-(1→4)-Rha-(1→2)-[Rha-(1→3)]-4-OAc-Fuc- |
| NP-017705 | Quillaic acid | | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | | Gal-(1→2)-[Xyl-(1→3)]-GlcA- | Glc-(1→3)-Xyl-(1→4)-Rha-(1→2)-[3-OAc--Rha-(1→3)]-Fuc- |
| SA1657 | Quillaic acid | | Gal-(1→2)-[Xyl-(1→3)]-GlcA- | Xyl-(1→3)-Xyl-(1→4)-Rha-(1→2)-[Qui-(1→4)]-Fuc- |
| AG2 | Quillaic acid | | Gal-(1→2)-[Xyl-(1→3)]-GlcA- | Glc-(1→3)-[Xyl-(1→4)]-Rha-(1→2)-[Qui-(1→4)]-Fuc- |
| GE1741 | Quillaic acid | | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | | Gal-(1→2)-[Xyl-(1→3)]-GlcA- | Glc-(1→3)-[Xyl-(1→4)]-Rha-(1→2)-Fuc- |
| SO1584 | Quillaic acid | | Gal-(1→2)-[Xyl-(1→3)]-GlcA- | 6-OAc-Glc-(1→3)-[Xyl-(1→4)]-Rha-(1→2)-Fuc- |
| SO1674 | Quillaic acid | | Gal-(1→2)-[Xyl-(1→3)]-GlcA- | Glc-(1→3)-[Xyl-(1→3)-Xyl-(1→4)]-Rha-(1→2)-Fuc- |
| SO1700³⁾ | Quillaic acid | | Gal-(1→2)-[Xyl-(1→3)]-GlcA- | Xyl-(1→4)-Rha-(1→2)-[Xyl-(1→3)-4-OAc-Qui-(1→4)]-Fuc- |
| Saponarioside B¹⁾ | Quillaic acid | | Gal-(1→2)-[Xyl-(1→3)]-GlcA- | Xyl-(1→3)-Xyl-(1→4)-Rha-(1→2)-[4-OAc-Qui-(1→4)]-Fuc- |
| SO1730³⁾ | Quillaic acid | | Gal-(1→2)-[Xyl-(1→3)]-GlcA- | Glc-(1→3)-Xyl-(1→4)-Rha-(1→2)-[-4-OAc-Qui-(1→4)]-Fuc- |
| SO1772³⁾ | Quillaic acid | | Gal-(1→2)-[Xyl-(1→3)]-GlcA- | 6-OAc-Glc-(1→3)-[Xyl-(1→4)]-Rha-(1→2)-[4-OAc-Qui-(1→4)]-Fuc-- |
| SO1832¹⁾ (protonated SO1831) = Saponarioside A | Quillaic acid | | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| SO1861 (deprotonated SO1862) | Quillaic acid | | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| SO1862 (protonated SO1861), also referred to as *Sapofectosid⁵⁾* | Quillaic acid | | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| SO1904³⁾ | Quillaic acid | | Gal-(1→2)-[Xyl-(1→3)]-GlcA- | 6-OAc-Glc-(1→3)-[Xyl-(1→4)]-Rha-(1→2)-[Xyl-(1→3)-4-OAc-Qui-(1→4)]-Fuc- |
| QS-7 (also referred to as QS1861) | Quillaic acid | | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-apio | Quillaic acid | | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | | Gal-(1→2)-[Xyl-(1→3)]-GlcA- | Xyl-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | | Gal-(1→2)-[Xyl-(1→3)]-GlcA- | Api-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 B-xylo | Quillaic acid | | Gal-(1→2)-[Xyl-(1→3)]-GlcA- | Xyl-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 | Quillaic acid | | Gal-(1→2)-[Xyl-(1→3)]-GlcA- | Combination of the carbohydrate chains depicted for QS-21 A-apio, A-xylo, B-apio, B-xylo, for this position at the aglycone (see also the structure depicted as (Scheme Q)) |
| Agrostemmoside E (AG1856, AG2.8)²⁾ | Quillaic acid | | Gal-(1→2)-[Xyl-(1→3)]-GlcA- | [4,6-di-OAc-Glc-(1→3)]-[Xyl-(1→4)]-Rha-(1→2)-[3,4-di-OAc-Qui-(1→4)]-Fuc- |
| | | | | |

| **Saponin Name** | **Aglycone core without an aldehyde group at the C-23 position** | | **Carbohydrate substituent at the C-3beta-OH group** | **Carbohydrate substituent at the C-28-OH group** |
|---|---|---|---|---|
| NP-005236 | 2alpha-Hydroxyoleanolic acid | | GlcA- | Glc/Gal- |
| AMA-1 | 16alpha-Hydroxyoleanolic acid | | Glc- | Rha-(1→2)-[Xyl-(1→4)]-Rha- |
| AMR | 16alpha-Hydroxyoleanolic acid | | Glc- | Rha-(1→2)-[Ara-(1→3)-Xyl-(1→4)]-Rha- |
| alpha-Hederin | Hederagenin (23-Hydroxyoleanolic acid) | | Rha-(1→2)-Ara- | Not present |
| NP-012672 | 16alpha,23-Dihydroxyoleanolic acid | Ara/Xyl-(1→4)-Rha/Fuc-(1→2)-Glc/Gal-(1→2)-Rha/Fuc-(1→2)-GlcA- | Ara/Xyl- | |
| beta-Aescin (described: Aescin la) | Protoaescigenin-21 (2-methylbut-2-enoate)-22-acetat | Glc-(1→2)-[Glc-(1→4)]-GlcA- | Not present | |
| Aescinate | Aglycone core without an aldehyde group at the C-23 position | present | Not present | |
| dipsacoside B | Aglycone core without an aldehyde group at the C-23 position | present | present | |
| esculentoside A | Aglycone core without an aldehyde group at the C-23 position | present | Not present | |
| Teaseed saponin I | 23-Oxo-barringtogenol C - 21,22-bis(2-methylbut-2-enoate) | Glc-(1→2)-Ara-(1→3)-[Gal-(1→2)]-GlcA- | Not present | |
| Teaseedsaponin J | 23-Oxo-barringtogenol C - 21,22-bis(2-methylbut-2-enoate) | Xyl-(1→2)-Ara-(1→3)-[Gal-(1→2)]-GlcA- | Not present | |
| Assamsaponin F | 23-Oxo-barringtogenol C - 21 (2-methylbut-2-enoate)-16,22-diacetat | Glc-(1→2)-Ara-(1→3)-[Gal-(1→2)]-GlcA- | Not present | |
| Primula acid 1 | 3,16,28-Trihydroxyoleanan-12-en | Rha-(1→2)-Gal-(1→3)-[Glc-(1→2)]-GlcA- | Not present | |
| AS64R | Gypsogenic acid | Absent | Glc-(1→3)-[Glc-(1→6)]-Gal- | |
| Macranthoidin A | Aglycone core without an aldehyde group at the C-23 position | present | present | |
| saikosaponin A | Aglycone core without an aldehyde group at the C-23 position | present | absent | |
| saikosaponin D | | Aglycone core without an aldehyde group at the C-23 position | present | absent |
| | | | | |
| | | | **Carbohydrate substituent at the C-23-OH group** | |
| AS6.2 | | Gypsogenic acid | Gal- | Glc-(1→3)-[Glc-(1→6)]-Gal- |
| a, b: Different names refer to different isolates of the same structure | | | | |
| c, d: Different names refer to different isolates of the same structure | | | | |
| 1) Jia et al., Major Triterpenoid Saponins from Saponaria officinalis, J. Nat. Prod. 1998, 61, 11, 1368-1373, Publication Date: September 19, 1998, https://doi.org/10.1021/np980167u | | | | |
| 2) The structure of Agrostemmoside E (also referred to as AG1856 or AG2.8) is given in Fig. 4 of J. Clochard et al, A new acetylated triterpene saponin from Agrostemma githago L. modulates gene delivery efficiently and shows a high cellular tolerance, International Journal of Pharmaceutics, Volume 589, 15 November 2020, 119822. | | | | |
| 3) Structures of SO1700, SO1730, SO1772, SO1904 are given in Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224). | | | | |
| 4) See for example: | | | | |
| | - thesis by Dr Stefan Böttger (2013): Untersuchungen zur synergistischen Zytotoxizität zwischen Saponinen und Ribosomen inaktivierenden Proteinen Typ I, and | | | |
| | - Sama et al., Structure-Activity Relationship of Transfection-Modulating Saponins - A Pursuit for the Optimal Gene Trafficker, Planta Med. Volume 85, pp. 513-518, 2019 (doi:10.1055/a-0863-4795) and | | | |
| | - Fuchs et al., Glycosylated Triterpenoids as Endosomal Escape Enhancers in Targeted Tumor Therapies, Biomedicine, Volume 5, issue 14, 2017 (doi:10.3390/biomedicines5020014). | | | |
| 5) Sama et al., Sapofectosid - Ensuring non-toxic and effective DNA and RNA delivery, International Journal of Pharmaceutics, Volume 534, Issues 1-2, 20 December 2017, Pages 195-205 (dx.doi.org/10.1016/j.ijpharm.2017.10.016) & Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224). | | | | |
| 6) See for example: doi:10.1016/s0040-4039(01)90658-6, Tetrahedron Letters No. 8, pp. 477-482, 1963 and pubchem.ncbi.nlm.nih.gov/compound/Gipsoside | | | | |

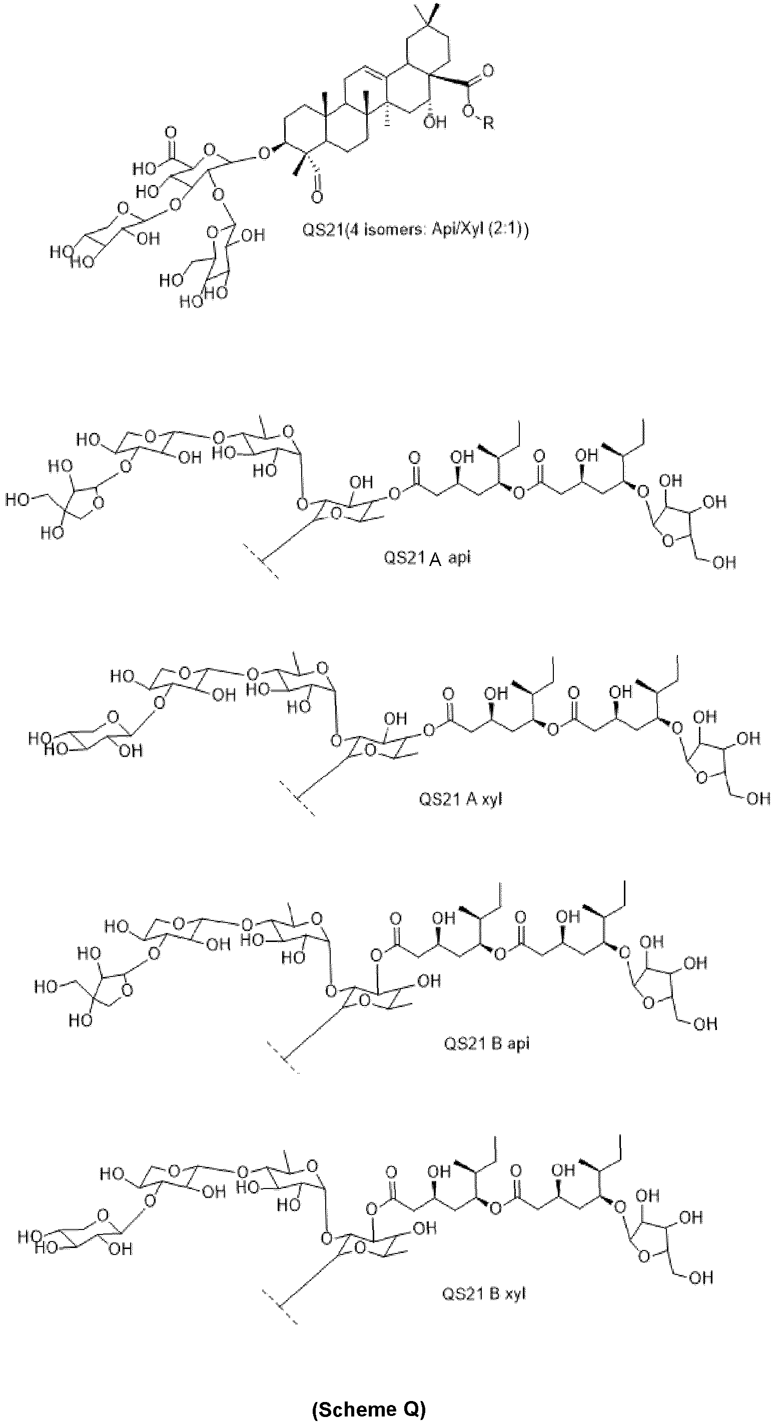

As can be seen from Table 1, many of the triterpenoid 12,13-dehydrooleanane-type saponin comprise an aldehyde group at position C-23 of the saponin's aglycone core structure.

Without wishing to be bound by any theory, it was observed that presence of said aldehyde group in the aglycone core structure of the saponin (here, also referred to as 'aglycone') is beneficial for the capacity of the saponin to stimulate and/or potentiate the endosomal escape of the therapeutic nucleic acids comprised by the conjugate of the invention.

Consequently, a composition for the disclosed herein therapeutic or prophylactic use or a conjugate according to the disclosure is provided, wherein the saponin either comprises an aldehyde group at position C-23 of the saponin's aglycone core structure, or a covalent bond at position C-23 of the saponin's aglycone core structure, the covalent bond covalently linking the saponin within the conjugate
preferably wherein the covalent bond at position C-23 is a cleavable bond that is subject to cleavage under conditions present in endosomes or lysosomes,
more preferably, wherein the cleavable covalent bond at position C-23 is adapted to restore aldehyde group at position C-23 upon cleavage;
or wherein the saponin used for preparing the conjugate in at least an unconjugated state e.g. prior to being covalently linked within the disclosed herein conjugates, e.g. in its natural form as existing or extracted from its source plant material, comprises an aldehyde group at position C-23 of the saponin's aglycone core structure.

Such saponins can be covalently linked to the remainder of the conjugate (said remainder comprising the nucleic acid and the ligand of an endocytic receptor on a muscle cell) by any functional group present in said saponin as suitable for conjugation as known in the art, or can be covalently liked by reacting said aldehyde group at position C-23 of the saponin's aglycone core structure, which reacting results in a conversion of the aldehyde group at position C-23 into a covalent bond at position C-23 wherein said covalent bond at position C-23 is covalently linking the saponin to the remainder of the conjugate.

Without wishing to be bound by any theory, it has been observed that such free aldehyde group is beneficial for a triterpenoid 12,13-dehydrooleanane-type saponin for its endosomal escape-stimulating properties, which is likely related to enhanced destabilisation of the vesicular membrane.

Consequently, for potentiating even further the endosomal escape-enhancing properties of the saponin, and thus for further improving the escaping of the therapeutic nucleic acid present therewith in the endosomal compartment as part of the disclosed conjugates, the covalent bond at position C-23 can be selected such that upon its cleavage (e.g. in response to conditions present in mammalian endosomes or lysosomes), the aldehyde group at position C-23 of the saponin's aglycone core structure is restored.

Examples of suitable bond types that can be designed for this aldehyde-group restoration purpose include one or more of: a semicarbazone bond, a hydrazone bond, an imine bond, an acetal bond including a 1,3-dioxolane bond, and/or an oxime bond. As shown herein below in examples, such functional design of a conjugate as disclosed herein has successfully been achieved with a saponin whereby the aldehyde group naturally present at position C-23 of the saponin, was converted into a semicarbazone or a hydrazone covalent bond at position C-23 of the saponin's aglycone core structure and linking the saponin within the conjugate. In response to acidic conditions, these bonds at position C-23 efficiently released the saponin from the conjugate, whereby the released saponin had the aldehyde group restored at position C-23.

In line with the above, endosomal-escape-enhancing properties of such saponins are also very pronounced when the aldehyde group is e.g. substituted by a maleimide-comprising moiety attached at said position C-23 with a cleavable covalent bond that cleaves off under acidic conditions present in endosomes and/or lysosomes of human cells, wherein said aldehyde group at position C-23 of the saponin's aglycone core structure is restored upon said cleavage under acidic conditions present in endosomes and/or lysosomes of human cells.

In advantageous embodiments, such cleavable covalent bond can be selected from a semicarbazone bond, a hydrazone bond, or an imine bond.

For example, in possible embodiments the maleimide-comprising moiety can be part of a molecule comprising or consisting of 4-(6-(2,5-dioxo-2,5-dihydro-1H-pyrrol-1-yl)hexanoyl)piperazine-1-carbohydrazide that is attached at position C-23 of the saponin's aglycone core structure upon forming a semicarbazone bond (further referred to as SC-Maleimide) or wherein the maleimide-comprising moiety is part of a molecule comprising or consisting of N-ε-maleimidocaproic acid hydrazide that is attached at position C-23 of the saponin's aglycone core structure upon forming a hydrazone bond (further referred to as EMCH).

Particularly suitable for the conjugates of the invention saponins of the 12,13-dehydrooleanane-type which naturally comprise the aldehyde group in position C-23 in their native or unconjugated form are saponins which aglycone core structure is either quillaic acid or gypsogenin. In line with this, it was observed that particularly suitable saponins for the conjugates of the invention are 12,13-dehydrooleanane-type saponins comprising a quillaic acid aglycone or a gypsogenin aglycone core structure, or if the C-23 aldehyde group of these aglycone core structures was used for conjugation, derivatives of said saponins wherein the aldehyde group at position C-23 of both of these aglycones has been converted to a covalent bond at the position C-23.

An example of an unconjugated saponin with the aldehyde group at position C-23 is depicted as SAPONIN A and illustrated by the following structure:

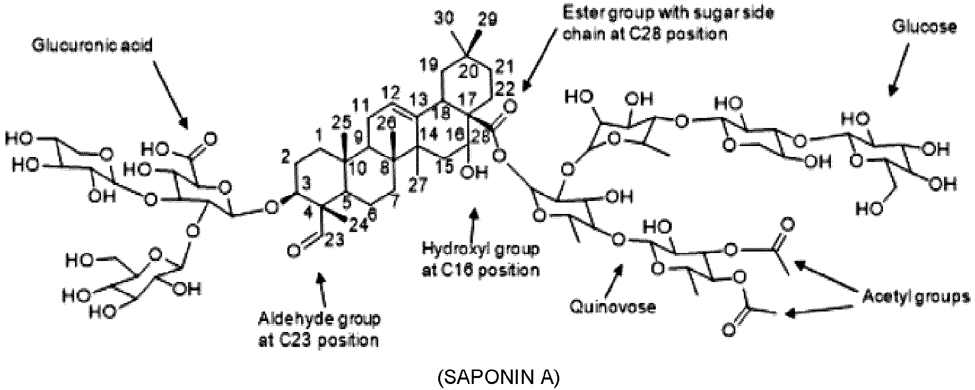

However, it should be noted that saponins comprising different 12,13-dehydrooleanane-type aglycone core structures were also observed to exhibit satisfactory endosomal-escape-enhancing properties, and consequently other aglycone structures listed in Table 1 can also be advantageous for the purposes of present disclosure.

Hence, in a further embodiment, a composition for the disclosed herein therapeutic or prophylactic use or a conjugate according to the disclosure is provided, wherein the saponin's aglycone core structure is selected from any one or more of:
quillaic acid;
(and/or including quillaic acid derivative wherein the aldehyde group at position C-23 of quillaic acid has been converted to a covalent bond at the position C-23, preferably wherein said covalent bond is the bond covalently linking the saponin within the conjugate;)
gypsogenin;
(and/or including gypsogenin derivative wherein the aldehyde group at position C-23 of gypsogenin has been converted to a covalent bond at the position C-23, preferably wherein said covalent bond is the bond covalently linking the saponin within the conjugate;) 2alpha-hydroxy oleanolic acid;
16alpha-hydroxy oleanolic acid;
hederagenin (23-hydroxy oleanolic acid);
16alpha,23-dihydroxy oleanolic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
3,16,28-trihydroxyoleanan-12-en;
gypsogenic acid; and
a derivative thereof,
preferably wherein the saponin's aglycone core structure is selected from
quillaic acid;
(and/or including quillaic acid derivative wherein the aldehyde group at position C-23 of quillaic acid has been converted to a covalent bond at the position C-23, preferably wherein said covalent bond is the bond covalently linking the saponin within the conjugate);
gypsogenin;
(and/or gypsogenin derivative wherein the aldehyde group at position C-23 of gypsogenin has been converted to a covalent bond at the position C-23, preferably wherein said covalent bond
is the bond covalently linking the saponin within the conjugate);
more preferably wherein the saponin's aglycone core structure is selected from quillaic acid (and/or including quillaic acid derivative wherein the aldehyde group at position C-23 of quillaic acid has been converted to a covalent bond at the position C-23, preferably wherein said covalent bond is the bond covalently linking the saponin within the conjugate).

Saponins can comprise one or more saccharide chains attached to the aglycone core structure. Preferred saponins of the compositions or conjugates of the disclosure comprise a single chain (i.e. are monodesmosidic) or two chains (i.e. are bidesmosidic) attached to the triterpene 12,13-dehydrooleanane aglycone core structure, optionally comprising an aldehyde group in position C-23.

It was postulated that the sugar chains also play a role in the endosomal-escape-enhancing properties.

In line with the above in a further embodiment, a composition for the disclosed herein therapeutic or prophylactic use or a conjugate according to the disclosure is provided, wherein the saponin's sugar fraction comprises a saccharide chain selected from any one of the saccharide chains as listed in group A or group B presented in the following Table 2:

| **Table 2. saccharide chains** |
|---|
| **Group A** |
| Ara/Xyl-(1→4)-Rha/Fuc-(1→2)-Glc/Gal-(1→2)-Rha/Fuc-(1→2)-GlcA- |
| Gal- |
| Gal-(1→2)-[Xyl-(1→3)]-GlcA- |
| Glc- |
| Glc-(1→2)-[Glc-(1→4)]-GlcA- |
| Glc-(1→2)-Ara-(1→3)-[Gal-(1→2)]-GlcA- |
| GlcA- |
| Rha-(1→2)-Ara- |
| Rha-(1→2)-Gal-(1→3)-[Glc-(1→2)]-GlcA- |
| Xyl-(1→2)-Ara-(1→3)-[Gal-(1→2)]-GlcA- |

| **Group B** |
|---|
| [4,6-di-OAc-Glc-(1→3)]-[Xyl-(1→4)]-Rha-(1→2)-[3,4-di-OAc-Qui-(1→4)]-Fuc- |
| 6-OAc-Glc-(1→3)-[Xyl-(1→4)]-Rha-(1→2)-Fuc- |
| 6-OAc-Glc-(1→3)-Xyl-(1→4)-Rha-(1→2)-[3-OAc-Rha-(1→3)]-Fuc- |
| Api-(1→3)-Xyl-(1→4)-[Glc-(1→3)]-Rha-(1→2)-[R-(→4)]-Fuc- |
| wherein R is 5-O-[5-O-Ara/Api-3,5-dihydroxy-6-methyl-octanoyl]-3,5-dihydroxy-6-methyl-octanoic acid |
| Api-(1→3)-Xyl-(1→4)-[Glc-(1→3)]-Rha-(1→2)-[Rha-(1→3)]-4OAc-Fuc- |
| Api-(1→3)-Xyl-(1→4)-[Glc-(1→3)]-Rha-(1→2)-[Rha-(1→3)]-4-OAc-Fuc- |
| Api-(1→3)-Xyl-(1→4)-[Glc-(1→3)]-Rha-(1→2)-Fuc- |
| Api-(1→3)-Xyl-(1→4)-Rha-(1→2)-[R-(→3)]-Fuc- |
| wherein R is 5-O-[5-O-Ara/Api-3,5-dihydroxy-6-methyl-octanoyl]-3,5-dihydroxy-6-methyl-octanoic acid |
| Api-(1→3)-Xyl-(1→4)-Rha-(1→2)-[R-(→4)]-Fuc- |
| wherein R is 5-O-[5-O-Ara/Api-3,5-dihydroxy-6-methyl-octanoyl]-3,5-dihydroxy-6-methyl-octanoic acid |
| Api-(1→3)-Xyl-(1→4)-Rha-(1→2)-[Rha-(1→3)]-4-OAc-Fuc- |
| Api/Xyl-(1→3)-Xyl-(1→4)-[Glc-(1→3)]-Rha-(1→2)-[R-(→4)]-Fuc- |
| wherein R is 5-O-[5-O-Ara/Api-3,5-dihydroxy-6-methyl-octanoyl]-3,5-dihydroxy-6-methyl-octanoic acid |
| Api/Xyl-(1→3)-Xyl-(1→4)-[Glc-(1→3)]-Rha-(1→2)-[R-(→4)]-Fuc- |
| wherein R is 5-O-[5-O-Rha-(1→2)-Ara/Api-3,5-dihydroxy-6-methyl-octanoyl]-3,5-dihydroxy-6-methyl-octanoic acid |
| Api/Xyl-(1→3)-Xyl-(1→4)-[Glc-(1→3)]-Rha-(1→2)-[Rha-(1→3)]-4OAc-Fuc- |
| Ara/Xyl- |
| Ara/Xyl-(1→3)-Ara/Xyl-(1→4)-Rha/Fuc-(1→2)-[4-OAc-Rha/Fuc-(1→4)]-Rha/Fuc- |
| Ara/Xyl-(1→4)-Rha/Fuc-(1→4)-[Glc/Gal-(1→2)]-Fuc- |
| Glc-(1→3)-[Glc-(1→6)]-Gal- |
| Glc-(1→3)-[Xyl-(1→3)-Xyl-(1→4)]-Rha-(1→2)-Fuc- |
| Glc-(1→3)-[Xyl-(1→4)]-Rha-(1→2)-[Qui-(1→4)]-Fuc- |
| Glc-(1→3)-[Xyl-(1→4)]-Rha-(1→2)-Fuc- |
| Glc-(1→3)-Xyl-(1→4)-[Glc-(1→3)]-Rha-(1→2)-4-OAc-Fuc- |
| Glc-(1→3)-Xyl-(1→4)-Rha-(1→2)-[3-OAc--Rha-(1→3)]-Fuc- |
| Glc-(1→3)-Xyl-(1→4)-Rha-(1→2)-[Xyl-(1→3)-4-OAc-Qui-(1→4)]-Fuc- |
| Glc-(1→3)-Xyl-(1→4)-Rha-(1→2)-4-OAc-Fuc- |
| Glc/Gal- |
| Rha-(1→2)-[Ara-(1→3)-Xyl-(1→4)]-Rha- |
| Rha-(1→2)-[Xyl-(1→4)]-Rha- |
| Xyl-(1→3)-Xyl-(1→4)-Rha-(1→2)-[3,4-di-OAc-Qui-(1→4)]-Fuc- |
| Xyl-(1→3)-Xyl-(1→4)-Rha-(1→2)-[Qui-(1→4)]-Fuc- |
| Xyl-(1→3)-Xyl-(1→4)-Rha-(1→2)-[R-(→3)]-Fuc- |
| wherein R is 5-O-[5-O-Ara/Api-3,5-dihydroxy-6-methyl-octanoyl]-3,5-dihydroxy-6-methyl-octanoic acid |
| Xyl-(1→3)-Xyl-(1→4)-Rha-(1→2)-[R-(→4)]-Fuc- |
| wherein R is 5-O-[5-O-Ara/Api-3,5-dihydroxy-6-methyl-octanoyl]-3,5-dihydroxy-6-methyl-octanoic acid |
| Xyl-(1→3)-Xyl-(1→4)-Rha-(1→2)-[Xyl-(1→3)-4-OAc-Qui-(1→4)]-Fuc- |
| Xyl-(1→4)-[Gal-(1→3)]-Rha-(1→2)-4-OAc-Fuc- |
| Xyl-(1→4)-[Gal-(1→3)]-Rha-(1→2)-Fuc- |
| Xyl-(1→4)-[Glc-(1→3)]-Rha-(1→2)-[R-(→4)]-3-OAc-Fuc- |
| wherein R is 4E-Methoxycinnamic acid) |
| Xyl-(1→4)-[Glc-(1→3)]-Rha-(1→2)-3,4-di-OAc-Fuc- |
| Xyl-(1→4)-[Glc-(1→3)]-Rha-(1→2)-Fuc- |
| Xyl-(1→4)-Rha-(1→2)-[R-(→4)]-Fuc- |
| wherein R is 4E-Methoxycinnamic acid |
| Xyl-(1→4)-Rha-(1→2)-[R-(→4)]-Fuc- |
| wherein R is 4Z-Methoxycinnamic acid |

In advantageous for e.g. conjugation-options embodiments, the saponin is a bidesmosidic saponin.

In a related embodiment, a composition for the disclosed herein therapeutic or prophylactic use or a conjugate according to the disclosure is provided, wherein the saponin is at least a bidesmosidic saponin comprising a first saccharide chain that is selected from the group A, and comprising a second saccharide chain that is selected from the group B; preferably wherein the first saccharide chain comprises a terminal glucuronic acid residue and/or wherein the second saccharide chain comprises at least four sugar residues in a branched configuration; more preferably wherein the first saccharide chain is Gal-(1→2)-[Xyl-(1→3)]-GlcA and/or wherein the branched second saccharide chain of at least four sugar residues comprises a terminal fucose residue and/or a terminal rhamnose residue.

In an advantageous embodiment of the conjugate of the invention, the saponin comprises one or both of: a first saccharide chain bound to the C-3 atom or to the C-28 atom of the aglycone core structure, preferably comprises one saccharide chain bound to the C-3 atom and a second saccharide chain bound to the C-28 atom of the aglycone core structure. In such instance, when the saponin comprised by the conjugate of the invention bears said two glycans (saccharide chains), the first saccharide chain is bound at position C-3 of the aglycone core structure and the second saccharide chain is typically bound at position C-28 of the aglycone core structure of the saponin, although for some saponins lacking the aldehyde group at position C-23 position, the second glycan can be bound at said C-23 position (see Table 1).

Hence, in a further related embodiment, a composition for the disclosed herein therapeutic or prophylactic use or a conjugate according to the disclosure is provided, wherein the saponin comprises the first saccharide chain at position C-3 of the saponin's aglycone core structure and/or the second saccharide chain at position C-28 of the saponin's aglycone core structure;
preferably wherein the first saccharide chain is a carbohydrate substituent at the C-3beta-OH group of the saponin's aglycone core structure and/or wherein the second saccharide chain is a carbohydrate substituent at the C-28-OH group of the saponin's aglycone core structure.

In a particularly preferred embodiment, a conjugate is provided wherein the saponin is a triterpenoid 12,13-dehydrooleanane-type saponin comprising in at least an unconjugated state an aldehyde group at position C-23 of the saponin's aglycone core structure and comprising as a carbohydrate substituent at the C-3beta-OH group of the saponin's aglycone core a saccharide chain selected from the group A and comprising a terminal glucuronic acid residue, the saccharide chain preferably being Gal-(1→2)-[Xyl-(1→3)]-GlcA.

In a next embodiment, a composition for the disclosed herein therapeutic or prophylactic use or a conjugate according to the disclosure is provided, wherein the conjugate comprises two or more molecules of the saponin, preferably being between 2-32 molecules of the saponin, even more preferably 4-16 molecules of the saponin, most preferably 4-8 molecules of the saponin.

These molecules can be identical saponins, or saponins of the same aglycone core structure and different saccharide chains, or can even be a mixture of different endosomal-escape enhancing saponins of the 12,13-dehydrooleanane-type, for example being a mixture of different saponins selected from Table 1.

In another embodiment, a composition for the disclosed herein therapeutic or prophylactic use or a conjugate according to the disclosure is provided, wherein the saponin is any one or more of:
a) saponin selected from any one or more of list A:
   - *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
   - *Saponinum album* saponin mixture, or a saponin isolated from *Saponinum album;*
   - *Saponaria officinalis* saponin mixture, or a saponin isolated from *Saponaria officinalis*; and
   - Quillaja bark saponin mixture, or a saponin isolated from Quillaja bark, for example Quil-A, QS-17-api, QS-17-xyl, QS-21, QS-21A, QS-21B, QS-7-xyl; or
b) a saponin comprising a gypsogenin aglycone core structure, selected from list B:
   SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C:
   AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, SO1832, SO1861, SO1862, SO1904, QS-7, QS-7 api, QS-17, QS-18, QS-21 A-apio, QS-21 A-xylo, QS-21 B-apio and QS-21 B-xylo; or
d) a saponin comprising a 12, 13-dehydrooleanane type aglycone core structure without an aldehyde group at the C-23 position of the aglycone, selected from list D:
   Aescin la, aescinate, alpha-Hederin, AMA-1, AMR, AS6.2, AS64R, Assamsaponin F, dipsacoside B, esculentoside A, macranthoidin A, NP-005236, NP-012672, Primula acid 1, saikosaponin A, saikosaponin D, Teaseed saponin I and Teaseedsaponin J,
preferably, the saponin is any one or more of a saponin selected from list A, B or C, more preferably, a saponin selected from list B or C, even more preferably, a saponin selected from list C.

In a particular embodiment, a composition for the disclosed herein therapeutic or prophylactic use or a conjugate according to the disclosure is provided, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* Saponarioside B, SO1542, SO1584, SO1658, SO1674, SO1700, SO1730, SO1772, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

In a more particular embodiment, a composition for the disclosed herein therapeutic or prophylactic use or a conjugate according to the disclosure is provided, wherein the saponin is a saponin isolated from *Saponaria officinalis,* preferably wherein the saponin is any one or more of Saponarioside B, SO1542, SO1584, SO1658, SO1674, SO1700, SO1730, SO1772, SO1832, SO1861, SO1862 and SO1904; more preferably wherein the saponin is any one or more of SO1542, SO1584, SO1658, SO1674, SO1700, SO1730, SO1772, SO1832, SO1861, SO1862 and SO1904; even more preferably wherein the saponin is any one or more of SO1832, SO1861 and SO1862; even more preferably wherein the saponin is SO1832 and SO1861; most preferably being SO1861.

In particular embodiments, conjugates and compositions comprising the same for the disclosed herein therapeutic or prophylactic use can be provided wherein one, two or three, preferably one or two, more preferably one, of:
i. an aldehyde group in the aglycone core structure of the at least one saponin has been derivatised when present,
ii. a carboxyl group of a glucuronic acid moiety in a first saccharide chain of the at least one saponin has been derivatised when present in the at least one saponin, and
iii. at least one acetoxy (Me(CO)O-) group in a second saccharide chain of the at least one saponin has been derivatised if present.

In more particular embodiments, conjugates and compositions comprising the same for the disclosed herein therapeutic or prophylactic use can be provided wherein the at least one saponin comprises:
i. an aglycone core structure comprising an aldehyde group which has been derivatised by:
   - reduction to an alcohol;
   - transformation into a hydrazone bond through reaction with N-ε-maleimidocaproic acid hydrazide (EMCH) wherein the maleimide group of the EMCH is optionally derivatised by formation of a thioether bond with mercaptoethanol;
   - transformation into a hydrazone bond through reaction with N-[ß-maleimidopropionic acid] hydrazide (BMPH) wherein the maleimide group of the BMPH is optionally derivatised by formation of a thioether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thioether bond with mercaptoethanol; or
ii. a first saccharide chain comprising a carboxyl group, preferably a carboxyl group of a glucuronic acid moiety, which has been derivatised by transformation into an amide bond through reaction with 2-amino-2-methyl-1,3-propanediol (AMPD) or N-(2-aminoethyl)maleimide (AEM); or
iii. a second saccharide chain comprising an acetoxy group (Me(CO)O-) which has been derivatised by transformation into a hydroxyl group (HO-) by deacetylation; or
iv. any combination of two or three derivatisations i., ii. and/or iii., preferably any combination of two derivatisations of i., ii. and iii.

In a specific embodiment, provided are conjugates and compositions comprising the same, wherein the at least one saponin comprises the first saccharide chain and comprises the second saccharide chain according to Group A and Group B of Table 2, respectively, wherein the first saccharide chain comprises more than one saccharide moiety and the second saccharide chain comprises more than one saccharide moiety, and wherein the aglycone core structure preferably is quillaic acid or gypsogenin, more preferably quillaic acid, wherein one, two or three, preferably one or two, of:
i. an aldehyde group in the aglycone core structure has been derivatised,
ii. a carboxyl group of a glucuronic acid moiety in the first saccharide chain has been derivatised, and
iii. at least one acetoxy (Me(CO)O-) group in the second saccharide chain has been derivatised.

An embodiment is the conjugate of the invention, wherein one, two or three, preferably one or two, more preferably one, of:
iv. an aldehyde group in the aglycone core structure of the at least one saponin has been derivatised when present,
v. a carboxyl group of a glucuronic acid moiety in a first saccharide chain of the at least one saponin has been derivatised when present in the at least one saponin, and
   at least one acetoxy (Me(CO)O-) group in a second saccharide chain of the at least one saponin has been derivatised if present.

In a specific embodiment, a conjugate or a composition for the disclosed herein therapeutic or prophylactic use is provided wherein the aldehyde function in position C-23 of the aglycone core structure of the at least one saponin is covalently bound to linker EMCH, which EMCH is covalently bound via a thio-ether bond to a sulfhydryl group in the oligomeric molecule or in the polymeric molecule of the covalent saponin conjugate, such as a sulfhydryl group of a cysteine. Binding of the EMCH linker to the aldehyde group of the aglycone of the saponin results in formation of a hydrazone bond. Such a hydrazone bond is a typical example of a cleavable bond under the acidic conditions inside endosomes and lysosomes. A saponin that is coupled to the ligand comprised by the conjugate or to the nucleic acid comprised by the conjugate etc. is releasable from the conjugate of the invention once delivered in the endosome or lysosome of a target muscle cell that exposes the endocytic receptor which the ligand can bind. This way, the saponin coupled to the ligand and to the nucleic acid in the conjugate is transferred from outside the cell into the endosome (or lysosome), and in the endosome (or the lysosome), the saponin is released from the remainder of the conjugate upon pH driven cleavage of the hydrazone bond. In the endosome (or the lysosome) the free saponin can exert its stimulatory activity when the delivery of the therapeutic nucleic acid comprised by the conjugate of the invention, into the cytosol of the muscle cell. As explained above, the inventors surprisingly realised that it is not a prerequisite for the saponin-mediated endosomal escape that the saponin is present in endosomes or lysosomes in a free form. Also, saponins comprised in the disclosed herein conjugates can potentiate the delivery of therapeutic nucleic acids out of the endosome / lysosome into the cytosol of the targeted muscle cell.

The development of the presented herein advantageous compositions was based on the surprising realisation that, thanks to the inclusion of the endosomal-escape-enhancing saponin in the conjugates of the invention, any nucleic acid can be delivered with an improved efficiently into muscle cells to aid the treatment and/or prophylaxis of muscle-wasting disorders.

In line with this, in an advantageous embodiment, the nucleic acid is a therapeutic nucleic acid adapted to target mutated transcript or a genomic mutation within a gene affected in a particular muscle cell-related genetic disorder. A list of such potentially targetable genetic targets and muscle cell-related genetic disorder associated therewith can be for instance found in Cardamone M, et al., 2008.

In a particular embodiment, such genetic target is the mutated human dystrophin underlying the dystrophinopathies such as DMD, for which the proof-of-concepts experiments demonstrating the potential of the disclosed herein compositions are presented in the continuation. However, other mutations in known genes can also be targeted, such as the ones in but not limited to: DUX4/double homeobox 4 underling facioscapulohumeral muscular dystrophy, or DMPK underlying myotonic dystrophy type 1, or EMD/emerin and LMNA/lamin A/C underling the Emery-Dreifuss muscular dystrophy, or MYOT/myotilin, LMNA/lamin A/C underling limb-girdle muscular dystrophy 1. Further examples LAMA2/merosin or laminin-α2 chain/ any of COL6A genes encoding for collagen 6A that become mutated in congenital muscular dystrophy or LMNA/lamin A/C in dilated familial cardiomyopathy. Further mutations that can be targeted by nuclei acids present in the disclosed herein conjugates and compositions can be found in genes like NEB/nebulin, ACTA/skeletal muscle alpha-actin, TPM3/alpha-tropomyosin-3, TPM2/beta-tropomyosin-2, TNNT1/troponin T1, LMOD3/leiomodin-3, MYPN/myopalladin etc. (nemalin myopathy) or TPM3/alpha-tropomyosin-3 , CTA/skeletal muscle alpha-actin, RYR1/ryanodine receptor channel (congenital fibre-type disproportion myopathy) or advantageously in TTN gene (titin).

In a particularly advantageous embodiment, a composition for the disclosed herein therapeutic or prophylactic use or a conjugate according to the disclosure is provided, wherein the nucleic acid is an oligonucleotide defined as a nucleic acid that is no longer than 150 nt, preferably wherein the oligonucleotide has a size of 5 - 150 nt, preferably being 8 - 100 nt, most preferably being 10 - 50 nt.

As used herein the term oligonucleotide shall be understood as encompassing both the oligomers that are made of naturally occurring nucleotides and hence, chemically are oligonucleotides, as well as oligomers comprising modified oligonucleotides or analogues thereof. For example, a synthetic oligomer may comprise e.g. 2' modified nucleosides which can be selected from: 2'-fluoro (2'-F), 2'-O-methyl (2'-O-Me), 2'-O-methoxyethyl (2'-MOE). 2'-O-aminopropyl (2'O-AP), 2'-O-dimethylaminoethyl (2'-O-DMAOE), 2'-0-dimethylaminopropyl (2'-O-DMAP),2'-O-dimethylaminoethyloxyethyl (2'-0-DMAE0E), 2'-O-N-methylacetamido (2'-O-NMA), locked nucleic acid (LNA), ethylene-bridged nucleic acid (ENA), and (S)-constrained ethylbridged nucleic acid (cEt), etc. In line with this, in a possible embodiment, the oligonucleotide can structurally or functionally be defined as any of: a deoxyribonucleic acid (DNA) oligomer, ribonucleic acid (RNA) oligomer, anti-sense oligonucleotide (ASO, AON), short interfering RNA (siRNA), anti-microRNA (anti-miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 3'-fluoro hexitol nucleic acid (FHNA), glycol nucleic acid (GNA), threose nucleic acid (TNA), BNA-based siRNA, and BNA-based antisense oligonucleotide (BNA-AON), an siRNA, such as BNA-based siRNA, selected from chemically modified siRNA, metabolically stable siRNA and chemically modified, metabolically stable siRNA, or any other category known in the art.

From functional perspective, in advantageous embodiments, a composition for the disclosed herein therapeutic or prophylactic use or a conjugate according to the disclosure is provided, wherein the oligonucleotide is an antisense oligonucleotide, more preferably being a mutation specific antisense oligonucleotide, most preferably being an oligonucleotide designed to induce exon skipping.

In a related embodiment, a composition for the disclosed herein therapeutic or prophylactic use or a conjugate according to the disclosure is provided, wherein the oligonucleotide comprises or consists of any one of the following: morpholino phosphorodiamidate oligomer (PMO), 2'-O-methyl (2'-OMe) phosphorothioate RNA, 2'-O-methoxyethyl (2'-O-MOE) RNA {2'-O-methoxyethyl-RNA (MOE)}, locked or bridged nucleic acid (LNA or BNA), 2'-O,4'-aminoethylene bridged nucleic acid (BNANC), peptide nucleic acid (PNA), 2'-deoxy-2'-fluoroarabino nucleic acid (FANA), 3'-fluoro hexitol nucleic acid (FHNA), glycol nucleic acid (GNA), threose nucleic acid (TNA), silencing RNA (siRNA), short hairpin RNA (shRNA), microRNA (miRNA), antagomir (miRNA antagonists), aptamer RNA or aptamer DNA, single-stranded RNA or single-stranded DNA, double-stranded RNA (dsRNA) or double-stranded DNA;

In particularly preferred embodiments, composition for the disclosed herein therapeutic or prophylactic use or a conjugate according to the disclosure is provided, wherein the oligonucleotide comprises or consists of a morpholino phosphorodiamidate oligomer (PMO) or a 2'-O-methyl (2'-OMe) phosphorothioate RNA.

In a particular and advantageous for DMD-specific embodiment, a composition for the disclosed herein therapeutic or prophylactic use or a conjugate according to the disclosure is provided, wherein the oligonucleotide is designed to induce exon skipping of the human dystrophin gene transcript, preferably wherein the exon skipping involves exon 51 skipping or exon 53 skipping or exon 45 skipping, preferably wherein the oligonucleotide is a 2'O-methyl-phosporothioate antisense oligonucleotide or a phosphorodiamidate morpholino oligomer antisense oligonucleotide that is designed to induce the exon 51 skipping or the exon 53 skipping or the exon 45 skipping.

In a very particular embodiment in line with the directly-preceding one, the oligonucleotide is selected from eteplirsen, drisapersen, golodirsen, viltolarsen, and casimersen.

In an advantageous embodiment, a composition for the disclosed herein therapeutic or prophylactic use or a conjugate according to the disclosure is provided, wherein the conjugate comprises two or more molecules of the nucleic acid. Such therapeutic combinations of two or more therapeutic nucleic acids are known in the art and for muscle-wasting disorders e.g. a combined approach based on two AONs for dual exon skipping in myostatin and dystrophin was proposed for the management of Duchenne muscular dystrophy [Kemaladewi el al, 2011].

In further advantageous embodiment, a composition for the disclosed herein therapeutic or prophylactic use or a conjugate according to the disclosure is provided, wherein preferably wherein the two or more molecules of the nucleic acid are 2-16 molecules, more preferably 2-8 molecules, possibly 2, 3, 4, 5, or 6 molecules.

In line with the clinical practice, in a preferred embodiment, a composition for the disclosed herein therapeutic or prophylactic use or a conjugate according to the disclosure is provided, wherein the two or more molecules of the nucleic acid are two or more oligonucleotides; possibly being two or more different oligonucleotides wherein at least one of the two or more different oligonucleotides is an antisense oligonucleotide.

With regard to particular embodiments relating to the ligand of an endocytic receptor on a muscle cell, it should be noted that may endocytic receptors expressed on the surface of muscle cells are known and are some of which like the transferrin receptor (CD71) or muscle-specific kinase (MuSK) are described in WO2018129384 or WO2020028857. Further examples of suitable receptors may include muscle-transmembrane transporters, for instance GLUT4 or ENT2 (described with many others in Ebner, 2015), or for example tetraspanin CD63 [Baik, 2021]. In fact, a lot of endocytic receptors present on the surface of muscle cells have been characterised so far, with the transferrin receptor (CD71) and perhaps insulin-like growth factor 1 (IGF-I) receptor (IGF1R) being the most investigated ones. The ligands for these receptors can be selected from natural ligands, such as transferrin (Tf) being a natural ligand of CD71, or LDL being a ligand of the LDL receptor. Alternatively, they can be non-naturally occurring ligands such as various types of antibodies or binding fragments thereof, or alternatively can be synthetic ligands like zymozan A that binds to endocytic mannose receptors, or synthetic fragments of naturally existing ligands such as fragments of IGF-I being a ligand of GF1R or fragments of IGF-II being a ligand of CI-MPR (also known as IGF2R).

In a preferred embodiment, a composition for the disclosed herein therapeutic or prophylactic use or a conjugate according to the disclosure is provided, wherein the endocytic receptor on a muscle cell to which the ligand binds is selected from: transferrin receptor (CD71), insulin-like growth factor 1 (IGF-I) receptor (IGF1R), tetraspanin CD63; muscle-specific kinase (MuSK), glucose transporter GLUT4, cation independent mannose 6 phosphate receptor (CI-MPR), and LDL receptor.

In a further preferred embodiment, a composition for the disclosed herein therapeutic or prophylactic use or a conjugate according to the disclosure is provided, wherein the ligand is selected from any one of:
insulin-like growth factor 1 (IGF-I) or fragments thereof;
insulin-like growth factor 2 (IGF-II) or fragments thereof
Mannose 6 phosphate
transferrin (Tf),
zymozan A, and
an antibody or a binding fragment thereof specific for binding to the endocytic receptor, wherein the endocytic receptor is preferably selected from: transferrin receptor (CD71), insulin-like growth factor 1 (IGF-I) receptor (IGF1R), tetraspanin CD63, muscle-specific kinase (MuSK), glucose transporter GLUT4, cation independent mannose 6 phosphate receptor (CI-MPR), and LDL receptor;
preferably wherein the ligand is an antibody or a binding fragment thereof that is specific for binding to a transferrin receptor,

In a related particularly advantageous embodiment, the ligand is a monoclonal antibody or a Fab' fragment or at least one single domain antibody specific for binding to a transferrin receptor, even more preferably wherein the ligand is a monoclonal antibody specific for binding to a transferrin receptor.

In further advantageous embodiment, the ligand is a monoclonal antibody such as a humanized or a human monoclonal antibody, an IgG, a molecule comprising or consisting of a single-domain antibody, at least one VHH domain, preferable a camelid VH, a variable heavy chain new antigen receptor (VNAR) domain, a Fab, an scFv, an Fv, a dAb, an F(ab)2 and a Fcab fragment.

In a specific embodiment, a conjugate of the invention is provided with the proviso that the ligand of an endocytic receptor on a muscle cell is not a ligand for CD71 or MuSK, preferably with the proviso that the ligand is not a CD71-binding antibody or a CD71-binding fragment thereof or is not a MuSK-binding antibody or a MuSK-binding fragment thereof.

As explained above, in further advantageous embodiments, a composition for the disclosed herein therapeutic or prophylactic use or a conjugate according to the disclosure is provided, wherein the covalent linking of the saponin within the conjugate is made via a first linker to which the saponin is covalently bound; preferably wherein the first linker comprises a covalent bond selected from any one or more of: a semicarbazone bond, a hydrazone bond, an imine bond, an acetal bond including a 1,3-dioxolane bond, a ketal bond, an ester bond, an oxime bond, a disulfide bond, a thio-ether bond, an amide bond, a peptide bond, and an ester bond, preferably being selected from any one or more of: a semicarbazone bond, a hydrazone bond, an imine bond, a disulfide bond, a thio-ether bond, an amide bond, a peptide bond, and an ester bond, more preferably a hydrazone bond or a semicarbazone bond; even more preferably wherein the saponin either comprises
an aldehyde group at position C-23 of the saponin's aglycone core structure, or
a covalent bond at position C-23 of the saponin's aglycone core structure, the covalent bond covalently linking the saponin within the first conjugate via the first linker and comprised as part of the first linker, preferably wherein the covalent bond at position C-23 is a cleavable bond that is subject to cleavage under conditions present in endosomes or lysosomes, more preferably, wherein the cleavable covalent bond at position C-23 is adapted to restore aldehyde group at position C-23 upon cleavage;
or wherein the saponin used for preparing the conjugate is a saponin that in at least an unconjugated state comprises an aldehyde group at position C-23 of the saponin's aglycone core structure and wherein following the preparing of the conjugate said aldehyde group has been engaged in forming the covalent bond with the first linker.

Hence, in a related advantageous embodiment, a composition for the disclosed herein therapeutic or prophylactic use or a conjugate according to the disclosure is provided, wherein the first linker is a cleavable linker subject to cleavage under acidic, reductive, enzymatic and/or light-induced conditions;
preferably wherein the first linker comprises a cleavable bond selected from:
   - a bond subject to cleavage under acidic conditions such as a semi-carbazone bond, a hydrazone bond, an imine bond, an acetal bond including a 1,3-dioxolane bond, a ketal bond, an ester bond, and/or an oxime bond,
   - a bond susceptible to proteolysis, for example amide or peptide bond, preferably subject to proteolysis by Cathepsin B;
   - a red/ox-cleavable bond such as a disulfide bond, or a thiol-exchange reaction-susceptible bond such as a thio-ether bond;
more preferably wherein the first linker comprises a cleavable bond selected from:
   - a bond subject to cleavage under acidic conditions such as a semicarbazone bond, or a hydrazone bond, and/or
   - a bond susceptible to proteolysis, for example proteolysis by Cathepsin B, and/or
   - a bond susceptible for cleavage under reductive conditions such as a disulfide bond.

Preferably, such bond is an acid-sensitive bond that is subject to cleavage *in vivo* under acidic conditions present in endosomes and/or lysosomes of human cells, preferably at pH 4.0 - 6.5, and more preferably at pH ≤ 5.5;

In advantageous embodiments, the acid-sensitive bond is selected from any one or more of: a semicarbazone bond, a hydrazone bond, an imine bond, an acetal bond including a 1,3-dioxolane bond, a ketal bond, an ester bond, and/or an oxime bond, even more preferably selected from a semicarbazone bond and a hydrazone bond; most preferably being a hydrazone bond.

In certain embodiments, when the saponin is conjugated by means of a covalent bond at position C-23 of the saponin's aglycone core structure (preferably being an acid-sensitive bond), it can be advantageous for said covalent bond at position C-23 to be selected such or adapted to restore the aldehyde group at position C-23 upon cleavage (e.g. under acidic conditions). Advantageously, such covalent bond can be selected from any one or more of: a semicarbazone bond, a hydrazone bond, an imine bond, an acetal bond including a 1,3-dioxolane bond, and/or an oxime bond, preferably being either a semi-carbazone bond or a hydrazone bond.

It is preferred that such a cleavable bond is not susceptible, or only to a minor extent, to cleavage when the conjugate is present outside the endosome and lysosome of the cell, such as outside the cell or in the endocytosed vesicle after the conjugate engaged with an endocytic receptor by binding. Preferably, the bond is efficiently cleaved *in vivo* under acidic conditions present in endosomes and/or lysosomes of human cells, preferably of pH ≤ 6.5, preferably pH ≤ 6, more preferably pH ≤ 5.5.

For example, the cleavable bond is preferably less susceptible to cleavage when the conjugate is present in the circulation of a human subject and/or is present extracellularly in an organ of the human subject, compared to the susceptibility for cleavage of the bond when the conjugate is in the endosome or in the lysosome of a target cell that endocytosed the conjugate. Therefore, preferred is a cleavable bond that is subject to cleavage *in vivo* under acidic conditions (i.e. an acid-sensitive bond) that are present in endosomes and/or lysosomes of human cells, preferably of pH ≤ 6.5, preferably pH ≤ 6, more preferably pH ≤ 5.5, more preferably being a bond selected from a semi-carbazone bond and a hydrazone bond; most preferably being a hydrazone bond. When the saponin is cleaved off from the remainder of the conjugate, the saponin improvingly exerts its endosomal escape enhancing activity to further molecules present in the endosome together with the saponin, such as the nucleic acid or the oligonucleotide of the conjugate of the disclosure.

Saponins comprising an aldehyde group at the C-23 position of the aglycone are particularly preferred since these saponins have potent endosomal escape enhancing activity towards nucleic acids such as oligonucleotides. Therefore, the saponins preferred for the conjugate that those that comprise or form an aldehyde group at position C-23 of the saponin's aglycone core structure under acidic conditions present in endosomes and/or lysosomes of human cells.

For example, when the saponins of Group B and Group C described above are covalently bound in the conjugate via linker chemistry involving the aldehyde group (e.g. formation of a hydrazone bond or a semicarbazone bond), it is preferred that the aldehyde group is re-formed (i.e. restored) in the endosome or lysosome when the conjugate is endocytosed and the saponin is cleaved off from the remainder of the conjugate by cleavage of a cleavable bond. Examples of such saponins suitable for this purpose are listed in Table 1, and are for example the saponins of Groups A-C, in particular Group B and Group C, as outlined here above.

For building complex covalent conjugates e.g. for including larger or a fixed number of ligands such as antibodies, or multiple nucleic acid molecules or, especially saponin molecules, molecular scaffolds can be employed comprising oligomeric or polymeric structures. One of the advantages of thereof is that the scaffold may be designed such that it comprises a defined number of molecules, for example saponins. For example, a scaffold may comprise exactly one saponin molecule but may also comprise a couple (e.g. two, three or four) of saponins or a multitude (e.g. 10, 20 or 100) of a relatively constant and defined number of saponins. Possibly such oligomeric/polymeric structure would be made of or poly(amines), e.g., polyethylenimine and poly(amidoamine), or alternatively polyethylene glycol, poly(esters), such as poly(lactides), poly(lactams), polylactide-co-glycolide copolymers, poly(dextrin), or a peptide or a protein, or natural and/or artificial polyamino acids, e.g. poly-lysine, DNA polymers, such as a DNA comprising 2-100 nucleotides, stabilized RNA polymers or PNA (peptide nucleic acid) polymers, for example comprising 2-200 nucleotides, either appearing as linear, branched or cyclic polymer, oligomer, dendrimer, dendron (for example any of a G2, G3, G4 or G5 dendron, for maximally covalently binding of 4, 8, 16 or 32 saponin moieties, respectively), dendronized polymer, dendronized oligomer or assemblies of these structures, either sheer or mixed. Preferably, such scaffolds can be made of oligomeric/polymeric structure such as a dendrimer, a dendron, a dendronized polymer, a dendronized oligomer, a DNA, for example 2-200 nucleic acids, a poly-ethylene glycol, an oligo-ethylene glycol (OEG), such as OEG₃, OEG₄ and OEG₅.

Hence, in an advantageous embodiment, a composition for the disclosed herein therapeutic or prophylactic use or a conjugate according to the disclosure is provided, wherein the first linker further comprises an oligomeric or polymeric structure either being a dendron such as a poly-amidoamine (PAMAM) dendrimer, or a poly-ethylene glycol such as any of PEG3 - PEG30; preferably the polymeric or oligomeric structure being any one of PEG4 - PEG12 or any one of a G2 dendron, a G3 dendron, a G4 dendron and a G5 dendron, more preferably being a G2 dendron or a G3 dendron or a PEG3-PEG30.

Such oligomeric/polymeric scaffold-forming structures are advantageously devoid of intrinsic biological activity. Typically, the scaffolds are made of inert molecules to avoid causing potential health risks. Driven by the number of selected saponins to be incorporated in particular embodiments of the presented herein muscle-targeting conjugates, the type and size or length of the oligomeric/polymeric structure can be appropriately selected. That is to say, the number of saponins to be coupled to the ligand and the nucleic acid as part of the conjugate, can determine the selection of a suitable oligomeric or polymeric structure, bearing the sufficient number of binding sites for coupling of the desired number of saponins, therewith providing a covalent saponin binding structure. For example, length of an OEG or size of a Dendron or poly-lysine molecule determines the maximum number of saponins which can be covalently linked to such oligomeric or polymeric structure potentially comprised as part of the first linker within the conjugate.

In advantageous embodiments, such scaffold-comprising conjugate would comprise a defined number or range of covalently-linked thereto saponins, rather than a random number thereof. This is especially advantageous for drug development in relation to obtaining marketing authorization. A defined number in this respect means that a conjugate could comprise a previously defined number of saponins. This is, e.g., achieved by designing a scaffold comprising the oligomeric/polymeric structure with a certain number of possible groups for engaging with the saponin(s). Under ideal circumstances, each one of these groups would engage with a saponin molecule thus resulting in a conjugate comprising a defined number of saponins. It is envisaged to offer a standard set of scaffolds, comprising, e.g., two, four, eight, sixteen, thirty-two, sixty-four, *etc.*

In an embodiment of the conjugate of the invention, a scaffold could be provided wherein the number of the saponin molecules would be defined as a range as, e.g., for non-ideal binding circumstances wherein not all group present in such oligomeric/polymeric would engage with a saponin molecule. Such ranges may for instance be 2 - 4 saponin molecules per scaffold, 3 - 6 saponin molecules per scaffold, 4 - 8 saponin molecules per scaffold, 6 - 8 saponin molecules per scaffold, 6 - 12 saponin molecules per scaffold and so on.

Such first linker comprising a number of saponins bound to the oligomeric or polymeric molecule in some embodiments could serves as a carrier (support, scaffold) for multiple saponin moieties, which can be bound to the ligand and the nucleic acid and thus form certain embodiments of the disclosed herein muscle-cell targeting therapeutic conjugates. In an advantageous embodiment, such oligomeric or polymeric molecule-comprising linker loaded with saponin molecules could be attached to the remainder of the muscle-cell targeting conjugate via a preferably cleavable bond.

In another advantageous embodiment, a composition for the disclosed herein therapeutic or prophylactic use or a conjugate according to the disclosure is provided, wherein the covalent linking of the nucleic acid within the conjugate is made via a second linker to which the nucleic acid is covalently bound; preferably wherein the second linker comprises or consists of linker succinimidyl 3-(2-pyridyldithio)propionate (SPDP); possibly wherein the second linker covalently links the nucleic acid to a lysine residue, preferably being a lysine residue comprised in the ligand, or to a glycan residue, preferably a partially-trimmed glycan.

Similarly to saponin conjugation, it can also be advantageous if the one or more of the nucleic acid molecules, e.g. oligonucleotide molecules, and in particular the double-stranded ones like siRNAs, are bound in the conjugate via a cleavable bond, wherein the cleavable bond is subject to cleavage under for example acidic, reductive, enzymatic and/or light-induced conditions. The cleavable bond being subject to cleavage under acidic conditions present in endosomes and/or lysosomes of human cells is preferred.

In line with the above, in a related and advantageous embodiment, a composition for the disclosed herein therapeutic or prophylactic use or a conjugate according to the disclosure is provided, wherein the second linker is a cleavable linker subject to cleavage under acidic, reductive, enzymatic and/or light-induced conditions;
preferably wherein the second linker comprises a cleavable bond selected from:
   - a bond subject to cleavage under acidic conditions such as a semicarbazone bond, a hydrazone bond, an imine bond, an acetal bond including a 1,3-dioxolane bond, a ketal bond, an ester bond, and/or an oxime bond,
   - a bond susceptible to proteolysis, for example amide or peptide bond, preferably subject to proteolysis by Cathepsin B;
   - a red/ox-cleavable bond such as a disulfide bond, or a thiol-exchange reaction-susceptible bond such as a thio-ether bond;
more preferably wherein the first linker comprises a cleavable bond selected from:
   - a bond subject to cleavage under acidic conditions such as a semicarbazone bond, or a hydrazone bond, and/or
   - a bond susceptible to proteolysis, for example proteolysis by Cathepsin B, and/or
   - a bond susceptible for cleavage under reductive conditions such as a disulfide bond.

Advantageously, the bond is acid sensitive, i.e. subject to cleavage *in vivo* under acidic conditions present in endosomes and/or lysosomes of human cells, preferably of pH ≤ 6.5, preferably pH ≤ 6, more preferably pH ≤ 5.5, more preferably being an acid-sensitive bond selected an acid-sensitive bond selected from any one or more of: a semicarbazone bond, a hydrazone bond, an imine bond, an acetal bond including a 1,3-dioxolane bond, a ketal bond, an ester bond, and/or an oxime bond, even more preferably selected from a semicarbazone bond and a hydrazone bond; most preferably being a hydrazone bond.

It is preferred that such a cleavable bond is not susceptible, or only to a minor extent, to cleavage when the conjugate is present outside the endosome and lysosome of the cell, such as outside the cell or in the endocytosed vesicle after the conjugate engaged with an endocytic receptor by binding of the ligand to its target endocytic receptor on a muscle cell. For example, the cleavable bond is preferably less susceptible to cleavage when the conjugate is present in the circulation of a human subject and/or is present extracellularly in an organ of the human subject, compared to the susceptibility for cleavage of the bond when the conjugate is in the endosome or in the lysosome of a target cell, herein being the muscle cells.

In a particular embodiment, a composition for the disclosed herein therapeutic or prophylactic use or a conjugate according to the disclosure is provided, wherein the first linker and/or wherein the second linker is directly or indirectly covalently linked to the ligand.

In a further and also advantageous embodiment, a composition for the disclosed herein therapeutic or prophylactic use or a conjugate according to the disclosure is provided, wherein the covalent linking of the ligand within the conjugate is made via a third linker to which the ligand is covalently bound; preferably wherein the ligand comprises a chain of amino acid residues comprising at least one cysteine residue and/or at least one lysine residue and wherein the third linker comprises a covalent bond made with said at least one cysteine residue or said at least one lysine residue; more preferably wherein the ligand comprises a chain of amino acid residues comprising a multicysteine repeat, possibly being a tetracysteine repeat represented by the sequence HRWCCPGCCKTF (SEQ ID NO.4), and wherein the third linker comprises a covalent bond with any one or more of the cysteine residues of the multicysteine repeat; possibly wherein more than one third linker is linked to one molecule of the ligand via binding of each of the more than one third linker to a separate cysteine residue of the multicysteine repeat comprised by the ligand.

In a further possible embodiment, a composition for the disclosed herein therapeutic or prophylactic use or a conjugate according to the disclosure is provided, wherein the conjugate comprises a central linker for effectuating covalent linking between at least one molecule of the saponin, at least one molecule of the nucleic acid, and at least one molecule of the ligand, wherein said covalent linking is effectuated either directly, or via the first linker, the second linker, and/or the third linker, respectively; preferably wherein the central linker is a trifunctional linker.

For example, in a related embodiment a composition for the disclosed herein therapeutic or prophylactic use or a conjugate according to the disclosure is provided, wherein the central linker is a trifunctional linker that in its non-conjugated form is represented by Structure A:

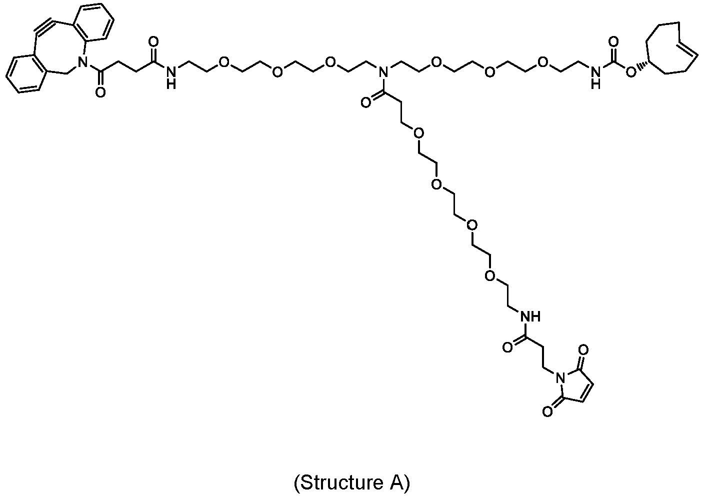

In a possible embodiment related to the above-described one, a composition for the disclosed herein therapeutic or prophylactic use or a conjugate according to the disclosure is provided, wherein the conjugate comprises 1-4 of the trifunctional linkers for every molecule of the ligand comprised by the conjugate, more preferably being 1-2 trifunctional linkers, most preferably being 1.2 - 1.8. trifunctional linkers on average.

In another related embodiment, a composition for the disclosed herein therapeutic or prophylactic use or a conjugate according to the disclosure is provided, wherein the trifunctional linker in its conjugated form is represented by Structure B: 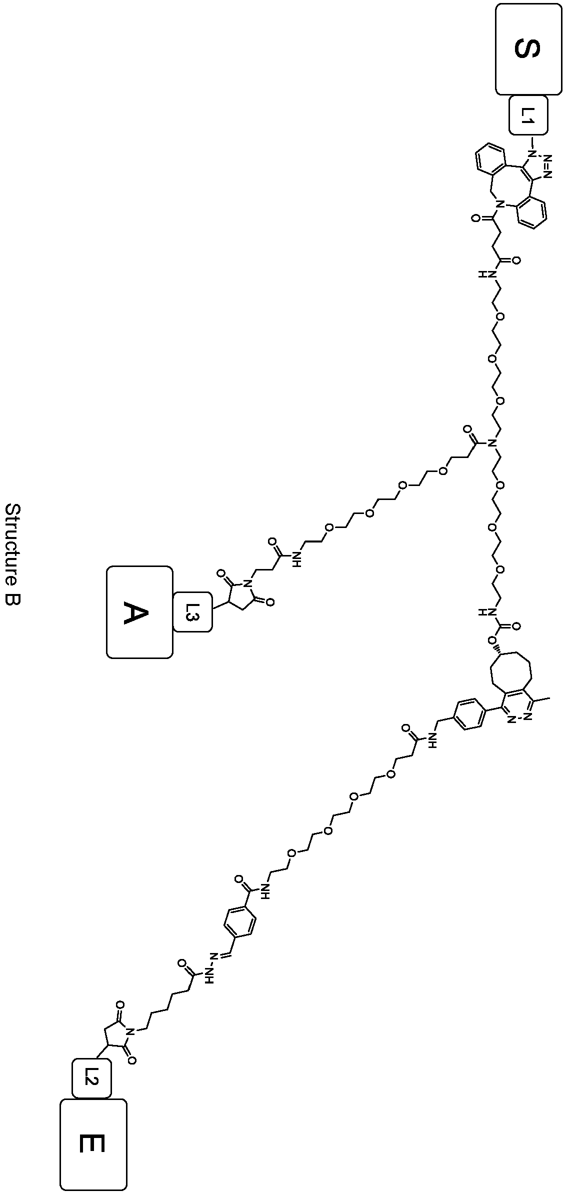 wherein:
S is the at least one molecule of the saponin, preferably selected from Group A, B or C,
L1 is the first linker to which said at least one molecule of the saponin is covalently bound,
E is at least one molecule of the nucleic acid,
L2 is the second linker to which said at least one molecule of the nucleic acid is covalently bound,

A is the least one molecule of the ligand, preferably being an antibody or a binding fragment thereof, possibly being one or more of single domain antibodies, and
L3 is the third linker to which said at least one molecule of the ligand is covalently bound,
wherein L1, L2 and L3 are the same or different.

In particular embodiments, L1 can optionally comprise the oligomeric or polymeric structure as described here above for certain embodiments, the structure preferably comprising PEG, more preferably selected from PEG3-PEG30, or a G2, G3 or G4 dendron.

In a particular embodiment, a conjugate is provided, wherein the at least one saponin is covalently bound via a thio-ether bond to a sulfhydryl group in one of the at least one ligand, preferably being an antibody or a binding fragment thereof, and/or in one of the at least one nucleic acid, preferably being an ASO, preferably via N-ε-maleimidocaproic acid hydrazide (EMCH) that is covalently bound to an aldehyde group in position C-23 of the aglycone core structure of the saponin and that is covalently bound to the sulfhydryl group in the ligand such as a sulfhydryl group of a cysteine.

In another particular embodiment, a conjugate is provided, wherein the at least one saponin is a bidesmosidic triterpene saponin or derivative thereof belonging to the type of a 12,13-dehydrooleanane with optionally an aldehyde function in position C-23 and comprising a glucuronic acid unit in a first saccharide chain bound at the C₃beta-OH group of the aglycone core structure of the saponin, wherein the saponin is covalently bound to an amino-acid residue of the ligand and/or of the at least one nucleic acid molecule via the carboxyl group of the glucuronic acid unit in the first saccharide chain, preferably via a linker, wherein the amino-acid residue preferably is selected from cysteine and lysine.

Different schematic representations of possible embodiments of the therapeutic muscle cell-targeting conjugates of the invention are shown and described in Figure 15 and its legend.

As explained above, one can envisage different exemplary embodiments of the disclosed herein muscle cell-targeting conjugates whereby different modes of conjugation of the saponin and/or the nucleic acid (preferably being an oligonucleotide) to the ligand and/or the oligomeric or polymeric structure (further termed scaffold) are envisaged. In line with the embodiments explained above, this conjugation can be either done via direct covalent bonding of at least two types of molecules comprised by the conjugate or made via linkers such as the described above first linker, second linker, and/or the third linker. A linker can be used to establish the covalent bonding of at least one molecule or at least one type of the molecules comprised by the conjugate to the remainder of the conjugate, or more linkers can be used for this purpose. In a particular embodiment, each of the distinct molecules comprised by the conjugate (i.e., the distinct molecules being the saponin, the nucleic acid, and the ligand) can be covalently bound to the remainder of the conjugate each by own linker (i.e., the first, the second, or the third linker, respectively), for example by being bound to a common scaffold. As explained above, in possible embodiments the linkers can be stable under the conditions present in the mammalian (e.g., human) endosomes/lysosomes, or labile (i.e., cleavable) under said conditions, the latter meaning that these linkers cleave in response to said conditions thus releasing the molecule covalently linked via such cleavable linker from the remainder of the conjugate.

Many examples of a cleavable first linker that covalently links the saponin within the conjugate are described above, including the described in more detail embodiments of cleavable first linkers bound to the saponin via an acid-sensitive bond at position C-23 of the saponin aglycone core, which acid-sensitive bonds have preferably been established by reacting the aldehyde group at position C-23 of such saponin's aglycone core and are configured to recover said group under the acidic conditions present in the mammalian (e.g., human) endosomes/lysosomes.

Alternatively, when receptor internalization rate is not a limiting factor, in other possible embodiments the first linker covalently linking the saponin within the conjugate of the invention can be a stable linker, which for example can be linked to the saponin via a glucuronic acid group, preferably and if present, via reacting with the glucuronic acid unit in a first saccharide chain bound at the C3beta-OH group of the aglycone core structure of the saponin. As a result of such reacting, a stable first linker comprising a stable (i.e., non cleavable) bond can be created at the first saccharide chain bound at the C3beta-OH group of the aglycone core structure of the saponin, the stable first linker covalently linking the saponin within the conjugate to e.g., the ligand (e.g., immunoglobulin like mAb, sdAb, VHH, etc.) or to the scaffold. In line with this, a possible embodiment is the conjugate as provided, wherein the saponin belongs to saponins comprising a glucuronic acid unit in the first saccharide chain at the C₃beta-OH group of the aglycone core structure of the saponin, wherein the glucuronic acid unit has been reacted to covalently bind a linker, preferably via an amide bond created at the first saccharide chain bound at the C3beta-OH group of the aglycone core structure of the saponin, more preferably to an amine group present in the ligand (such as an amine group of a lysine or an N-terminus of a proteinaceous ligand such as an immunoglobulin) or to the scaffold. The glucuronic acid function is particularly advantageous as it can be reacted to establish the covalent linking of the saponin to the ligand or to the scaffold of the conjugates of the invention, either via a direct covalent bond, or via a linker, wherein the linker is a stable linker, but can also be designed to be a cleavable linker.

The choice between the cleavable first linker and the stable first linker is entirely up to the skilled person and can be made e.g., depending on the choice of the ligand and the desired release rates for any of the distinct molecules comprised by the conjugate. A saponin conjugated via a cleavable first linker or a stable first linker can be part of any embodiment of the conjugate of the invention, for example an embodiment wherein the nucleic acid (e.g., oligonucleotide, preferably a PMO or ASO) is linked via either a cleavable second linker or a stable second linker. Examples of both stable and cleavable second linkers were provided above and are both very much possible for being used for conjugation of nucleic acids within the conjugates of the invention. The choice between them will very much depend on the type of nucleic acid used and the intended therapy. For example, a stable linker can very easily be used for conjugating just one of the strands of a therapeutic nucleic acid that will be designed to act in a single stranded form in the cell. Two strands of such therapeutic nucleic acids can be selected so as to dissociate in response to conditions present in the endosome/lysosome, thus releasing the therapeutic strand from the conjugate to enter the cytosol in a manner enhanced by the presence of the described herein endosomal-escape-enhancing saponin. For double stranded nucleic acids, in some embodiments, a cleavable second linker could be advantageous and can be considered, as either being conjugated to the ligand or being conjugated to the scaffold.

In a further particular embodiment, a composition for the disclosed herein therapeutic or prophylactic use or a conjugate according to the disclosure is provided, wherein the saponin is or comprises at least one molecule of SO1861, the nucleic acid is drisapersen or eteplirsen, and the ligand is anti-CD71 antibody or a binding fragment thereof.

In another embodiment, a composition for the disclosed herein therapeutic or prophylactic use is provided, for use in intravenous or subcutaneous administration to a human subject.

In a yet another embodiment, a composition for the disclosed herein therapeutic or prophylactic use or a conjugate according to the disclosure is provided comprising a pharmaceutically acceptable excipient and/or pharmaceutically acceptable diluent.

In a specific aspect, a composition or a conjugate according to the disclosure is provided for use as a medicament.

### EXAMPLES

### Materials:

SO1861 was isolated and purified by Extrasynthese, France and/or Analyticon Discovery GmbH, Germany from raw plant extract obtained from *Saponaria officinalis L.*

An antisense oligonucleotide with the sequence 5'-UCAAGGAAGAUGGCAUUUCU-3' [SEQ ID NO: 1], and an ASO with the same sequence and a thiol modification (DMD-ASO and 5'-thiol-DMD-ASO, respectively) were custom-made and purchased from Hanugen Therapeutics Pvt Ltd. A PMO with the sequence 5'-CTCCAACATCAAGGAAGATGGCATTTCTAG-3' (DMD-PMO or DMD-PMO(1)) [SEQ ID NO: 2], and a PMO with the same sequence with a disulfide amide modification (5'-disulfidamide-DMD-PMO or 5'-disulfideamide-DMD-PMO(1)), were custom-made and purchased from Gene Tools, LLC. A PMO with the sequence 5'-CTCCAACATCAAGGAAGATGGCATTTCTAG-3' (DMD-PMO(1)) [SEQ ID NO: 2] and a disulfide amide modification on the 3' (3'-disulfidamide-DMD-PMO(1)) was custom-made and purchased from Gene Tools. A PMO with the sequence 5'-GGCCAAACCTCGGCTTACCTGAAAT-3' (M23D) [SEQ ID NO: 3], and a PMO with the same sequence with a disulfide amide modification (3'-disulfideamide-M23D) were custom-made and purchased from Gene Tools, LLC. A PMO with the sequence 5'-GTGTCACCAGAGTAACAGTCTGAGTAGGAG-3' (DMD-PMO(2)) [SEQ ID NO: 16] and a disulfide amide modification on the 3' (3'-disulfidamide-DMD-PMO(2)) was custom-made and purchased from Gene Tools. A PMO with the sequence 5'-GGCAGTTTCCTTAGTAACCACAGGTTGTGT-3' (DMD-PMO(3)) [SEQ ID NO: 17] and a disulfide amide modification on the 3' (3'-disulfidamide-DMD-PMO(3)) was custom-made and purchased from Gene Tools. A PMO with the sequence 5'-GTTGCCTCCGGTTCTGAAGGTGTTC-3' (DMD-PMO(4)) [SEQ ID NO: 18] and a disulfide amide modification on the 3' (3'-disulfidamide-DMD-PMO(4)) was custom-made and purchased from Gene Tools. A PMO with the sequence 5'-CCTCCGGTTCTGAAGGTGTTC-3' (DMD-PMO(5)) [SEQ ID NO: 19] and a disulfide amide modification on the 3' (3'-disulfidamide-DMD-PMO(5)) was custom-made and purchased from Gene Tools.

Anti-CD71 antibody (clone OKT9) targeting human CD71 (hCD71) and anti-CD71 antibody (clone R17 217.1.3) targeting murine (mCD71) were both purchased from BioXCell. Anti-CD63 antibody (clone H5C6) targeting human CD63 (hCD63) and anti-CD63 antibody (clone NVG-2) targeting murine (mCD63) were both purchased from Biolegend. IGF-1 ligand was purchased from PeproTech.

Tris(2-carboxyethyl)phosphine hydrochloride (TCEP, 98%, Sigma-Aldrich), 5,5-Dithiobis(2-nitrobenzoic acid) (DTNB, Ellman's reagent, 99%, Sigma-Aldrich), Zeba^{™} Spin Desalting Columns (2 mL, Thermo-Fisher), NuPAGE^{™} 4-12% Bis-Tris Protein Gels (Thermo-Fisher), NuPAGE^{™} MES SDS Running Buffer (Thermo-Fisher), Novex^{™} Sharp Pre-stained Protein Standard (Thermo-Fisher), PageBlue^{™} Protein Staining Solution (Thermo-Fischer), Pierce^{™} BCA Protein Assay Kit (Thermo-Fisher), N-Ethylmaleimide (NEM, 98%, Sigma-Aldrich), 1,4-Dithiothreitol (DTT, 98%, Sigma-Aldrich), Sephadex G25 (GE Healthcare), Sephadex G50 M (GE Healthcare), Superdex 200P (GE Healthcare), Isopropyl alcohol (IPA, 99.6%, VWR), Tris(hydroxymethyl)aminomethane (Tris, 99%, Sigma-Aldrich), Tris(hydroxymethyl)aminomethane hydrochloride (Tris.HCL, Sigma-Aldrich), L-Histidine (99%, Sigma-Aldrich), D-(+)-Trehalose dehydrate (99%, Sigma-Aldrich), Polyethylene glycol sorbitan monolaurate (TWEEN 20, Sigma-Aldrich), Dulbecco's Phosphate-Buffered Saline (DPBS, Thermo-Fisher), Guanidine hydrochloride (99%, Sigma-Aldrich), Ethylenediaminetetraacetic acid disodium salt dihydrate (EDTA-Na₂, 99%, Sigma-Aldrich), sterile filters 0.2 µm and 0.45 µm (Sartorius), Vivaspin T4 and T15 concentrator (Sartorius), Superdex 200PG (GE Healthcare), Tetra(ethylene glycol), Dimethyl sulfoxide (DMSO, 99%, Sigma-Aldrich), N-(2-Aminoethyl)maleimide trifluoroacetate salt (AEM, 98%, Sigma-Aldrich), L-Cysteine (98.5%, Sigma-Aldrich), deionized water (DI) was freshly taken from Ultrapure Lab Water Systems (MilliQ, Merck), Nickel-nitrilotriacetic acid agarose (Ni-NTA agarose, Protino), Glycine (99.5%, VWR), 5,5-Dithiobis(2-nitrobenzoic acid (Ellman's reagent, DTNB, 98 %, Sigma-Aldrich), S-Acetylmercaptosuccinic anhydride Fluorescein (SAMSA reagent, Invitrogen) Sodium bicarbonate (99.7%, Sigma-Aldrich), Sodium carbonate (99.9%, Sigma-Aldrich), PD MiniTrap desalting columns with Sephadex G-25 resin (GE Healthcare), PD10 G25 desalting column (GE Healthcare), Zeba Spin Desalting Columns in 0.5, 2, 5, and 10 mL (Thermo-Fisher), Vivaspin Centrifugal Filters T4 10 kDa MWCO, T4 100 kDa MWCO, and T15 (Sartorius), Biosep s3000 aSEC column (Phenomenex), Vivacell Ultrafiltration Units 10 and 30 kDa MWCO (Sartorius), Nalgene Rapid-Flow filter (Thermo-Fisher), dichlormethan (Sigma-Aldrich), methanol (Sigma-Aldrich), diethyl ether (Sigma-Aldrich), acetonitrile (Sigma-Aldrich), Pyridine 2-thione (Sigma-Aldrich), Goat anti-Human IgG - HRP (Southern Biotech), Goat anti-Human Kappa - HRP (Southern Biotech), Tris concentrate (Thermo-Fisher), MOPS running buffer (20x, Thermo-Fisher), LDS sample buffer (4x, Thermo-Fisher), TBS Blocking Buffer (Thermo-Fisher), Tris (Tris(hydroxymethyl)aminomethane, Merck), Tris HCl (Sigma-Aldrich), Minisart RC15 0.2 µm filter (Sartorius), Minisart 0.45 µm filter (Sartorius), PD Minitrap G25 (Cytiva), TNBS (2,4,6-trinitrobenzene sulfonic acid, Sigma-Aldrich), Sodium Dodecyl Sulfate (SDS, Sigma-Aldrich), SMCC (succinimidyl 4-(N-maleimidomethyl)cyclohexane-1-carboxylate, Thermo-Fisher), THPP (Tris(hydroxypropyl)phosphine, Sigma-Aldrich), DBCO-NHS (CAS 1353016-71-3, BroadPharm), PEG4-SPDP (2-Pyridyldithiol-tetraoxatetradecane-N-hydroxysuccinimide, Thermo-Fisher), Novex^{™} TBE-Urea Gels, 15% (Thermo-Fisher), TBE buffer (ris-Borat-EDTA, Thermo-Fisher), GlyCLICK^{™} (10 mg) Azide Activation Kit (Genovis), and Immobilized GlycINATOR^{™} column (from GlyCLICK^{™} Azide Activation Kit), (Genovis) were used.

### Materials related to hCD71 conjugates

| **Description** | **Supplier** |
|---|---|
| hCD71 human anti-CD71 antibody | BioXcell |
| SO1861-SC-Maleimide | Symeres |
| DMD-PMO(1)-5'-SS-amide (5'-DMD-PMO(1)) | Gene Tools |
| DMD-PMO(1)-SS-amide (3'-DMD-PMO(1)) | Gene Tools |
| DMD-PMO(2)-SS-amide (3'-DMD-PMO(2)) | Gene Tools |
| DMD-PMO(3)-SS-amide (3'-DMD-PMO(3)) | Gene Tools |
| DMD-PMO(4)-SS-amide (3'-DMD-PMO(4)) | Gene Tools |
| DMD-PMO(5)-SS-amide (3'-DMD-PMO(5)) | Gene Tools |
| DMD-ASO-thiol (5'-DMD-ASO) | Hanugen Therapeutics Pvt Ltd |
| Goat anti-Human IgG - HRP | Southern Biotech |
| Goat anti-Human Kappa - HRP | Southern Biotech |
| Conjugation buffer (TBS pH 7.5) | Fleet Bioprocessing Ltd |
| Dulbecco's PBS pH 7.5 | Fleet Bioprocessing Ltd |
| SEC analysis buffer (DPBS:IPA 85:15) | Fleet Bioprocessing Ltd |
| 6M Guanidine.HCl | Fleet Bioprocessing Ltd |
| 0.5M NaOH | Fleet Bioprocessing Ltd |
| PBS-T (Dulbecco's PBS pH 7.5 with Tween-20) | Fleet Bioprocessing Ltd |
| MOPS running buffer (20X) | Fleet Bioprocessing Ltd |
| LDS sample buffer (4X) | Thermo |
| NuPAGE Transfer Buffer (10X) | Fleet Bioprocessing Ltd |
| TBS Blocking Buffer | Thermo |
| TCEP | Thermo |
| THPP | Sigma |
| DTT | Sigma |
| PEG4-SPDP | Thermo |
| NEM | Sigma |
| Glycine | VWR |
| DMSO | Sigma |
| Ellman's reagent | Thermo |
| 0.2µm Minisart Filter | Sartorius |
| 0.45µm Minisart Filter | Sartorius |
| 0.2µm Spin X Filter | Corning |
| Zeba 2ml 7K MWCO | Thermo |
| Zeba 10ml 40K MWCO | Thermo |
| PD10 G25 Sephadex | Cytiva |
| 2.6 × 30cm Sephadex G25 | Fleet Bioprocessing Ltd |
| 2.6 × 40cm Superdex 200 | Fleet Bioprocessing Ltd |
| 2.6 × 60cm Superdex 200 | Fleet Bioprocessing Ltd |
| Vivaspin 20 (10K MWCO) | Sartorius |
| Vivaspin T15 (10K MWCO) | Sartorius |
| Vivacell 100 (10K MWCO) | Sartorius |
| Novex protein standards ladder | Life Technologies |
| 4-12% Bis-Tris gel | Thermo |
| Nitrocellulose membrane | Thermo |
| PageBlue protein stain | Thermo |
| Ultra TMB Blotting Solution | Thermo |
| BCA assay kit | Thermo |
| 2mg/ml BGG standard | Thermo |
| Biosep s3000 aSEC column | Phenomenex |

### Materials related to hCD63 conjugates

| **Description** | **Supplier** |
|---|---|
| hCD63 human anti-CD63 antibody | Biolegend |
| SO1861-SC-Maleimide | Symeres |
| DMD-PMO(1)-SS-amide (3'-DMD-PMO(1)) | Gene Tools |
| DMD-PMO(2)-SS-amide (3'-DMD-PMO(2)) | Gene Tools |
| DMD-PMO(3)-SS-amide (3'-DMD-PMO(3)) | Gene Tools |
| DMD-PMO(4)-SS-amide (3'-DMD-PMO(4)) | Gene Tools |
| DMD-ASO-thiol (5'-DMD-ASO) | Hanugen Therapeutics Pvt |
| Goat anti-Human IgG - HRP | Southern Biotech |
| Goat anti-Human Kappa - HRP | Southern Biotech |
| Conjugation buffer (TBS pH 7.5) | Fleet Bioprocessing Ltd |
| Presentation buffer (Dulbecco's PBS pH 7.5) | Fleet Bioprocessing Ltd |
| SEC analysis buffer (DPBS:IPA 85:15) | Fleet Bioprocessing Ltd |
| 6M Guanidine.HCl | Fleet Bioprocessing Ltd |
| PBS-T | Fleet Bioprocessing Ltd |
| MOPS running buffer (20X) | Fleet Bioprocessing Ltd |
| LDS sample buffer (4X) | Thermo |
| Transfer Buffer (1X) | Fleet Bioprocessing Ltd |
| TBS Blocking Buffer | Thermo |
| TCEP | Thermo |
| THPP | Sigma |
| DTT | Sigma |
| PEG4-SPDP | Thermo |
| NEM | Sigma |
| Glycine | VWR |
| DMSO | Thermo |
| | Sigma |
| Ellman's reagent | Thermo |
| 0.2µm Minisart Filter | Sartorius |
| 0.2µm Spin Filter | Millipore |
| Zeba 2ml 7K MWCO | Thermo |
| PD10 G25 Sephadex | Cytiva |
| 2.6 × 30 cm Sephadex G25 | Fleet Bioprocessing Ltd |
| 2.6 × 40 cm Superdex 200 | Fleet Bioprocessing Ltd |
| 2.6 × 60 cm Superdex 200 | Fleet Bioprocessing Ltd |
| Vivaspin 20 (10K MWCO) | Sartorius |
| Vivaspin T15 (10K MWCO) | Sartorius |
| Novex protein standards ladder | Life Technologies |
| 4-12% Bis-Tris gel | Thermo |
| Nitrocellulose membrane | Thermo |
| PageBlue protein stain | Thermo |
| Ultra TMB Blotting Solution | Thermo |
| BCA assay kit | Thermo |
| 2mg/ml BGG standard | Thermo |
| Biosep s3000 aSEC column | Phenomenex |

### Abbreviations

- Ab: Antibody
- Ac: Acetyl
- AON: Antisense oligonucleotide
- ASO: Antisense oligonucleotide
- BCA: Bicinchoninic acid
- BGG: Bovine gamma globulin
- aSEC: Analytical size exclusion chromatography
- DAR: Drug-antibody ratio
- DBCO: Dibenzocyclooctyne
- DBCO-NHS: Dibenzocyclooctyne- N-hydroxysuccinimide ester
- DCM: Dichloromethane
- DIPEA: N,N-diisopropylethylamine
- DMEM: Dulbecco's modified Eagles medium
- DMF: N,N-dimethylformamide
- DMSO: Dimethylsulfoxide
- DPBS: Dulbecco's phosphate-buffered saline
- DTME: Dithiobismaleimidoethane
- DTNB: 5,5'-dithiobis-(2-nitrobenzoic acid)
- DTT: Dithiothreitol
- EDTA: Ethylenediaminetetraacetic acid
- EDCl.HCl: 1-(3-Dimethylaminopropyl)-3-ethylcarbodiimide hydrochloride
- EMCH.TFA: N-(ε-maleimidocaproic acid) hydrazide, trifluoroacetic acid salt
- Equiv.: Equivalent
- EtBr: Ethidium bromide
- FBS: Fetal bovine serum
- GaIT: Galactose-1-phosphate uridylyl transferase
- HATU: 1-[Bis(dimethylamino)methylene]-1H-1,2,3-triazolo[4,5-b]pyridinium 3-oxide hexafluorophosphate
- HRP: Horse-radish peroxidase
- IPA: Isopropyl alcohol
- LC-MS: Liquid chromatography - mass spectrometry
- LDS: Lithium dodecyl sulfate
- LRMS: Low resolution mass spectrometry
- NEM: N-ethylmaleimide
- NHS: N-hydroxy succinimide ester
- mAb: Monoclonal antibody
- min: Minutes
- MOPS: 3-(Morpholin-4-yl)propane-1-sulfonic acid

- MPLC: Medium pressure liquid chromatography
- MWCO: Molecular weight cut-off
- NMM: 4-Methylmorpholine
- PBS: Phosphate-buffered saline
- PBS-T: Phosphate-buffered saline with Tween-20
- PEG4-SPDP: 2-Pyridyldithiol-tetraoxatetradecane-N-hydroxysuccinimide
- PMO: Phosphorodiamidate Morpholino Oligomer
- PDT: Pyridine 2-thione
- rpm: Revolutions per minute
- RT: Room temperature
- r.t.: Retention time
- SDS: Sodium dodecyl sulfate
- SEC: Size exclusion chromatography
- SMCC: Succinimidyl 4-(N-maleimidomethyl)cyclohexane-1-carboxylate
- TBEU: (Tris-(hydroxymethyl)-aminomethane)-Borate-EDTA-rea
- TBS: Tris buffered saline
- TCEP: Tris(2-carboxyethyl)phosphine hydrochloride
- TCO4 - NHS: Trans-Cyclooctene - N-hydroxy succinimide
- Temp: Temperature
- TFA: Trifluoroacetic acid
- THPP: Tris(hydroxypropyl)phosphine
- TMB: 3,3',5,5'-tetramethylbenzidine
- TNBS: 2,4,6-trinitrobenzene sulfonic acid
- Tris: Tris(hydroxymethyl)aminomethane
- UDP-GalNAz: Uridine diphosphate-N-azidoacetylgalactosamine

### Methods (as performed in Examples 1 - 6):

### SO1861-Maleimides, SO1861-NHS synthesis

SO1861 was from *Saponaria officinalis L* (Extrasynthese, France and/or Analyticon Discovery GmbH, Germany), and was coupled to respective handles by Symeres (NL), according to methods known in the art.

### Conjugation of SO1861 to antibodies and proteins

Custom production of IGF-1-SO1861, mCD71-SO1861 and hCD71-SO1861 was performed by Fleet Bioprocessing (UK).

### Conjugation of 5'-disulfidamide-DMD-PMO, 5'-thiol-DMD-ASO, and 3'-disulfidamide-M23D to antibodies

Custom production of mCD71-M23D, mCD71-M23D-SO1861, and hCD71-DMD-ASO, hCD71-DMD-PMO, hCD71-DMD-ASO-SO1861, and hCD71-DMD-PMO-SO1861 was performed by Fleet Bioprocessing (UK).

### Analytical and Preparative methods

### LC-MS method 1

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: neg or neg/pos within in a range of 1500-2400 or 2000-3000; ELSD: gas pressure 40 psi, drift tube temp: 50°C; column: Acquity C18, 50×2.1 mm, 1.7 µm Temp: 60°C, Flow: 0.6 mL/min, lin. Gradient depending on the polarity of the product:
^{A}t₀ = 2% A, t_{5.0min} = 50% A, t_{6.0min} = 98% A
^{B}t₀ = 2% A, t_{5.0min} = 98% A, t_{6.0min} = 98% A
Posttime: 1.0 min, Eluent A: acetonitrile, Eluent B: 10 mM ammonium bicarbonate in water (pH=9.5).

### LC-MS method 2

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50°C; column: Waters XSelect^{™} CSH C18, 50×2.1 mm, 2.5 µm, Temp: 25°C, Flow: 0.5 mL/min, Gradient: t₀ₘᵢₙ = 5% A, t_{2.0min} = 98% A, t_{2.7min} = 98% A, Posttime: 0.3 min, Eluent A: acetonitrile, Eluent B: 10 mM ammonium bicarbonate in water (pH=9.5).

### LC-MS method 3

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product pos/neg 105-800, 500-1200 or 1500-2500; ELSD: gas pressure 40 psi, drift tube temp: 50°C; column: Waters XSelect^{™} CSH C18, 50×2.1mm, 2.5µm, Temp: 40°C, Flow: 0.5 mL/min, Gradient: t₀ₘᵢₙ = 5% A, t_{2.0min} = 98% A, t_{2.7min} = 98% A, Posttime: 0.3 min, Eluent A: 0.1% formic acid in acetonitrile, Eluent B: 0.1% formic acid in water.

### LC-MS method 4

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50°C column: Waters Acquity Shield RP18, 50×2.1 mm, 1.7 µm, Temp: 25°C, Flow: 0.5 mL/min, Gradient: t₀ₘᵢₙ = 5% A, t_{2.0min} = 98% A, t_{2.7min} = 98% A, Posttime: 0.3 min, Eluent A: acetonitrile, Eluent B: 10 mM ammonium bicarbonate in water (pH = 9.5).

### Preparative MP-LC method 1

Instrument type: Reveleris^{™} prep MPLC; column: Waters XSelect^{™} CSH C18 (145×25 mm, 10µm); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient:
^{A}t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B
^{A}t₀ₘᵢₙ = 5% B, t₁ₘᵢₙ = 5% B, t₂ₘᵢₙ = 20% B, t₁₇ₘᵢₙ = 60% B, t₁₈ₘᵢₙ = 100% B, t₂₃ₘᵢₙ = 100% B; Detection UV: 210, 235, 254 nm and ELSD.

### Preparative MP-LC method 2

Instrument type: Reveleris^{™} prep MPLC; Column: Phenomenex LUNA C18(3) (150×25 mm, 10µm); Flow: 40 mL/min; Column temp: room temperature; Eluent A: 0.1% (v/v) Formic acid in water, Eluent B: 0.1% (v/v) Formic acid in acetonitrile; Gradient:
^{A}t₀ₘᵢₙ = 5% B, t₁ₘᵢₙ = 5% B, t₂ₘᵢₙ = 20% B, t₁₇ₘᵢₙ **=** 60% B, t₁₈ₘᵢₙ = 100% B, t₂₃ₘᵢₙ = 100% B
^{B}t₀ₘᵢₙ = 2% B, t₁ₘᵢₙ = 2% B, t₂ₘᵢₙ = 2% B, t₁₇ₘᵢₙ = 30% B, t₁₈ₘᵢₙ = 100% B, t₂₃ₘᵢₙ = 100% B
^{C}t₀ₘᵢₙ = 5% B, t₁ₘᵢₙ **=** 5% B, t₂ₘᵢₙ = 10% B, t₁₇ₘᵢₙ **=** 50% B, t₁₈ₘᵢₙ = 100% B, t₂₃ₘᵢₙ = 100% B
^{D}t₀ₘᵢₙ = 5% B, t₁ₘᵢₙ **=** 5% B, t₂ₘᵢₙ = 5% B, t₁₇ₘᵢₙ = 40% B, t₁₈ₘᵢₙ = 100% B, t₂₃ₘᵢₙ = 100% B; Detection UV : 210, 235, 254 nm and ELSD.

### Preparative LC-MS method 3

MS instrument type: Agilent Technologies G6130B Quadrupole; HPLC instrument type: Agilent Technologies 1290 preparative LC; Column: Waters XSelect^{™} CSH (C18, 150×19mm, 10µm); Flow: 25 ml/min; Column temp: room temperature; Eluent A: 100% acetonitrile; Eluent B: 10 mM ammonium bicarbonate in water pH=9.0; Gradient:
^{A}t₀ = 20% A, t_{2.5min} = 20% A, t₁₁ₘᵢₙ = 60% A, t₁₃ₘᵢₙ = 100% A, t₁₇ₘᵢₙ = 100% A
^{B}t₀ = 5% A, t_{2.5min} = 5% A, t₁₁ₘᵢₙ = 40% A, t₁₃ₘᵢₙ = 100% A, t₁₇ₘᵢₙ = 100% A; Detection: DAD (210 nm); Detection: MSD (ESI pos/neg) mass range: 100 - 800; Fraction collection based on DAD.

### Preparative LC-MS method 4

MS instrument type: Agilent Technologies G6130B Quadrupole; HPLC instrument type: Agilent Technologies 1290 preparative LC; Column: Waters XBridge Protein (C4, 150×19mm, 10µm); Flow: 25 ml/min; Column temp: room temperature; Eluent A: 100% acetonitrile; Eluent B: 10 mM ammonium bicarbonate in water pH=9.0; Gradient:
^{A}t₀ = 2% A, t_{2.5min} = 2% A, t₁₁ₘᵢₙ = 30% A, t₁₃ₘᵢₙ = 100% A, t₁₇ₘᵢₙ = 100% A
^{B}t₀ = 10% A, t_{2.5min} = 10% A, t₁₁ₘᵢₙ = 50% A, t₁₃ₘᵢₙ = 100% A, t₁₇ₘᵢₙ = 100% A
^{C}t₀ = 5% A, t_{2.5min} = 5% A, t₁₁ₘᵢₙ = 40% A, t₁₃ₘᵢₙ = 100% A, t₁₇ₘᵢₙ = 100% A; Detection: DAD (210 nm); Detection: MSD (ESI pos/neg) mass range: 100 - 800; Fraction collection based on DAD

### Flash chromatography

Grace Reveleris X2^{™} C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50 bar (725 psi); Detection: UV 200-400 nm, combination of up to 4 UV signals and scan of entire UV range, ELSD; Column sizes: 4-330g on instrument, luer type, 750g up to 3000g with optional holder.

### UV-vis spectrophotometry

Concentrations were determined using either a Thermo Nanodrop 2000 spectrometer or Perkin Elmer Lambda 365 Spectrophotometer and the following mass Extinction Coefficient (EC) values: Experimentally determined molar ε495 = 58,700 M-1 cm-1 and Rz280:495 = 0.428 were used for SAMSA-fluorescein.
M23D-SS-amide; mass EC265 = 259,210 M-1 cm-1
Ellman's reagent (TNB); molar EC412 = 14,150 M-1 cm-1
DMD-PMO; molar EC265 = 318,050 135,027 M-1 cm-1
DMD-ASO; molar EC265 = 310,000 252,512 M-1 cm-1
Pyridine 2-thione (PDT); molar ε363 = 8,080 M-1 cm-1

### TNBS assay

Glycine standards (0, 2.5, 5, 10, 15 and 20 µg/ml) were freshly prepared using DPBS pH 7.5. TNBS assay reagent was prepared by combining TNBS (40 µl) and DPBS pH 7.5 (9.96 ml). 10% w/v SDS prepared using DI water. For the assay, 60 µl of each sample (singlicate) and standard (triplicate) was plated out. To each well was added TNBS reagent (60 µl) and the plate shaker-incubated for 3 hours at 37°C and 600rpm. After, 50 µl of 10% SDS and 25 µl 1M HCl was added and the plate was analysed at 340 nm. SO1861-hydrazone-NHS incorporation was determined by depletion of lysine concentration of conjugate with respect to unmodified protein.

### SEC

The conjugates were analysed by SEC using an Akta purifier 10 system and Biosep SEC-s3000 column eluting with DPBS:IPA (85:15). Conjugate purity was determined by integration of the conjugate peak with respect to impurities/aggregate forms.

### SDS-PAGE and Western Blotting

Native proteins and conjugates were analysed under heat denaturing non-reducing and reducing conditions by SDS-PAGE against a protein ladder using a 4-12% bis-tris gel and MOPS as running buffer (200V, ~40 minutes). Samples were prepared to 0.5 mg/ml, comprising LDS sample buffer and MOPS running buffer as diluent. For reducing samples, DTT was added to a final concentration of 50mM. Samples were heat treated for 2 minutes at 90-95°C and 5 µg (10 µl) added to each well. Protein ladder (10 µl) was loaded without pre-treatment. Empty lines were filled with 1× LDS sample buffer (10 µl). After the gel was run, it was washed thrice with DI water (100 ml) with shaking (15 minutes, 200 rpm). Coomassie staining was performed by shaker-incubating the gel with PAGEBlue protein stain (30 ml) (60 minutes, 200 rpm). Excess staining solution was removed, rinsed twice with DI water (100 ml) and destained with DI water (100 ml) (60 minutes, 200 rpm). The resulting gel was imaged and processed using ImageJ (ImageJ (Rasband, W.S., ImageJ, U. S. National Institutes of Health, Bethesda, Maryland, USA) and MyCurveFit (point-to-point correlation of protein ladder).

### Western Blotting

From SDS-PAGE, the gel was transferred to nitrocellulose membrane using the X-Cell blot module with the following setup ((-)BP-BP-FP-Gel-NC-BP-BP-BP(+)) and conditions (30V, 60 minutes) using freshly prepared transfer buffer. BP - blotting pad; FP - Filter pad; NC - Nitrocellulose membrane. After that, the NC were washed thrice with PBS-T (100 ml) with shaking (5 minutes, 200 rpm), non-specific sites blocked with blocking buffer (50 ml) with shaking (30 minutes, 200 rpm) then active sites labelled with a combination of Goat anti-Human Kappa - HRP (1:2000) and Goat anti-Human IgG - HRP (1:2000) (50 ml) diluted in blocking buffer with shaking (30 minutes, 200 rpm). After that, the NC was washed once with PBS-T (100 ml) with shaking (5 minutes, 200 rpm) and complexed antibody detected with freshly prepared, freshly filtered CN/DAB substrate (25 ml). Colour development was observed visually, and after 2 minutes development was stopped by washing the NC with water, and the resulting blot photographed.

### TBEU-PAGE

Oligonucleotide conjugates and oligonucleotide standards were analysed under heat denaturing, non-reducing conditions by TBE-Urea PAGE against an oligo ladder using a 15% TBE-Urea gel and TBE as running buffer (180V, ~60 minutes). Samples were prepared to 0.5 mg/ml, and standards were prepared to 50 to 5 µg/ml, respectively, all comprising TBE Urea sample buffer and purified H₂O as diluent. Samples and standards were heat treated for 3 minutes at 70°C and 10 µl added to each well, equating to 5 µg of protein and conjugate samples, and 0.5/0.2/0.1/0.5 µg (DMD-ASO) or 0.2/0.1/0.05 µg (DMD-PMO) of oligonucleotide, per lane. Oligo ladder reconstituted to 0.1 µg/band/ml in TE pH 7.5 (2 µl) was loaded without pre-treatment. After the gel was run, it was stained with freshly prepared ethidium bromide solution (1 µg/ml) with shaking (40 minutes, 200 rpm). The resulting gel was visualised by UV epi-illumination (254nm), imaged and processed using ImageJ (Rasband, W.S., ImageJ, U. S. National Institutes of Health, Bethesda, Maryland, USA).

### mCD71-M23D

An aliquot of mCD71 (42.9 mg, 4.20 ml) was buffer exchanged into DPBS pH 7.5 and normalised to 2.5 mg/ml. To an aliquot of mCD71 (34.4 mg, 0.23 µmol, 2.53 mg/ml) was added an aliquot of freshly prepared SMCC solution (2.0 mg/ml, 3.53 mole equivalents, 0.81 µmol), the mixture vortexed briefly then incubated for 60 minutes at 20°C with roller-mixing. After incubation, the reaction was quenched by the addition of an aliquot of a freshly prepared glycine solution (2.0 mg/ml, ~20 mole equivalents, 4.05 µmol), the mixture vortexed briefly then incubated for >15 minutes at 20 °C with roller-mixing. The conjugate was purified by Superdex 200 column eluting with TBS pH 7.5 and analysed by UV-vis to give purified mCD71-SMCC (31.7 mg, yield: 96%, 0.942 mg/ml, SMCC to mCD71 ratio = 2.1).

Separately, an aliquot of M23D-SS-amide (17.2 mg, 1.99 µmol, 10.0 mg/ml) reconstituted using TBS pH 7.5 was added an aliquot of freshly prepared THPP solution (50 mg/ml, 10 mole equivalents, 19.9 µmol, 82.8 µl), the mixture vortexed briefly then incubated for 60 minutes at 37°C with roller-mixing. After incubation, the oligo was purified by PD10 Sephadex G25M column eluting with TBS pH 7.5, to afford M23D-SH (14.8 mg, yield: 86%, thiol to M23D ratio = 0.98).

To an aliquot of mCD71-SMCC (31.7 mg, 0.21 µmol, 0.942 mg/ml) was added an aliquot of M23D-SH (4.122 mg/ml, 4.0 mole equivalents, 0.85 µmol, 1.771 ml), the mixture vortexed briefly then incubated at 20°C with roller-mixing. After ca. 72 hours, the conjugate mixture was concentrated and purified by Superdex 200PG column eluting with DPBS pH 7.5 to give purified mCD71-M23D conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a mCD71-M23D conjugate (total yield = 25.7 mg, 73%, M23D to mCD71 ratio = 1.2).

### mCD71-SO1861

For the generation of mCD71-SO1861, either SO1861-SC-Maleimide or SO1861-EMCH has been used to generate two different mCD71-SO1861 conjugates. The procedure is exemplary described for the conjugation between mCD71 and SO1861-EMCH.

An aliquot mCD71 (55.1 mg, 0.37 µmol, 8.10 mg/ml, 6.80 ml) was normalised to 5 mg/ml with DPBS pH 7.5 and then was added 30 µl/ml (330 µl) of a pre-mixed Tris/Tris.HCl/EDTA concentrate comprising Tris concentrate (127 mg/ml, 1.05M), Tris.HCl concentrate (623 mg/ml, 3.95 M) and EDTA.2Na.2H₂O concentrate (95 mg/ml, 0.26M) combined 1:1:1 v/v, to give a 50 mM TBS, 2.5 mM EDTA buffer pH ~7.5. To mCD71 (50 mg, 0.33 µmol, 5.044 mg/ml) was added an aliquot of freshly prepared TCEP solution (2.00 mg/ml, 2.74 mole equivalents, 0.912 µmol), the mixture vortexed briefly then incubated for 210 minutes at 20°C with roller-mixing. After incubation (prior to addition of SO1861-EMCH), the mCD71-SH was dispensed out for multiple conjugations and a 1.0 mg (0.201 ml) aliquot was removed and purified by gel filtration using Zeba spin desalting column into TBS pH 7.5. This aliquot was characterised by UV-vis analysis and Ellman's assay (3.248 mg/ml, thiol to mCD71 ratio = 3.97). To an aliquot of mCD71-SH (42 mg, 0.28 µmol, 4.978 mg/ml) was added an aliquot of freshly prepared SO1861-EMCH solution (2.0 mg/ml, 8 mole equivalents, 2.24 µmol, 2.32 ml), the mixture vortexed briefly then incubated for 120 minutes at 20°C. Besides the conjugation reaction, two aliquots of desalted mCD71-SH (0.25 mg, 0.077 ml, 1.67 nmol) were reacted with NEM (8.00 equivalents, 134 nmol, 6.7 µl of a 0.25 mg/ml solution) or TBS pH 7.5 buffer (6.7 µl) for 120 minutes at 20°C, as positive and negative controls, respectively. After incubation, a *ca.* 0.4 mg aliquot of mCD71-SO8161 mixture was removed, purified by gel filtration using Zeba spin desalting column into TBS pH 7.5 and characterised by Ellman's assay alongside positive and negative controls to obtain SO1861 incorporation. To the bulk mCD71-SO1861 mixture was added an aliquot of freshly prepared NEM solution (5 mole equivalents, 1.40 µmol, 70.1 µl of a 2.5 mg/ml solution) to quench the reaction. The conjugate was purified by Superdex 200 column eluting with DPBS pH 7.5 to give purified mCD71-SO1861 conjugate. The product was concentrated then normalised to 2.5 mg/ml, filtered through 0.2 µm and then dispensed into aliquots for characterisation, product testing and further conjugation. The result was mCD71-SO1861 conjugate (total yield = 37.3 mg, 89%, SO1861 to mCD71 ratio = 3.8).

### IGF-1-SO1861

IGF-1 (4 mg) was dissolved in DPBS pH 7.5 (1.60 ml). To IGF-1 (3.88 mg, 0.51 µmol, 4.821 mg/ml) was added an aliquot of freshly prepared SO1861-hydrazone-NHS solution (2.0 mg/ml, 5 mole equivalents, 2.53 µmol, 3.80 ml), the mixture vortexed briefly then incubated for 60 minutes at 20°C. After conjugation, an aliquot of a freshly prepared glycine solution (25 equivalents, 13 µmol, 95 µl of a 10 mg/ml solution) was added and then the conjugate was purified by Zeba 10ml 7K MWCO spin desalting column eluting with DPBS pH 7.5 to give purified IGF-1-SO1861 conjugate. The aliquot was analysed by BCA colorimetric assay to ascertain a new EC value, then concentrated by centrifugal filtration (3,000 g, 20°C, 10-minute intervals), normalised to 2.5 mg/ml, filtered through 0.2 µm and dispensed into aliquots for characterisation and customer testing. The result was IGF-1-SO1861 conjugate (total yield = 2.83 mg, 73%, SO1861 to IGF-1 ratio = 2.7).

### mCD71-M23D-SO1861

To an aliquot of mCD71-SO1861 with a DAR3.8 (20 mg, 0.126 µmol, 2.53 mg/ml) was added an aliquot of freshly prepared SMCC solution (2.0 mg/ml, 3.53 mole equivalents, 0.447 µmol), the mixture vortexed briefly then incubated for 60 minutes at 20°C with roller-mixing. After incubation, the reaction was quenched by the addition of an aliquot of a freshly prepared glycine solution (2.0 mg/ml, ~20 mole equivalents, 2.5 µmol), the mixture vortexed briefly then incubated for >15 minutes at 20°C with roller-mixing. The conjugate was purified by Superdex 200 column eluting with TBS pH 7.5 and analysed by UV-vis to give purified mCD71-SO1861-SMCC (18.6 mg, yield: 93%, 0.942 mg/ml, SMCC to mCD71 ratio = 2.6).

Separately, an aliquot of M23D-SS-amide (17.2 mg, 1.99 µmol, 10.0 mg/ml) reconstituted using TBS pH 7.5 was added an aliquot of freshly prepared THPP solution (50 mg/ml, 10 mole equivalents, 19.9 µmol, 82.8 µl), the mixture vortexed briefly then incubated for 60 minutes at 37°C with roller-mixing. After incubation, the oligo was purified by PD10 Sephadex G25M column eluting with TBS pH 7.5, to afford M23D-SH (14.8 mg, yield: 86%, thiol to M23D ratio = 0.98).

To an aliquot of mCD71-SO1861-SMCC (17.2 mg, 0.11 µmol, 0.95 mg/ml) was added an aliquot of M23D-SH (4.1 mg/ml, 4.0 mole equivalents, 0.44 µmol, 0.86 ml), the mixture vortexed briefly then incubated at 20°C with roller-mixing. After ca. 72 hours, the conjugate mixture was concentrated and purified by Superdex 200PG column eluting with DPBS pH 7.5 to give purified mCD71-SO1861-M23D conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a mCD71-SO1861-M23D conjugate (total yield = 15.2 mg, 79%, M23D to mCD71-SO1861 ratio = 1.6).

### hCD71-DMD-ASO

An aliquot of hCD71 (60 mg, 4.20 ml) was buffer exchanged into DPBS pH 7.5 and normalised to 2.5 mg/ml. To an aliquot of hCD71 (58 mg, 0.38 µmol, 2.53 mg/ml) was added an aliquot of freshly prepared PEG4-SPDP solution (10 mg/ml, 10 mole equivalents, 3.8 µmol), the mixture vortexed briefly then incubated for 60 minutes at 20°C with roller-mixing. After incubation, the reaction was quenched by the addition of an aliquot of a freshly prepared glycine solution (2.0 mg/ml, 50 mole equivalents, 19 µmol), the mixture vortexed briefly then incubated for >15 minutes at 20°C with roller-mixing. The conjugate was purified by Zeba 40K spin desalting column eluting with TBS pH 7.5 and analysed by UV-vis to give purified hCD71-PEG4-SPDP (51.3 mg, yield: 88%, 0.95 mg/ml, PEG4-SPDP to hCD71 ratio = 4.5).

Separately, an aliquot of DMD-ASO-SH (14.4 mg, 2 µmol, 10.0 mg/ml) reconstituted using TBS pH 7.5 was added an aliquot of freshly prepared THPP solution (50 mg/ml, 10 mole equivalents, 20 µmol, 82.8 µl), the mixture vortexed briefly then incubated for 60 minutes at 37°C with roller-mixing. After incubation, the oligo was purified by PD10 Sephadex G25M column eluting with TBS pH 7.5, to afford reduced DMD-ASO-SH (13.2 mg, yield: 92%, thiol to DMD-ASO ratio = 0.91).

To an aliquot of hCD71-PEG4-SPDP (25 mg, 0.16 µmol, 0.95 mg/ml) was added an aliquot of DMD-ASO-SH (4 mg/ml, 4.0 mole equivalents, 0.65 µmol, 1.17 ml), the mixture vortexed briefly then incubated at 20°C with roller-mixing. Reaction progression was measured by PDT displacement. After 16 hours, the conjugate mixture was concentrated and purified by Superdex 200PG column eluting with DPBS pH 7.5 to give purified hCD71-DMD-ASO conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a hCD71-DMD-ASO conjugate (total yield = 23.1 mg, 82%, DMD-ASO to hCD71 ratio = 3.5). In a second synthesis, a hCD71-DMD-ASO conjugate was synthesized with the same methods as described and a DMD-ASO to hCD71 ratio = 2.1).

### hCD71-DMD-PMO

An aliquot of hCD71 (60 mg, 4.20 ml) was buffer exchanged into DPBS pH 7.5 and normalised to 2.5 mg/ml. To an aliquot of hCD71 (58 mg, 0.38 µmol, 2.53 mg/ml) was added an aliquot of freshly prepared PEG4-SPDP solution (10 mg/ml, 10 mole equivalents, 3.8 µmol), the mixture vortexed briefly then incubated for 60 minutes at 20 °C with roller-mixing. After incubation, the reaction was quenched by the addition of an aliquot of a freshly prepared glycine solution (2.0 mg/ml, 50 mole equivalents, 19 µmol), the mixture vortexed briefly then incubated for >15 minutes at 20°C with roller-mixing. The conjugate was purified by Zeba 40K spin desalting column eluting with TBS pH 7.5 and analysed by UV-vis to give purified hCD71-PEG4-SPDP (51.3 mg, yield: 88%, 0.95 mg/ml, PEG4-SPDP to hCD71 ratio = 4.1).

Separately, an aliquot of DMD-PMO-SS-amide (20.2 mg, 2 µmol, 10.0 mg/ml) reconstituted using TBS pH 7.5 was added an aliquot of freshly prepared THPP solution (50 mg/ml, 10 mole equivalents, 20 µmol, 82.8 µl), the mixture vortexed briefly then incubated for 60 minutes at 37°C with roller-mixing. After incubation, the oligo was purified by PD10 Sephadex G25M column eluting with TBS pH 7.5, to afford DMD-PMO-SH (16.4 mg, yield: 81%, thiol to DMD-PMO ratio = 0.97).

To an aliquot of hCD71-PEG4-SPDP (25 mg, 0.16 µmol, 0.95 mg/ml) was added an aliquot of DMD-PMO-SH (4.1 mg/ml, 4.0 mole equivalents, 0.65 µmol, 1.59 ml), the mixture vortexed briefly then incubated at 20°C with roller-mixing. Reaction progression was measured by PDT displacement. After ca 16 hours, the conjugate mixture was concentrated and purified by Superdex 200PG column eluting with DPBS pH 7.5 to give purified hCD71-DMD-PMO conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a hCD71-DMD-PMO conjugate (total yield = 28.2 mg, 92%, DMD-PMO to hCD71 ratio = 3.9). In a second synthesis, a hCD71-DMD-PMO conjugate was synthesized with the same method, and a DMD-PMO to hCD71 ratio = 3.2).

### hCD71-SO1861

The hCD71 (55.1 mg, 0.37 µmol, 8.10 mg/ml, 6.80 ml) as supplied was buffer exchanged using a Zeba spin desalting column eluting with TBS pH 7.5, and normalised to 3 mg/ml. To hCD71 (50 mg, 0.33 µmol, 5.044 mg/ml) was added an aliquot of freshly prepared TCEP solution (2.00 mg/ml, 3 mole equivalents, 1 µmol), the mixture vortexed briefly then incubated for 210 minutes at 20 °C with roller-mixing. After incubation (prior to addition of SO1861-SC-Maleimide), a 1.0 mg (0.201 ml) aliquot of hCD71 solution was removed and purified by gel filtration using Zeba spin desalting column into TBS pH 7.5. This aliquot was characterised by UV-vis analysis and Ellman's assay (2.23 mg/ml, thiol to hCD71 ratio = 4.35). To an aliquot of bulk hCD71-SH (42 mg, 0.28 µmol, 4.978 mg/ml) was added an aliquot of freshly prepared SO1861-SC-Maleimide solution (2.0 mg/ml, 8 mole equivalents, 2.24 µmol, 2.32 ml), the mixture vortexed briefly then incubated for 120 minutes at 20°C. Besides the conjugation reaction, two aliquots of desalted hCD71-SH (0.25 mg, 0.077 ml, 1.67 nmol) were reacted with NEM (8.00 equivalents, 13.4 nmol, 6.7 µl of a 0.25 mg/ml solution) or TBS pH 7.5 buffer (6.7 µl) for 120 minutes at 20°C, as positive and negative controls, respectively. After incubation, a *ca.* 0.4 mg aliquot of hCD71-SO8161 mixture was removed, purified by gel filtration using Zeba spin desalting column into TBS pH 7.5 and characterised by Ellman's assay alongside positive and negative controls to obtain SO1861 incorporation. To the bulk hCD71-SO1861 mixture was added an aliquot of freshly prepared NEM solution (5 mole equivalents, 1.4 µmol of a 2.5 mg/ml solution) to quench the reaction. The conjugate was purified by Zeba 40K MWCO spin desalting columns eluting with DPBS pH 7.5 to give purified hCD71-SO1861 conjugate. An aliquot of the product was analysed by BCA colorimetric assay to ascertain a new EC280 value. Then, the product was normalised to 2.5 mg/ml, filtered through 0.2 µm and then dispensed into aliquots for characterisation, product testing and further conjugation. The result was hCD71-SO1861 conjugate (total yield = 37.8 mg, 89%, SO1861 to hCD71 ratio = 4.2).

### hCD71-DMD-PMO-SO1861

To an aliquot of hCD71-SO1861 with a DAR 4.2 (20 mg, 0.128 µmol, 2.53 mg/ml) in DPBS pH 7.5 was added an aliquot of freshly prepared PEG4-SPDP solution (10 mg/ml, 10 mole equivalents, 1.28 µmol), the mixture vortexed briefly then incubated for 60 minutes at 20°C with roller-mixing. After incubation, the reaction was quenched by the addition of an aliquot of a freshly prepared glycine solution (2.0 mg/ml, 50 mole equivalents, 6.4 µmol), the mixture vortexed briefly then incubated for >15 minutes at 20 °C with roller-mixing. The conjugate was purified by Zeba 40K spin desalting column eluting with TBS pH 7.5 and analysed by UV-vis to give purified hCD71-SO1861-PEG4-SPDP (17.2 mg, yield: 84%, 0.93 mg/ml, PEG4-SPDP to hCD71 ratio = 3.7).

Separately, an aliquot of DMD-PMO-SS-amide (20.2 mg, 2 µmol, 10.0 mg/ml) reconstituted using TBS pH 7.5 was added an aliquot of freshly prepared THPP solution (50 mg/ml, 10 mole equivalents, 20 µmol, 82.8 µl), the mixture vortexed briefly then incubated for 60 minutes at 37°C with roller-mixing. After incubation, the oligo was purified by PD10 Sephadex G25M column eluting with TBS pH 7.5, to afford DMD-PMO-SH (16.4 mg, yield: 81%, thiol to DMD-PMO ratio = 0.97).

To an aliquot of hCD71-SO1861-PEG4-SPDP (15 mg, 95 nmol, 0.98 mg/ml) was added an aliquot of DMD-PMO-SH (4 mg/ml, 4.0 mole equivalents, 0.38 µmol, 0.95 ml), the mixture vortexed briefly then incubated at 20°C with roller-mixing. After 72 hours, the conjugate mixture was concentrated and purified by Superdex 200PG column eluting with DPBS pH 7.5 to give purified hCD71-DMD-PMO-SO1861 conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a hCD71-DMD-PMO-SO1861 conjugate (total yield = 11.8 mg, 49%, DMD-PMO to hCD71-SO1861 ratio = 2.6).

### hCD71-DMD-ASO-SO1861

To an aliquot of hCD71-SO1861 with a DAR 4.2 (20 mg, 0.128 µmol, 2.53 mg/ml) in DPBS pH 7.5 was added an aliquot of freshly prepared PEG4-SPDP solution (10 mg/ml, 10 mole equivalents, 1.28 µmol), the mixture vortexed briefly then incubated for 60 minutes at 20°C with roller-mixing. After incubation, the reaction was quenched by the addition of an aliquot of a freshly prepared glycine solution (2.0 mg/ml, 50 mole equivalents, 6.4 µmol), the mixture vortexed briefly then incubated for >15 minutes at 20°C with roller-mixing. The conjugate was purified by Zeba 40K spin desalting column eluting with TBS pH 7.5 and analysed by UV-vis to give purified hCD71-SO1861-PEG4-SPDP (17.2 mg, yield: 84%, 0.93 mg/ml, PEG4-SPDP to hCD71 ratio = 3.7).

Separately, an aliquot of DMD-ASO-SH (14.4 mg, 2 µmol, 10.0 mg/ml) reconstituted using TBS pH 7.5 was added an aliquot of freshly prepared THPP solution (50 mg/ml, 10 mole equivalents, 20 µmol, 82.8 µl), the mixture vortexed briefly then incubated for 60 minutes at 37°C with roller-mixing. After incubation, the oligo was purified by PD10 Sephadex G25M column eluting with TBS pH 7.5, to afford reduced DMD-ASO-SH (13.2 mg, yield: 92%, thiol to DMD-ASO ratio = 0.91).

To an aliquot of hCD71-SO1861-PEG4-SPDP (15 mg, 95 nmol, 0.98 mg/ml) was added an aliquot of DMD-ASO-SH (4 mg/ml, 4.0 mole equivalents, 0.38 µmol, 0.68 ml), the mixture vortexed briefly then incubated at 20°C with roller-mixing. After 72 hours, the conjugate mixture was concentrated and purified by Superdex 200PG column eluting with DPBS pH 7.5 to give purified hCD71-DMD-ASO-SO1861 conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a hCD71-DMD-ASO-SO1861 conjugate (total yield = 14.3 mg, 84%, DMD-ASO to hCD71-SO1861 ratio = 2.1).

### Cell culture (human)

Immortalized human myoblasts from non-DMD donors (KM155) and myoblasts from a DMD-affected donor (DM8036) were cultured in Skeletal Muscle Cell Growth Medium (PromoCell, Germany) with supplementary pack according to manufacturer's instructions, further supplemented with 15% fetal bovine serum (Gibco, United Kingdom) and 0.5% gentamicin (Sigma-Aldrich, USA). For differentiation, cells were seeded on a 0.5% gelatin-coated surface, and at ~70 - 80% confluence, the proliferation medium was replaced by DMEM (Gibco) supplemented with 2% FBS (Gibco), 2% GlutaMAX, and 1% glucose (Sigma-Aldrich) and 0.5% gentamycin. Treatments were started after at least 3 days up to a maximum of 5 days of differentiation (based on the presence of differentiated myotubes).

### Cell culture and in vitro experiments (murine)

Murine myoblast cell line C2C12 was maintained in 10% FBS DMEM medium + Pen/Strep and plated at 240,000 cells per well (cpw) in 24-well plates or 40,000 cpw in 96-well plates (wp) in maintenance medium (10% FBS in DMEM medium + Pen/Strep) and incubated at 37°C with 5% CO₂. Twenty-four hours after seeding, cells were switched to differentiation media (2% horse serum in DMEM) and incubated for 3 days before refreshing the medium. After another 24 hours, medium was refreshed again and compounds were added and incubated for 48 hours. Differentiation medium was then refreshed (without compounds), and cells were incubated for another 24 hours. At 72 hours total post treatment, cells in the 24-wp were harvested for exon skip analysis and the cell viability was assessed on the 96-wp.

### Exon skip analysis and quantification (human)

RNA was isolated with the TRIsure Isolation Reagent (Bioline) and chloroform extraction; isopropanol precipitation of RNA from the aqueous phase was performed as known to someone skilled in the art. For cDNA synthesis, 1000 ng of total RNA was used and diluted in an appropriate amount of RNase-free water to yield 8 µl RNA dilution. The priming premixed contained 1 µl dNTP mix (10 mM each) and 1 µl specific reverse primer (for KM155, h53R 5'- CTCCGGTTCTGAAGGTGTTC-3' [SEQ ID NO: 5]; for DM8036, h55R 5'-ATCCTGTAGGACATTGGCAGTT-3' [SEQ ID NO: 6]). This mixture was heated for 5 min at 70 °C, then chilled on ice for at least 1 min. A reaction mixture was prepared containing 0.5 µl rRNasin (Promega), 4.0 µl RT buffer, 1.0 µl Tetro RT (Bioline), and 4.5 µl RNase-free water, and was added to the chilled mixture to yield a total volume of 20 µl per reaction. The RT-PCR was run for 60 min at 42 °C, then 5 min at 85 °C, and chilled on ice. For skip analysis, a nested PCR approach was followed. To this end, in the first PCR, 3 µl cDNA were added to a mix of 2.5 µl 10x SuperTaq PCR buffer, 0.5 µl dNTP mix (10 mM each), 0.125 µl Taq DNA polymerase TAQ-RO (5U/µl; Roche) 16.875 µl RNase-free water and 1 µl (10 pmol/ µl) of each primer flanking the targeted exons were used: for KM155, h48F 5'- AAAAGACCTTGGGCAGCTTG-3' [SEQ ID NO: 7] and h53R 5'-CTCCGGTTCTGAAGGTGTTC-3' [SEQ ID NO: 5]; for DM8036, h47F2 5'-TGAAACTGGAGGACCCGTG -3' [SEQ ID NO: 8] and h54R 5'-CCAAGAGGCATTGATATTCTC -3' [SEQ ID NO: 9]. These samples were subjected to a PCR run of 5 min at 94°C, then 25 cycles with 40 sec at 94°C, 40 sec at 60°C, 180 sec at 72°C, after which for 7 min at 72°C. For the second PCR, 1.5 µl PCR1 sample were added to a mix of 5 µl 10x SuperTaq PCR buffer, 1 µl dNTP mix (10 mM each), 0.25 µl Taq DNA polymerase TAQ-RO (5 U/µl; Roche), 38.25 µl RNase-free water and 2 µl (10 pmol/ µl) of each primer flanking the targeted exons were used: for KM155, h49F 5'- CCAGCCACTCAGCCAGTG-3'[SEQ ID NO: 10] and h52R2 5'- TTCTTCCAACTGGGGACGC-3' [SEQ ID NO: 11]; for DM8036, h47F 5'-CCCATAAGCCCAGAAGAGC-3' [SEQ ID NO: 12] and h53R 5'- CTCCGGTTCTGAAGGTGTTC-3' [SEQ ID NO: 13]. These samples were subjected to a PCR run of 5 min at 94°C, then 32 cycles with 40 sec at 94°C, 40 sec at 60°C, 60 sec at 72°C, after which for 7 min at 72°C. Exon skipping levels were quantified with the Femto Pulse System using the Ultra Sensitivity NGS Kit (Agilent), according to the manufacturer's instructions. Alternatively, the specific PCR fragments were analyzed using Bioanalyzer 2100 with DNA1000 chip (lab-on-a-chip; Agilent), or separated on 2% agarose gels run at 120 V. Gels were imaged and band intensities were quantified using ImageJ. The expected non-skipped product has a size of 408 bp (KM155) and 475 bp (DM8036), and the skip product of 175 bp (KM155) or 242 bp (DM8036), respectively.

### Exon skip analysis and quantification (murine vitro and vivo)

For analysis of skip from murine cell lines, cells were harvested and RNA was isolated using 0.5 ml TRIzol^{™} Reagent (Thermo Scientific) per sample, according to the manufacturer's instruction. For analysis of tissue, 30-50 mg frozen tissue was first cut into smaller pieces and mRNA was isolated using TRIzol^{™} Reagent (Thermo Scientific) and a TissueLyser LT (Qiagen) according to the manufacturer's instruction. For cDNA synthesis per sample, to 0.5 µg RNA in 5.0 µl, 10.0 µl ddH₂O, 4.0 µl 5x iScript^{™} Reaction Mix and 1.0 µl iScript^{™} Reverse Transcriptase (BioRad) were added to yield a total volume of 20.0 µl per reaction. The RT-PCR was run for 5 min at 25°C, 60 min at 46°C, and 2 min at 95°C. For skip analysis, SapphireAmp^{™} Fast PCR Master Mix (TakaraBio) was used according to the manufacturer's instructions. To this end 9.7 µl RNAse free water, 12.5 µl of 2x Master Mix, 0.4 µl of 10 µM FW primer 5'-ACCCAGTCTACCACCCTATC-3' [SEQ ID NO: 14], and 0.4 µl of 10 µM RV primer 5'-CTCTTTATCTTCTGCCCACCTT-3' [SEQ ID NO: 15] were added to a PCR tube, mixed, after which 2 µl cDNA (50 ng) was added to yield a total volume of 25 µl. These samples were subjected to a PCR run of 1 min at 94°C, 35 cycles of 5 sec at 98°C, 5 sec at 55°C, 5 sec at 72°C, followed by 1 min at 72°C. Samples were mixed with 2 µl 6x loading buffer, then 16 µl were loaded onto an 2% agarose gel and ran for 60-90 min at 80 V. Gels were imaged and band intensities were quantified with a ChemiDoc^{™} XRS+ System and Image Lab^{™} Software (BioRad). The expected non-skipped product has a size of 788 bp, and the skip product of 575 bp, respectively.

### Cell viability (murine vitro)

After treatment, the cell viability was determined with a CellTiter-Glo^{®} 2.0 assay, performed according to the manufacturer's instruction (Promega). The luminescence signal was measured on a SpectraMax ID5 plate reader (Molecular Devices). For quantification, the background signal of 'medium only' wells was subtracted from all other wells, before the cell viability percentage of treated/untreated cells was calculated by dividing the background corrected signal of treated wells over the background corrected signal of the untreated wells (x 100).

### In vivo studies

Per group, 9 male CD-1 mice, aged 6 - 7 weeks at dosing, were given a single injection intravenously (iv) of the compounds or vehicle listed in Table A2. Doses were normalized to obtain similar PMO doses in group 2 and group 3 to allow comparison of efficacy. During the treatment period, animals were regularly weighed (on the day before dosing, and twice weekly post dosing) and any clinical observations were recorded. At day 4, day 14, and day 28, respectively, 3 mice per group were sacrificed and terminal bleeds and samples from different tissues and organs were harvested for analysis. Serum was prepared and ALT (AU480, Beckman Coulter) and creatinine levels (colorimetric method, Beckman Coulter) were analyzed. Tissues were preserved in RNALater and snap frozen until analysis. In heart, diaphragm and gastrocnemius samples, dystrophin skip levels were determined.

**Table A2: Dosing groups in vivo efficacy and tolerability**

| Group | Compound Name | # animals | Total compound [mg/kg] | **Relative PMO amount in total dose [mg/kg]** |
|---|---|---|---|---|
| 1 | Vehicle (PBS) | 9 | 0.0 | N/A |
| 2 | mCD71-M23D (DAR1.2) | 9 | 44.0 | 2.80 |
| 3 | mCD71-M23D-SO1861 (DAR1.6) | 9 | 36.6 | 2.91 |
| 4 | mCD71-M23D-SO1861 (DAR1.6) | 9 | 5.2 | 0.42 |
| 5 | mCD71-M23D-SO1861 (DAR1.6) | 9 | 1.8 | 0.15 |

### Results (example 1 - 6)

### Example 1. DMD-PMO + SO1861 and DMD-ASO + SO1861

DMD-ASO (a 2'O-methyl-phosporothioate antisense oligonucleotide that induces exon 51 skipping of human dystrophin and has the same sequence and chemistry modifications as drisapersen) and DMD-PMO (a phosphorodiamidate morpholino oligomer antisense oligonucleotide that induces exon 51 skipping of human dystrophin and has the same sequence but not 5'-modifications as eteplirsen) were assessed for exon skipping activity in combination with the endosomal escape enhancer SO1861-EMCH (4 µM) in differentiated human myotubes derived from a non-DMD (healthy) donor (KM155). Surprisingly, this revealed strongly enhanced exon skipping of exon 51 only in combination with SO1861 after 72 hrs (Table A3): while exposure to 20 µM DMD-ASO alone showed no skipping activity, already 0.08 µM DMD-ASO + SO1861-EMCH revealed 40% exon skip, indicating that the improvement factor is exceeding 250-fold (Figure 1A). Likewise, exposure to 20 µM DMD-PMO resulted in 4% skip, while already 1.25 µM DMD-ASO + SO1861-EMCH achieved 5% skip, and 34% at 20 µM, indicating an increase in potency of 16-fold for the untargeted oligos with untargeted SO1861-EMCH (Figure 1B).

In conclusion, co-administration of SO1861-EMCH strongly enhances the on-target delivery of DMD-ASO and DMD-PMO and induces marked exon 51 skipping at one to two orders of magnitude lower exposure concentrations compared to conditions where SO1861-EMCH is not present.

**Table A3: Skip efficacy of MD oligos with and without SO1861-EMCH co-administration in human myotubes (KM155)**

| | | **Concentration DMD oligo** | | | | |
|---|---|---|---|---|---|---|
| **% exon skipping** (Lab-on-a-chip or Femto analysis) | | **20 µM** | **5 µM** | **1.25 µM** | **0.31 µM** | **0.08 µM** |
| DMD-ASO | Lab-on-a-chip | 1% | 1% | 0% | 0% | 0% |
| DMD-ASO + SO1861-EMCH | Lab-on-a-chip | 52% | 61% | 67% | 53% | 40% |
| DMD-PMO | Femto | 4% | 0% | 0% | 0% | 0% |
| DMD-PMO + SO1861-EMCH | Femto | 34% | 19% | 5% | 0% | 0% |

### Example 2. hCD71-DMD-ASO + SO1861-EMCH or hCD71-DMD-PMO + SO1861-EMCH or mCD71-M23D + SO1861-EMCH

DMD-ASO-SH and DMD-PMO-SH were conjugated as shown in Figure 2B and Figure 2C, respectively to PEG4-SPDP-modified human anti-CD71 monoclonal antibody (hCD71-PEG4-SPDP, Figure 2A) to produce: hCD71-DMD-ASO (DAR2.1) and hCD71-DMD-PMO (DAR3.2). The resultant compounds were tested for enhanced cytoplasmic DMD oligo delivery and enhanced dystrophin exon 51 skipping either without or in combination with 4 µM SO1861-EMCH on the differentiated human myotubes from a non-DMD donor (KM155) and a DMD-affected donor (DM8036). This revealed strongly enhanced exon skipping of exon 51 in combination with 4 µM SO1861-EMCH at markedly lower concentrations compared to hCD71-DMD-ASO alone, and to hCD71-DMD-PMO alone, respectively after 72 hrs of treatment (Table A4 and A5): hCD71-DMD-ASO resulted in low skip (3%) in KM155 at 2181 nM (Figure 3A, left panel), which is an improvement compared to non-targeted DMD-ASO (Table A3), while hCD71-DMD-ASO + SO1861 furthermore increased skip to 18 - 24% already at 18.2 - 109 nM in KM155 (Figure 3A, right panel). Likewise, hCD71-DMD-PMO resulted in no skip at 2022 nM (0%) in KM155 (Figure 3B, left panel), while with addition of SO1861-EMCH, exon skip was visible at a concentration of 2.8 - 16.9 nM hCD71-DMD-PMO (Figure 3B, right panel). More importantly, in differentiated myotubes from a DMD-affected donor (DM8036), only 17% exon 51 skip was observed at 363 nM hCD71-DMD-ASO (Figure 4A, left panel), while, surprisingly, the 720-fold lower concentration of 0.50 nM hCD71-DMD-ASO + 4 µM SO1861-EMCH already resulted in comparable skip (15%). The skip efficiency strikingly increased up to 64 - 68% at only 18.2 - 109 nM exposure concentration of hCD71-DMD-ASO (Figure 4A, right panel). Likewise, also in DM8036 myotubes, hCD71-DMD-PMO alone reached 21% skip at 337 nM (Figure 4B, left panel), whereas already an exposure concentration of 0.08 - 0.47 nM hCD71-DMD-PMO + 4 µM SO1861-EMCH resulted in clearly measurable skip (7 - 13%), which increased up to 33% at 101 nM (Figure 4B, right panel) realizing an improvement of one to two orders of magnitude.

Taken together, this shows that co-administration of SO1861-EMCH to targeted oligonucleotides hCD71-DMD-ASO and hCD71-DMD-PMO markedly improves on-target cytoplasmic delivery and specifically, in relevant cell systems such as differentiated myotubes from DMD-affected donors.

**Table A4: Skip efficacy of anti-CD71-conjugated DMD oligos in human myotubes (top concentration for DMD-ASO-conjugate, bottom for DMD-PMO-conjugate)**

| **% exon skipping** (Femto analysis) | [nM conjugate] | | | | | | |
|---|---|---|---|---|---|---|---|
| | 2181/ 2022 | 363/ 337 | 61/ 56 | 10.1/ 9.4 | 1.68/1.5 6 | 0.28/ 0.26 | |
| hCD71-DMD-ASO (DAR2.1) (KM155) | 3 | 0 | 0 | 0 | 0 | 0 | % DMD exon 51 skipping |
| hCD71-DMD-PMO (DAR3.2) (KM155) | 0 | 0 | 0 | 0 | 0 | 0 | |
| hCD71-DMD-ASO (DAR2.1) (DM8036) | 24 | 17 | 0 | 0 | 0 | 0 | |
| hCD71-DMD-PMO (DAR3.2) (DM8036) | 45 | 21 | 0 | 0 | 0 | 0 | |

**Table A5: Skip efficacy of anti-CD71-conjugated DMD oligos with SO1861-EMCH co-administration in human myotubes (top concentration for DMD-ASO-conjugate, bottom for DMD-PMO-conjugate)**

| **% exon skipping** (Femto analysis) | [nM conjugate] | | | | | | |
|---|---|---|---|---|---|---|---|
| | 109/ 101 | 18.2/16. 9 | 3.0/2.8 | 0.50/0.4 7 | 0.084/ 0.078 | 0.014/0. 013 | |
| hCD71-DMD-ASO (DAR2.1) + SO1861-EMCH (KM155) | 18 | 24 | 0 | 0 | 0 | 0 | |
| hCD71-DMD-PMO (DAR3.2) + SO1861-EMCH (KM155) | 0 | 3 | 2 | 0 | 0 | 0 | % DMD exon 51 skipping |
| hCD71-DMD-ASO (DAR2.1) + SO1861-EMCH (DM8036) | 64 | 68 | 62 | 15 | 1 | 0 | |
| hCD71-DMD-PMO (DAR3.2) + SO1861-EMCH (DM8036) | 33 | 34 | 15 | 13 | 7 | 0 | |

Next, M23D-SS-amide (a phosphorodiamidate morpholino oligomer antisense oligonucleotide that induces exon 23 skipping of mouse dystrophin) was conjugated to anti-CD71 monoclonal antibody targeting murine CD71 modified with SMCC-linker (mCD71-SMCC, Figure 2D) to produce mCD71-M23D (DAR1.2) (Figure 2E). Co-administration of mCD71-M23D + 8 µM SO1861-SC-Mal was tested on differentiated C2C12 myotubes and this revealed strong exon skipping enhancement with at least one order of magnitude lower concentrations mCD71-M23D (clear band until 27 nM, Figure 5, right panel) in the combination with SO1861-SC-Mal, whereas mCD71-M23D alone showed no activity at all concentration tested up to 433 nM (Figure 5, left panel).

This shows that co-administration of SO1861-compounds is broadly applicable cross-species to effectively increase potency of targeted PMOs with different targeting sequences (different exons, different sequences).

### Example 3. mCD71-SO1861 + M23D or IGF-1-SO1861 + M23D

To TCEP-treated anti-CD71 monoclonal antibody targeting murine CD71 (mCD71-SH, Figure 6B), either SO1861-EMCH or SO1861-SC-Maleimide (Figure 6A) were conjugated, respectively, to produce two different conjugates of mCD71-SO1861 (DAR3.8) (Figure 6C). Each of the conjugates was co-administered, in a concentration range from 0 - 720 nM, with a fixed concentration of 500 nM M23D to C2C12 differentiated myotubes. This revealed similarly strong enhanced M23D efficacy in exon 23 skipping for the combinations with mCD71-SO1861 (SO1861-EMCH) + 500 nM M23D (12% skip at 710 nM, 8% skip at 142 nM) (Figure 7A, left panel) and mCD71-SO1861 (SO1861-SC-Mal)+ 500 nM M23D (19% skip at 710 nM and 10% skip at 142 nM), whereas 500 nM M23D alone (0 nM conjugate) only showed 5% skip (Figure 7A, right panel). This data shows that addition of a mCD71-SO1861-Mal conjugate, independent of the conjugation and linkers, markedly increases the on-target effect, i.e. exon skip, mediated by M23D.

Next, IGF-1 ligand was conjugated to SO1861-hydrazone-NHS via lysines to produce: IGF-1-SO1861 (DAR2.7), as shown in Figure 6D. IGF-1-SO1861 was tested in co-administration, in a concentration range from 0 - 3333 nM, with 500 nM M23D on C2C12 differentiated myotubes. This revealed enhanced M23D cytoplasmic delivery, i.e. dystrophin exon 23 skipping for the combinations with IGF-1-SO1861 + 500 nM M23D (19% skip at 3333 nM, 11% skip at 667 nM, 8% skip at 133 nM) (Figure 7B).

Taken together, these data show that antibody and non-antibody ligand-conjugated, i.e. targeted SO1861, leads to marked potency enhancement, i.e. on-target delivery of a PMO payload in muscle cells in a co-administration setting.

### Example 4. hCD71-DMD-ASO-SO1861 or hCD71-DMD-PMO-SO1861

To TCEP-treated anti-CD71 monoclonal antibody targeting human CD71 (hCD71-SH, Figure 8A), SO1861-SC-Mal was conjugated via cysteines (Figure 8B) and PEG4-SPDP to lysines to yield hCD71-SO1861-PEG4-SPDP (Figure 8C), to which either DMD-ASO-SH or DMD-PMO-SH were conjugated to produce: hCD71-DMD-ASO-SO1861 (DAR2.1/4.2) (Figure 8D) and hCD71-DMD-PMO-SO1861 (DAR2.6/4.2) (Figure 8E); DMD-ASO-SH or DMD-PMO-SH were also (as previously described in Figure 2B and Figure 2C) conjugated to anti-CD71 monoclonal antibody targeting human CD71 (hCD71) to yield hCD71-DMD-ASO (DAR2.2) and hCD71-DMD-PMO (DAR3.1). Conjugates, including controls, were tested on differentiated human myotubes of a non-DMD donor (KM155) and of a DMD-affected donor (DM8036). As expected, these treatments revealed no or only very minor exon skip in KM155 myotubes in the concentration range from 0.084 nM - 651 nM for hCD71-DMD-ASO (Figure 9A, left panel; Table A6) and from 0.078 nM - 610 nM hCD71-DMD-PMO (Figure 9B, left panel; Table A6) at 72 hrs post dose, ranging from 0 - 2.4%. However, when co-administering 4 µM SO1861-SC-Mal to either hCD71-DMD-ASO (Figure 9A, right panel; Table A7) or to hCD71-DMD-PMO (Figure 9B, right panel; Table A7), strongly enhanced exon skip was observed in differentiated human myotubes: already at ca. 0.5 nM, 0-5% and 0-4% exon skip were observed for hCD71-DMD-ASO and hCD71-DMD-PMO, respectively. This increased to 29-40% at 109 nM hCD71-DMD-ASO and 4-5% at 102 nM hCD71-DMD-PMO, respectively, constituting a several order of magnitude improvement in potency, compared to conditions without SO1861-SC-Mal addition.

Most strikingly, incubation with a conjugate incorporating both the ASO and SO1861 conjugated to hCD71, hCD71-DMD-ASO-SO1861, revealed already exon 51 skip (8.5-8.6%) at 10.0 nM conjugate, and increasing up to 36-53% at 361 nM, and thus constituting a marked improvement of potency compared to conditions without SO1861 and in the same order of magnitude compared to improvement with co-administered SO1861-SC-Mal (Figure 9C; Table A8).

More relevantly, in differentiated myotubes from a DMD-affected donor, treatments with targeted conjugates hCD71-DMD-ASO and hCD71-DMD-PMO at 72 hrs post dose again revealed maximally 7-11% skip at 651 nM hCD71-DMD-ASO (Figure 9A, left panel; Table A7) and 610 nM hCD71-DMD-PMO (Figure 9B, left panel; Table A7), respectively. However, strikingly, and when co-administering 4 µM SO1861-SC-Mal to either hCD71-DMD-ASO or hCD71-DMD-PMO, strongly enhanced exon skip was observed in differentiated human myotubes from a DMD-affected donor: with 92-96% skip at 109 nM hCD71-DMD-ASO + SO1861-SC-Mal (Figure 10A, right panel) and 37-57% skip at 102 nM hCD71-DMD-PMO + SO1861-SC-Mal (Figure 10B, right panel). An effect was still measurable down to at least 0.50 nM hCD71-DMD-ASO, and even at 0.013 nM, skip was observed for hCD71-DMD-PMO + SO1861-SC-Mal. Again, most strikingly, incubation with a conjugate incorporating the ASO and SO1861 conjugated to hCD71, hCD71-DMD-ASO-SO1861, revealed strongly enhanced exon 51 skip (94-96%) at 361 nM, and still 30-40% at 10.0 nM conjugate (Figure 9C; Table A8), and thus constituting a marked improvement of potency compared to conditions without SO1861 and in the same order of magnitude compared to improvement with co-administered SO1861-SC-Mal.

**Table A6: Skip efficacy of anti-CD71-conjugated DMD oligos in human myotubes (top concentration for DMD-ASO-conjugate, bottom for DMD-PMO-conjugate)**

| **Exon skip%** | | **[nM conjugate]** | | | | | | |
|---|---|---|---|---|---|---|---|---|
| ImageJ and Femto analysis | | **651/ 610** | **109/ 102** | **18.1/ 16.9** | **3.0/ 2.8** | **0.50/ 0.47** | **0.084/ 0.078** | |
| hCD71-DMD-ASO (DAR2.2) (KM155) | ImageJ | 1.9 | 0.0 | 0.5 | 1.0 | 1.1 | 1.5 | % DMD exon 51 skipping |
| | Femto | 0.0 | 0.0 | 0.0 | 0.0 | 0.0 | 0.0 | |
| hCD71-DMD-PMO (DAR3.1) (KM155) | ImageJ | 0.3 | 1.4 | 2.4 | 2.0 | 1.8 | 2.0 | |
| | Femto | 0.0 | 0.0 | 0.0 | 0.0 | 0.0 | 0.0 | |
| hCD71-DMD-ASO (DAR2.2) (DM8036) | ImageJ | 6.9 | 1.6 | 1.4 | 1.0 | 1.3 | 1.4 | |
| | Femto | 11.1 | 0.0 | 0.0 | 0.0 | 0.0 | 0.0 | |
| hCD71-DMD-PMO (DAR3.1) (DM8036) | ImageJ | 7.3 | 3.5 | 3.2 | 2.8 | 3.3 | 2.2 | |
| | Femto | 7.9 | 0.0 | 0.0 | 0.0 | 0.0 | 0.0 | |

**Table A7: Skip efficacy of anti-CD71-conjugated DMD oligos with SO1861-SC-Mal co-administration in human myotubes (top concentration for DMD-ASO, bottom for DMD-PMO)**

| **Exon skip%** | | **[nM conjugate]** | | | | | | |
|---|---|---|---|---|---|---|---|---|
| ImageJ and Femto analysis | | **109/ 102** | **18.1/ 16.9** | **3.0/ 2.8** | **0.50/ 0.47** | **0.084/ 0.078** | **0.014/ 0.013** | |
| hCD71-DMD-ASO (DAR2.2) + SO1861-SC-Mal (KM155) | ImageJ | 28.8 | 29.4 | 13.9 | 5.1 | 3.0 | 3.0 | % DMD exon 51 skipping |
| | Femto | 40.3 | 38.4 | 13.5 | 0.0 | 0.0 | 0.0 | |
| hCD71-DMD-PMO (DAR3.1) + SO1861-SC-Mal (KM155) | ImageJ | 4.6 | 5.2 | 5.2 | 4.1 | 2.8 | 1.9 | |
| | Femto | 3.9 | 4.4 | 2.0 | 0.0 | 0.0 | 0.0 | |
| hCD71-DMD-ASO (DAR2.2) + SO1861-SC-Mal (DM8036) | ImageJ | 92.2 | 56.6 | 34.1 | 7.0 | 2.2 | 0.0 | |
| | Femto | 96.1 | 88.0 | 53.8 | 7.4 | 0.0 | 0.0 | |
| hCD71-DMD-PMO (DAR3.1) + SO1861-SC-Mal (DM8036) | ImageJ | 37.1 | 38.3 | 34.7 | 28.9 | 25.2 | 6.1 | |
| | Femto | 57.2 | 65.0 | 55.8 | 46.5 | 40.9 | 9.9 | |

**Table A8: Skip efficacy of hCD71-DMD-ASO-SO1861 in human myotubes**

| **Exon skip%** | | **[nM conjugate]** | | | | | | |
|---|---|---|---|---|---|---|---|---|
| ImageJ and Femto analysis | | **361** | **60.1** | **10.0** | **1.7** | **0.28** | **0.046** | |
| hCD71-DMD-ASO-SO1861 (DAR2.1/4.2) (KM155) | ImageJ | 35.5 | 31.6 | 8.5 | 2.0 | 1.3 | 0.2 | % DMD exon 51 skipping |
| | Femto | 52.8 | 43.4 | 8.6 | 0.0 | 0.0 | 0.0 | |
| hCD71-DMD-ASO-SO1861 (DAR2.1/4.2) (DM8036) | ImageJ | 94.4 | 78.3 | 29.7 | 4.2 | 2.0 | 1.2 | |
| | Femto | 96.1 | 93.5 | 40.2 | 0.0 | 0.0 | 0.0 | |

### Example 5. mCD71-M23D-SO1861 (in vitro)

To anti-CD71 monoclonal antibody targeting murine CD71 (mCD71), SO1861-EMCH was conjugated via cysteines and M23D-thiol was conjugated via lysines to produce: mCD71-M23D-SO1861 (DAR1.6/3.8) (Figure 8F, 8G) and mCD71-M23D (DAR1.2) (Figure 2D) and tested for dystrophin exon 23 skipping on differentiated murine C2C12 myotubes. Treatment did not affect cell viability, as determined with a CTG assay, but revealed enhanced exon 23 skipping at 336 nM (40% skip), 84 nM (28% skip), 21 nM (21% skip), 5 nM (12% skip), 1 nM (6% skip) (Figure 11), while as previously shown no appreciable skip was observed at any concentration tested for mCD71-M23D (Figure 5, left panel). These data show that mCD71-M23D-SO1861 improves on-target delivery of M23D in myotubes at least one to two orders of magnitude.

### Example 6. mCD71-M23D-SO1861 (in vivo efficacy)

CD-1 male mice received a single injection of mCD71-M23D-SO1861 (DAR1.6/3.8) (Figure 8C, 8D) or mCD71-M23D (DAR1.2) (Figure 2D), normalized to a similar nominal dose of the exon-skip active compound M23D (2.91 and 2.80 mg/kg, respectively). Additionally, 2 dose groups with lower doses of mCD71-M23D-SO1861 were also included, as well as a vehicle control group.

As Figure 12 (A-C) and Figure 13 show, animals receiving mCD71-M23D (2.80 mg/kg PMO; group 2) showed no exon 23 skip in any of the tested tissues or any time point (neither at day 4, day 14 nor day 28). Importantly however, marked skip in all animals and in all tested muscle tissues was observed in the group dosed with mCD71-M23D-SO1861 (2.91 mg/kg PMO, group 3): at day 4, 8.8% skip in gastrocnemius, 6.4% in diaphragm, and even 17.1% in cardiac muscle; at day 14, skip increased to 19.3% skip in gastrocnemius, 10.2% in diaphragm, and 18.4% in cardiac muscle. The effect of a single administration was durable as even at day 28, 8.5% skip in gastrocnemius, 9.0% in diaphragm, and 7.6% skip were measured in cardiac muscle (all % are mean for n = 2/3 per tissue, +/- standard error of the mean) for only 2.91 mg/kg absolute PMO payload administered. Notably, low-level skip was observed in several tissues and time points for the mid and even low-dose, and strikingly, 3.8% in cardiac muscle at day 14 for the mid-dose, containing only 0.42 mg/kg absolute PMO payload.

This data shows that conjugates containing a targeting ligand, an oligonucleotide payload and most importantly SO1861 are highly efficacious and already a single dose of 2.91 mg/kg of a DMD-targeting PMO can achieve substantial exon skip in different skeletal, but most importantly, also cardiac muscle, while conjugates without SO1861 do not achieve any skip at comparable doses. Even doses containing as little as only 0.42 mg/kg absolute PMO payload can result in measurable skip. This data supports a potency increase of at least one to two orders of magnitude in an in vivo wild-type content.

### In vivo tolerability of mCD71-M23D-SO1861 in CD-1 mice

The conjugates mCD71-M23D-SO1861 and mCD71-M23D were produced as described in Figure 8C, Figure 8D and were dosed as detailed in Table A2. During the course of the study, mCD71-M23D-SO1861 conjugates at all doses were well tolerated and showed no treatment-specific adverse findings or any specific clinical observations indicative of the conjugates not being tolerated. One animal treated with mCD71-M23D however (of six remaining in group 2) was found dead on day 11, also resulting in missing biomarker data for one of three mice at day 28. At the day 14 sacrifice, two (of three) mice dosed with mCD71-M23D in group 2 showed kidney abnormalities and elevated serum creatinine (Figure 14A), while importantly, no abnormalities in groups dosed with mCD71-M23D-SO1861 were observed. No marked or lasting changes in the kidney biomarker ALT were obvious, with one result at 161 U/L and two results at 30 and 34 U/L, respectively, in the highest mCD71-M23D-SO1861 dose (group 3), resulting in high variation in this small group of N = 3 (Figure 14B). Notably, after 14 days and 28 days, ALT levels in all dosing groups, especially for mCD71-M23D-SO1861 were comparable to vehicle controls (group 1). In conclusion, mCD71-M23D-SO1861 was well tolerated at all doses tested. Taken together, these data show that while highly efficacious, mCD71-M23D-SO1861 conjugates are also well tolerated in this study and show a more favourable tolerability profile than mCD71-M23D.

### Methods (as performed in Examples 7 - 9):

### SO1861

SO1861 was from *Saponaria officinalis L* (Extrasynthese, France) and was coupled to respective handle by Symeres (NL), according to methods known in the art.

### Synthesis of DBCO-(M23D)₂

DBCO-(M23D)₂ synthesis was performed by Symeres (NL).

### Conjugation of SO1861 to antibodies

Custom conjugate production of mCD63-SO1861 was performed by Abzena (UK).

### Conjugation of DBCO-(M23D)₂ to antibodies

Custom conjugate productions of mCD71-M23D and mCD63-M23D were performed by Abzena (UK).

### Conjugation of DBCO-(M23D)₂ to SO1861-antibodies

Custom conjugate production of mCD63-M23D-SC-SO1861 was performed by Abzena (UK).

### Conjugation of 3'-disulfidamide-M23D to SO1861-antibodies

Custom conjugate production of mCD71-M23D-EMCH-SO1861(2) was performed by Fleet Bioprocessing (UK).

### Analytical and Preparative methods

### Analytical methods

### SEC Method 1

Apparatus for reaction analysis: Analytical SEC Instrument DIONEX Ultimate 3000 UPLC (DIONEX 6); Column: Waters Protein BEH SEC Column, 200 Å, 1.7 µm, 4.6 mm X 150 mm; Mobile Phase: Buffer A (0.2 M Potassium Phosphate buffer, pH 6.8, 0.2M KCI, 15% isopropanol in ultra-pure water); Method: Isocratic buffer A for 10 min; Flow Rate: 0.35 ml/min; Run Time: 10 min; Detection UV: 214 nm, 248 nm, 260 nm and 280 nm; Column Oven: 30 °C; Auto Sampler: ambient; Injection Volume: 10 µL; Sample preparation: final sample was analyzed by diluting sample to 1.0 mg/ml with DPBS.

### LC-MS Method 1

Mass Spectrometry (LC-MS) Instrument: XEVO-G2XS TOF; Column: Agilent Poroshell 300SB-C3 guard, 5 µm RP column; Mobile Phases: Buffer A (Water, 0.1% formic acid) Buffer B (MeCN, 0.1% formic acid); Inlet Method: 0-2.0 min, 10% B 2.0-7.0 min, 10-80% B 7.0-8.0 min, 100% B 8.0-8.01 min, 10% B 8.01-10.0 min, 10% B; MS Method: Capillary voltage: 3.0 kV, Cone voltage: 120 V, Cone temperature: 140 °C, Desolvation temperature: 450 °C,: Flow Rate: 0.4 ml/min: Run Time: 10 min: Detection: TIC; Column Oven: 60 °C; Auto Sampler: Ambient; Injection Volume: 10 µL; Sample preparation: a. Final samples, intermediates, and reaction mixtures were analyzed by diluting sample to 0.05 mg/ml in DPBS.

### LC-MS method 2

Instrument: Agilent 1260 Infinity II, 1260 G7112B Bin. Pump, 1260 G7167A Multisampler, 1290 MCT G7116B Column Comp. 1260 G7115A DAD (210, 220 and 210-320 nm), PDA (210-320 nm), G6130B MSD (ESI pos/neg) mass range 90-1500, Column: XSelect CSH C18 (30x2.1mm 3.5µm) Flow: 1 ml/min, Column temp: 40 °C, Eluent A: 0.1% formic acid in Water, Eluent B: 0.1% formic acid in acetonitrile, Gradient: t₀ₘᵢₙ = 5% B, t_{1.6min} =98%B, t₃ₘᵢₙ = 98% B, Postrun: 1.3 min.

### LC-MS method 3

Instrument: Agilent 1260 Infinity II, 1260 G7112B Bin. Pump, 1260 G7167A Multisampler, 1260 MCT G7116A Column Comp. 1260 G7115A DAD (210, 220 and 210-320nm), PDA (210-320nm), G6130B MSD (ESI pos/neg) mass range 90-1500, Column: XSelect CSH C18 (30x2.1mm 3.5µm), Flow: 1 ml/min, Column temp: 25 °C, Eluent A: 10mM ammoniumbicarbonate in water (pH 9.5), Eluent B: acetonitrile, Gradient: t₀ₘᵢₙ = 5% B, t_{1.6min} =98%B, t₃ₘᵢₙ = 98% B, Postrun: 1.2 min.

### LC-MS method 4

Instrument: Agilent 1260 Infinity II, 1260 G7112B Bin. Pump, 1260 G7167A Multisampler, 1290 MCT G7116B Column Comp. 1260 G7115A DAD (210-320 nm, 210 and 220nm), PDA (210-320 nm), G6130B MSD (ESI pos/neg) mass range 90-1500, Column: Waters C4 BEH (50x2.1mm 3.5µm) Flow: 1 ml/min; Column Temp: 40 °C, Eluent A: 0.1% formic acid in water, Eluent B: 0.1% formic acid in acetonitrile, Gradient:
^{A}t₀ₘᵢₙ = 5% B, t_{2.5min} =98%B, t₄ₘᵢₙ = 98% B
^{B}t₀ₘᵢₙ = 5% B, t_{0.05min} = 5% B, t₅ₘᵢₙ =98%B, t₆ₘᵢₙ = 98% B
Postrun: 1.5 min.

### Preparative methods

### Preparative MP-LC method 1

Instrument type: Buchi Reveleris^{™} prep MPLC; column: Waters XSelect^{™} CSH C18 (145×25 mm, 10µm); Flow: 40 ml/min; Column temp: room temperature; Eluent A: 10 mM ammoniumbicarbonate in water pH = 9.0); Eluent B: 99% acetonitrile + 1% 10 mM ammoniumbicarbonate in water; Gradient: t₀ₘᵢₙ = 50% B, t₄ₘᵢₙ = 50% B, t₁₆ₘᵢₙ = 100% B, t₂₁ₘᵢₙ = 100% B; Detection UV: 220, 254, 270 nm; Fraction collection based on UV.

### Preparative MP-LC method 2

Instrument type: Buchi Reveleris^{™} prep MPLC; Column: Phenomenex LUNA C18(3) (150×25 mm, 10µm); Flow: 40 ml/min; Column temp: room temperature; Eluent A: 0.1% (v/v) formic acid in water, Eluent B: 0.1% (v/v) formic acid in acetonitrile; Gradient: t₀ₘᵢₙ = 5% B, t₁ₘᵢₙ = 5% B, t₂ₘᵢₙ = 20% B, t₁₇ₘᵢₙ = 60% B, t₁₈ₘᵢₙ = 100% B, t₂₃ₘᵢₙ = 100% B; Detection UV: 220, 240, 280 nm; Fraction collection based on UV.

### Preparative LC-MS method

MS instrument type: Agilent Technologies G6120AA Quadrupole; HPLC instrument type: Agilent Technologies 1200 preparative LC; Column: Waters XBridge Protein (C4, 150x19mm, 10µ); Flow: 25 ml/min; Column temp: room temperature; Eluent A: 0.1% formic acid in water; Eluent B: 100% acetonitrile; Gradient: t₀ = 10% A, t_{2.5min} = 10% A, t₁₁ₘᵢₙ = 50% A, t₁₃ₘᵢₙ = 100% A, t₁₇ₘᵢₙ = 100% A; Detection: DAD (220-320 nm); Detection: MSD (ESI pos/neg) mass range: 100 - 1000; Fraction collection based on DAD.

### Flash chromatography

Grace Reveleris X2^{™} C-815 Flash; Solvent delivery system: 3-piston pump with auto-priming, 4 independent channels with up to 4 solvents in a single run, auto-switches lines when solvent depletes; maximum pump flow rate 250 ml/min; maximum pressure 50bar (725psi); Detection: UV 200-400nm, combination of up to 4 UV signals and scan of entire UV range, ELSD; Column sizes: 4-330g on instrument, luer type, 750g up to 3000g with optional holder.

### mCD71-M23D-EMCH-SO1861(2)

To an aliquot of mCD71-EMCH-SO1861 with a DAR4.5 (20 mg, 0.126 µmol, 2.53 mg/ml) was added an aliquot of freshly prepared SMCC solution (2.0 mg/ml, 7 mole equivalents, 0.886 µmol), the mixture vortexed briefly then incubated for 60 minutes at 20°C with roller-mixing. After incubation, the reaction was quenched by the addition of an aliquot of a freshly prepared glycine solution (2.0 mg/ml, ~20 mole equivalents, 2.5 µmol), the mixture vortexed briefly then incubated for >15 minutes at 20°C with roller-mixing. The conjugate was purified by Superdex 200 column eluting with TBS pH 7.5 and analysed by UV-vis to give purified mCD71-SO1861-SMCC (18.6 mg, yield: 93%, 0.942 mg/ml, SMCC to mCD71 ratio = 3.9).

Separately, an aliquot of M23D-SS-amide (17.2 mg, 1.99 µmol, 10.0 mg/ml) reconstituted using TBS pH 7.5 was added an aliquot of freshly prepared THPP solution (50 mg/ml, 5 mole equivalents, 10 µmol, 41 µl), the mixture vortexed briefly then incubated for 60 minutes at 37°C with roller-mixing. After incubation, the oligo was purified by PD10 Sephadex G25M column eluting with TBS pH 7.5, to afford M23D-SH (14.8 mg, yield: 86%, thiol to M23D ratio = 0.98).

To an aliquot of mCD71-SO1861-SMCC (17.2 mg, 0.11 µmol, 0.95 mg/ml) was added an aliquot of M23D-SH (4.1 mg/ml, 8 mole equivalents, 0.88 µmol, 1.72 ml), the mixture vortexed briefly then incubated at 20°C with roller-mixing. After ca. 72 hours, the conjugate mixture was concentrated and purified by Superdex 200PG column eluting with DPBS pH 7.5 to give purified mCD71-M23D-EMCH-SO1861(2) conjugate. The aliquot was analysed by BCA colorimetric assay and assigned a new EC value for the conjugate, then concentrated and normalised to 2.5 mg/ml, filtered through 0.2 µm and then dispensed into an aliquot for characterisation and an aliquot for product testing. The result was a mCD71-M23D-EMCH-SO1861(2) conjugate (total yield = 15.1 mg, 79%, M23D to mCD71-SO1861 ratio = 2.1).

### DBCO-(M23D)₂ synthesis

### Intermediate 1 (tert-butyl N-[2-(4-{2-azatricyclo[10.4.0.0⁴,⁹]hexadeca-1(12),4(9),5,7,13,15-hexaen-10-yn-2-yl}-N-(2-{[(tert-butoxy)carbonyl]amino}ethyl)-4-oxobutanamido)ethyl]carbamate)

To a solution of DBCO-acid (50.0 mg, 0.164 mmol) in DMF (1.00 ml) was added DIPEA (34.0 µL, 0.195 mmol) and HATU (62.3 mg, 0.164 mmol) and the mixture was stirred for 15 min. Next, di-tert-butyl (azanediylbis(ethane-2,1-diyl))dicarbamate (59.6 mg, 0.197 mmol) was added and the reaction mixture was stirred at room temperature. After 30 min, the reaction mixture was added to water (10.0 ml). The resulting dense suspension was centrifuged (5000 RPM, 3 min) to yield a clear solution with solids on the top. The solution was removed with a pipette and the solids were dissolved in acetonitrile (10.0 ml). The resulting solution was concentrated *in vacuo.* The residue was purified by flash chromatography (DCM - methanol/DCM (1/9, v/v) gradient 100:0 rising to 0:50) to give the title product (90.0 mg, 93%) as a colorless solidifying oil. Purity based on LC-MS 100%.
LRMS (m/z): 591 [M+H]¹⁺
LC-MS r.t. (min): 2.12¹

### Intermediate 2 (N,N-bis(2-azaniumylethyl)-4-{2-azatricyclo[10.4.0.0⁴,⁹]hexadeca-1(12),4(9),5,7,13,-15-hexaen-10-yn-2-yl}-4-oxobutanamide ditrifluoroacetate)

Tert-butyl N-[2-(4-{2-azatricyclo[10.4.0.0⁴,⁹]hexadeca-1(12),4(9),5,7,13,15-hexaen-10-yn-2-yl}-N-(2-{[(tert-butoxy)carbonyl]amino}ethyl)-4-oxobutanamido)ethyl]carbamate (90.0 mg, 0.152 mol) was dissolved in DCM (2.00 ml) and cooled to 0 °C. Next, TFA (2.00 ml, 26.0 mmol) was added and the reaction mixture was stirred at 0 °C for 40 min. The reaction mixture was allowed to reach room temperature over the course of 10 min. As next, the reaction mixture was cooled to 0 °C and diluted with toluene (5 ml). The resulting solution was concentrated *in vacuo* and co-evaporated with DCM (2 x 5 ml) to give the crude title product as a slightly pink oil, which was used directly in the next step. Purity based on LC-MS 88%.
LRMS (m/z): 196 [M+2]²⁺, 391 [M+1]¹⁺
LC-MS r.t. (min): 1.17 (LC-MS method 2)

### Intermediate 3 (1S,4E)-cyclooct-4-en-1-yl N-[2-(4-{2-azatricyclo[10.4.0.0⁴,⁹]hexadeca-1(12),4(9),5,-7,13,15-hexaen-10-yn-2-yl}-N-[2-({[(1S,4E)-cyclooct-4-en-1-yloxylcarbonyl}amino)ethyl]-4-oxobutan-amido)ethyl]carbamate

To a solution of crude N,N-bis(2-azaniumylethyl)-4-{2-azatricyclo[10.4.0.0⁴,⁹]hexadeca-1(12),4(9),-5,7,13,15-hexaen-10-yn-2-yl}-4-oxobutanamide ditrifluoroacetate (0.152 mmol) in DMF (1.00 ml) and DIPEA (200 µL, 1.15 mmol) was added TCO4 - NHS carbonate (102 mg, 0.380 mmol) and the mixture was stirred at room temperature. After 30 min the reaction mixture was submitted to preparative MP-LC (Preparative MP-LC method 1). Fractions corresponding to the product were immediately pooled together, frozen and lyophilized overnight to give the title compound (90.0 mg, 85%) as a slightly brown oil. Purity based on LC-MS 99%.
LRMS (m/z): 696 [M+1]¹⁺
LC-MS r.t. (min): 2.35 (LC-MS method 3)

### Intermediate 4 2-[4-(6-methyl-1,2,4,5-tetrazin-3-yl)phenyl]-N-[2-(pyridin-2-yldisulfanyl)ethyl]acetamide

To a solution of methyltetrazine-NHS ester (50.0 mg, 0.153 mmol) in DMF (800 µL) was added 2-(2-pyridinyldisulfanyl)ethanamine hydrochloride (40.8 mg, 0.183 mmol) and DIPEA (53.0 µL, 0.306 mmol). The resulting mixture was stirred at room temperature. After two hours the reaction mixture was submitted to preparative MP-LC (Preparative MP-LC method 2). Fractions corresponding to the product were immediately pooled together, frozen and lyophilized overnight to give the title compound (53.6 mg, 88%) as a pink solid. Purity based on LC-MS 100%.
LRMS (m/z): 399 [M+1]¹⁺
LC-MS r.t. (min): 1.87 (LC-MS method 3)

### Intermediate 5: M23D-mTz

To a solution of M23D-DSA (294 mg, 34.0 µmol) in water (15.0 ml) was added DTT (100 mg, 648 µmol). The reaction mixture was stirred at room temperature. After two hours, the reaction mixture was divided in six equal fractions and poured in acetonitrile (6 x 45 ml). The resulting suspensions were shaken and left standing for 30 min. Next, the suspensions were centrifuged (7830 RPM, 20 min). The solutions were decanted and the residues were treated with acetonitrile (each vial 20 ml). The resulting suspensions were centrifuged (7830 RPM, 3 min). The residues were dissolved in water (total 10.0 ml) and the solutions were combined. Next, a solution of 2-[4-(6-methyl-1,2,4,5-tetrazin-3-yl)phenyl]-N-[2-(pyridin-2-yldisulfanyl)ethyl]acetamide (54.2 mg, 136 µmol) in acetonitrile (4.00 ml) was added. The reaction mixture was stirred at room temperature. After two hours the reaction mixture was equally divided and poured in acetonitrile (6 x 45 ml). The resulting suspensions were shaken and centrifuged (7830 RPM, 3 min). The solutions were decanted and the residues were dissolved in water/acetonitrile (6 x 2 ml, 1/1, v/v). The resulting solutions were poured in acetonitrile (6 x 20 ml). Next, the suspensions were centrifuged (7830 RPM, 3 min). The solutions were decanted and the residues were dissolved in water/acetonitrile (total 15 ml, 1/1, v/v) and lyophilized overnight to give the title compound (340 mg, quant.) as a pink solid. Purity based on LC-MS 99%.
LRMS (m/z): 1468 [M+6]⁶⁺, 1259 [M+7]⁷⁺, 1102 [M+8]⁸⁺, 979 [M+9]⁹⁺, 881 [M+10]¹⁰⁺
LC-MS r.t. (min): 1.75 (LC-MS method 4A)

### DBCO-(M23D)₂

To a solution of M23D-mTz (16.4 mg, 1.86 µmol) in water (1.00 ml) and acetonitrile (0.400 ml) was added a stock solution of (1S,4E)-cyclooct-4-en-1-yl N-[2-(4-{2-azatricyclo[10.4.0.0⁴,⁹]hexadeca-1(12),4(9),5,7,13,15-hexaen-10-yn-2-yl}-N-[2-({[(1S,4E)-cyclooct-4-en-1-yloxy]carbonyl}amino)ethyl]-4-oxobutanamido)ethyl]carbamate (0.67 mg, 0.964 µmol) in acetonitrile (675 µl) and the reaction mixture was stirred at room temperature. After every addition, the reaction progress was monitored with LC-MS^{3A}. In this way, complete conversion to the title product was obtained. The addition of the stock solution was as following; 400 µl, after 10 min - 100 µl, after 10 min - 100 µl, after 10 min - 25 µl, after 10 min - 25 µl, after 10 min - 25 µl. Next, the reaction mixture was submitted to preparative LC-MS. Fractions corresponding to the product were immediately pooled together, frozen and lyophilized overnight to give the title compound (10.5 mg, 62%) as a white solid. Purity based on LC-MS 100% (broad peak).
LRMS (m/z): 1142 [M+16]¹⁶⁺, 1075[M+17]¹⁷⁺, 1015 [M+18]¹⁸⁺, including multiple m/z values of known fragments
LC-MS r.t. (min): 2.84 (LC-MS method 4B)

### Conjugation of murine anti-CD63 (mCD63) mAb to DBCO-(M23D)₂

### 1. Preparation of mCD63

mCD63 was buffer exchanged into TBS using a Vivaspin (50 kDa MWCO), to a final concentration of 10.0 mg/ml.

### 2. Modification of the carbohydrate on antibody-Fc domain

The immobilized GlycINATOR^{™} column (from GlyCLICK^{™} Azide Activation Kit, Genovis) was equilibrated and prepared according to the vendor's indications. The sample was then loaded on the column, the medium was resuspended, and the mixture was incubated and mixed at RT for 1 h. The column was then centrifuged, and the sample eluted.

UDP-GalNAz (from GlyCLICK^{™} Azide Activation Kit, Genovis) was reconstituted with TBS according to the vendor's indications and transferred to the pooled eluate together with GaIT (from GlyCLICK^{™} Azide Activation Kit, Genovis). The mixture was incubated overnight, in the dark, at 30 °C. Afterwards, the reaction was loaded onto a pre-conditioned desalting column (according to the indications of the vendor) and centrifuged to collect the flow-through, containing the azido-modified mAb. This was stored in the dark at 4 C, until later use for conjugation.

### 3. Conjugation with DBCO-(M23D)₂

mCD63 was buffer exchanged into DPBS using a Vivaspin (50 kDa MWCO) to a final concentration of 10.0 mg/ml DBCO-(M23D)₂ (5.0 equi., 1 mM in DPBS, pH 7.4) and was added to the mAb solution. The reaction mixture was incubated for 24 h at 37 °C, then directly purified by preparative SEC (HiLoad 26/600 Superdex 200 pg, DPBS). The conjugate was characterized by SEC-UV (DAR determination). The pooled fractions were concentrated using the aforementioned Vivaspin to a final concentration of > 10.0 mg/ml, sterile filtered over 0.22 µm filter units, and stored at 4 °C until further use.

| **Test** | **Result** |
|---|---|
| SEC (% monomers) | 98.4 |
| LC-MS (MW) | N/A |
| HIC (average DAR) | N/A |
| LC-MS (average DAR) | N/A |
| UV (average DAR) | 3.42 |
| Endotoxin (EU/mg) | 0.517 |
| Volume (ml) | 5.0 |
| Concentration (mg/ml) | 12.24 |
| Amount (mg) | 61.2 |
| Yield (%) | 76.5 |

### Conjugation of murine anti-CD63 (mCD63) mAb to SO1861

### 1. Preparation of mCD63

mCD63 was buffer exchanged into DPBS + 5 mM EDTA, pH 7.4, using a Vivaspin (50 kDa MWCO), to a final concentration of 8.0 mg/ml.

### 2. Conjugation of mCD63

mCD63 was pre-incubated at 37 °C for ~15 minutes, followed by TCEP (3.8 equi.) addition. The reaction mixture was diluted to 6.5 mg/ml, then incubated at 37 °C for 1 h. The reduction of mCD63 was monitored by denaturing LC-MS. The reaction was equilibrated to 22 °C, and SO1861 (8.0 equi.) was added. Reaction monitoring was performed via denaturing LC-MS, and when the reaction was complete, it was quenched with DTT (100 equi.).

### 3. Purification of mCD63 conjugate

The reaction mixture was directly purified by P2 desalting columns using DPBS. The conjugate was characterized by SEC and denaturing LC-MS (DAR determination, final extinction coefficient), then concentrated above 10 mg/ml using a Vivaspin (50 kDa MWCO), sterile filtered over 0.22 µm filter units, and stored at +4 °C until further use.

| **Test** | **Result** |
|---|---|
| SEC (% monomers) | 100 |
| LC-MS (MW) | Conform to structure |
| HIC (average DAR) | N/A |
| LC-MS (average DAR) | 4.08 |
| UV (average DAR) | N/A |
| Endotoxin (EU/mg) | 0.629 |
| Volume (ml) | 1.65 |
| Concentration (mg/ml) | 10.0 |
| Amount (mg) | 16.5 |
| Yield (%) | 74.0 |

### Conjugation of murine anti-CD63 (mCD63) mAb-SO1861 to DBCO-(M23D)₂

### 1. Preparation of mCD63

The mCD63-SO1861 conjugate was buffer exchanged into TBS using a Vivaspin (50 kDa MWCO), to a final concentration of 10.0 mg/ml.

### 2. Modification of the carbohydrate on antibody-Fc domain

The immobilized GlycINATOR^{™} column (from GlyCLICK^{™} Azide Activation Kit, Genovis) was equilibrated and prepared according to the vendor's indications. The sample was then loaded on the column, the medium was resuspended, and the mixture was incubated and mixed at RT for 1 h. The column was then centrifuged, and the sample eluted.

UDP-GalNAz (from GlyCLICK^{™} Azide Activation Kit, Genovis) was reconstituted with TBS according to the vendor's indications and transferred to the pooled eluate together with GaIT (from GlyCLICK^{™} Azide Activation Kit, Genovis). The mixture was incubated overnight, in the dark, at 30 °C. Afterwards, the reaction was loaded onto a pre-conditioned desalting column (according to the indications of the vendor) and centrifuged to collect the flow-through, containing the azido-modified mCD63. This was stored in the dark at 4 C, until later use for conjugation.

### Step 3. Conjugation with DBCO-(M23D)₂

mCD63 was buffer exchanged into DPBS using a Vivaspin (50 kDa MWCO) to a final concentration of 10.0 mg/ml. DBCO-(M23D)₂ (5.0 equi., 1 mM in DPBS, pH 7.4) was added to the mCD63 solution, and the reaction mixture was incubated for 24 h at 37 °C, then directly purified by preparative SEC (HiLoad 26/600 Superdex 200 pg, DPBS). The conjugate was characterized by SEC-UV (DAR determination). The pooled fractions were concentrated using the aforementioned Vivaspin to a final concentration of > 10.0 mg/ml, sterile filtered over 0.22 µm filter units, and stored at 4 °C until further use.

| **Test** | **Result** |
|---|---|
| SEC (% monomers) | 98.6 |
| LC-MS (MW) | Conform to structure for SO1861 |
| HIC (average DAR) | N/A |
| LC-MS (average DAR) | 4.08 (SO1861) |
| UV (average DAR) | 3.62 (M23D) |
| Endotoxin (EU/mg) | 0.430 |
| Volume (ml) | 3.5 |
| Concentration (mg/ml) | 10.87 |
| Amount (mg) | 38.12 |
| Yield (%) | 76.2 |

### Conjugation of murine anti-CD71 (mCD71) mAb to DBCO-(M23D)₂

### 1. Preparation of mCD71 mCD71 was buffer exchanged into TBS using a Vivaspin (50 kDa MWCO), to a final concentration of 10.0 mg/ml.

### 2. Modification of the carbohydrate on antibody-Fc domain

The immobilized GlycINATOR^{™} column (from GlyCLICK^{™} Azide Activation Kit, Genovis) was equilibrated and prepared according to the vendor's indications. The sample was then loaded on the column, the medium was resuspended, and the mixture was incubated and mixed at RT for 1 h. The column was then centrifuged, and the sample eluted.

UDP-GalNAz (from GlyCLICK^{™} Azide Activation Kit, Genovis) was reconstituted with TBS according to the vendor's indications and transferred to the pooled eluate together with GaIT (from GlyCLICK^{™} Azide Activation Kit, Genovis). The mixture was incubated overnight, in the dark, at 30 °C. Afterwards, the reaction was loaded onto a pre-conditioned desalting column (according to the instructions of the vendor) and centrifuged to collect the flow-through, containing the azido-modified mCD71. This was stored in the dark at 4 °C, until later use for conjugation.

### 3. Conjugation with DBCO-(M23D)₂

mCD71 was buffer exchanged into DPBS using a Vivaspin (50 kDa MWCO) to a final concentration of 10.0 mg/ml. DBCO-(M23D)₂ (5.0 equi., 1 mM in DPBS, pH 7.4) was added to the mCD71 solution, and the reaction mixture was incubated for 24 h at 37 °C, then directly purified by preparative SEC (HiLoad 26/600 Superdex 200 pg, DPBS). The conjugate was characterized by SEC-UV (DAR determination). The pooled fractions were concentrated using the aforementioned Vivaspin to a final concentration of > 10.0 mg/ml, sterile filtered over 0.22 µm filter units, and stored at 4 °C until further use.

| **Test** | **Result** |
|---|---|
| SEC (% monomers) | 99.8 |
| LC-MS (MW) | N/A |
| HIC (average DAR) | N/A |
| LC-MS (average DAR) | N/A |
| UV (average DAR) | 3.49 |
| Endotoxin (EU/mg) | 1.418 |
| Volume (ml) | 4.6 |
| Concentration (mg/ml) | 10.76 |
| Amount (mg) | 49.54 |
| Yield (%) | 69.7 |

### Cell culture and in vitro experiments (murine)

Murine myoblast cell line C2C12 was maintained in 10% FBS DMEM medium + Pen/Strep and plated at 240,000 cells or 180,000 cells per well (cpw) in 24-well plates, or at 40,000 or 30,000 cpw in 96-well plates (wp), in maintenance medium (10% FBS in DMEM medium + Pen/Strep) and incubated at 37°C with 5% CO₂. Twenty-four hours after seeding, cells were switched to differentiation media (2% horse serum in DMEM) and incubated for 3 days before refreshing the medium. After another 24 hours, medium was refreshed again and compounds were added and incubated for 48 or 72 hours. At 48 or 72 hours total post treatment, cells in the 24-wp were harvested for exon skip analysis and the cell viability was assessed on the 96-wp.

### Exon skip analysis and quantification (murine in vitro)

Performed as described above in the Methods (as performed in Examples 1-6).

### Cell viability (murine in vitro)

Performed as described above in the Methods (as performed in Examples 1-6).

### Results (example 7 - 9)

### Example 7. mCD71-M23D + SO1861-SC-Mal or mCD63-M23D + SO1861-SC-Mal (in vitro)

DBCO-(M23D)₂, a branched scaffold bearing two M23D (a phosphorodiamidate morpholino oligomer antisense oligonucleotide that induces exon 23 skipping of mouse dystrophin) oligonucleotide payloads, was produced as shown in Figure 16. DBCO-(M23D)₂ was conjugated to either anti-CD71 monoclonal antibody targeting murine CD71 or anti-CD63 monoclonal antibody targeting murine CD63, to produce mCD71-M23D (DAR 3.5) and mCD63-M23D (DAR 3.4), respectively (for conjugation procedure see Figure 17). Either mCD71-M23D conjugate or mCD63-M23D conjugate was co-administered with a fixed concentration of 8 µM of the endosomal escape enhancer SO1861-SC-Mal and tested for dystrophin exon 23 skipping on differentiated C2C12 murine myotubes following 48h of treatment. Co-administration of 8 µM SO1861-SC-Mal revealed strong exon 23 skipping enhancement for both mCD71-M23D (clear band until 0.2 nM, an improvement of at least three orders of magnitude) (Figure 18A, right panel) and mCD63-M23D (clear band until 6.0 nM, an improvement of two orders of magnitude) (Figure 18B, right panel). mCD71-M23D alone showed no activity at all concentrations tested up to 758 nM (Figure 18A, left panel), and mCD63-M23D alone showed only 2% skip at 755 nM (Figure 18B, left panel). Treatments did not affect cell viability, as determined with a CTG assay. These data show that co-administration of SO1861-SC-Mal improves CD71- and CD63-targeted delivery of M23D in myotubes by at least two to three orders of magnitude.

### Example 8. mCD63-SC-SO1861 + M23D or mCD71-M23D (in vitro)

To anti-CD63 monoclonal antibody targeting murine CD63, SO1861-SC-Mal was conjugated to produce mCD63-SC-SO1861 (DAR 4.1) (for conjugation procedure see Figure 19). mCD63-SC-SO1861 was co-administered with a fixed concentration of 500 nM M23D (a phosphorodiamidate morpholino oligomer antisense oligonucleotide that induces exon 23 skipping of mouse dystrophin) to differentiated C2C12 murine myotubes. This revealed enhanced exon 23 skipping with 28% skip at 662 nM and 2% skip at 1.6 nM mCD63-SC-SO1861 after 48h of treatment (Figure 20A), whereas 500 nM M23D alone showed no exon skip activity (Figure 20C). These data demonstrate that mCD63-SC-SO1861 induces on-target enhanced cytoplasmic delivery of M23D, inducing enhanced exon 23 skipping.

Next, mCD63-SC-SO1861 (Figure 19) was co-administered with a fixed concentration of 80 nM mCD71-M23D (Figure 17). Treatment with 80 nM mCD71-M23D alone showed no exon skip (Figure 20C). Co-administration of mCD63-SC-SO1861 with 80 nM mCD71-M23D revealed enhanced exon 23 skipping with 10% skip at 662 nM and still 4% skip at 5.3 nM mCD63-SC-SO1861 after 48h of treatment (Figure 20B).

Taken together, these data show that ligand1-conjugated SO1861 (i.e., targeted SO1861), in combination with ligand2-conjugated PMO payload (i.e., targeted M23D) leads to marked potency enhancement (example of a targeted 2-component system).

### Example 9. mCD71-M23D-EMCH-SO1861(2) or mCD63-M23D-SC-SO1861 (in vitro)

To anti-CD71 monoclonal antibody targeting murine CD71, both SO1861-EMCH and M23D-thiol were conjugated to produce mCD71-M23D-EMCH-SO1861(2) (DAR 2.1/4.5) (conjugation procedure previously described in Figure 8F, 8G). To anti-CD63 monoclonal antibody targeting murine CD63, both SO1861-SC-Mal and DBCO-(M23D)₂ were conjugated to produce mCD63-M23D-SC-SO1861 (DAR 3.6/4.1) (for conjugation procedure see Figure 21). Treatment of differentiated C2C12 murine myotubes with these conjugates for 72h revealed enhanced exon 23 skipping for mCD71-M23D-EMCH-SO1861(2) at 1200 nM (65% skip), 240 nM (27% skip), 48 nM (17% skip), 9.6 nM (6% skip), and 1.9 nM (7% skip) (Figure 22A). For mCD63-M23D-SC-SO1861, enhanced exon 23 skipping was observed at 1200 nM (53% skip), 240 nM (22% skip), and 48 nM (11% skip) (Figure 22B). Treatments did not affect cell viability, as determined with a CTG assay. These data show that mCD71-M23D-EMCH-SO1861(2) and mCD63-M23D-SC-SO1861 improve on-target delivery of M23D in myotubes at least one to three orders of magnitude (example of a targeted 1-component system).

### Methods (example 10 - 12)

### SO1861-SC-Maleimide

SO1861 was from *Saponaria officinalis L* (Extrasynthese, France) and was coupled to respective handles by Symeres (NL) according to methods known in the art.

### Conjugation of SO1861 to antibodies

Custom conjugate productions of hCD71-SC-SO1861 and hCD63-SC-SO1861 were performed by Fleet Bioprocessing (UK).

### Conjugation of 5'-thiol-DMD-ASO, 5'-disulfidamide-DMD-PMO(1), and 3'-disulfidamide-DMD-PMO(1, 2, 3, 4 and 5) to antibodies

Custom conjugate productions of hCD71-5'-SS-DMD-ASO, hCD71-5'-SS-DMD-PMO(1), hCD71-3'-SS-DMD-PMO(1), hCD71-3'-SS-DMD-PMO(2), hCD71-3'-SS-DMD-PMO(3), hCD71-3'-SS-DMD-PMO(4), hCD71-3'-SS-DMD-PMO(5), and hCD63-5'-SS-DMD-ASO were performed by Fleet Bioprocessing (UK).

### Conjugation of 5'-thiol-DMD-ASO and 3'-disulfidamide-DMD-PMO(1, 2, 3, 4 and 5) to SO1861-antibodies

Custom conjugate productions of hCD71-5'-SS-DMD-ASO-SC-SO1861, hCD71-3'-SS-DMD-PMO(1)-SC-SO1861, hCD71-3'-SS-DMD-PMO(2)-SC-SO1861, hCD71-3'-SS-DMD-PMO(3)-SC-SO1861, hCD71-3'-SS-DMD-PMO(4)-SC-SO1861, hCD71-3'-SS-DMD-PMO(5)-SC-SO1861, hCD63-5'-SS-DMD-ASO-SC-SO1861, hCD63-3'-SS-DMD-PMO(1)-SC-SO1861, hCD63-3'-SS-DMD-PMO(2)-SC-SO1861, hCD63-3'-SS-DMD-PMO(3)-SC-SO1861, and hCD63-3'-SS-DMD-PMO(4)-SC-SO1861 were performed by Fleet Bioprocessing (UK).

### Analytical and Preparative methods

The conjugates were characterised via UV-vis spectrophotometry, BCA colorimetric assay, analytical SEC, SDS-PAGE, Western Blotting, and Urea-PAGE gel electrophoresis. Prior to conjugation with SO1861-SC-Maleimide, reduced mAb-SH (either hCD71-SH or hCD63-SH) was analysed via UV-vis spectrophotometry and Ellman's assay.

### UV-vis spectrophotometry

Concentrations were determined using either a Thermo Nanodrop 2000 spectrometer or Perkin Elmer Lambda 365 Spectrophotometer with the following mass Extinction Coefficient (EC) values: Experimentally determined molar ε495 = 58,700 M-1 cm-1 and Rz280:495 = 0.428 were used for SAMSA-fluorescein.
Ellman's reagent (TNB); molar ε412 = 14,150 M-1 cm-1
Pyridine 2-thione (PDT); molar ε363 = 8,080 M-1 cm-1

Additionally, the DMD oligonucleotide incorporation was determined by UV-vis spectrophotometry and BCA colorimetric assay using literature ε²⁶⁵values:
DMD-PMO(1); molar ε363 = 318,050 (mg/ml)-1 cm-1
DMD-PMO(2); molar ε363 = 318,120 (mg/ml)-1 cm-1
DMD-PMO(3); molar ε363 = 308,180 (mg/ml)-1 cm-1
DMD-PMO(4); molar ε363 = 247,710 (mg/ml)-1 cm-1
DMD-PMO(5); molar ε363 = 207,890 (mg/ml)-1 cm-1
DMD-ASO; molar ε363 = 310,00 (mg/ml)-1 cm-1

### SEC

The conjugates were analysed by SEC using an Akta purifier 10 system and Biosep SEC-s3000 column eluting with DPBS: IPA (85: 15). Conjugate purity was determined by integration of the conjugate peak with respect to impurities/aggregate forms.

### SDS-PAGE

Native proteins and conjugates were analysed under heat denaturing non-reducing and reducing conditions by SDS-PAGE against a protein ladder using a 4-12% bis-tris gel and MOPS as running buffer (200 V, ~ 40 minutes). Samples were prepared to 0.5 mg/ml, comprising LDS sample buffer and MOPS running buffer as diluent. For reducing samples, DTT was added to a final concentration of 50 mM. Samples were heat treated for 15 minutes at 90 - 95 °C and 2.5 µg (5 µL) was added to each well. Protein ladder (10 µL) was loaded without pre-treatment. Empty lines were filled with 1× LDS sample buffer (10 µL). After the gel was run, it was washed thrice with DI water (100 ml) with shaking (15 minutes, 200 rpm). Coomassie staining was performed by shaker-incubating the gel with PAGEBlue protein stain (30 ml) (60 minutes, 200 rpm). Excess staining solution was removed, rinsed twice with DI water (100 ml) and de-stained with DI water (100 ml) (60 minutes, 200 rpm). The resulting gel was imaged and processed using ImageJ (ImageJ (Rasband, W.S., ImageJ, U. S. National Institutes of Health, Bethesda, Maryland, USA) and MyCurveFit (point-to-point correlation of protein ladder).

### Western Blotting

From SDS-PAGE, the gel was transferred to nitrocellulose membrane using the X-Cell blot module with the following setup ((-)BP-BP-FP-Gel-NC-BP-BP-BP(+)) and conditions (30V, 60 minutes) using freshly prepared transfer buffer. BP - blotting pad; FP - Filter pad; NC - Nitrocellulose membrane. After, the NC were washed thrice with PBS-T (100 ml) with shaking (5 minutes, 200 rpm), non-specific sites blocked with blocking buffer (50 ml) with shaking (30 minutes, 200 rpm) then active sites labelled with a combination of Goat anti-Human Kappa - HRP (1:2000) and Goat anti-Human IgG - HRP (1:2000) (50 ml) diluted in blocking buffer with shaking (30 minutes, 200 rpm). After that, the NC was washed once with PBS-T (100 ml) with shaking (5 minutes, 200 rpm) and complexed antibody detected with Ultra TMB-Blotting Solution (25 ml). Colour development was observed visually, and development was stopped by washing the NC with water, and the resulting blot was photographed.

### Urea-PAGE gel electrophoresis

This characterisation of conjugates was carried out under denaturing non-reducing conditions with EtBr or SYBR Green staining, compared against oligonucleotide standards and oligonucleotide standards ladder (report residual 'free' oligonucleotide).

### hAb-SC-SO1861

A general description of the conjugation of hAb-SO1861, such as hCD71-SC-SO1861 and hCD63-SC-SO1861 is shown below. The quantities given in brackets and italics are shown for hCD71-SC-SO1861, as an example.

An aliquot of hAb was buffer exchanged into TBS pH 7.5 and normalized to 5 mg/ml. To an aliquot of hAb (*95.8 mg,* 6.39 × *10⁻⁴ mmol, 4.94 mg*/*ml*) was added an aliquot of TCEP *(3.18 equiv., 2.03* × *10⁻³ mmol, 0.58 mg, 0.582 ml*) freshly prepared in TBS pH 7.5 (1 mg/ml) with gentle swirling. The mixture was incubated at 20°C for 210 minutes with roller-mixing. After incubation, a 1.0 mg aliquot (0.203 ml) of the reaction mixture was removed and purified by Zeba 7K spin desalting column eluting with TBS pH 7.5 and characterized by UV-vis and Ellman's assay (*3.407 mg*/*ml, SH to hAb ratio* = 4.8). To the bulk reaction was added an aliquot of SO1861-SC-Maleimide *(6 mol equiv., 3.79* × *10⁻³ mmol, 8.3 mg, 4.14 ml*) freshly prepared in TBS pH 7.5 (2 mg/ml) with gentle swirling, the mixture vortexed briefly then incubated for 120 minutes at 20 °C. 2 × 0.25 mg of desalted hAb-SH (1.67 × *10⁻⁶ mmol, 0.073 ml*) were dispensed out and reacted with NEM (8 mol equiv., 1.34 × 10⁻⁵ mmol, 6.7 µl, 0.25 mg/ml) or TBS pH 7.5 (6.7 µl), then incubated alongside the bulk conjugation, as positive and negative reaction controls, respectively. After incubation, a 0.5 mg aliquot (ca. 0.100 ml) of the hAb-SC-SO1861 mixture was removed, purified by Zeba 7K spin desalting column eluting with TBS pH 7.5 and characterized by Ellman's assay alongside positive and negative controls to obtain SO1861 incorporation. After reaction, to the bulk hAb-SC-SO1861 mixture was added an aliquot of freshly prepared NEM solution (*5 mol equiv., 3.16* × *10⁻³ mmol, 158 µl of a 2.5 mg*/*ml solution*) to quench the reaction. The quenched reaction mixture was purified by using a sanitized 2.6 × 40 cm Superdex 200 column eluting with DPBS pH 7.5. The purified hAb-SC-SO1861 was collected and analysed by UV-vis spectrophotometry. It was then concentrated to >2.5 mg/ml using a Vivaspin 20 centrifugal filter, normalized to 2.5 mg/ml, and filtered to 0.2 µm under laminar flow. The product was dispensed into aliquots for product testing, characterization, and further conjugation work. The results were:
*hCD71-SC-SO1861 (total yield = 78.4 mg,* 82%, *SO1861 to hCD71 ratio = 4.0)*
*hCD63-SC-SO1861 conjugate (total yield = 58.8 mg, SO1861 to hCD63 ratio = 4.8).*

### hAb-DMD-oligonucleotide

A general description of the conjugation of hAb targeted DMD oligonucleotides, such as hCD71-5'-SS-DMD-ASO, hCD71-5'-SS-DMD-PMO(1), hCD71-3'-SS-DMD-PMO(1-5), and hCD63-5'-SS-DMD-ASO, is shown below. The quantities given in brackets and *italics* are shown for hCD71-5'-SS-DMD-PMO(1), as an example.

An aliquot of hAb was buffer exchanged into DPBS pH 7.5 and normalized to 2.5 mg/ml. To an aliquot of hAb (*hCD71, 20.0 mg,* 1.33 × *10-4 mmol, 2.5 mg*/*ml*) was added an aliquot of freshly prepared PEG4-SPDP solution (*10.0 mg*/*ml, 10.0 mole equivalents, 1.33* × *10-3 mmol, 0.075 ml*), the mixture was vortexed briefly and then incubated for 60 minutes at 20 °C with roller-mixing. After incubation, the reaction was quenched by the addition of an aliquot of a freshly prepared glycine solution (*10 mg*/*ml, 50 mole equivalents, 6.65* × *10-3 mmol, 8.1 µl*), the mixture vortexed briefly, then incubated for > 15 minutes at 20°C with roller-mixing. The conjugate was purified (*using Zeba 40K spin desalting columns eluting with TBS pH 7.5, filtered to 0.45 µm*), then analysed by UV-vis to give purified hAb-SPDP (*hCD71-SPDP, 21.2 mg, 2.04 mg*/*ml, SPDP to hCD71 ratio* = *3.9*). hAb-SPDP was used immediately.

Separately, the desired DMD oligonucleotide (*DMD-PMO(1)-5'-amide, 20.2 mg, 1.99* × *10-3 mmol, 10.00 mg*/*mll*) was reconstituted using TBS pH 7.5, pooled into a single aliquot and analysed by UV-vis to ascertain ε₂₈₀, ε₂₆₀ and their ratio ε₂₆₀/ε₂₈₀. To this was added an aliquot of freshly prepared THPP solution (*50 mg*/*ml, 10 mole equivalents, 1.99* × *10-2 mmol, 83 µl*), the mixture was vortexed briefly, then incubated for 60 minutes at 37°C with roller-mixing. After incubation, the oligonucleotide was purified using PD10 Sephadex G25 columns eluting with TBS pH 7.5, to afford the reduced DMD oligonucleotide-SH (*DMD-PMO(1)-5'-SH, 16.4 mg, 81%, thiol to DMD-PMO(1) ratio = 1.02*)*.*

To an aliquot of hAb-SPDP (*hCD71-SPDP, 10.2 mg, 6.79* × *10-5 mmol, 2.04 mg*/*ml*) was added an aliquot of oligonucleotide-SH (*DMD-PMO(1)-SH, 2.73 mg*/*ml, 8.0 mole equivalents, 5.43* × *10-4 mmol, 2.01 ml*), the mixture was vortexed briefly and then incubated overnight at 20°C with roller-mixing. After *ca.* 16 hours, the conjugate mixture was analysed by UV-vis to ascertain incorporation by PDT displacement and then purified using a sanitised 2.6 × 60 cm Superdex 200PG column eluting with DPBS pH 7.5. The conjugate was analysed by UV-vis and BCA colorimetric assay and assigned a new ε₂₈₀. The material was concentrated (*using a Vivacell 100 (30 K MWCO), to the maximum concentration obtainable*) and then dispensed into aliquots for product testing and characterisation. The result was a hCD71-5'-SS-DMD-PMO(1) conjugate (*total yield = 65%; DMD-PMO(1) to hCD71 ratio = 2.2*), as an example. The other results were:
*hCD71-3'-SS-DMD-PMO(1) (total yield = 58%, DMD-PMO(1) to hCD71 ratio = 2.1)*
*hCD71-3'-SS-DMD-PMO(2) (total yield = 70%, DMD-PMO(2) to hCD71 ratio = 3.0)*
*hCD71-3'-SS-DMD-PMO(3) (total yield = 65%, DMD-PMO(3) to hCD71 ratio = 2.6)*
*hCD71-3'-SS-DMD-PMO(4) (total yield =* 68%, *DMD-PMO(4) to hCD71 ratio = 2.3)*
*hCD71-3'-SS-DMD-PMO(5) (total yield = 67%, DMD-PMO(5) to hCD71 ratio = 2.1)*
*hCD71-5'-SS-DMD-ASO (total yield =* 76%, *DMD-ASO to hCD71 ratio = 2.1)*
*hCD63-5'-SS-DMD-ASO (total yield = 60%, DMD-ASO to hCD63 ratio = 2.3)*

### hAb-DMD-oligonucleotide-SC-SO1861

A general description of the conjugation of hAb-DMD-oligo-SC-SO1861, such as hCD71-5'-SS-DMD-ASO-SC-SO1861, hCD63-5'-SS-DMD-ASO-SC-SO1861, hCD71-3'-SS-DMD-PMO(1-5)-SC-SO1861 and hCD63-3'-SS-DMD-PMO(1-5)-SC-SO1861, is shown below.

hAb-SC-SO1861 (DAR4) was conjugated to a series of DMD oligonucleotides *via* lysine residues targeting DAR values of 4. Bulk activation of hAb-SC-SO1861 was carried out and the resulting SPDP intermediates were split into aliquots for conjugation to each oligonucleotide. In the beginning, scales of *ca.* 100 mg hAb was PEG4-SPDP functionalised, then purified by gel filtration using a sanitised 5 × 50 cm Superdex 200 column and characterised by UV-vis to obtain hAb-SPDP. Prior to the coupling of DMD oligonucleotide, each oligonucleotide was activated by treatment with e.g. THPP and then analysed by UV-vis and Ellman's assay to ascertain the presence of a thiol.

Each aliquot of hAb-SPDP was reacted with 8 equiv. of the corresponding oligonucleotide (*i.e*., 2 equiv. per hAb-SPDP; 0.37-0.54 mg of oligonucleotide-SH is required per 1 mg of hAb-SPDP) to give hAb-oligonucleotide *via* a labile disulfide linkage. Oligonucleotide incorporation was estimated by PDT displacement, measured by UV-vis. The conjugates were purified by gel filtration using a dedicated 2.6 × 40 cm Superdex 200 column eluting with Dulbecco's PBS pH 7.5. The purified conjugates were analysed by BCA colorimetric assay to ascertain hAb concentration and UV-vis spectrometry, then concentrated and normalized prior to terminal filtration and dispensing under laminar flow. A small aliquot (*e.g*., 0.25 mg) of each was retained for characterization via aSEC, SDS-PAGE, Western Blotting and Urea Page Electrophoresis. The results were:
*hCD71-3'-SS-DMD-PMO(1)-SC-SO1861 (total yield = 67%, SO1861 to hCD71 ratio = 4.0, DMD-PMO(1) to hCD71 ratio = 2.4)*
*hCD71-3'-SS-DMD-PMO(2)-SC-SO1861 (total yield = 57%, SO1861 to hCD71 ratio = 4.0, DMD-PMO(2) to hCD71 ratio = 3.1)*
*hCD71-3'-SS-DMD-PMO(3)-SC-SO1861 (total yield = 61%, SO1861 to hCD71 ratio = 4.0, DMD-PMO(3) to hCD71 ratio = 2.5)*
*hCD71-3'-SS-DMD-PMO(4)-SC-SO1861 (total yield = 61%, SO1861 to hCD71 ratio = 4.0, DMD-PMO(4) to hCD71 ratio = 2.0)*
*hCD71-3'-SS-DMD-PMO(5)-SC-SO1861 (total yield = 54%, SO1861 to hCD71 ratio = 4.0, DMD-PMO(5) to hCD71 ratio = 2.0)*
*hCD71-5'-SS-DMD-ASO-SC-SO1861 (total yield = 62%, SO1861 to hCD71 ratio = 4.0, DMD-ASO to hCD71 ratio = 2.3)*
*hCD63-3'-SS-DMD-PMO(1)-SC-SO1861 (total yield = 69%, SO1861 to hCD63 ratio = 4.8, DMD-PMO(1) to hCD63 ratio = 2.7)*
*hCD63-3'-SS-DMD-PMO(2)-SC-SO1861 (total yield = 59%, SO1861 to hCD63 ratio = 4.8, DMD-PMO(2) to hCD63 ratio = 2.7)*
*hCD63-3'-SS-DMD-PMO(3)-SC-SO1861 (total yield = 67%, SO1861 to hCD63 ratio = 4.8, DMD-PMO(3) to hCD63 ratio = 2.8)*
*hCD63-3'-SS-DMD-PMO(4)-SC-SO1861 (total yield = 58%, SO1861 to hCD63 ratio = 4.8, DMD-PMO(4) to hCD63 ratio = 2.4)*
*hCD63-5'-SS-DMD-ASO-SC-SO1861 (total yield = 51%, SO1861 to hCD63 ratio = 4.8, DMD-ASO to hCD63 ratio = 2.2)*

### Cell culture (human)

Immortalized human myoblasts from non-DMD donors (KM155) were cultured as described above in the Methods (as performed in Examples 1-6).

Immortalized human myoblasts from a DMD-affected donor (KM1328) were cultured in homemade growth medium, containing 80 ml 199 medium (Thermo Fisher Scientific) and 320 ml DMEM (Thermo Fisher Scientific), supplemented with 20% fetal bovine serum (Gibco, United Kingdom), 50 µg/ml gentamycin (Thermo Fisher Scientific), 25 µg/ml fetuin (Thermo Fisher Scientific), 5 ng/ml human epidermal growth factor (Thermo Fisher Scientific), 0.5 ng/ml basis fibroblast growth factor (Thermo Fisher Scientific), 5 µg/ml insulin (Sigma), and 0.2 µg/ml dexamethasone (Sigma). For differentiation, cells were seeded on a Matrigel-coated surface, which was prepared by incubation with 0.1 mg Corning^{™} Matrigel^{™} Basement Membrane Matrix (Corning) per ml DMEM (Gibco) for 60 min at 37°C. At 100% confluence, the growth medium was replaced by DMEM (Gibco) supplemented with 2% fetal bovine serum (Gibco), 10 µg/ml insulin (Sigma), and 50 µg/ml gentamycin (Thermo Fisher Scientific). Treatments were started after at least 3 days up to a maximum of 5 days of differentiation based on the presence of differentiated myotubes).

### Exon skip analysis and quantification (human)

RNA was isolated with the TRIsure Isolation Reagent (Bioline) and chloroform extraction; isopropanol precipitation of RNA from the aqueous phase was performed as known to someone skilled in the art. For cDNA synthesis, 1000 ng of total RNA was used and diluted in an appropriate amount of RNase-free water to yield 8 µl RNA dilution.

For exon 51 and exon 53 skip analysis in KM155 myotubes, the priming premix contained 1 µl dNTP mix (10 mM each) and 1 µl specific reverse primer (for KM155, exon 51: h53R 5'-CTCCGGTTCTGAAGGTGTTC-3' [SEQ ID NO: 5]; exon 53: h55R 5'-ATCCTGTAGGACATTGGCAGTT-3 [SEQ ID NO: 6]. This mixture was heated for 5 min at 70°C, then chilled on ice for at least 1 min. A reaction mixture was prepared containing 0.5 µl rRNasin (Promega), 4.0 µl 5x RT buffer (Promega), 1.0 µl M-MLV RT (Promega), and 4.5 µl RNase-free water, and was added to the chilled mixture to yield a total volume of 20 µl per reaction. The RT-PCR was run for 60 min at 42 °C, then 5 min at 85 °C, and chilled on ice. For skip analysis, a nested PCR approach was followed. To this end, in the first PCR, 3 µl cDNA was added to a mix of 2.5 µl 10x SuperTaq PCR buffer, 0.5 µl dNTP mix (10 mM each), 0.125 µl Taq DNA polymerase TAQ-RO (5U/µl; Roche), 16.875 µl RNase-free water and 1 µl (10 pmol/ µl) of each primer flanking the targeted exons. The following primers were used: for KM155, exon 51: h48F 5'- AAAAGACCTTGGGCAGCTTG-3' [SEQ ID NO: 7] and h53R 5'- CTCCGGTTCTGAAGGTGTTC-3' [SEQ ID NO: 5]; exon 53: h50F 5'-AGGAAGTTAGAAGATCTGAGC-3' [SEQ ID NO: 20] and h55R 5'-ATCCTGTAGGACATTGGCAGTT-3' [SEQ ID NO: 6]. These samples were subjected to a PCR run of 5 min at 94°C, then 25 cycles with 40 sec at 94°C, 40 sec at 60°C, 180 sec at 72°C, after which for 7 min at 72°C. For the second PCR, 1.5 µl PCR1 sample was added to a mix of 5 µl 10x SuperTaq PCR buffer, 1 µl dNTP mix (10 mM each), 0.25 µl Taq DNA polymerase TAQ-RO (5 U/µl; Roche), 38.25 µl RNase-free water and 2 µl (10 pmol/ µl) of each primer flanking the targeted exons. The following primers were used: for KM155, exon 51: h49F 5'- CCAGCCACTCAGCCAGTG-3' [SEQ ID NO: 10] and h52R2 5'- TTCTTCCAACTGGGGACGC-3' [SEQ ID NO: 11], exon 53: h52F 5'-CCCCAGTTGGAAGAACTCATT-3' [SEQ ID NO: 21] and h54R 5'-CCAAGAGGCATTGATATTCTC -3' [SEQ ID NO: 9]. These samples were subjected to a PCR run of 5 min at 94°C, then 32 cycles with 40 sec at 94°C, 40 sec at 60°C, 60 sec at 72°C, after which for 7 min at 72°C. Exon skipping levels were quantified using the Femto Pulse System using the Ultra Sensitivity NGS Kit (Agilent), according to the manufacturer's instructions. Alternatively, the specific PCR fragments were analysed using Bioanalyzer 2100 with DNA1000 chip (lab-on-a-chip; Agilent). For exon 51 skipping, the expected non-skipped product has a size of 408 bp (KM155) and the skip product of 175 bp (KM155). For exon 53 skipping, the expected non-skipped product has a size of 438 bp (KM155) and the skip product of 226 bp (KM155).

For exon 51 and exon 53 skip analysis in KM1328 myotubes, the priming premixed contained 1 µl dNTP mix (10 mM each) and 1 µl specific reverse primer (for KM1328, exon 51 and 53: h57R 5'-TCTGAACTGCTGGAAAGTCG-3' [SEQ ID NO: 22]). This mixture was heated for 5 min at 70 °C, then chilled on ice for at least 1 min. A reaction mixture was prepared containing 0.5 µl rRNasin (Promega), 4.0 µl 5x RT buffer (Promega), 1.0 µl M-MLV RT (Promega), and 4.5 µl RNase-free water, and was added to the chilled mixture to yield a total volume of 20 µl per reaction. The RT-PCR was run for 60 min at 42 °C, then 10 min at 70 °C, and chilled on ice. For skip analysis, a nested PCR approach was followed. To this end, in the first PCR, 3 µl cDNA was added to a mix of 2.5 µl 10x SuperTaq PCR buffer, 0.5 µl dNTP mix (10 mM each), 0.125 µl Taq DNA polymerase TAQ-RO (5U/µl; Roche) 16.875 µl RNase-free water and 1 µl (10 pmol/ µl) of each primer flanking the targeted exons. The following primers were used: for KM1328, exon 51 and exon 53: h48F 5'- AAAAGACCTTGGGCAGCTTG-3' [SEQ ID NO: 7] and h57R 5'-TCTGAACTGCTGGAAAGTCG-3' [SEQ ID NO: 22]. These samples were subjected to a PCR run of 5 min at 94°C, then 25 cycles with 40 sec at 94°C, 40 sec at 60°C, 180 sec at 72°C, after which for 7 min at 72°C. For the second PCR, 1.5 µl PCR1 samples were added to a mix of 5 µl 10x SuperTaq PCR buffer, 1 µl dNTP mix (10 mM each), 0.25 µl Taq DNA polymerase TAQ-RO (5 U/µl; Roche), 38.25 µl RNase-free water and 2 µl (10 pmol/ µl) of each primer flanking the targeted exons. The following primers were used: for KM1328, exon 51 and 53: h49F2 5'-AAACTGAAATAGCAGTTCAAGC-3' [SEQ ID NO: 23] and h54R 5'-CCAAGAGGCATTGATATTCTC-3'

[SEQ ID NO: 9]. These samples were subjected to a PCR run of 5 min at 94°C, then 32 cycles with 40 sec at 94°C, 40 sec at 60°C, 90 sec at 72°C, after which for 7 min at 72°C. Exon skipping levels were quantified by analysing the specific PCR fragments using Bioanalyzer 2100 with DNA1000 chip (lab-on-a-chip; Agilent). For exon 51 skipping, the expected non-skipped product has a size of 793 bp (KM1328) and the skip product of 560 bp (KM1328). For exon 53 skipping, the expected non-skipped product has a size of 793 bp (KM1328) and the skip product of 581 bp (KM1328).

### Results (example 10 - 12)

### Example 10. hCD71-5'-SS-DMD-ASO + SO1861-SC-Mal or hCD71-5'-SS-DMD-PMO(1) + SO1861-SC-Mal or hCD71-3'-SS-DMD-PMO(1, 2, 3, 4, or 5) + SO1861-SC-Mal (in vitro)

To anti-CD71 monoclonal antibody targeting human CD71, DMD-ASO-SH (activated form of a 2'O-methyl-phosporothioate antisense oligonucleotide that induces exon 51 skipping of human dystrophin and has the same sequence and chemistry modifications as drisapersen) or DMD-PMO(1)-SH (activated form of a phosphorodiamidate morpholino oligomer [PMO] antisense oligonucleotide that induces exon 51 skipping of human dystrophin and has the same sequence but not 5'-modifications as eteplirsen) was conjugated through disulfide bond formation on the 5' to produce hCD71-5'-SS-DMD-ASO (DAR2.1) and hCD71-5'-SS-DMD-PMO(1) (DAR2.2), respectively (for conjugation procedure see Figure 23A-D). The resultant compounds were tested for dystrophin exon 51 skipping, either without or in combination with 4 µM of the endosomal escape enhancer SO1861-SC-Mal, on differentiated human myotubes from a non-DMD donor (KM155). This revealed strongly enhanced skipping of exon 51 in combination with 4 µM SO1861-SC-Mal after 72 hrs of treatment: while hCD71-5'-SS-DMD-ASO alone resulted in low exon 51 skipping (2.6%) at 600 nM (Figure 24A, left panel; Table A9), already 0.46 - 100 nM hCD71-5'-SS-DMD-ASO + SO1861-SC-Mal revealed 13.4 - 43.6 % exon 51 skipping (Figure 24A, right panel; Table A10). Likewise, while exposure to hCD71-5'-SS-DMD-PMO(1) resulted in very minor exon 51 skipping at 16.6 - 600 nM (0.4 - 1.1%) (Figure 24B, left panel; Table A9), exon 51 skipping increased to 10.4 - 12.5% at a 6-fold lower exposure concentration of 2.78 - 100 nM hCD71-5'-SS-DMD-PMO(1) + SO1861-SC-Mal (Figure 24B, right panel; Table A10), constituting a marked improvement in potency compared to conditions without SO1861-SC-Mal. Thus, co-administration of SO1861-SC-Mal with hCD71-5'-SS-DMD-ASO and hCD71-5'-SS-DMD-PMO(1), i.e. targeted DMD oligonucleotides that are conjugated to hCD71 on the 5', improves on-target delivery and induces marked exon 51 skipping.

Next, targeted DMD-PMOs that are conjugated to anti-hCD71 on the 3' were tested for exon 51 skipping activity on human myotubes. To anti-CD71 monoclonal antibody targeting human CD71, DMD-PMO(1)-SH, DMD-PMO(2)-SH (activated form of a PMO antisense oligonucleotide that induces exon 51 skipping of human dystrophin, as described in Echigoya et al. (2017)) or DMD-PMO(3)-SH (activated form of a PMO antisense oligonucleotide that induces exon 51 skipping of human dystrophin, as described in Echigoya et al. (2017)) was conjugated through disulfide bond formation on the 3' to produce hCD71-3'-SS-DMD-PMO(1) (DAR2.1), hCD71-3'-SS-DMD-PMO(2) (DAR3.0), and hCD71-3'-SS-DMD-PMO(3) (DAR2.6), respectively (for conjugation procedure see Figure 23A-D). The resultant compounds were tested for dystrophin exon 51 skipping, either without or in combination with 4 µM SO1861-SC-Mal, on differentiated human myotubes from a non-DMD donor (KM155). As shown for hCD71-5'-SS-DMD-PMO(1), this revealed enhanced exon 51 skipping for hCD71-3'-SS-DMD-PMO(1) in combination with 4 µM SO1861-SC-Mal after 72 hrs of treatment: while exposure to hCD71-3'-SS-DMD-PMO(1) alone resulted in very minor exon 51 skipping at 0.077 - 600 nM (0.4 - 2.2%) (Figure 25A, left panel; Table A9), exon 51 skipping increased to 8.2 - 9.7% at an exposure concentration of 2.78 - 100 nM hCD71-3'-SS-DMD-PMO(1) + SO1861-SC-Mal (Figure 25A, right panel; Table A10). More strikingly, exon 51 skipping was strongly enhanced for hCD71-3'-SS-DMD-PMO(2) in combination with 4 µM SO1861-SC-Mal: exposure to hCD71-3'-SS-DMD-PMO(2) + SO1861-SC-Mal revealed already exon 51 skipping (7.8%) at 0.013 nM conjugate, which increased up to 75.6 - 80.4% at 2.78 - 100 nM conjugate (Figure 25B, right panel; Table A10), while exposure to hCD71-3'-SS-DMD-PMO(2) alone resulted only in 5.7% exon 51 skipping at 600 nM conjugate (Figure 25B, left panel; Table A9), constituting a four orders of magnitude improvement compared to conditions without SO1861-SC-Mal. Also exposure to hCD71-3'-SS-DMD-PMO(3) in combination with 4 µM SO1861-SC-Mal resulted in strongly enhanced exon 51 skipping: hCD71-3'-SS-DMD-PMO(3) + SO1861-SC-Mal revealed already exon 51 skipping (9.6%) at 0.077 nM conjugate, which increased up to 28.3 - 29.2% at 2.78 - 100 nM (Figure 25C, right panel; Table A10), while exposure to hCD71-3'-SS-DMD-PMO(2) alone resulted in no exon 51 skipping (0.0%) at all concentrations tested (up to 600 nM) (Figure 25C, left panel; Table A9). Thus, co-administration of SO1861-SC-Mal to hCD71-3'-SS-DMD-PMO(1), hCD71-3'-SS-DMD-PMO(2), and hCD71-3'-DMD-PMO(3), i.e. targeted DMD oligonucleotides that are conjugated to hCD71 on the 3', improves on-target delivery and induces marked exon 51 skipping.

Also targeted DMD-PMOs that induce exon 53 skipping of human dystrophin were tested on human myotubes. To anti-CD71 monoclonal antibody targeting human CD71, DMD-PMO(4)-SH (activated form of a PMO antisense oligonucleotide that induces exon 53 skipping of human dystrophin and has the same sequence and chemistry modifications as golodirsen) or DMD-PMO(5)-SH (activated form of a PMO antisense oligonucleotide that induces exon 53 skipping of human dystrophin and has the same sequence and chemistry modifications as viltolarsen) was conjugated through disulfide bond formation on the 3' to produce hCD71-3'-SS-DMD-PMO(4) (DAR2.3) and hCD71-3'-SS-DMD-PMO(5)

(DAR2.1), respectively (for conjugation procedure see Figure 23A-D). The resultant compounds were tested for dystrophin exon 53 skipping, either without or in combination with 4 µM SO1861-SC-Mal, on differentiated human myotubes from a non-DMD donor (KM155). This revealed enhanced exon 53 skipping only in combination with SO1861-SC-Mal after 72 hrs of treatment: while exposure to 600 nM hCD71-3'-SS-DMD-PMO(4) alone resulted in only 0.4% exon 53 skipping (Figure 26A, left panel; Table A9), already 2.78 nM hCD71-3'-SS-DMD-PMO(4) + SO1861-SC-Mal resulted in 5.4% exon 53 skipping (Figure 26A, right panel; Table A10). Likewise, while exposure to 600 nM hCD71-3'-SS-DMD-PMO(5) alone resulted in 0.3% exon 53 skipping (Figure 26B, left panel; Table A9), already 2.78 nM hCD71-3'-SS-DMD-PMO(5) + SO1861-SC-Mal (Figure 26B, right panel; Table A10) resulted in 6.0% exon 53 skipping, which increased up to 8.4% at 100 nM hCD71-3'-SS-DMD-PMO(5), realizing an improvement of one to two orders of magnitude. These data show that co-administration of SO1861-SC-Mal to hCD71-3'-SS-DMD-PMO(4) and hCD71-3'-SS-DMD-PMO(5), i.e. targeted DMD oligonucleotides that are conjugated to hCD71 on the 3', enhances their on-target delivery and induces exon 53 skipping.

Taken together, these data show that co-administration of SO1861-SC-Mal is broadly applicable to effectively increase the potency of hCD71-targeted DMD oligonucleotides with different targeting sequences (different exons, different sequences) and conjugation methods (either on the 5' or 3' terminus) in human myotubes.

**Table A9: Skip efficacy of anti-CD71-conjugated DMD oligonucleotides in human myotubes**

| **% exon skipping** (Femto or Lab-on-a-chip analysis) | | **[nM conjugate]** | | | | | | |
|---|---|---|---|---|---|---|---|---|
| | | **600** | **100** | **16.6** | **2.78** | **0.46** | **0.077** | |
| hCD71-5'-SS-DMD-ASO (DAR2.1) (KM155) | Femto | 2.6 | 0.4 | 0.3 | 0.5 | 1.5 | 0.4 | % DMD exon 51 skipping |
| hCD71-5'-SS-DMD-PMO(1) (DAR2.2) (KM155) | Femto | 0.5 | 0.4 | 1.1 | 0.3 | 0.3 | 0.3 | |
| hCD71-3'-SS-DMD-PMO(1) (DAR2.1) (KM155) | Femto | 2.2 | 0.4 | 0.5 | 0.4 | 1.6 | 0.5 | |
| hCD71-3'-SS-DMD-PMO(2) (DAR3.0) (KM155) | Femto | 5.7 | 1.3 | 2.6 | 0.3 | 0.4 | 0.5 | |
| hCD71-3'-SS-DMD-PMO(3) (DAR2.6) (KM155) | Lab-on-a-chip | 0.0 | 0.0 | 0.0 | 0.0 | 0.0 | 0.0 | |
| hCD71-3'-SS-DMD-PMO(4) (DAR2.3) (KM155) | Femto | 0.4 | 0.0 | 0.0 | 0.0 | 0.0 | 0.0 | % DMD exon 53 skipping |
| hCD71-3'-SS-DMD-PMO(5) (DAR2.1) (KM155) | Femto | 0.3 | 0.0 | 0.5 | 0.0 | 0.0 | 0.0 | |

**Table A10: Skip efficacy of anti-CD71-conjugated DMD oligonucleotides with SO1861-SC-Mal co-administration in human myotubes**

| **% exon skipping** (Femto or Lab-on-a-chip analysis) | | **[nM conjugate]** | | | | | | |
|---|---|---|---|---|---|---|---|---|
| | | **100** | **16.6** | **2.78** | **0.46** | **0.07 7** | **0.013** | |
| hCD71-5'-SS-DMD-ASO (DAR2.1) + SO1861-SC-Mal (KM155) | Femto | 43.6 | 63.8 | 21.9 | 13.4 | 0.0 | 0.0 | % DMD exon 51 skipping |
| hCD71-5'-SS-DMD-PMO(1) (DAR2.2) + SO1861-SC-Mal (KM155) | Femto | 10.4 | 12.5 | 12.3 | 5.3 | 1.8 | 1.0 | |
| hCD71-3'-SS-DMD-PMO(1) (DAR2.1) + SO1861-SC-Mal (KM155) | Femto | 9.5 | 9.7 | 8.2 | 5.4 | 2.2 | 0.7 | |
| hCD71-3'-SS-DMD-PMO(2) (DAR3.0) + SO1861-SC-Mal (KM155) | Femto | 79.1 | 80.4 | 75.6 | 50.7 | 23.4 | 7.8 | |
| hCD71-3'-SS-DMD-PMO(3) (DAR2.6) + SO1861-SC-Mal (KM155) | Lab-on-a-chip | 29.2 | 29.1 | 28.3 | 14.6 | 9.6 | 1.0 | |
| hCD71-3'-SS-DMD-PMO(4) (DAR2.3) + SO1861-SC-Mal (KM155) | Femto | 4.4 | 5.0 | 5.4 | 1.3 | 0.6 | 0.1 | % DMD exon 53 skipping |
| hCD71-3'-SS-DMD-PMO(5) + SO1861-SC-Mal (DAR2.1) (KM155) | Femto | 8.4 | 5.4 | 6.0 | 2.1 | 1.8 | 0.0 | |

### Example 11. hCD71-5'-SS-DMD-ASO + hCD63-SC-SO1861 or hCD63-5'-SS-DMD-ASO + hCD71-SC-SO1861 (in vitro)

DMD-ASO-SH was conjugated to either anti-CD71 monoclonal antibody targeting human CD71 or anti-CD63 monoclonal antibody targeting human CD63 to yield hCD71-5'-SS-DMD-ASO (DAR2.1) and hCD63-5'-SS-DMD-ASO (DAR2.3), respectively (for conjugation procedure see Figure 23A-D). SO1861-SC-Mal was also conjugated to either anti-CD71 monoclonal antibody targeting human CD71 or anti-CD63 monoclonal antibody targeting human CD63 to produce hCD71-SC-SO1861 (DAR 4.0) and hCD63-SC-SO1861 (DAR 4.8), respectively (for conjugation procedure see Figure 27). hCD71-5'-SS-DMD-ASO and hCD63-5'-SS-DMD-PMO were co-administered with a fixed concentration of 100 nM hCD63-SC-SO1861 or hCD71-SC-SO1861, respectively, on differentiated human myotubes from a non-DMD (healthy) donor (KM155). Strikingly, co-administration of hCD71-5'-SS-DMD-ASO with 100 nM hCD63-SC-SO1861 revealed enhanced exon 51 skipping with already 28.7% exon skip at 0.46 nM hCD71-5'-SS-DMD-ASO, which increased up to 50.0 - 68.1% exon skip at 16.6 - 100 nM hCD71-5'-SS-DMD-ASO (Figure 28A; Table A11). Co-administration of hCD63-5'-SS-DMD-ASO with 100 nM hCD71-SC-SO1861 revealed enhanced exon 51 skipping with 29.2% exon skip at 16.6 nM (Figure 28B; Table A11). These data show that hCD63-SC-SO1861 and hCD71-SC-SO1861 induce on-target enhanced cytoplasmic delivery of hCD71- or hCD63-targeted DMD-ASO, inducing enhanced exon 51 skipping.

Next, the titrated conjugate and the conjugate co-administered with a fixed concentration were switched. Differentiated human myotubes from a non-DMD (healthy) donor (KM155) were treated with hCD63-SC-SO1861 in combination with a fixed concentration of 55.6 nM hCD71-5'-SS-DMD-ASO. This revealed enhanced exon 51 skipping with 72.6% exon skip at 16.6 nM, 53.1% exon skip at 2.78 nM, and still 21.0% exon skip at 0.46 nM hCD63-SC-SO1861 (Figure 29A; Table A12). More strikingly and relevantly, in differentiated myotubes from a DMD-affected donor (KM1328), co-administration of hCD63-SC-SO1861 with a fixed concentration of 55.6 nM hCD71-5'-SS-DMD-ASO resulted in already 53.8% skip at 0.46 nM hCD63-SC-SO1861, which increased up to 93.4% skip at 16.6 nM and 97.0% skip at 600 nM hCD63-SC-SO1861 (Figure 29B, Table A12).

Taken together, these data show that ligand1-conjugated oligonucleotide payload (i.e. either hCD71- or hCD63-targeted DMD-ASO), in combination with ligand2-conjugated SO1861 (i.e. either hCD71- or hCD63-targeted SO1861), or vice versa, i.e., ligand2-conjugated oligonucleotide payload in combination with ligand1-conjugated SO1861, lead to marked potency enhancement in human myotubes, and specifically in a disease-relevant cell system such as differentiated myotubes from a DMD-affected donor (example of a 2-target, 2-component system).

**Table A11: Skip efficacy of anti-CD71-conjugated DMD oligonucleotide with hCD61-SC-SO1861 and anti-CD63-conjugated DMD oligonucleotide with hCD71-SC-SO1861 co-administration in human myotubes**

| **% exon skipping** (Femto analysis) | **[nM hCD71-5'-SS-DMD-ASO or hCD63-5'-SS-DMD-ASO]** | | | | | | |
|---|---|---|---|---|---|---|---|
| | **100** | **16.6** | **2.78** | **0.46** | **0.077** | **0.013** | |
| hCD71-5'-SS-DMD-ASO (DAR2.1) + 100 nM hCD63-SC-SO1861 (DAR4.8) (KM155) | 50.0 | 68.1 | 29.8 | 28.7 | 0.0 | 0.0 | % DMD exon 51 skipping |
| hCD63-5'-SS-DMD-ASO (DAR2.3) + 100 nM hCD71-SC-SO1861 (DAR4.0) (KM155) | 6.9 | 29.2 | 0.0 | 0.6 | 0.0 | 0.0 | |

**Table A12: Skip efficacy of anti-CD61-SC-SO1861 with anti-CD71-conjugated DMD oligo co-administration in human myotubes**

| **% exon skipping** (Femto or lab-on-a-chip analysis) | | **[nM hCD63-SC-SO1861]** | | | | | | |
|---|---|---|---|---|---|---|---|---|
| | | **600** | **100** | **16.6** | **2.78** | **0.46** | **0.077** | |
| hCD63-SC-SO1861 (DAR4.8) + 55.6 nM hCD71-5'-SS-DMD-ASO (DAR2.1) (KM155) | Femto | 61.3 | 58.9 | 72.6 | 53.1 | 21.0 | 0.7 | % DMD exon 51 skipping |
| hCD63-SC-SO1861 (DAR4.8) + 55.6 nM hCD71-5'-SS-DMD-ASO (DAR2.1) (KM1328) | Lab-on-a-chip | 97.0 | 95.5 | 93.4 | 89.3 | 53.8 | 0.7 | |

### Example 12. hCD71-5'-SS-DMD-ASO-SC-SO1861 or hCD63-5'-SS-DMD-ASO-SC-SO1861 or hCD71-3'-SS-DMD-PMO(1, 2, 3, 4, or 5)-SC-SO1861 or hCD63-DMD-PMO(1, 2, 3, or 4)-SC-SO1861 (in vitro)

To either anti-CD71 monoclonal antibody targeting human CD71 (hCD71) or anti-CD63 monoclonal antibody targeting human CD63 (hCD63), SO1861-SC-Mal was conjugated and then DMD-ASO-SH was conjugated through disulfide bond formation on the 5' to produce: hCD71-5'-SS-DMD-ASO-SC-SO1861 (DAR2.3/4.0) and hCD63-5'-SS-DMD-ASO-SC-SO1861 (DAR2.2/4.8), respectively (for conjugation procedure see Figure 30A-C). Strikingly, treatment of differentiated human myotubes from a DMD-affected donor (KM1328) with these conjugates revealed extremely enhanced exon 51 skipping: hCD71-5'-SS-DMD-ASO-SC-SO1861 showed 99.6% skip at 600 nM, 96.3% skip at 100 nM, 64.7% skip at 16.6 nM, and still 79.2% skip at 2.78 nM conjugate (Figure 31A, left panel; Table A13). Exon skipping was still measurable down to at least 0.46 nM hCD71-5'-SS-DMD-ASO-SC-SO1861. Likewise, hCD63-5'-SS-DMD-ASO-SC-SO1861 showed 100.0% exon skip at 100 - 600 nM, 74.6% skip at 16.6 nM, and still 62.4% skip at 2.78 nM conjugate (Figure 31A, right panel; Table A13). These data show that incubation with a conjugate incorporating DMD-ASO and SO1861-SC-Mal conjugated to either hCD71 or hCD63, constitutes an outstanding improvement in the maximum percentage of dystrophin exon 51 skipping achieved in human myotubes from a DMD-affected donor.

Next, conjugates incorporating DMD-PMO(1, 2, or 3) and SO1861-SC-Mal conjugated to either hCD71 or hCD63 were tested for dystrophin exon 51 skipping on human myotubes from a DMD-affected donor (KM1328). To either anti-CD71 monoclonal antibody targeting human CD71 or anti-CD63 monoclonal antibody targeting human CD63, SO1861-SC-Mal was conjugated and then DMD-PMO(1)-SH, DMD-PMO(2)-SH, or DMD-PMO(3)-SH was conjugated through disulfide bond formation on the 3' to produce: hCD71-3'-SS-DMD-PMO(1)-SC-SO1861 (DAR2.4/4.0), hCD63-3'-SS-DMD-PMO(1)-SC-SO1861 (DAR2.7/4.8), hCD71-3'-SS-DMD-PMO(2)-SC-SO1861 (DAR3.1/4.0), hCD63-3'-SS-DMD-PMO(2)-SC-SO1861 (DAR2.7/4.8), hCD71-3'-SS-DMD-PMO(3)-SC-SO1861 (DAR2.5/4.0), and hCD63-3'-SS-DMD-PMO(3)-SC-SO1861 (DAR2.8/4.8) (for conjugation procedure see Figure 30A-C). Treatment with these conjugates all revealed extremely enhanced exon 51 skipping in human myotubes from a DMD-affected donor (KM1328) (Table A13): with 75.8% exon skip at 600 nM hCD71-3'-SS-DMD-PMO(1)-SC-SO1861 (Figure 31B, left panel) and 96.5% exon skip at 600 nM hCD63-3'-SS-DMD-PMO(1)-SC-SO1861 (Figure 31B, right panel). Exon 51 skipping was still 76.9% at 2.78 nM hCD63-3'-SS-DMD-PMO(1)-SC-SO1861, and an effect was measurable down to at least 0.46 nM hCD63-3'-SS-DMD-PMO(1)-SC-SO1861. Even more strikingly, hCD63-3'-SS-DMD-PMO(2)-SC-SO1861 revealed 100.0% exon skip at 2.78 - 600 nM and still 53.6% skip at 0.46 nM (Figure 31C, right panel). Likewise, hCD71-3'-SS-DMD-PMO(2)-SC-SO1861 revealed strongly enhanced exon 51 skipping with 100.0% exon skip at 600 nM, 97.6% skip at 100 nM, 92.1% skip at 16.6 nM, 76.3% skip at 2.78 nM, and still 28.3% skip at 0.46 nM (Figure 31C, left panel). Finally, also hCD71-3'-SS-DMD-PMO(3)-SC-SO1861 and hCD63-3'-SS-DMD-PMO(3)-SC-SO1861 showed strongly enhanced exon 51 skipping with 86.7% skip at 600 nM hCD71-3'-SS-DMD-PMO(3)-SC-SO1861 (Figure 31D, left panel) and 89.6 - 98.2% exon skip at 2.78 - 600 nM hCD63-3'-SS-DMD-PMO(3)-SC-SO1861 (Figure 31D, right panel). Marked exon 51 skipping was measurable down to at least 0.46 nM (20.0%) hCD63-3'-SS-DMD-PMO(3)-SC-SO1861. Together, these data show that incubation with conjugates incorporating both DMD-PMO and SO1861-SC-Mal conjugated to either hCD71 or hCD63, constitute an outstanding improvement in the maximum percentage of exon 51 skipping achieved in human myotubes from a DMD-affected donor, with different targeting sequences for DMD-PMO.

In addition, conjugates incorporating DMD-PMO(4 or 5) and SO1861-SC-Mal conjugated to either hCD71 or hCD63 were tested for dystrophin exon 53 skipping on human myotubes from a DMD-affected donor (KM1328). To either anti-CD71 monoclonal antibody targeting human CD71 or anti-CD63 monoclonal antibody targeting human CD63, SO1861-SC-Mal was conjugated and then DMD-PMO(4)-SH or DMD-PMO(5)-SH was conjugated through disulfide bond formation on the 3' to produce: hCD71-3'-SS-DMD-PMO(4)-SC-SO1861 (DAR2.0/4.0), hCD63-3'-SS-DMD-PMO(4)-SC-SO1861 (DAR2.4/4.8), and hCD71-3'-SS-DMD-PMO(5)-SC-SO1861 (DAR2.0/4.0) (for conjugation procedure see Figure 30A-C). Treatment with these conjugates all revealed extremely enhanced exon 53 skipping in human myotubes from a DMD-affected donor (KM1328) (Table A13): with 93.6% exon skip at 600 nM hCD71-3'-SS-DMD-PMO(4)-SC-SO1861 (Figure 32A, left panel) and 98.0% skip at 600 nM hCD63-3'-SS-DMD-PMO(4)-SC-SO1861 (Figure 32A, right panel). Exon 53 skipping was still 90.6% at 2.78 nM hCD63-3'-SS-DMD-PMO(4)-SO1861, and an effect was measurable down to at least 0.46 nM hCD63-3'-SS-DMD-PMO(4)-SC-SO1861. Likewise, hCD71-3'-SS-DMD-PMO(5)-SC-SO1861 revealed 94.4% exon skip at 600 nM and still 19.1% skip at 2.78 nM (Figure 32B). These data show that incubation with conjugates incorporating both DMD-PMO and SO1861-SC-Mal conjugated to either hCD71 or hCD63, constitute an outstanding improvement in the maximum percentage of exon 53 skipping achieved in human myotubes from a DMD-affected donor, with different targeting sequences for DMD-PMO.

In conclusion, conjugates incorporating a DMD oligonucleotide and SO1861-SC-Mal conjugated to either hCD71 or hCD63 lead to marked potency enhancement, with different oligonucleotide targeting sequences (different exons, different sequences), in a relevant cell system such as differentiated myotubes from a DMD-affected donor (example of a targeted 1-component system).

**Table A13: Skip efficacy of hCD71-5'-SS-DMD-ASO-SC-SO1861, hCD63-5'-SS-DMD-ASO-SC-SO1861, hCD71-3'-SS-DMD-PMO(1-5)-SC-SO1861, and hCD63-3'-SS-DMD-PMO(1-5)-SC-SO1861 in human myotubes**

| **% exon skipping** (Lab-on-a-chip analysis) | **[nM conjugate]** | | | | | | |
|---|---|---|---|---|---|---|---|
| | **600** | **100** | **16.6** | **2.78** | **0.46** | **0.077** | |
| hCD71-5'-SS-DMD-ASO-SC-SO1861 (DAR2.3/4.0) (KM1328) | 99.6 | 96.3 | 64.7 | 79.2 | 3.7 | 0.8 | % DMD exon 51 skipping |
| hCD63-5'-SS-DMD-ASO-SC-SO1861 (DAR2.2/4.8) (KM1328) | 100.0 | 100.0 | 74.6 | 62.4 | 2.0 | 0.7 | |
| hCD71-3'-SS-DMD-PMO(1)-SC-SO1861 (DAR2.4/4.0) (KM1328) | 75.8 | 43.4 | 20.1 | 4.7 | 1.5 | 0.9 | |
| hCD63-3'-SS-DMD-PMO(1)-SC-SO1861 (DAR2.7/4.8) (KM1328) | 96.5 | 93.5 | 89.5 | 76.9 | 8.6 | 0.7 | |
| hCD71-3'-SS-DMD-PMO(2)-SC-SO1861 (DAR3.1/4.0) (KM1328) | 100.0 | 97.6 | 92.1 | 76.3 | 28.3 | 0.5 | |
| hCD63-3'-SS-DMD-PMO(2)-SC-SO1861 (DAR2.7/4.8) (KM1328) | 100.0 | 100.0 | 100.0 | 100.0 | 53.6 | 0.6 | |
| hCD71-3'-SS-DMD-PMO(3)-SC-SO1861 (DAR2.5/4.0) (KM1328) | 86.7 | 71.0 | 54.3 | 18.7 | 7.3 | 0.8 | |
| hCD63-3'-SS-DMD-PMO(3)-SC-SO1861 (DAR2.8/4.8) (KM1328) | 98.2 | 96.4 | 95.3 | 89.6 | 20.0 | 0.7 | |
| hCD71-3'-SS-DMD-PMO(4)-SC-SO1861 (DAR2.0/4.0) (KM1328) | 93.6 | 59.6 | 42.8 | 9.5 | 6.4 | 0.8 | % DMD exon 53 skipping |
| hCD63-3'-SS-DMD-PMO(4)-SC-SO1861 (DAR2.4/4.8) (KM1328) | 98.0 | 97.4 | 96.6 | 90.6 | 6.6 | 0.7 | |
| hCD71-3'-SS-DMD-PMO(5)-SC-SO1861 (DAR2.0/4.0) (KM1328) | 94.4 | 63.7 | 31.2 | 19.1 | 4.8 | 0.5 | |

### LITERATURE REFERENCES

Cardamone M, et al., 2008 - Cardamone M; Darras BT; Ryan MM, 2008, 'Inherited myopathies and muscular dystrophies', Seminars in Neurology, vol. 28, pp. 250 - 259, http://dx.doi.org/10.1055/s-2008-1062269
Shadrin et al, 2016 - Striated muscle function, regeneration, and repair, Cell Mol Life Sci. 2016 November; 73(22): 4175-4202. doi:10.1007/s00018-016-2285-z.
Dumont NA, Wang YX, Rudnicki MA. Intrinsic and extrinsic mechanisms regulating satellite cell function. Development. 2015;142(9):1572-1581, doi: 10.1242/dev.114223
Uygur and Lee, Mechanisms of Cardiac Regeneration. Dev Cell. 2016 Feb 22; Volume 36(4): pp. 362-374, doi: 10.1016/j.devcel.2016.01.018
Slørdal L, Spigset O. Heart failure induced by non-cardiac drugs, Drug Saf 2006; Volume 29(7): pp. 567-86, doi: 10.2165/00002018-200629070-00003
Ebner et al., Strategies for Skeletal Muscle Targeting in Drug Discovery, Current Pharmaceutical Design 2015, Volume 21(10), pp. 1327-1336, DOI:10.2174/1381612820666140929095755
González-Jamett et al., Hereditary Myopathies, 2018, DOl: 10.5772/intechopen.76076, www.intechopen.com/chapters/60283
Savarese et al., Is Gene-Size an Issue for the Diagnosis of Skeletal Muscle Disorders?, 2020, Volume 7(3), pp. 203-216, DOl: 10.3233/JND-190459
Duan et al., Duchenne muscular dystrophy, Nat Rev Dis Primers, 2021 Feb 18; Volume 7(1):13, doi: 10.1038/s41572-021-00248-3
Bladen, C. L. et al., The TREAT-NMD Duchenne muscular dystrophy registries: conception, design, and utilization by industry and academia. Hum. Mutat. 34, 1449-1457 (2013), DOl: 10.1002/humu.22390.
Goemans, N. et al., A randomized placebo-controlled phase 3 trial of an antisense oligonucleotide, drisapersen, in Duchenne muscular dystrophy. Neuromuscul. Disord. Volume 28(1), pp. 4-15 (2018), doi: 10.1016/j.nmd.2017.10.004.
Mendell, J. R. et al., Assessment of systemic delivery of rAAVrh74.MHCK7.micro-dystrophin in children with duchenne muscular dystrophy: a nonrandomized controlled trial. JAMA Neurol. Volume 77(9), pp. 1122-1131 (2020), doi: 10.1001/jamaneurol.2020.1484.
Mendell, J. R. et al, A phase I/IIa follistatin gene therapy trial for Becker muscular dystrophy, Mol. Ther. Volume 23(1), pp. 192-201 (2015), doi: 10.1038/mt.2014.200.
Wasala, N. B. et al., Single SERCA2a therapy ameliorated dilated cardiomyopathy for 18 months in a mouse model of Duchenne muscular dystrophy, Mol. Ther. Volume 28(3), pp. 845-854 (2020), doi: 10.1016/j.ymthe.2019.12.011
Aránega, et al., Review MiRNAs and Muscle Regeneration: Therapeutic Targets in Duchenne Muscular Dystrophy, Int J Mol Sci. (2021 Apr), Volume 22(8): 4236, doi: 10.3390/ijms22084236
Chemello, et al., Correction of muscular dystrophies by CRISPR gene editing, J. Clin. Invest. Volume 130(6), pp. 2766-2776 (2020), doi: 10.1172/JCI136873
Nelson, et al., Genome engineering: a new approach to gene therapy for neuromuscular disorders, Nat. Rev. Neurol. Volume 13(11), pp. 647-661 (2017), doi: 10.1038/nrneurol.2017.126
Cerro-Herreros et al., Therapeutic Potential of AntagomiR-23b for Treating Myotonic Dystrophy, Mol Ther Nucleic Acids, 2020 Sep 4; Volume 21, pp. 837-849, doi.org/10.1016/j.omtn.2020.07.021
Bladen, C. L. et al., The TREAT-NMD DMD global database: analysis of more than 7000 Duchenne muscular dystrophy mutations. Hum. Mutat. Volume 36(4), pp. 395-402 (2015), doi: 10.1002/humu.22758
Hanahan and Weinberg, Hallmarks of cancer: the next generation, Cell (2011, Mar 4); Volume 144(5), pp. 646-674, doi: 10.1016/j.cell.2011.02.013.
Wang et al., Saponins as Natural Adjuvant for Antisense Morpholino Oligonucleotides Delivery In Vitro and in mdx Mice, Molecular Therapy: Nucleic Acids, 2018, Volume 11, pp. 192-202, doi: 10.1016/j.omtn.2018.02.004
Wang et al., Saponins enhance exon skipping of 2'-O-methyl phosphorothioate oligonucleotide in vitro and in vivo, Drug Design, Development and Therapy Volume 12: pp. 3705-3715 (2018), DOI:10.2147/DDDT.S179008
Kemaladewi et al, Dual exon skipping in myostatin and dystrophin for Duchenne muscular dystrophy, BMC Medical Genomics, Volume 4, 36 (2011), DOI: 10.1186/1755-8794-4-36
Baik et al, Cell type-selective targeted delivery of a recombinant lysosomal enzyme for enzyme therapies, Molecular Therapy, Volume 29(12), pp. 3512-3524 (2021), DOI: 10.1016/j.ymthe.2021.08.020
Echigoya et al., Quantitative antisense screening and optimization for exon 51 skipping in Duchenne muscular dystrophy, Molecular Therapy, Volume 25(11), pp. 2561 - 2572 (2017), DOI: 10.1016/j.ymthe.2017.07.014

## Claims

1. A pharmaceutical composition for use in the treatment or prophylaxis of a muscle wasting disorder, the composition comprising
a covalently-linked conjugate comprising
a saponin,
an oligonucleotide, and
a ligand of an endocytic receptor on a muscle cell,
wherein the saponin is a triterpenoid 12,13-dehydrooleanane-type saponin.

2. Composition for use according to claim 1, wherein the muscle wasting disorder is a muscle cell-related genetic disorder, preferably being a congenital myopathy or a muscular dystrophy;
preferably wherein the congenital myopathy is selected from nemaline myopathy or congenital fiber-type disproportion myopathy, and/or wherein the muscular dystrophy is selected from a dystrophinopathy, facioscapulohumeral muscular dystrophy, myotonic dystrophy, Emery-Dreifuss muscular dystrophy, limb-girdle muscular dystrophy 1B, congenital muscular dystrophy; or dilated familial cardiomyopathy;
most preferably wherein the muscle wasting disorder is a muscle cell-related genetic disorder being a dystrophinopathy, preferably being Duchenne muscular dystrophy.

3. Composition for use according to claim 1 or 2, wherein the treatment or prophylaxis of the muscle wasting disorder involves antisense therapy, preferably involving exon skipping.

4. Composition for use according to any one of the preceding claims, wherein the saponin in at least an unconjugated state comprises an aldehyde group at position C-23 of the saponin's aglycone core structure.

5. Composition for use according to any one of the preceding claims, wherein the saponin's aglycone core structure is selected from any one or more of:
quillaic acid;
gypsogenin;
2alpha-hydroxy oleanolic acid;
16alpha-hydroxy oleanolic acid;
hederagenin (23-hydroxy oleanolic acid);
16alpha,23-dihydroxy oleanolic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
3,16,28-trihydroxyoleanan-12-en;
gypsogenic acid; and
a derivative thereof,
preferably wherein the saponin's aglycone core structure is selected from quillaic acid, gypsogenin, and a derivative thereof, more preferably the saponin's aglycone core structure is quillaic acid.

6. Composition for use according to any one of the preceding claims, wherein the conjugate comprises two or more molecules of the saponin, preferably being between 2-32 molecules of the saponin, even more preferably 4-16 molecules of the saponin, most preferably 4-8 molecules of the saponin.

7. Composition for use according to any one of the preceding claims, wherein the saponin is any one or more of:
a) saponin selected from any one or more of list A:
- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Saponinum album* saponin mixture, or a saponin isolated from *Saponinum album;*
- *Saponaria officinalis* saponin mixture, or a saponin isolated from *Saponaria officinalis*; and
- Quillaja bark saponin mixture, or a saponin isolated from Quillaja bark, for example Quil-A, QS-17-api, QS-17-xyl, QS-21, QS-21A, QS-21B, QS-7-xyl; or
b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, SO1832, SO1861, SO1862, SO1904, QS-7, QS-7 api, QS-17, QS-18, QS-21 A-apio, QS-21 A-xylo, QS-21 B-apio and QS-21 B-xylo; or
d) a saponin comprising a 12, 13-dehydrooleanane type aglycone core structure without an aldehyde group at the C-23 position of the aglycone, selected from list D:
Aescin la, aescinate, alpha-Hederin, AMA-1, AMR, AS6.2, AS64R, Assamsaponin F, dipsacoside B, esculentoside A, macranthoidin A, NP-005236, NP-012672, Primula acid 1, saikosaponin A, saikosaponin D, Teaseed saponin I and Teaseedsaponin J,
preferably, the saponin is any one or more of a saponin selected from list A, B or C, more preferably, a saponin selected from list B or C, even more preferably, a saponin selected from list C,
and/or
wherein the saponin is any one or more of AG1856, GE1741, a saponin isolated from *Quillaja saponaria,* Quil-A, QS-17, QS-21, QS-7, SA1641, a saponin isolated from *Saponaria officinalis,* Saponarioside B, SO1542, SO1584, SO1658, SO1674, SO1700, SO1730, SO1772, SO1832, SO1861, SO1862 and SO1904;
preferably wherein the saponin is any one or more of QS-21, SO1832, SO1861, SA1641 and GE1741; more preferably wherein the saponin is QS-21, SO1832 or SO1861;
even more preferably wherein the saponin is SO1832 and SO1861;
most preferably being SO1861.

8. Composition for use according to any one of the preceding claims, wherein the oligonucleotide is defined as a nucleic acid that is no longer than 150 nt, preferably wherein the oligonucleotide has a size of 5 - 150 nt, preferably being 8 - 100 nt, most preferably being 10 - 50 nt; preferably wherein the oligonucleotide is an antisense oligonucleotide, more preferably being a mutation specific antisense oligonucleotide, most preferably being an oligonucleotide designed to induce exon skipping;
and/or
wherein the oligonucleotide comprises or consists of any one of the following: morpholino phosphorodiamidate oligomer (PMO), 2'-O-methyl (2'-OMe) phosphorothioate RNA, 2'-O-methoxyethyl (2'-O-MOE) RNA {2'-O-methoxyethyl-RNA (MOE)}, locked or bridged nucleic acid (LNA or BNA), 2'-O,4'-aminoethylene bridged nucleic acid (BNANC), peptide nucleic acid (PNA), 2'-deoxy-2'-fluoroarabino nucleic acid (FANA), 3'-fluoro hexitol nucleic acid (FHNA), glycol nucleic acid (GNA), threose nucleic acid (TNA), silencing RNA (siRNA), short hairpin RNA (shRNA), microRNA (miRNA), antagomir (miRNA antagonists), aptamer RNA or aptamer DNA, single-stranded RNA or single-stranded DNA, double-stranded RNA (dsRNA) or double-stranded DNA;
preferably wherein the oligonucleotide comprises or consists of a morpholino phosphorodiamidate oligomer (PMO) or a 2'-O-methyl (2'-OMe) phosphorothioate RNA.

9. Composition for use according to any one of the preceding claims, wherein the oligonucleotide is designed to induce exon skipping of the human dystrophin gene transcript, preferably wherein the exon skipping involves exon 51 skipping or exon 53 skipping or exon 45 skipping;
more preferably wherein the oligonucleotide is a 2'O-methyl-phosporothioate antisense oligonucleotide or a phosphorodiamidate morpholino oligomer antisense oligonucleotide that is designed to induce the exon 51 skipping or the exon 53 skipping or the exon 45 skipping,
even more preferably wherein the oligonucleotide is selected from eteplirsen, drisapersen, golodirsen, viltolarsen, and casimersen.

10. Composition for use according to any one of the preceding claims, wherein the conjugate comprises two or more molecules of the nucleic acid,
preferably wherein the two or more molecules of the nucleic acid are 2-16 molecules, more preferably 2-8 molecules, possibly 2, 3, 4, 5, or 6 molecules;
most preferably wherein the two or more molecules of the nucleic acid are two or more oligonucleotides; possibly being two or more different oligonucleotides wherein at least one of the two or more different oligonucleotides is an antisense oligonucleotide.

11. Composition for use according to any one of the preceding claims, wherein the conjugate comprises 1 - 16 molecules of the saponin and 1 - 5 molecules of the nucleic acid per 1 molecule of the ligand; preferably wherein the conjugate comprises 2 - 8 molecules of the saponin per 1 molecule of the ligand; more preferably 3 - 6 molecules of the saponin per 1 molecule of the ligand;
even more preferably 4 - 5 molecules of the saponin per 1 molecule of the ligand;
most preferably wherein the conjugate comprises on average 4-4.5 molecules of the saponin per 1 molecule of the ligand;
and/or
preferably wherein the conjugate comprises 2 - 5 molecules of the nucleic acid per 1 molecule of the ligand;
more preferably 3 - 4 molecules of the nucleic acid per 1 molecule of the ligand;
even more preferably wherein the conjugate comprises on average 4 molecules of the nucleic acid per 1 molecule of the ligand.

12. Composition for use according to any one of the preceding claims, wherein the endocytic receptor on a muscle cell to which the ligand binds is selected from: transferrin receptor (CD71), insulin-like growth factor 1 (IGF-I) receptor (IGF1R), insulin-like growth factor 2 (IGF-II) receptor (IGF2R), tetraspanin CD63; muscle-specific kinase (MuSK), glucose transporter GLUT4, cation independent mannose 6 phosphate receptor (CI-MPR), and LDL receptor;
and/or
wherein the ligand is selected from any one of:
insulin-like growth factor 1 (IGF-I) or fragments thereof;
insulin-like growth factor 2 (IGF-II) or fragments thereof;
mannose 6 phosphate
transferrin (Tf),
zymozan A, and
an antibody or a binding fragment thereof specific for binding to the endocytic receptor, wherein the endocytic receptor is preferably selected from: transferrin receptor (CD71), insulin-like growth factor 1 (IGF-I) receptor (IGF1R), insulin-like growth factor 2 (IGF-II) receptor (IGF2R), tetraspanin CD63, muscle-specific kinase (MuSK), glucose transporter GLUT4, cation independent mannose 6 phosphate receptor (CI-MPR), and LDL receptor;
preferably wherein the ligand is an antibody or a binding fragment thereof that is specific for binding to a transferrin receptor,
more preferably wherein the ligand is a monoclonal antibody or a Fab' fragment or at least one single domain antibody specific for binding to a transferrin receptor, even more preferably wherein the ligand is a monoclonal antibody specific for binding to a transferrin receptor.

13. Composition for use according to any one of the preceding claims, wherein the covalent linking of the saponin within the conjugate is made via a first linker to which the saponin is covalently bound;
preferably wherein the first linker comprises a covalent bond selected from any one or more of:
a semicarbazone bond, a hydrazone bond, an imine bond, an acetal bond including a 1,3-dioxolane bond, a ketal bond, an ester bond, an oxime bond, a disulfide bond, a thio-ether bond, an amide bond, a peptide bond, and an ester bond, preferably being a hydrazone bond or a semicarbazone bond;
more preferably wherein the saponin is a saponin that in at least an unconjugated state comprises an aldehyde group at position C-23 of the saponin's aglycone core structure and wherein said aldehyde group has been engaged in forming the covalent bond with the first linker.

14. Composition for use according to claim 13, wherein the first linker is a cleavable linker subject to cleavage under acidic, reductive, enzymatic and/or light-induced conditions;
preferably wherein the first linker comprises a cleavable bond selected from:
• a bond subject to cleavage under acidic conditions such as a semicarbazone bond, a hydrazone bond, an imine bond, an acetal bond including a 1,3-dioxolane bond, a ketal bond, an ester bond, and/or an oxime bond,
• a bond susceptible to proteolysis, for example amide or peptide bond, preferably subject to proteolysis by Cathepsin B;
• a red/ox-cleavable bond such as a disulfide bond, or a thiol-exchange reaction-susceptible bond such as a thio-ether bond;
preferably being an acid-sensitive bond subject to cleavage *in vivo* under acidic conditions present in endosomes and/or lysosomes of human cells, preferably at pH 4.0 - 6.5, and more preferably at pH ≤ 5.5;
more preferably being an acid-sensitive bond selected from any one or more of: a semicarbazone bond, a hydrazone bond, an imine bond, an acetal bond including a 1,3-dioxolane bond, a ketal bond, an ester bond, and/or an oxime bond, even more preferably selected from a semicarbazone bond and a hydrazone bond; most preferably being a hydrazone bond.

15. Composition for use according to any one of the preceding claims, wherein the saponin is or comprises at least one molecule of SO1861, the nucleic acid is drisapersen or eteplirsen or golodirsen or viltolatrsen, preferably drisapersen or eteplirsen, and the ligand is anti-CD71 antibody or a binding fragment thereof.
